# EUROPEAN PATENT APPLICATION

(11) **EP 4 276 097 A1**
(43) Date of publication of application: **15.11.2023**
(21) Application number: 22386025.5
(22) Date of filing: 10.05.2022
(51) Int. Cl.: C07D 401/14, C07K 1/00, A61P 25/16

(54) **AMINOPYRIMIDINYL DERIVATIVES FOR THE TREAMENT OF PARKINSON'S DISEASE**

(71) Applicant: University Of Dundee, Dundee DD1 4HN (GB)
(72) Inventor: Ciulli, Alessio, Dundee, DD1 5EH (GB); Liu, Xingui, Dundee, DD1 5EH (GB); Makukhin, Nikolai, London, E1 2AX (GB); Alessi, Dario, Dundee, DD1 5EH (GB); Kalogeropulou, Alexia, Dundee, DD1 5EH (GB)
(74) Representative: HGF

(57) **Abstract**

Disclosed are heterobifunctional compounds that can induce the degradation of leucine-rich repeat kinase 2 (LRRK2) and PDE6D. These compounds can engage LRRK2 on one end and bind to an ubiquitin E3 ligase (e.g. cereblon, Von Hippel-Lindau, or Cellular Inhibitor of Apoptosis (clAP)) on the other end and therefore bring LRRK2 in close proximity to the E3 ligase and induce the ubiquitination and degradation of the LRRK2 protein. Also disclosed are pharmaceutical composition comprising the heterobifunctional compounds and methods of using the compounds to treat LRRK2 and PDE6D-related diseases and disorders.

## Description

### Field

Described herein are novel small-molecule Proteolysis Targeting Chimeras (PROTACs) targeting and inducing rapid and selective degradation of Leucine-Rich Repeat Kinase 2 (LRRK2) at nanomolar range of concentrations in multiple cells.

### Background

Parkinson's disease (PD) is the second most common neurodegenerative disorder currently affecting about 10 million people worldwide. This number is projected to be 17.5 million by 2040 (Dorsey et al., J Parkinsons Dis. 2018;8(s1):S3-S8.) due to the fast-growing aging population, bringing serious public health problems and economic burdens (Feigin et al., Lancet Neurol. 2019 May;18(5):459-480.). As thus far, all attempts to slow the progression of PD have failed and successful treatment remains a critically important unmet medical need.

Leucine-rich repeat kinase 2 (LRRK2), encoded by LRRK2 gene, is a large (286 kDa), multi-domain protein. The armadillo repeat motif (ARM), ankyrin repeat domain (ANK), leucine-rich repeat domain and the WD40 repeat domain are involved in protein-protein interactions; the Ras of complex protein (ROC) and C terminus of ROC (COR) tandem domain functions as GTPase; the kinase domain, which is the most-studied domain of LRRK2, is responsible for autophosphorylation at Ser-1292 (Sheng et al., Sci Transl Med. 2012 Dec 12;4(164):164ra161.) and for phosphorylating a group of Rab GTPases that are involved in vesicle trafficking (Steger et al. eLife 2016; 5:e12813). LRRK2 is found to play roles in both sporadic PD and familial PD (Tolosa et al., Nat. Rev. Neurol. 2020; 16(2):97-107.). Pathological mutations in the RocCOR GTPase domain (R1441C/G/H, N1437H, Y1699C) and the kinase domain (G2019S, I2020T) result in altered GTPase and/or kinase activity, leading to functional alterations in vesicular trafficking, cytoskeleton dynamics, autophagy, lysosomal degradation, neurotransmission, mitochondrial function and many other pathogenic hallmarks associated with PD (Tolosa et al., Nat. Rev. Neurol. 2020; 16(2):97-107.). Targeting the GTPase and kinase activities of LRRK2 are therefore considered as potential disease-modifying PD therapies (Tolosa et al., Nat. Rev. Neurol. 2020; 16(2):97-107.), with numerous LRRK2 kinase inhibitors described in the past, including LRRK2-IN-1 (Deng et al., Nat. Chem. Biol. 2011; 7(4):203-5., HG-10-102-01 (Choi et al., ACS Med Chem Lett. 2012; 3(8):658-662.), MLi-2 (Fell et al., J. Pharmacol. Exp. Ther. 2015; 355(3):397-409.), PF-06447475 (Henderson et al., J. Med. Chem. 2015; 58(1):419-32.), and DNL201 and DNL151 which are the first two LRRK2 kinase inhibitors in clinical trial (Tolosa et al., Nat. Rev. Neurol. 2020; 16(2):97-107.). However, all these LRRK2 kinase inhibitors are ATP-competitive type 1 kinase inhibitors that enhance LRRK2 mislocalization and microtubule association by preferably binding to the closed conformation of LRRK2 ( Deniston et al., Nature 2020; 588(7837):344-349; Watanabe et al. Cell 2020; 182(6):1508-1518.e16.), underlying undesirable effects on vesicular trafficking and the on-target side-effects observed on lung and kidney ( Andersen et al. Toxicology . 2018; 395:15-22; Baptista et al. Sci. Transl. Med. 2020 Apr 22;12(540):eaav0820.).

Alternative LRRK2 targeting strategies, such as G2019S LRRK2 selective inhibitors (Garofalo et al., J. Med. Chem. 2020; 63(23):14821-14839; Lesniak et al., Eur. J. Med. Chem. 2022; 229:114080.), LRRK2 dimerization inhibitors (Helton et al., ACS Chem. Biol. 2021; 16(11):2326-2338.), GTPase inhibitors, antisense oligonucleotide, type 2 LRRK2 kinase inhibitors (Tasegian et al. Biochem J. 2021; 478(19):3555-3573.), and LRRK2 proteolysis targeting chimeras (PROTACs) (Konstantinidou et al., ChemMedChem 2021; 16(6):959-965.) (WO2020081682A1, WO2021194878A1, and WO2021194879A1) have therefore been proposed and explored. However, to date no potent, fast and selective LRRK2 degraders have been reported.

Induced target protein degradation is a paradigm-shifting drug discovery approach. Heterobifunctional degraders (also known as PROTACs) induce target protein degradation by recruiting E3 ubiquitin ligase in proximity to the target protein, resulting the ubiquitination and subsequent degradation of the target protein (Schapira et al. Nat. Rev. Drug Discov. 2019; 18(12):949-963.). Previous efforts on developing LRRK2 PROTACs resulted in three patents (WO2020081682A1, WO2021194878A1, and WO2021194879A1) and one publication (Konstantinidou et al., ChemMedChem 2021; 16(6):959-965.). However, all these reported compounds are cereblon-based that showed at best modest LRRK2 degradation and/or are slow degraders.

There is a need for identifying faster, more potent, and orally bioavailable and/or blood brain barrier penetrant LRRK2 PROTAC degraders targeting the GTPase and kinase activities of LRRK2., in particular for the treatment of Parkinson's Disease and related diseases.

### Summary

In a first aspect, there is provided a compound of formula (I):

X-L1-L2-Y Formula (I)

Wherein X is:

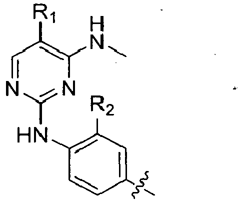

Wherein R₁ is selected from: F, Cl, Br, CF₃, CN, NO₂, CHF₂, CH₂F;

Wherein R₂ is selected from: - Me, CD₃, -OMe, OCD₃, OCF₃, -OCHF₂, Ethyl, -OCH₂CH₃, isopropyl, cyclopropane, and the following groups:

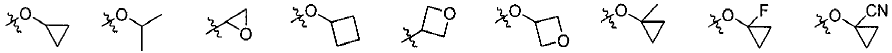

Wherein L1 is selected from:

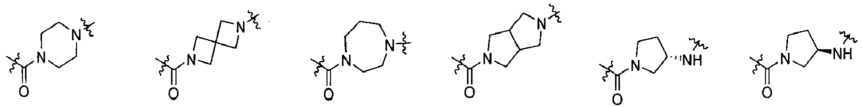

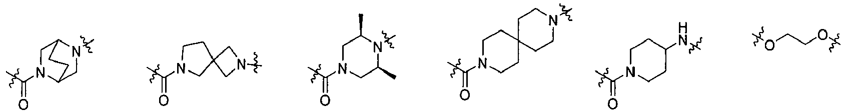

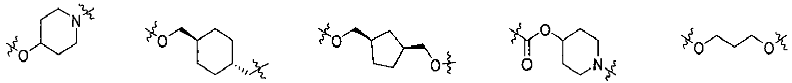

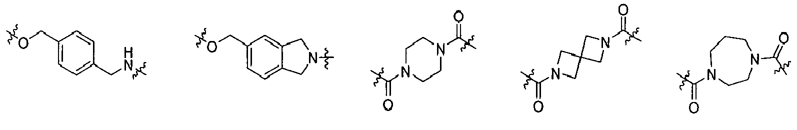

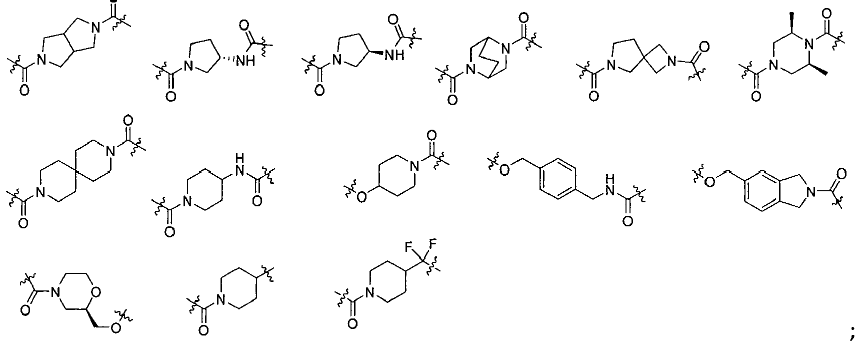

Wherein L2 is selected from: 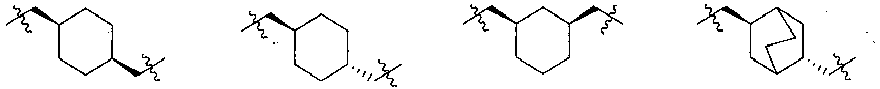 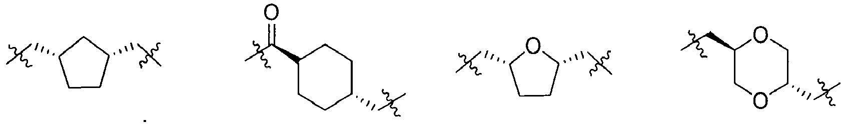 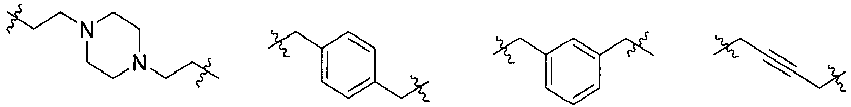 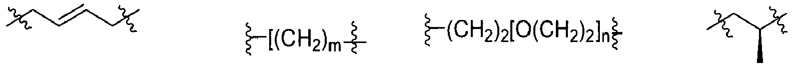 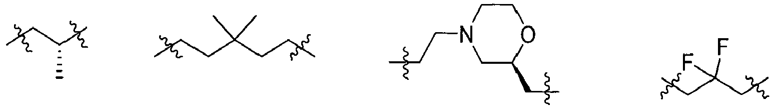 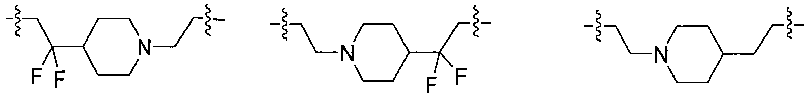 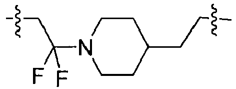
wherein m is independently selected from 2, 3, 4, 5, 6, 7, and 8; and
wherein n is independently selected from 1, 2, 3, 4, 5, and 6;

Wherein Y is selected from: 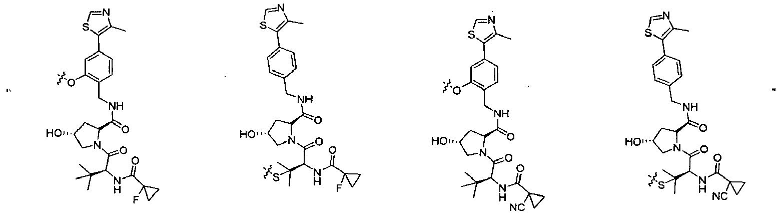 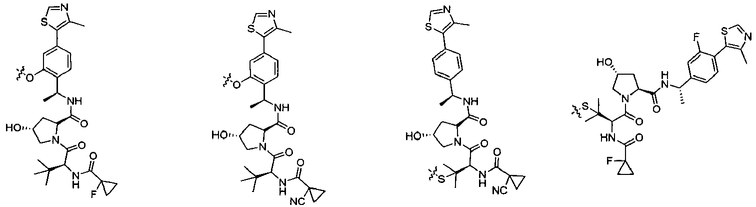 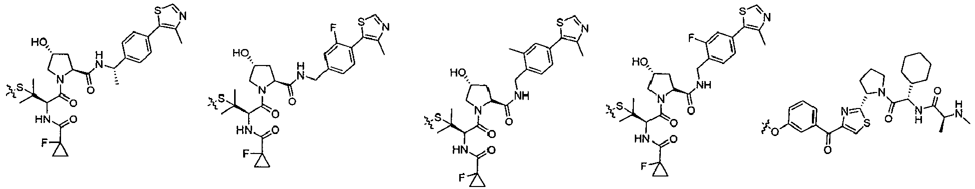 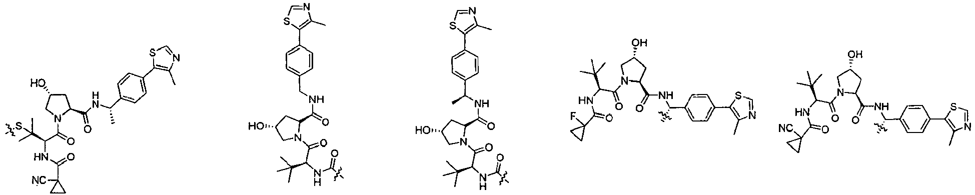 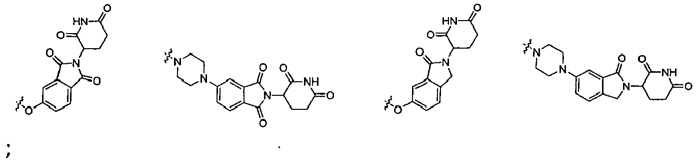 or a pharmaceutically acceptable, salt, enantiomer, stereoisomer, hydrate, solvate, or polymorph thereof.

In the compound of formula (I) X may be selected from

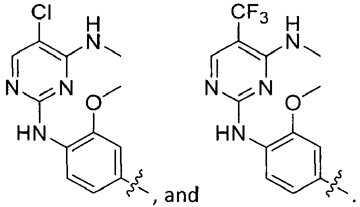

In the compound of formula (I), L1 may be selected from:

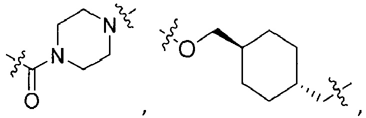

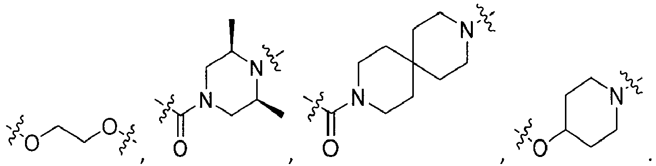

In preferred embodiments, L1 may be selected from 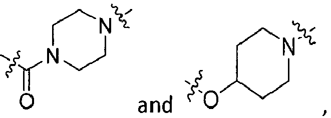 preferably

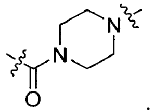

In the compound of formula (I), L2 may be selected from: 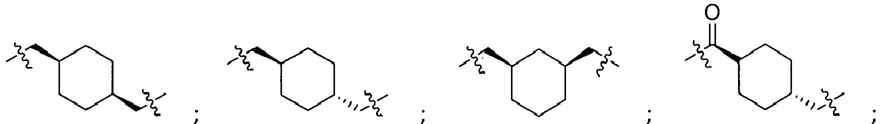 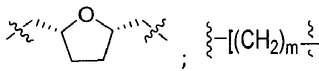 wherein m is independently selected from 3, 4, and 5; 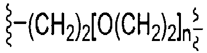 wherein n is independently selected from 1, 2, 3, and 4;

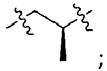

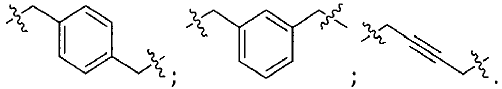

In preferred embodiments, L2 may be selected from 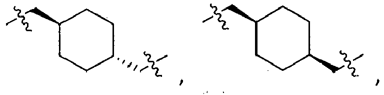 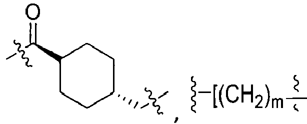 wherein m is independently selected from 3, 4, and 5 (especially wherein m is 3), 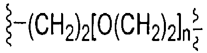 wherein n is independently selected from 1, 2, 3, and 4 (especially when n is 1or 2), and 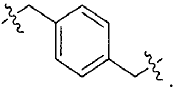 In further preferred embodiments, L2 may be selected from 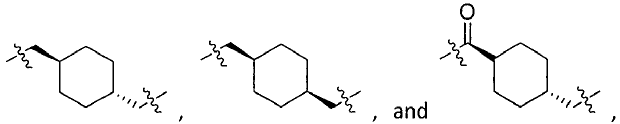 more particularly 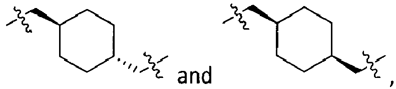 and most particularly

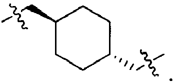

In the compound of formula (I), Y may be selected from:

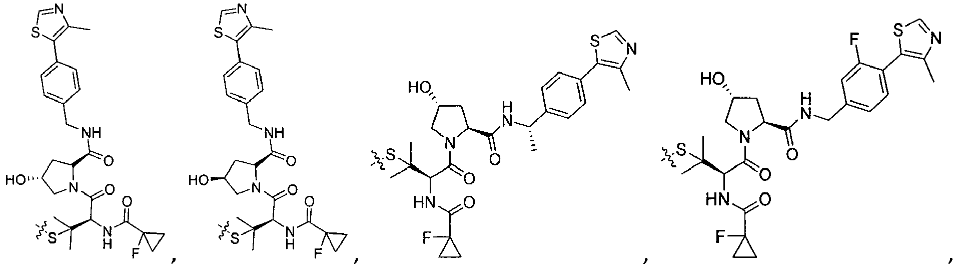

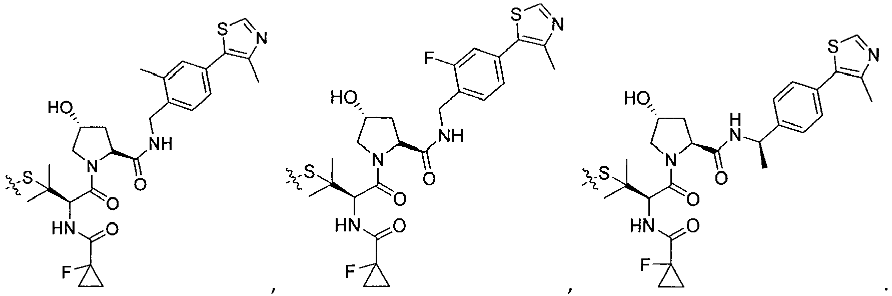

In further preferred embodiments, Y may be selected from 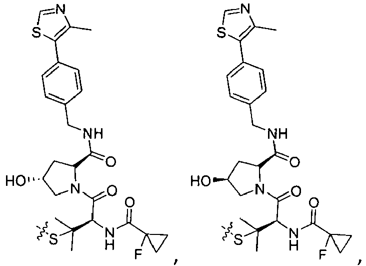 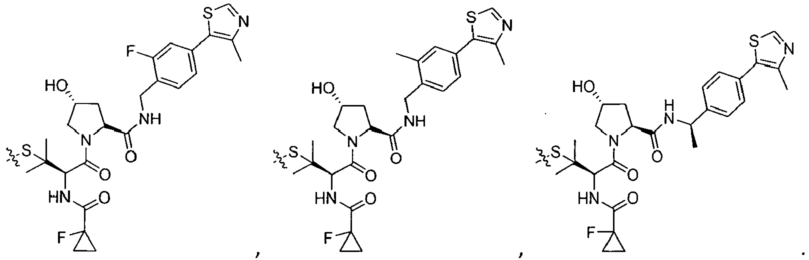 In particularly preferred embodiments, , Y may be selected from 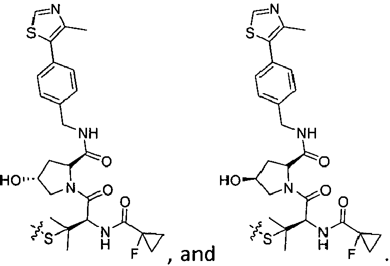 In most preferred embodiments, Y may be

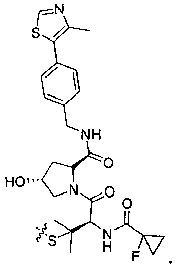

Preferred compounds of formula (I) may be selected from: 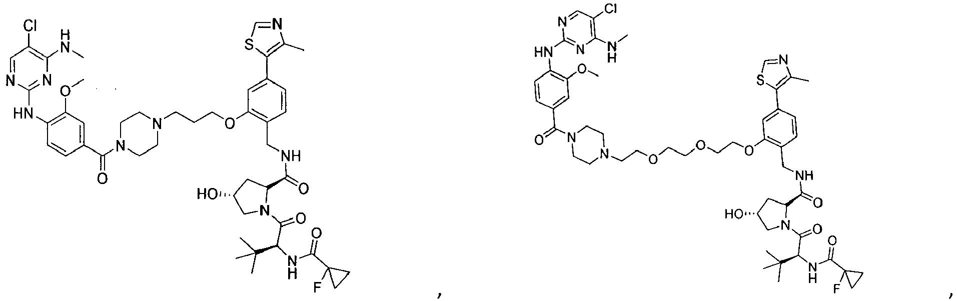 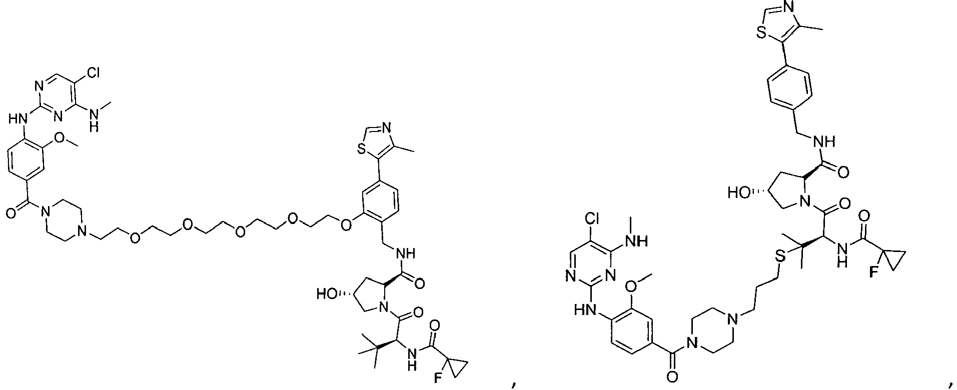 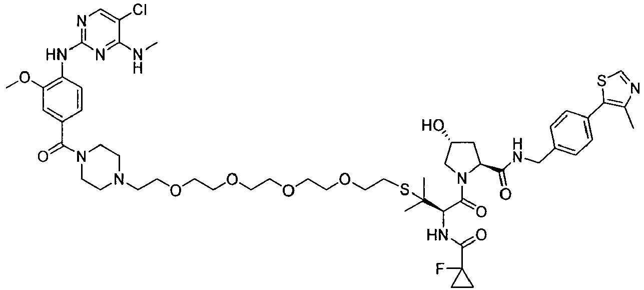 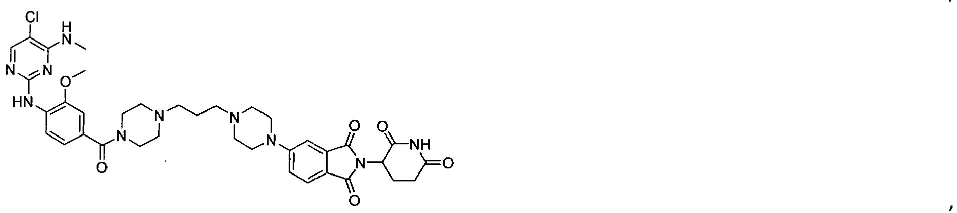 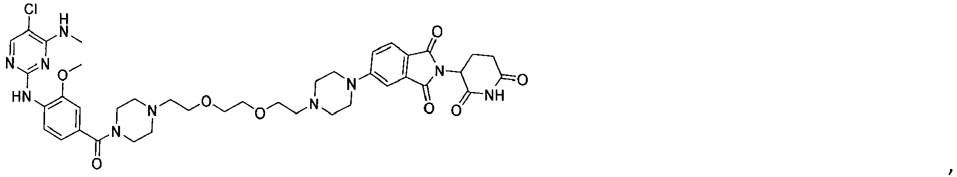 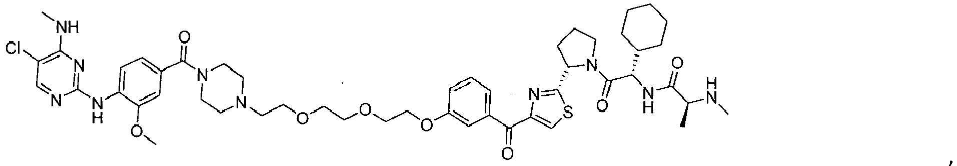 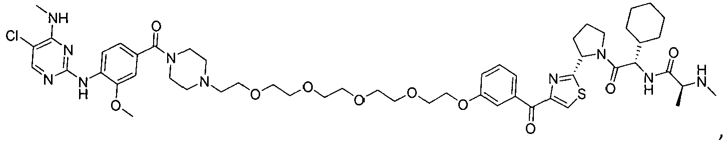 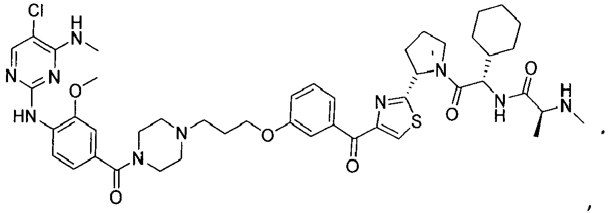 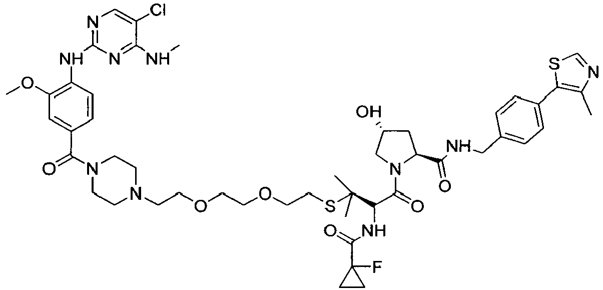 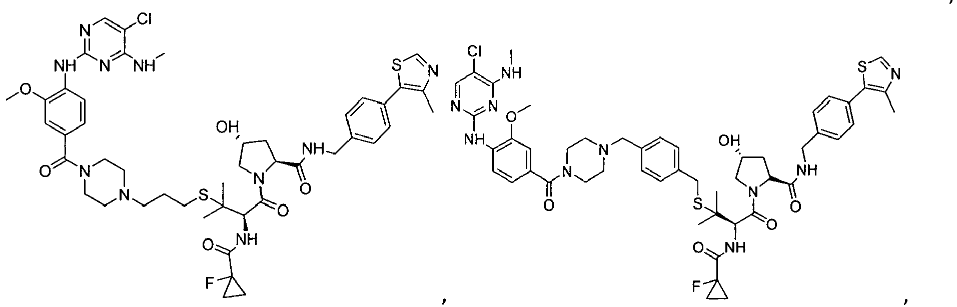 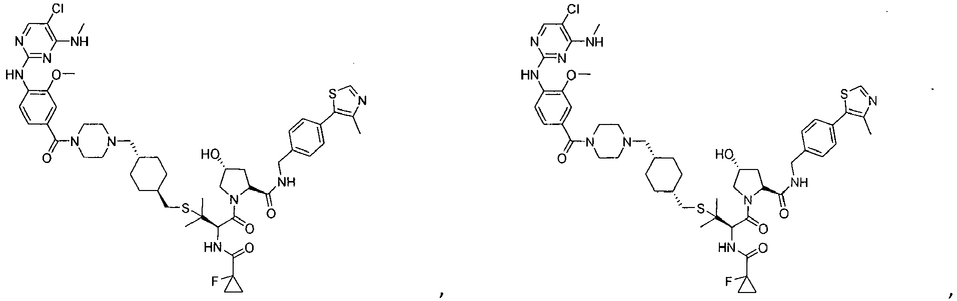 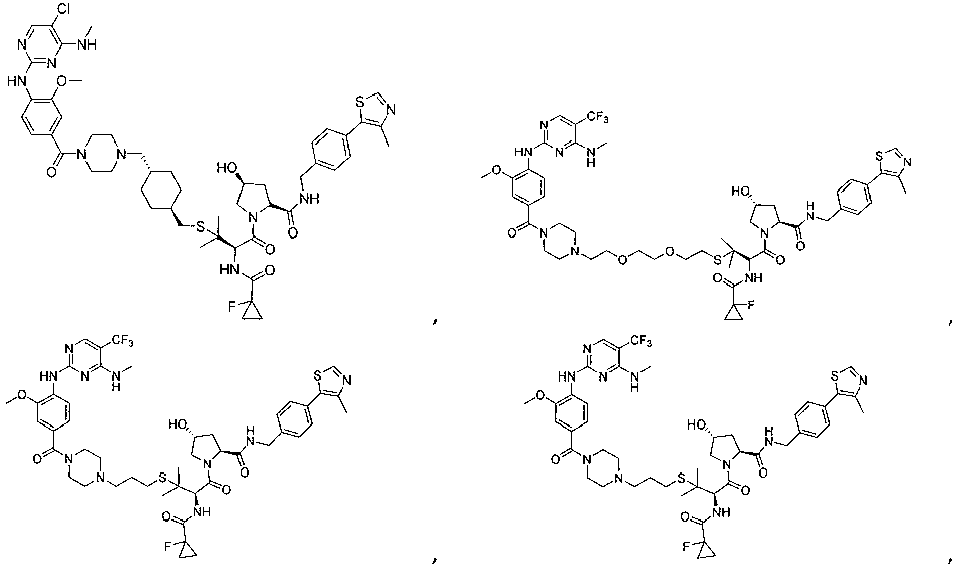 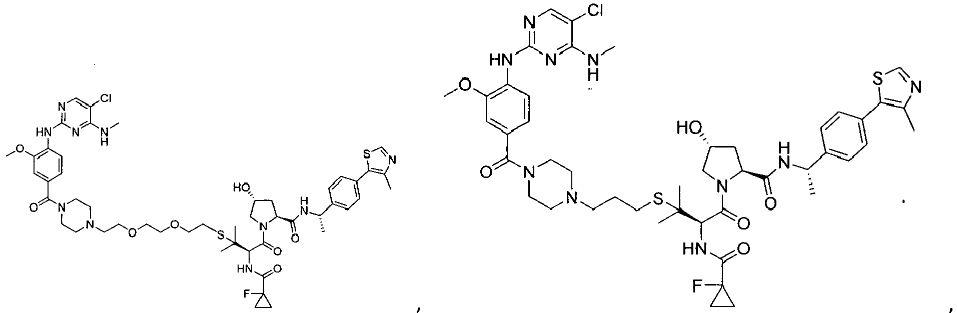 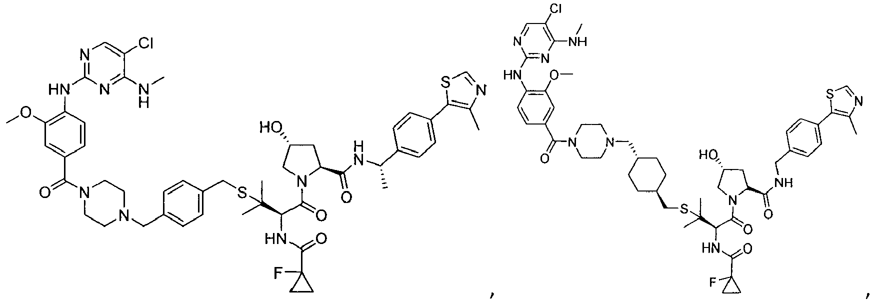 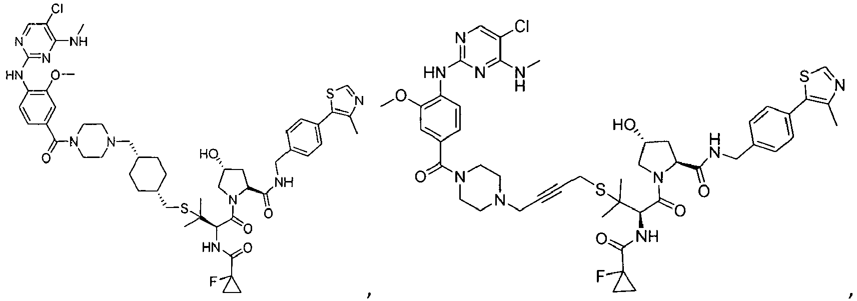 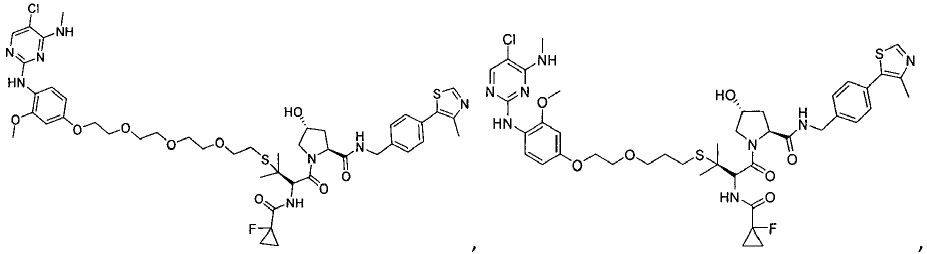 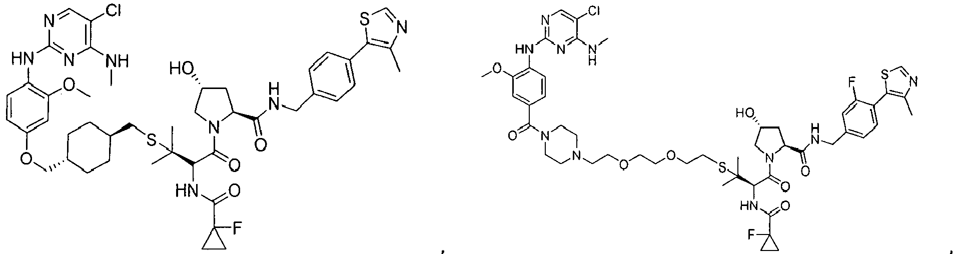 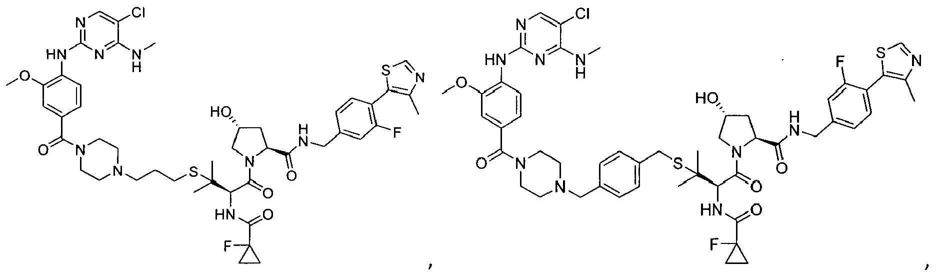 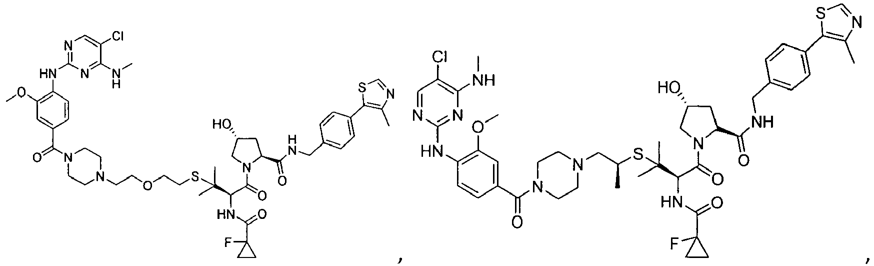 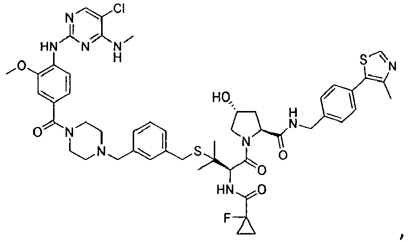 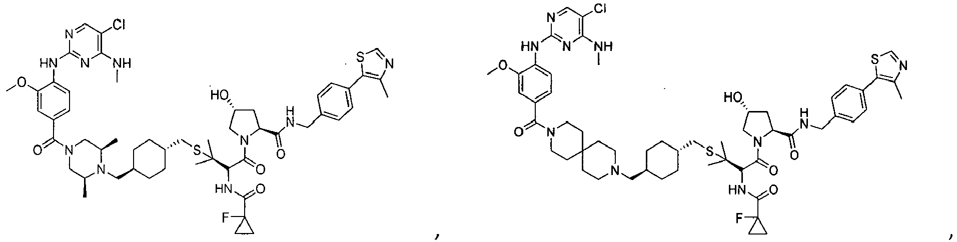 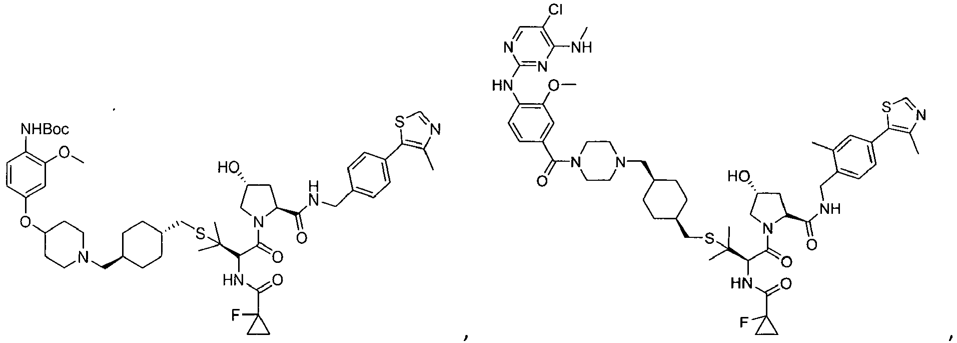 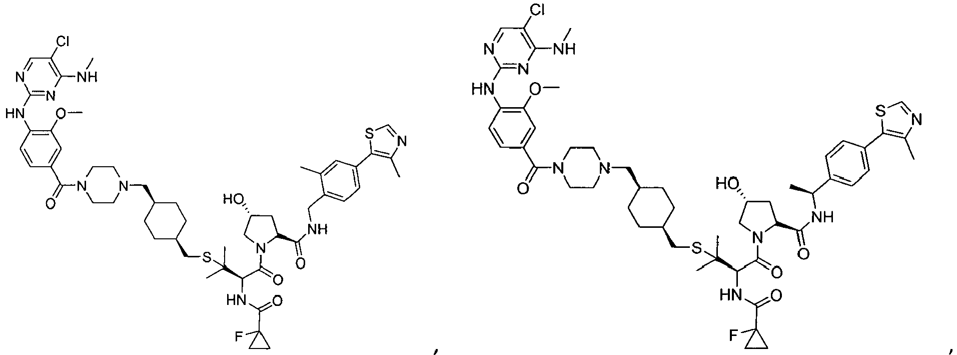 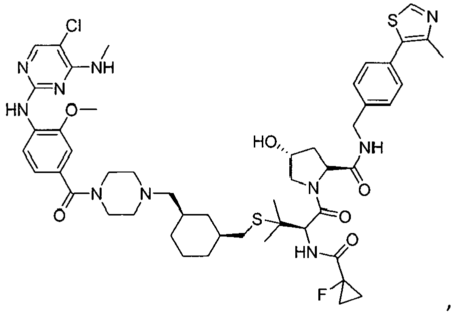 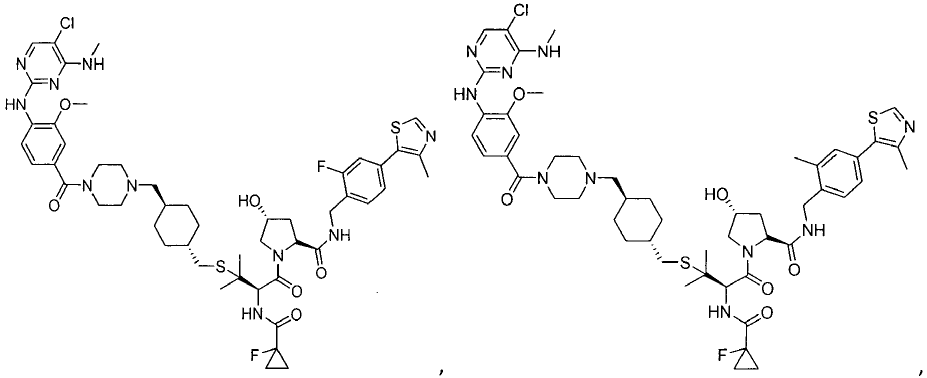 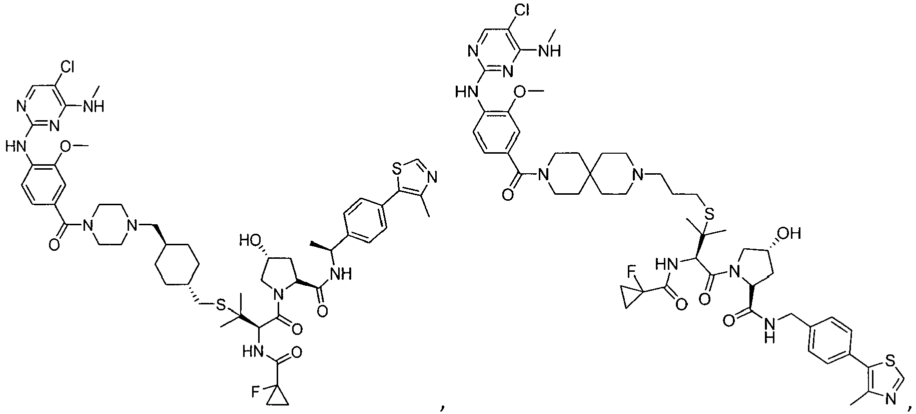 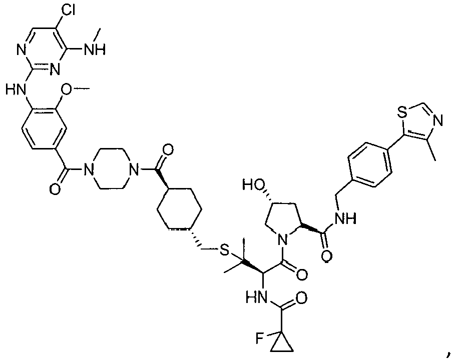 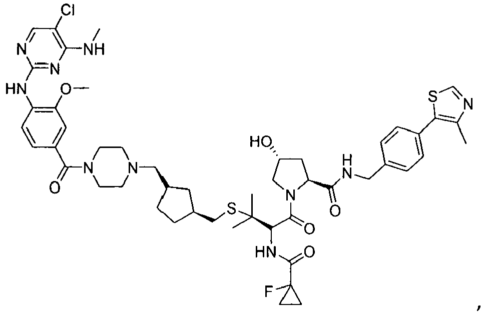 or a pharmaceutically acceptable, salt, enantiomer, stereoisomer, hydrate, solvate, or polymorph thereof.

Further preferred compounds of formula (I) may be selected from: 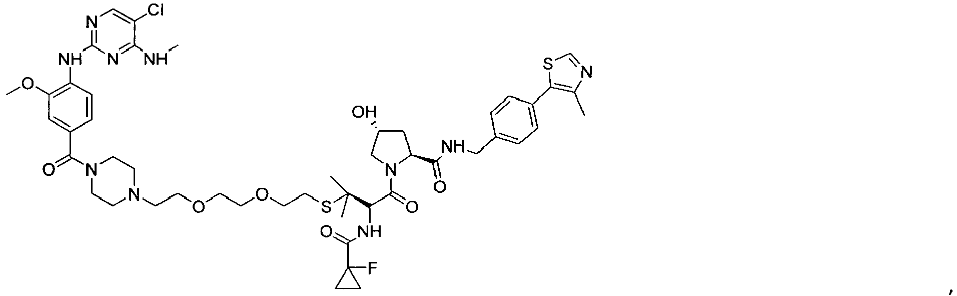 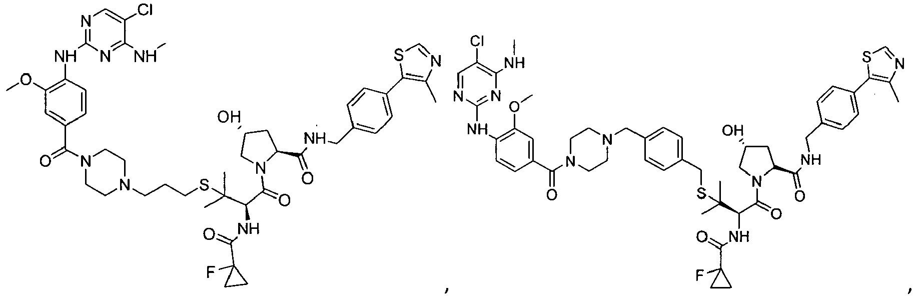 or a pharmaceutically acceptable, salt, enantiomer, stereoisomer, hydrate, solvate, or polymorph thereof.

Also, the compounds of formula (I) may be selected from: 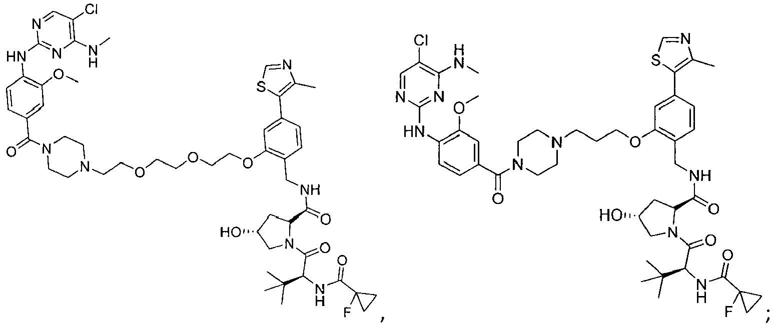 or a pharmaceutically acceptable, salt, enantiomer, stereoisomer, hydrate, solvate, or polymorph thereof.

Also preferred compounds of formula (I) may be selected from: 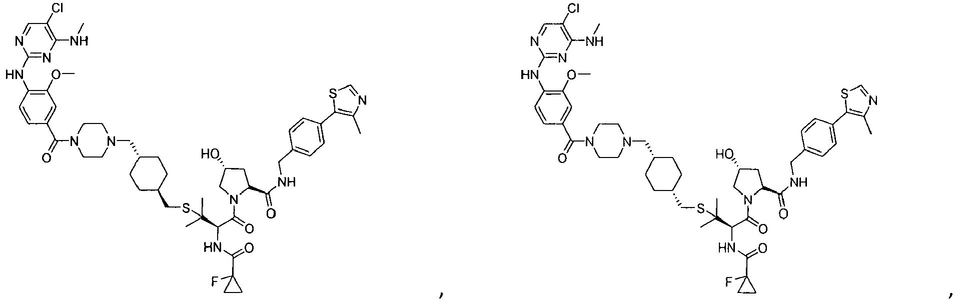 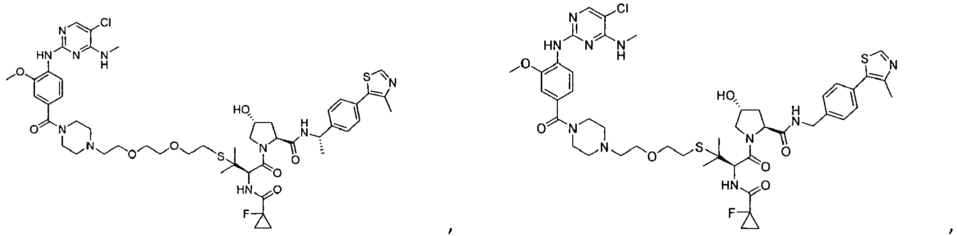 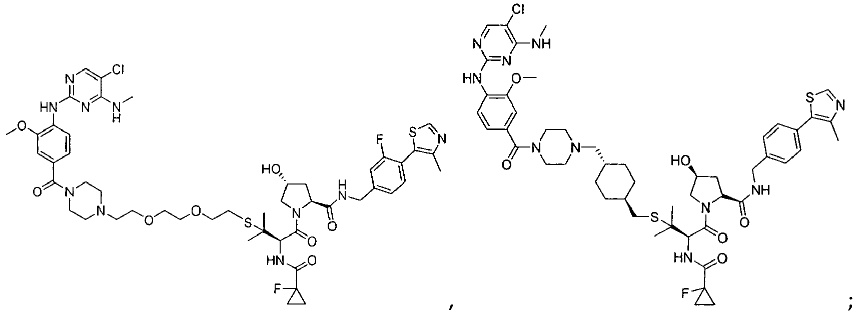 or a pharmaceutically acceptable, salt, enantiomer, stereoisomer, hydrate, solvate, or polymorph thereof.

The compound of formula (I) may further be selected from: 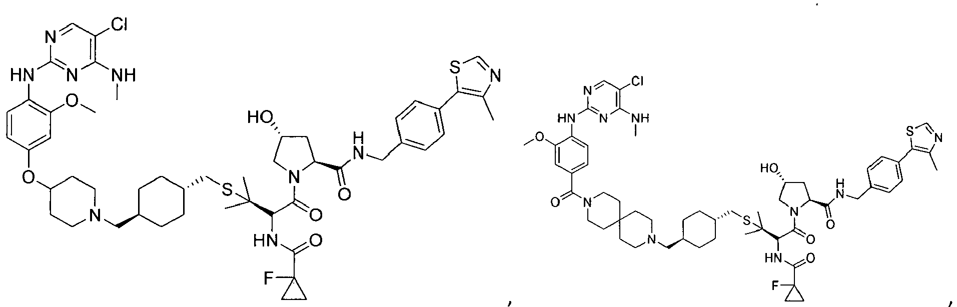 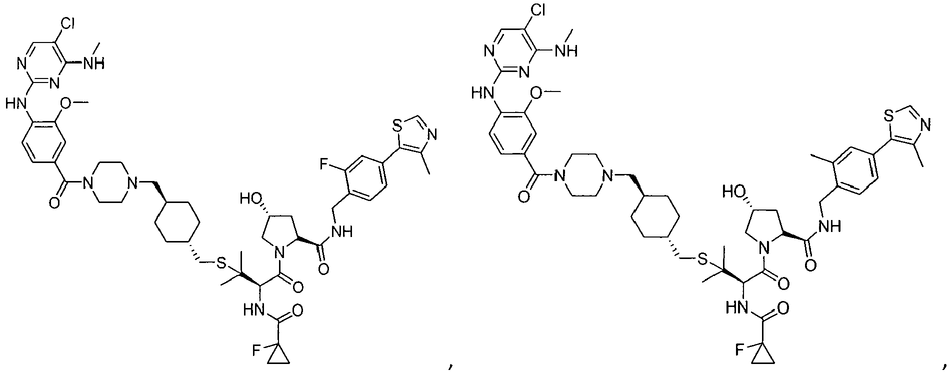 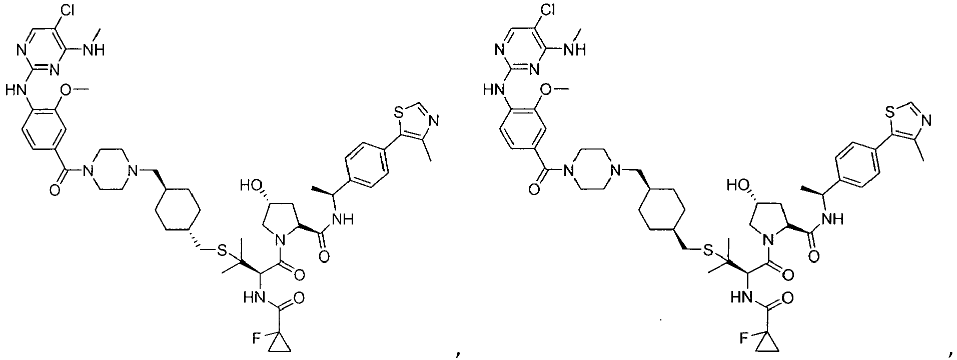 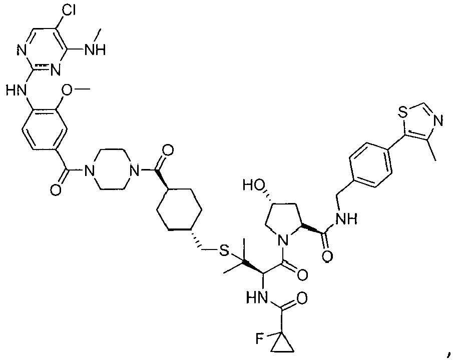 or a pharmaceutically acceptable, salt, enantiomer, stereoisomer, hydrate, solvate, or polymorph thereof.

Some other preferred compounds of formula (I) may be selected from: 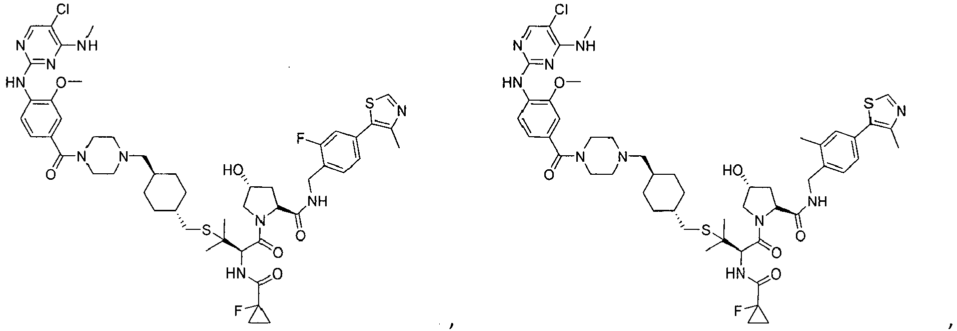 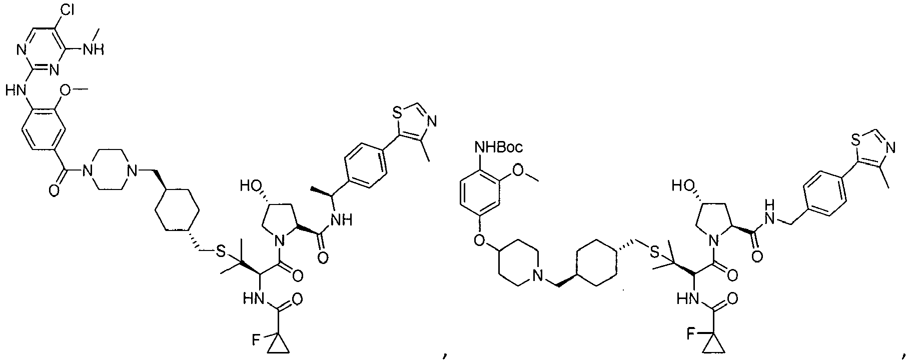 and 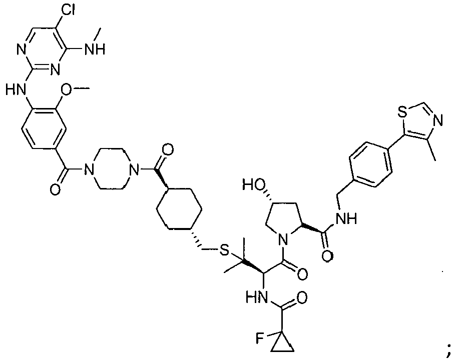 or a pharmaceutically acceptable, salt, enantiomer, stereoisomer, hydrate, solvate, or polymorph thereof.

Also, the compound formula (I) may be: 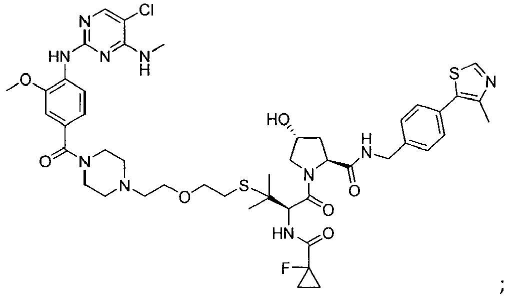 or a pharmaceutically acceptable, salt, enantiomer, stereoisomer, hydrate, solvate, or polymorph thereof.

Some other preferred compounds of formula (I) may be selected from: 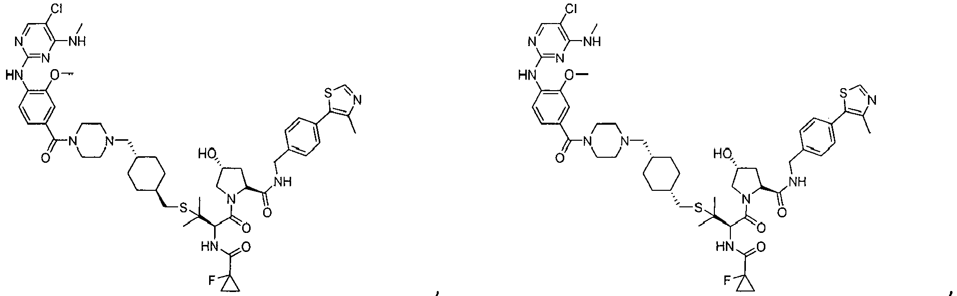 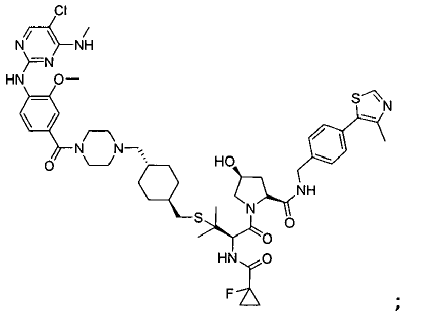 or a pharmaceutically acceptable, salt, enantiomer, stereoisomer, hydrate, solvate, or polymorph thereof.

A particularly preferred compound of formula (I) may be: 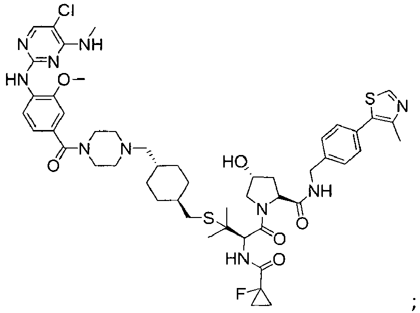 or a pharmaceutically acceptable, salt, enantiomer, stereoisomer, hydrate, solvate, or polymorph thereof.

In other words, preferred compounds of Formula (I) may be selected from:
*(2R,4R)-N-(2-(3-(4-(4-((5-chloro-4-(methylamino)pyrimidin-2-yi)amino)-3-methoxybenzoyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide* (SD13);
*(2R,4R)-N-(2-(2-(2-(2-(4-(4-((5-chloro-4-(methylamino)pyrimidin-2-yl)omino)-3-methoxybenzoyl)piperazin-1-yl)ethoxy)ethoxy)ethoxy)-4-(4-methylthiazol-5-yl)benzyl)-1-((S)-2-(1-fluorocyclopropane-1-corboxamido)-3,3-dimethylbutanoyl)-4-hydroxypyrrolidine-2-carboxamide (***SD12***);*
*(2R,4R)-N-(2-((14-(4-(4-((5-chloro-4-(methylamino)pyrimidin-2-yl)amino)-3-methoxybenzoyl)piperazin-1-yl)-3,6,9,12-tetraoxatetradecyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (***SD79***);*
*(2R,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide (***SD82***);*
*(2R,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropone-1-carboxamido)-3-methylbutanoyl)-4-hydroxy-N-(4-(4-methylthiazol-5-yl)benzyl)pyrrolidine-2-corboxamide (***SD75*****)**;*
*(2S,4R)-1-((R)-1-(4-(4-((5-chloro-4-(methylamino)pyrimidin-2-yl)amino)-3-methoxybenzoyl)piperazin-1-yl)-17-(1-fluorocyclopropane-1-carboxamido)-16,16-dimethyl-3,6,9,12-tetraoxa-15-thiaoctodecon-18-oyl)-4-hydroxy-N-(4-(4-methylthiazol-5-yl)benzyl)pyrrolidine-2-carboxamide (***SD74***);*
*(2R,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-corboxamido)-3-methylbutanoyl)-4-hydroxy-N-(4-(4-methylthiazol-5-yl)benzyl)pyrrolidine-2-carboxamide (***SD100)*;***
*5-(4-(3-(4-(4-((5-chloro-4-(methylamino)pyrimidin-2-yl)amino)-3-methoxybenzoyl)piperazin-1-yl)propyl)piperazin-1-yl)-2-(2, 6-dioxopiperidin-3-yl)isoindoline-1,3-dione (***SD11*****)**;*
*5-(4-(2-(2-(2-(4-(4-((5-chloro-4-(methylamino)pyrimidin-2-yl)amino)-3-methoxybenzoyl)piperazin-1-yl)ethoxy)ethoxy)ethyl)piperazin-1-yl)-2-(2,6-dioxopiperidin-3-yl)isoindoline-1,3-dione (***SD10***);*
*(S)-N-((S)-2-((S)-2-(4-(3-(2-(2-(2-(4-(4-((5-chloro-4-(methylamino)pyrimidin-2-yl)omino)-3-methoxybenzoyl)piperazin-1-yl)ethoxy)ethoxy)ethoxy)benzoyl)thiazol-2-yl)pyrrolidin-1-yl)-1-cyclohexyl-2-oxoethyl)-2-(methylamino)propenamide HCl salt (***SD113***);*
*(S)-N-((S)-2-((S)-2-(4-(3-((14-(4-(4-((5-chloro-4-(methylamino)pyrimidin-2-yl)amino)-3-methoxybenzoyl)piperazin-1-yl)-3,6,9,12-tetraoxatetradecyl)oxy)benzoyl)thiazol-2-yl)pyrrolidin-1-yl)-1-cyclohexyl-2-oxoethyl)-2-(methylamino)propenamide (***SD112***);*
*(S)-N-((S)-2-((S)-2-(4-(3-(3-(4-(4-((5-chloro-4-(methylamino)pyrimidin-2-yl)amino)-3-methoxybenzoyl)piperazin-1-yl)propoxy)benzoyl)thiazol-2-yl)pyrrolidin-1-yl)-1-cyclohexyl-2-oxoethyl)-2-(methylamino)propenamide (**SD114**);*
*(2S,4R)-1-((S)-2-(1-fluorocyclopropane-1-carboxamido)-3-((2-(2-(2-(4-(3-methoxy-4-((4-(methylamino)-5-(trifluoromethyl)pyrimidin-2-yl)amino)benzoyl)piperazin-1-yl)ethoxy)ethoxy)ethyl)thio)-3-methylbutanoyl)-4-hydroxy-N-(4-(4-methylthiazol-5-yl)benzyl)pyrrolidine-2-carboxamide (***XL01078B***);*
*(2S,4R)-1-((R)-2-(1-fluorocyclopropone-1-carboxamido)-3-((3-(4-(3-methoxy-4-((4-(methylamino)-5-(trifluoromethyl)pyrimidin-2-yl)amino)benzoyl)piperazin-1-yl)propyl)thio)-3-methylbutanoyl)-4-hydroxy-N-(4-(4-methylthiazol-5-yl)benzyl)pyrrolidine-2-carboxamide (***XL01072***);*
*(2R,4R)-1-((R)-2-(1-fluorocyclopropane-1-carboxamido)-3-((4-((4-(3-methoxy-4-((4-(methylamino)-5-(trifluoromethyl)pyrimidin-2-yl)amino)benzoyl)piperazin-1-yl)methyl)benzyl)thio)-3-methylbutanoyl)-4-hydroxy-N-(4-(4-methylthiazol-5-yl)benzyl)pyrrolidine-2-carboxamide (***XL01070B***);*
*(2S,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide (***XL01119***);*
*(2S,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropone-1-carboxamido)-3-methylbutanoyl)-4-hydroxy-N-((S)-1-(4-(4-methylthiazol-5-yl)phenyl)ethyl)pyrrolidine-2-carboxamide (***XL01118***);*
*(2S,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-corboxamido)-3-methylbutanoyl)-4-hydroxy-N-((S)-1-(4-(4-methylthiazol-5-yl)phenyl)ethyl)pyrrolidine-2-carboxamide (***XL01120***);*
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL01126***);*
*(2R,4R)-1-((R)-3-((((1S,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide (***XL01134)***;*
*(2S,4R)-1-((R)-3-((4-(4-(4-((5-chloro-4-(methylamino)pyrimidin-2-yl)amino)-3-methoxybenzoyl)piperazin-1-yl)but-2-yn-1-yl)thio)-2-(1-fluorocyclopropone-1-corboxamido)-3-methylbutanoyl)-4-hydroxy-N-(4-(4-methylthiazol-5-yl)benzyl)pyrrolidine-2-carboxamide (***XL01076***);*
*(2S,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropane-1-carboxamido)-3-methylbutanoyl)-N-(3-fluoro-4-(4-methylthiazol-5-yl)benzyl)-4-hydroxypyrrolidine-2-carboxamide* **(XL01123);**
*(2S,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropone-1-carboxamido)-3-methylbutanoyl)-N-(3-fluoro-4-(4-methylthiazol-5-yl)benzyl)-4-hydroxypyrrolidine-2-carboxamide (***XL01122***);*
*(2S,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-carboxamido)-3-methylbutanoyl)-N-(3-fluoro-4-(4-methylthiazol-5-yl)benzyl)-4-hydroxypyrrolidine-2-carboxamide* **(XL01121);**
*(2S,4R)-1-((R)-3-((2-(2-(4-(4-((5-chloro-4-(methylamino)pyrimidin-2-yl)amino)-3-methoxybenzoyl)piperazin-1-yl)ethoxy)ethyl)thio)-2-(1-fluorocyclopropane-1-corboxamido)-3-methylbutanoyl)-4-hydroxy-N-(4-(4-methylthiazol-5-yl)benzyl)pyrrolidine-2-carboxamide (***XL01131)***;*
*(2R,4R)-1-((R)-3-(((S)-1-(4-(4-((5-chloro-4-(methylamino)pyrimidin-2-yl)amino)-3-methoxybenzoyl)piperazin-1-yl)propan-2-yl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL01140***);*
*(2S,4R)-1-((R)-3-((3-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-corboxamido)-3-methylbutanoyl)-4-hydroxy-N-(4-(4-methylthiazol-5-yl)benzyl)pyrrolidine-2-carboxamide (***XL01111***);*
*(2S,4R)-1-((S)-1-(4-((5-chloro-4-(methylamino)pyrimidin-2-yl)amino)-3-methoxyphenoxy)-14-(1-fluorocyclopropane-1-corboxamido)-13,13-dimethyl-3,6,9-trioxa-12-thiopentodecon-15-oyl)-4-hydroxy-N-(4-(4-methylthiazol-5-yl)benzyl)pyrrolidine-2-carboxamide (***XL01145***);*
*(2S,4R)-1-((S)-3-((3-(2-(4-((5-chloro-4-(methylamino)pyrimidin-2-yl)amino)-3-methoxyphenoxy)ethoxy)propyl)thio)-2-(1-fluorocyclopropane-1-corboxamido)-3-methylbutanoyl)-4-hydroxy-N-(4-(4-methylthiazol-5-yl)benzyl)pyrrolidine-2-carboxamide (***XL01149***);*
*(2S,4R)-1-((S)-3-((((1R,4S)-4-((4-((5-chloro-4-(methylamino)pyrimidin-2-yl)amino)-3-methoxyphenoxy)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL01168***);*
*(2S,4R)-1-((S)-3-((((1R,4S)-4-((4-(4-((5-chloro-4-(methylamino)pyrimidin-2-yl)amino)-3-methoxyphenoxy)piperidin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL02017***);*
*(2S,4R)-1-((S)-3-((((1R,4S)-4-(((2R,6S)-4-(4-((5-chloro-4-(methylamino)pyrimidin-2-yl)amino)-3-methoxybenzoyl)-2,6-dimethylpiperazin-1-yl)methyl)cyclohexyl)methyl)thio)-2-(1-fluorocyclopropane-1-corboxamido)-3-methylbutanoyl)-4-hydroxy-N-(4-(4-methylthiazol-5-yl)benzyl)pyrrolidine-2-carboxamide (***XL02031***);*
*(2S,4R)-1-((S)-3-((((1R,4S)-4-((9-(4-((5-chloro-4-(methylamino)pyrimidin-2-yl)amino)-3-methoxybenzoyl)-3,9-diazaspiro[5.5]undecan-3-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL02038***);*
*(2R,4R)-1-((R)-3-((((1r,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-N-(2-fluoro-4-(4-methylthiazol-5-yl)benzyl)-4-hydroxypyrrolidine-2-carboxamide (**XL02047**);*
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(2-methyl-4-(4-methylthiazol-5-yl)benzyl)pyrrolidine-2-carboxamide (***XL02048)***;*
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide (***XL02049***);*
*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-N-(2-fluoro-4-(4-methylthiazol-5-yl)benzyl)-4-hydroxypyrrolidine-2-carboxamide (***XL02058***);*
*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(2-methyl-4-(4-methylthiazol-5-yl)benzyl)pyrrolidine-2-carboxamide (***XL02059***);*
*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide (***XL02060***);*
*(2R,4R)-1-((R)-3-((((1S,3R)-3-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide (***XL02063***);*
*(2R,4R)-1-((R)-3-((3-(9-(4-((5-chloro-4-(methylamino)pyrimidin-2-yl)amino)-3-methoxybenzoyl)-3,9-diazospiro[5.5]undecan-3-yl)propyl)thio)-2-(1-fluorocyclopropane-1-corboxamido)-3-methylbutanoyl)-4-hydroxy-N-(4-(4-methylthiazol-5-yl)benzyl)pyrrolidine-2-carboxamide (***XL02067B***);*
*(2R,4R)-1-((R)-3-((((1R,4R)-4-(4-(4-((5-chloro-4-(methylamino)pyrimidin-2-yl)amino)-3-methoxybenzoyl)piperazine-1-carbonyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL02068***);*
*(2S,4R)-1-((S)-3-((((1S,3S)-3-((4-(4-((5-chloro-4-(methylamino)pyrimidin-2-yl)amino)-3-methoxybenzoyl)piperazin-1-yl)methyl)cyclopentyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide* **(XL02093);**
or a pharmaceutically acceptable, salt, enantiomer, stereoisomer, hydrate, solvate, or polymorph thereof.

Further preferred compounds be selected from:
*(2R,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***SD75***);*
*(2R,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide (***SD82***);*
*(2R,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-corboxamido)-3-methylbutanoyl)-4-hydroxy-N-(4-(4-methylthiazol-5-yl)benzyl)pyrrolidine-2-carboxamide (***SD100***);*
or a pharmaceutically acceptable, salt, enantiomer, stereoisomer, hydrate, solvate, or polymorph thereof.

Further preferred compounds may also be selected from:
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL01126***);*
*(2R,4S)-1-((R)-3-((((1r,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide* (**cis-XL01126**);
*(2R,4R)-1-((R)-3-((((1S,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL01134***);*
or a pharmaceutically acceptable, salt, enantiomer, stereoisomer, hydrate, solvate, or polymorph thereof.

Further preferred compounds may also be selected from:
*(2S,4R)-1-((S)-3-((((1R,4S)-4-((4-(4-((5-chloro-4-(methylamino)pyrimidin-2-yl)amino)-3-methoxyphenoxy)piperidin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***XL02017***);*
*(2R,4R)-1-((R)-3-((((1r,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-N-(2-fluoro-4-(4-methylthiazol-5-yl)benzyl)-4-hydroxypyrrolidine-2-carboxamide (**XL02047**);*
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-(2-methyl-4-(4-methylthiazol-5-yl)benzyl)pyrrolidine-2-carboxamide (***XL02048***);*
*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide (***X102049***);*
*(2R,4R)-1-((R)-3-((((1R,4R)-4-(4-(4-((5-chloro-4-(methylamino)pyrimidin-2-yl)amino)-3-methoxybenzoyl)piperazine-1-carbonyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (***X102068***);*
or a pharmaceutically acceptable, salt, enantiomer, stereoisomer, hydrate, solvate, or polymorph thereof.

Particularly preferred compounds of formula (I) may be selected from **XL01126, Cis-XL01126,** and **XL01134,** particularly **XL01126,** or a pharmaceutically acceptable, salt, enantiomer, stereoisomer, hydrate, solvate, or polymorph thereof.

Also preferred compounds of formula (I) may be selected from **XL02047,** XL02048, **XL02049, XL02017,** and **XL02068,** or a pharmaceutically acceptable, salt, enantiomer, stereoisomer, hydrate, solvate, or polymorph thereof.

Also preferred compounds of formula (I) may be selected from **SD82, SD75,** and **DS100,** or a pharmaceutically acceptable, salt, enantiomer, stereoisomer, hydrate, solvate, or polymorph thereof.

Also preferred compounds of formula (I) may be selected from **SD12, SD13,** and **XL01131,** or a pharmaceutically acceptable, salt, enantiomer, stereoisomer, hydrate, solvate, or polymorph thereof.

In a second aspect there is provided a pharmaceutical composition comprising a compound as defined herein above and a pharmaceutically acceptable vehicle or diluent therefor.

The pharmaceutical composition may comprise an effective amount of a compound as defined hereinabove in combination with a pharmaceutically acceptable carrier, additive or excipient and optionally in further combination with an additional bioactive agent.

In a third aspect there is provided a compound as defined herein for use in medicine. Also provided herein is the use of a compound as defined herein in medicine.

In a fourth aspect there is provided a compound as defined herein for use in the prophylaxis or treatment of a disease or condition associated with altered GTPase and/or kinase activity of the protein Leucine-rich repeat kinase 2 (LRRK2) comprising the administration of a PROTAC compound of Formula (I) to a subject suffering from or likely to be exposed to said disease or condition. Also provided herein is the use of a compound as defined herein for the prophylaxis or treatment of a disease or condition associated with altered GTPase and/or kinase activity of the protein Leucine-rich repeat kinase 2 (LRRK2) comprising the administration of a PROTAC compound of Formula (I) to a subject suffering from or likely to be exposed to said disease or condition.

In a fifth aspect there is provided a compound as defined herein for use in the prophylaxis or treatment of a disease or condition associated with altered PDE6D activity, comprising the administration of a PROTAC compound of Formula (I) as defined herein to a subject suffering from or likely to be exposed to a PDE6D-related disease or condition. Also provided herein there is provided the use of a compound as defined herein for the prophylaxis or treatment of a disease or condition associated with altered PDE6D activity, comprising the administration of a PROTAC compound of Formula (I) as defined herein to a subject suffering from or likely to be exposed to a PDE6D-related disease or condition.

In a sixth aspect there is provided a compound as defined herein for use in the prophylaxis or treatment of a disease or condition independently selected from: Parkinson's Disease, idiopathic Parkinson's Disease, idiopathic late-onset Parkinson's Disease, familial Parkinson's Disease, LRRK2 mutation associated Parkinson's disease, Lewy body dementia, primary tauopathy, or inflammation-associated disease such as Leprosy, neuroinflammation, and Crohn's disease. Also provided herein is the use of a compound as defined herein for the prophylaxis or treatment of a disease or condition independently selected from: Parkinson's Disease, idiopathic Parkinson's Disease, idiopathic late-onset Parkinson's Disease, familial Parkinson's Disease, LRRK2 mutation associated Parkinson's disease, Lewy body dementia, primary tauopathy, or inflammation-associated disease such as Leprosy, neuroinflammation, and Crohn's disease.

In a seventh aspect there is provided a method of regulating protein activity of protein Leucine-rich repeat kinase 2 (LRRK2) in a patient in need comprising administering to said patient an amount of a compound as described herein.

In an eight aspect there is provided a method of degrading a Leucine-rich repeat kinase 2 (LRRK2) in a patient in need comprising administering to said patient an effective amount of a compound as described herein.

In a further aspect there is provided a method of regulating protein activity of protein PDE6D in a patient in need comprising administering to said patient an amount of a compound as described herein.

In a further aspect there is provided a method of degrading a protein PDE6D in a patient in need comprising administering to said patient an effective amount of a compound as described herein.

In a further aspect there is provided a method of treating a patient suffering from a disease or condition associated with altered GTPase and/or kinase activity of the protein Leucine-rich repeat kinase 2 (LRRK2) comprising the administration of a PROTAC compound of Formula (I) to a subject suffering from or likely to be exposed to said disease or condition

In a further aspect there is provided a method of treating a patient suffering from a disease or condition associated with altered PDE6D activity, comprising the administration of a PROTAC compound of Formula (I) as defined herein to a subject suffering from or likely to be exposed to a PDE6D-related disease or condition, the method comprising administering a therapeutically effective dose of the compound as described herein or the pharmaceutical composition as described herein.

In a further aspect there is provided a method of treating a patient suffering from a disease or condition independently selected from: Parkinson's Disease, idiopathic Parkinson's Disease, idiopathic late-onset Parkinson's Disease, familial Parkinson's Disease, LRRK2 mutation associated Parkinson's disease, Lewy body dementia, primary tauopathy, or inflammation-associated disease such as Leprosy, neuroinflammation, and Crohn's disease, the method comprising administering a therapeutically effective dose of the compound as described herein or the pharmaceutical composition as described herein.

### Brief Description of the Figures

FIGURE 1 is an image of western blots that show the degradation of LRRK2 total, the inhibition of phosphorylation of LRRK2 at Ser935 and the inhibition of phosphorylation of Rab10 at Thr73 after 33 nM/4h and 1µM/4h treatment of the first-generation degraders on WT mouse embryonic fibroblasts (MEFs) and LRRK2^{G2019S} MEFs.
FIGURE 2 is an image of western blots that show the degradation of LRRK2 total, the inhibition of phosphorylation of LRRK2 at Ser935 and the inhibition of phosphorylation of RablO at Thr73 after 33 nM/24h and 1µM/24h treatment of the first-generation degraders on WT MEFs and LRRK2^{G2019S} MEFs.
FIGURE 3 is an image of western blots that show the degradation of LRRK2 total, the inhibition of phosphorylation of LRRK2 at Ser935 and the inhibition of phosphorylation of RablO at Thr73 after 33 nM/4h and 1µM/4h treatment of the second generation (set 1) degraders on WT MEFs and LRRK2^{G2019S} MEFs.
FIGURE 4 is an image of western blots that show the degradation of LRRK2 total, the inhibition of phosphorylation of LRRK2 at Ser935 and the inhibition of phosphorylation of RablO at Thr73 after 33 nM/24h and 1µM/24h treatment of the second generation (set 1) degraders on WT MEFs and LRRK2^{G2019S} MEFs.
FIGURE 5 is an image of western blots that show the degradation of LRRK2 total, the inhibition of phosphorylation of LRRK2 at Ser935 and the inhibition of phosphorylation of RablO at Thr73 after 33 nM/4h and 1µM/4h treatment of the second generation (set 2) degraders on WT MEFs and LRRK2^{G2019S} MEFs.
FIGURE 6 is an image of western blots that show the degradation of LRRK2 total, the inhibition of phosphorylation of LRRK2 at Ser935 and the inhibition of phosphorylation of RablO at Thr73 after 33 nM/24h and 1µM/24h treatment of the second generation (set 2) degraders on WT MEFs and LRRK2^{G2019S} MEFs.
FIGURE 7 is an image of western blots that show the degradation of LRRK2 total, the inhibition of phosphorylation of LRRK2 at Ser935 and the inhibition of phosphorylation of RablO at Thr73 after 33 nM/4h and 1µM/4h treatment of the third generation (set 1) degraders on WT MEFs and LRRK2^{G2019S} MEFs.
FIGURE 8 is an image of western blots that show the degradation of LRRK2 total, the inhibition of phosphorylation of LRRK2 at Ser935 and the inhibition of phosphorylation of RablO at Thr73 after 33 nM/24h and 1µM/24h treatment of the third generation (set 1) degraders on WT MEFs and LRRK2^{G2019S} MEFs.
FIGURE 9 is an image of western blots that show the degradation of LRRK2 total, the inhibition of phosphorylation of LRRK2 at Ser935 and the inhibition of phosphorylation of RablO at Thr73 after 33 nM/4h and 1µM/4h treatment of the third generation (set 2) degraders on WT MEFs and LRRK2^{G2019S} MEFs.
FIGURE 10 is an image of western blots that show the degradation of LRRK2 total, the inhibition of phosphorylation of LRRK2 at Ser935 and the inhibition of phosphorylation of Rab10 at Thr73 after 33 nM/24h and 1µM/24h treatment of the third generation (set 2) degraders on WT MEFs and LRRK2^{G2019S} MEFs.
FIGURE 11A is an image of western blots that show the degradation of LRRK2 total, the inhibition of phosphorylation of LRRK2 at Ser935 and the inhibition of phosphorylation of RablO in a dose dependent manner by SD75 after 24 h treatment on WT MEFs and LRRK2^{G2019S} MEFs.
FIGURE 11B is a graph that shows the degradation of LRRK2^{WT} total and LRRK2^{G2019S} total in a dose dependent manner by SD75 after 24 h treatment on WT MEFs and LRRK2^{G2019S} MEFs separately.
FIGURE 11C is a graph that shows the inhibition of phosphorylation of RablO at Thr73 in a dose dependent manner by SD75 after 24 h treatment on WT MEFs and LRRK2^{G2019S} MEFs separately.
FIGURE 12A is an image of western blots that show the degradation of LRRK2 total, the inhibition of phosphorylation of LRRK2 at Ser935 and the inhibition of phosphorylation of RablO in a dose dependent manner by XL01134 after 4 h treatment on WT MEFs and LRRK2^{G2019S} MEFs.
FIGURE 12B is a graph that shows the degradation of LRRK2^{WT} total and LRRK2^{G2019S} total in a dose dependent manner by XL01134 after 4 h treatment on WT MEFs and LRRK2^{G2019S} MEFs separately.
FIGURE 12C is a graph that shows the inhibition of phosphorylation of RablO at Thr73 in a dose dependent manner by XL01134 after 4 h treatment on WT MEFs and LRRK2^{G2019S} MEFs separately.
FIGURE 13A is an image of western blots that show the degradation of LRRK2 total, the inhibition of phosphorylation of LRRK2 at Ser935 and the inhibition of phosphorylation of RablO in a dose dependent manner by XL01126 after 4 h treatment on WT MEFs and LRRK2^{G2019S} MEFs.
FIGURE 13B is a graph that shows the degradation of LRRK2^{WT} total and LRRK2^{G2019S} total in a dose dependent manner by XL01126 after 4 h treatment on WT MEFs and LRRK2^{G2019S} MEFs separately.
FIGURE 13C is a graph that shows the inhibition of phosphorylation of RablO at Thr73 in a dose dependent manner by XL01126 after 4 h treatment on WT MEFs and LRRK2^{G2019S} MEFs separately.
FIGURE 14A is an image of western blots that show the degradation of LRRK2 total, the inhibition of phosphorylation of LRRK2 at Ser935 and the inhibition of phosphorylation of RablO in a time-dependent manner by SD75 after treating WT MEFs and LRRK2^{G2019S} MEFs with 1000 nM of SD75.
FIGURE 14B is a graph that shows the degradation of LRRK2^{WT} total and LRRK2^{G2019S} total in a time-dependent manner by SD75 after treating WT MEFs and LRRK2^{G2019S} MEFs with 1000 nM of SD75 separately.
FIGURE 14C is a graph that shows the inhibition of phosphorylation of RablO at Thr73 in a time-dependent manner by SD75 after treating WT MEFs and LRRK2^{G2019S} MEFs with 1000 nM SD75 separately.
FIGURE 15A is an image of western blots that show the degradation of LRRK2 total, the inhibition of phosphorylation of LRRK2 at Ser935 and the inhibition of phosphorylation of RablO in a time-dependent manner by XL01134 after treating WT MEFs and LRRK2^{G2019S} MEFs with 300 nM of XL01134.
FIGURE 15B is a graph that shows the degradation of LRRK2^{WT} total and LRRK2^{G2019S} total in a time-dependent manner by XL01134 after treating WT MEFs and LRRK2^{G2019S} MEFs with 300 nM of XL01134 separately.
FIGURE 15C is a graph that shows the inhibition of phosphorylation of RablO at Thr73 in a time-dependent manner by XL01134 after treating WT MEFs and LRRK2^{G2019S} MEFs with 300 nM XL01134 separately.
FIGURE 16A is an image of western blots that show the degradation of LRRK2 total, the inhibition of phosphorylation of LRRK2 at Ser935 and the inhibition of phosphorylation of RablO in a time-dependent manner by XL01126 after treating WT MEFs and LRRK2^{G2019S} MEFs with 300 nM of XL01126.
FIGURE 16B is a graph that shows the degradation of LRRK2^{WT} total and LRRK2^{G2019S} total in a time-dependent manner by XL01126 after treating WT MEFs and LRRK2^{G2019S} MEFs with 300 nM of XL011126 separately.
FIGURE 16C is a graph that shows the inhibition of phosphorylation of RablO at Thr73 in a time-dependent manner by XL01126 after treating WT MEFs and LRRK2^{G2019S} MEFs with 300 nM XL01126 separately.
FIGURE 17A is an image of a western blot showing the degradation of LRRK2^{WT,} and the dephosphorylation of LRRK2 and RablO in the presence of VHL Ligand (VH101), MLN4924, and MG132.
FIGURE 17B is a graph showing that XL01126 induced degradation of LRRK2^{WT} total can be rescued by VHL Ligand (VH101), MLN4924, and MG132 pretreatment.
FIGURE 17C is a graph showing that XL01126 induced dephosphorylation of RablO at Thr73 on WT MEFs can partially be rescued by VHL Ligand (VH101), MLN4924, and MG132 pretreatment.
FIGURE 17D is a graph showing that XL01126 induced LRRK2^{WT} dephosphorylation at Ser935 can partially be rescued by VHL Ligand (VH101), MLN4924, and MG132 pretreatment.
FIGURE 18A is an image of a western blot showing the degradation of LRRK2^{G20195S,} and the dephosphorylation of LRRK2^{G2019S} and RablO on LRRK2^{G2019S} MEFs in the presence of VHL Ligand (VH101), MLN4924, and MG132.
FIGURE 18B is a graph showing that XL01126 induced degradation of LRRK2^{G2019S} total can be rescued by VHL Ligand (VH101), MLN4924, and MG132 pretreatment.
FIGURE 18C is a graph showing that XL01126 induced dephosphorylation of RablO at Thr73 on LRRK2^{G2019S} MEFs can partially be rescued by VHL Ligand (VH101), MLN4924, and MG132 pretreatment.
FIGURE 18D is a graph showing that XL01126 induced LRRK2^{G2019S} dephosphorylation at Ser935 can partially be rescued by VHL Ligand (VH101), MLN4924, and MG132 pretreatment.
FIGURE 19A is an image of a western blot showing that XL01126 can degrade LRRK2^{R1441C} and inhibit the phosphorylation of LRRK2^{R1441C} at Ser935 and phosphorylation of RablO at Thr73 in a dose-dependent manner after 4h treatment on LRRK2^{R1441C} MEFs, while Cis-XL01126 can only inhibit the phosphorylation of LRRK2^{R1441C}at Ser935 and phosphorylation of RablO at Thr73 in a dose-dependent manner.
FIGURE 19B is a graph which shows that XL01126 can degrade LRRK2^{R1441C}in a dose-dependent manner after 4h treatment on LRRK2^{R1441C} MEFs, while Cis-XL01126 cannot degrade LRRK2^{R1441C}.
FIGURE 19C is a graph which shows that XL01126 is more potent than Cis-XL01126 in inhibiting the phosphorylation of RablO at Thr73.
FIGURE 20A is an image of a western blot which shows that XL01126 can degrade LRRK2 total in macrophages in a dose-dependent manner, while Cis-XL01126 cannot degrade LRRK2 in macrophages.
FIGURE 20B is a graph which shows that XL01126 can degrade LRRK2 total in macrophages in a dose-dependent manner, while Cis-XL01126 cannot degrade LRRK2 in macrophages.
FIGURE 21A is an image of a western blot showing that XL01126 degrades LRRK2 and inhibits the phosphorylation of LRRK2 at Ser935 and phosphorylation of RablO at Thr73 in a dose-dependent manner after 4h treatment on human peripheral blood mononuclear cell (PBMC), while Cis-XL01126 can only inhibit the phosphorylation of LRRK2at Ser935 and phosphorylation of RablO at Thr73 in a dose-dependent manner.
FIGURE 21B is a graph showing that XL01126 degrades LRRK2, in a dose-dependent manner after 4h treatment on human peripheral blood mononuclear cell (PBMC), while Cis-XL01126 cannot degrade LRRK2 at the concentrations tested.
FIGURE 21C is a graph showing that both XL01126 and Cis-XL01126 can inhibit the phosphorylation of RablO on human peripheral blood mononuclear cells (PBMC) in a dose dependent manner after 4h treatment, but XL01126 is more potent.
FIGURE 22A is an image of a western blot showing that XL01126 degrades LRRK2 and inhibits the phosphorylation of LRRK2 at Ser935 and phosphorylation of RablO at Thr73 in a dose-dependent manner after 24h treatment on human peripheral blood mononuclear cells (PBMC), while Cis-XL01126 can only inhibit the phosphorylation of LRRK2at Ser935 and phosphorylation of RablO at Thr73 in a dose-dependent manner.
FIGURE 22B is a graph showing that XL01126 degrade LRRK2, in a dose-dependent manner after 24h treatment on human peripheral blood mononuclear cell (PBMC), while Cis-XL01126 degraded little LRRK2 at the concentrations tested.
FIGURE 22C is a graph showing that both XL01126 and Cis-XL01126 can inhibit the phosphorylation of RablO on human peripheral blood mononuclear cells (PBMC) in a dose dependent manner after 24h treatment, but XL01126 is more potent.
FIGURE 23A is an image of a western blot showing that XL01126 degrades LRRK2 and inhibits the phosphorylation of LRRK2 at Ser935 and the phosphorylation of RablO at Thr73 in a time-dependent manner after treating human peripheral blood mononuclear cell (PBMC) with 300 nM XL01126. 300 nM Cis-XL01126 cannot degrade LRRK2 and barely inhibits the phosphorylation of LRRK2 at Ser935 and the phosphorylation of RablO at Thr73.
FIGURE 23B is a graph showing that XL01126 degrades LRRK2 and inhibits the phosphorylation of LRRK2 at Ser935 and phosphorylation of RablO at Thr73 in a time-dependent manner after treating human peripheral blood mononuclear cell (PBMC) with 300 nM XL01126.
FIGURE 23C is a graph showing that 300 nM Cis-XL01126 cannot degrade LRRK2 and barely inhibits the phosphorylation of LRRK2 at Ser935 and phosphorylation of RablO at Thr73.
FIGURE 24 is a graph of quantitative proteomic analysis that shows the intracellular individual protein level ratio in cells (WT MEFs) treated with XL01126 VS Cis-XL01126
FIGURE 25A is an image of western blots that show the degradation of LRRK2 total, the inhibition of phosphorylation of LRRK2 at Ser935 and the inhibition of phosphorylation of RablO in a dose dependent manner by XL02047 after 4 h treatment on WT MEFs and LRRK2^{G2019S} MEFs.
FIGURE 25B is a graph that shows the degradation of LRRK2^{WT} total and LRRK2^{G2019S} total in a dose dependent manner by XL02047 after 4 h treatment on WT MEFs and LRRK2^{G2019S} MEFs separately.
FIGURE 25C is a graph that shows the inhibition of phosphorylation of RablO at Thr73 in a dose dependent manner by XL02047 after 4 h treatment on WT MEFs and LRRK2^{G2019S} MEFs separately.
FIGURE 26A is an image of western blots that show the degradation of LRRK2 total, the inhibition of phosphorylation of LRRK2 at Ser935 and the inhibition of phosphorylation of RablO in a dose dependent manner by XL02048 after 4 h treatment on WT MEFs and LRRK2^{G2019S} MEFs.
FIGURE 26B is a graph that shows the degradation of LRRK2^{WT} total and LRRK2^{G2019S} total in a dose dependent manner by XL02048 after 4 h treatment on WT MEFs and LRRK2^{G2019S} MEFs separately.
FIGURE 26C is a graph that shows the inhibition of phosphorylation of RablO at Thr73 in a dose dependent manner by XL02048 after 4 h treatment on WT MEFs and LRRK2^{G2019S} MEFs separately.
FIGURE 27A is an image of western blots that show the degradation of LRRK2 total, the inhibition of phosphorylation of LRRK2 at Ser935 and the inhibition of phosphorylation of RablO in a dose dependent manner by XL02049 after 4 h treatment on WT MEFs and LRRK2^{G2019S} MEFs.
FIGURE 27B is a graph that shows the degradation of LRRK2^{WT} total and LRRK2^{G2019S} total in a dose dependent manner by XL02049 after 4 h treatment on WT MEFs and LRRK2^{G2019S} MEFs separately.
FIGURE 27C is a graph that shows the inhibition of phosphorylation of RablO at Thr73 in a dose dependent manner by XL02049 after 4 h treatment on WT MEFs and LRRK2^{G2019S} MEFs separately.
FIGURE 28A is an image of western blots that show the degradation of LRRK2 total, the inhibition of phosphorylation of LRRK2 at Ser935 and the inhibition of phosphorylation of RablO in a dose dependent manner by XL02017 after 4 h treatment on WT MEFs and LRRK2^{G2019S} MEFs.
FIGURE 28B is a graph that shows the degradation of LRRK2^{WT} total and LRRK2^{G2019S} total in a dose dependent manner by XL02017 after 4 h treatment on WT MEFs and LRRK2^{G2019S} MEFs separately.
FIGURE 28C is a graph that shows the inhibition of phosphorylation of RablO at Thr73 in a dose dependent manner by XL02017 after 4 h treatment on WT MEFs and LRRK2^{G2019S} MEFs separately.
FIGURE 29A is an image of western blots that show the degradation of LRRK2 total, the inhibition of phosphorylation of LRRK2 at Ser935 and the inhibition of phosphorylation of RablO in a dose dependent manner by XL02038 after 4 h treatment on WT MEFs and LRRK2^{G2019S} MEFs.
FIGURE 29B is a graph that shows the degradation of LRRK2^{WT} total and LRRK2^{G2019S} total in a dose dependent manner by XL02038 after 4 h treatment on WT MEFs and LRRK2^{G2019S} MEFs separately.
FIGURE 29C is a graph that shows the inhibition of phosphorylation of RablO at Thr73 in a dose dependent manner by XL02038 after 4 h treatment on WT MEFs and LRRK2^{G2019S} MEFs separately.
FIGURE 30A is an image of western blots that show the degradation of LRRK2 total, the inhibition of phosphorylation of LRRK2 at Ser935 and the inhibition of phosphorylation of RablO in a dose dependent manner by XL02068 after 4 h treatment on WT MEFs and LRRK2^{G2019S} MEFs.
FIGURE 30B is a graph that shows the degradation of LRRK2^{WT} total and LRRK2^{G2019S} total in a dose dependent manner by XL02068 after 4 h treatment on WT MEFs and LRRK2^{G2019S} MEFs separately.
FIGURE 30C is a graph that shows the inhibition of phosphorylation of RablO at Thr73 in a dose dependent manner by XL02068 after 4 h treatment on WT MEFs and LRRK2^{G2019S} MEFs separately.
FIGURE 31A is an image of western blots that show the degradation of LRRK2 total, the inhibition of phosphorylation of LRRK2 at Ser935 and the inhibition of phosphorylation of RablO in a dose dependent manner by XL02059 after 4 h treatment on WT MEFs and LRRK2^{G2019S} MEFs.
FIGURE 31B is a graph that shows the degradation of LRRK2^{WT} total and LRRK2^{G2019S} total in a dose dependent manner by XL02059 after 4 h treatment on WT MEFs and LRRK2^{G2019S} MEFs separately.
FIGURE 31C is a graph that shows the inhibition of phosphorylation of RablO at Thr73 in a dose dependent manner by XL02059 after 4 h treatment on WT MEFs and LRRK2^{G2019S} MEFs separately.
FIGURE 32A is an image of western blots that show the degradation of LRRK2 total, the inhibition of phosphorylation of LRRK2 at Ser935 and the inhibition of phosphorylation of RablO in a dose dependent manner by XL02060 after 4 h treatment on WT MEFs and LRRK2^{G2019S} MEFs.
FIGURE 32B is a graph that shows the degradation of LRRK2^{WT} total and LRRK2^{G2019S} total in a dose dependent manner by XL02060 after 4 h treatment on WT MEFs and LRRK2^{G2019S} MEFs separately.
FIGURE 32C is a graph that shows the inhibition of phosphorylation of RablO at Thr73 in a dose dependent manner by XL02060 after 4 h treatment on WT MEFs and LRRK2^{G2019S} MEFs separately.
FIGURE 33 is a graph which shows the plasma concentrations of XL01126 in mice after the mice were given XL01126 through intravenous (5 mg/Kg), intraperitoneal (30 mg/Kg), and orally (30 mg/Kg) separately.
FIGURE 34 is a graph that shows the brain concentrations of XL01126 in mice after the mice were given XL01126 through intravenous (5 mg/Kg), intraperitoneal (30 mg/Kg), and oral (30 mg/Kg) separately.
FIGURE 35 is a graph that shows the cerebrospinal fluid concentrations of XL01126 in mice after the mice were given XL01126 through intravenous (5 mg/Kg), intraperitoneal (30 mg/Kg), and oral (30 mg/Kg) separately.
FIGURE 36 is a graph showing the binary binding of VH101, XL01126, SD75, XL01134 and Cis-XL01126 to VHL as tested by fluorescence polarization assay.
FIGURE 37A and FIGURE 37B are graphs VHL engagement assay showing VH101, XL01126, SD75, and XL01134 can engage with VHL in the permeabilized and live HEK293 cells.
FIGURE 38A and FIGURE 38B are graphs of LRRK2 engagement assay showing HG-10-102-01, XL01126, SD75, and XL01134 can engage with LRRK2 in the permeabilized and live HEK293 cells.
FIGURE 39A, FIGURE 39B and FIGURE 39C are graphs of LRRK2 engagement assay of XL01126, SD75, and XL01134 in the presence and absence of VCB protein, using HG-10-102-01 as reference compound.
FIGURE 40 is a graph showing the ternary complex formation induced by Cis-XL01126, XL01126, SD75, and XL01134 with VHL and LRRK2.

### Detailed Description

The present invention is directed to identify a faster and more potent, and potentially orally bioavailable and/or blood brain barrier penetrant LRRK2 PROTAC degraders and qualify them as chemical probe to study LRRK2 biology, further validate the target as therapeutic concept in PD, and offer a potential treatment or prophylaxis of PD and related diseases.

Using HG-10-102-01 as the warhead and recruiting multiple different E3 ligase ligand, the inventors developed a first generation of LRRK2 PROTACs.

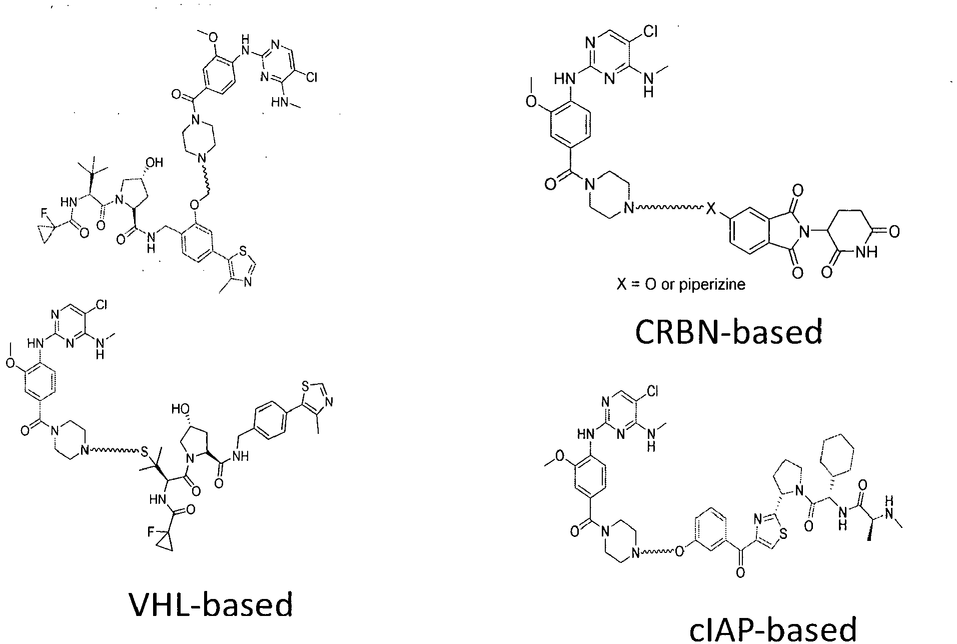

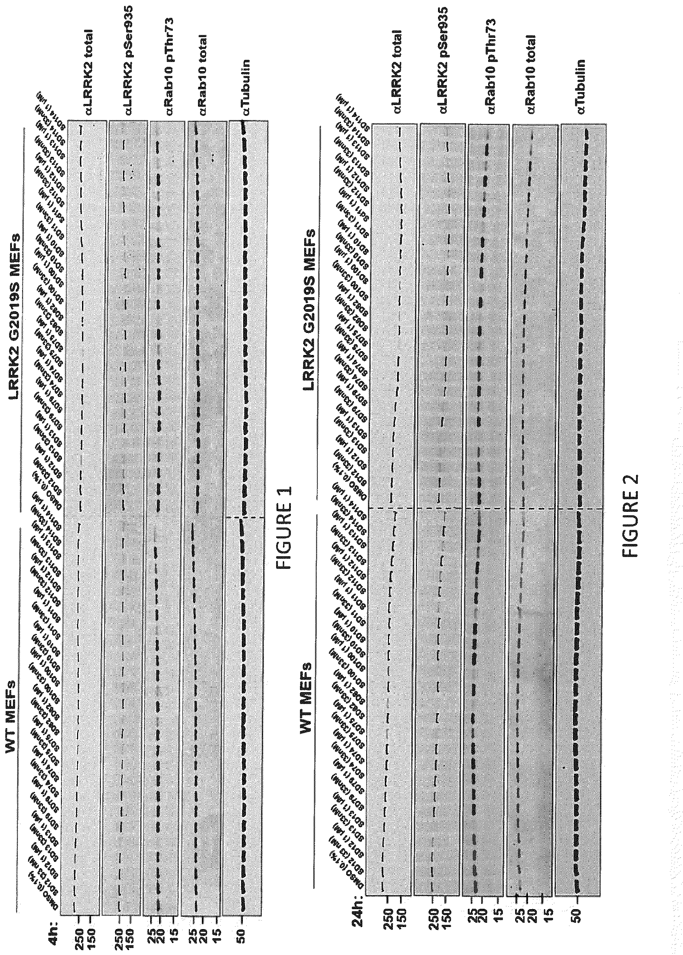

Among the first-generation compounds, SD75, SD82, and SD100 showed moderate LRRK2 degradation (FIGURE 1 and 2):

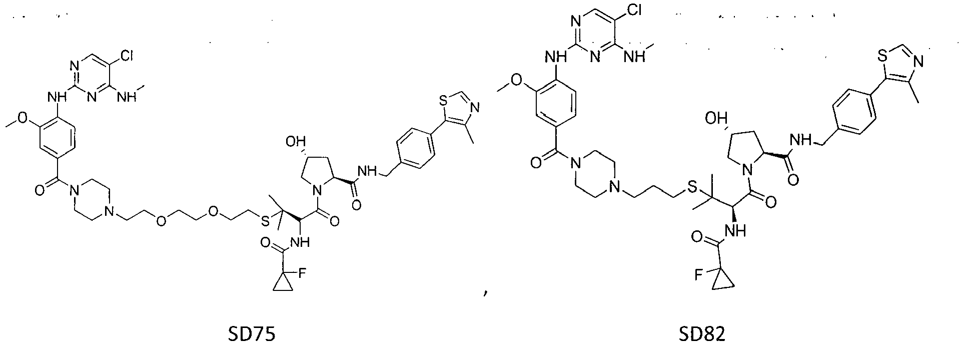

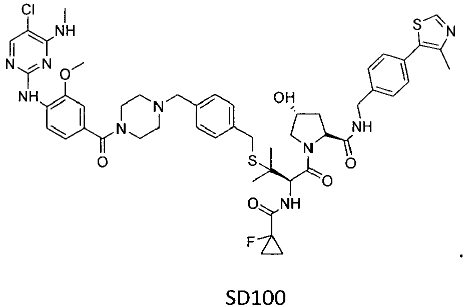

A second generation of LRRK2 PROTACs were then designed and synthesised based on the most potent first-generation LRRK2 PROTACs SD75, SD82, and SD100-set.

Two sets of second generation LRRK2 PROTACs were designed and synthesised:

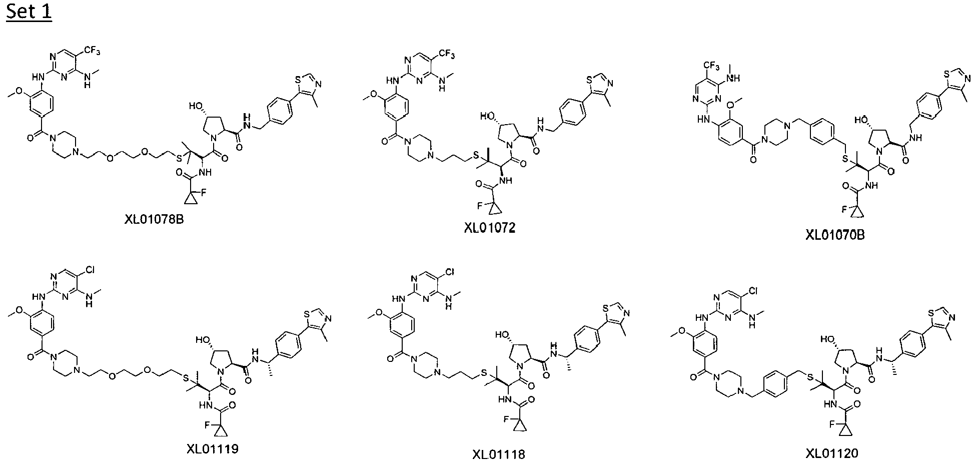

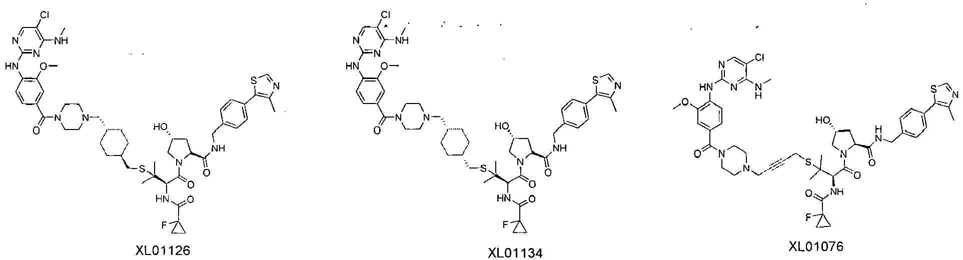

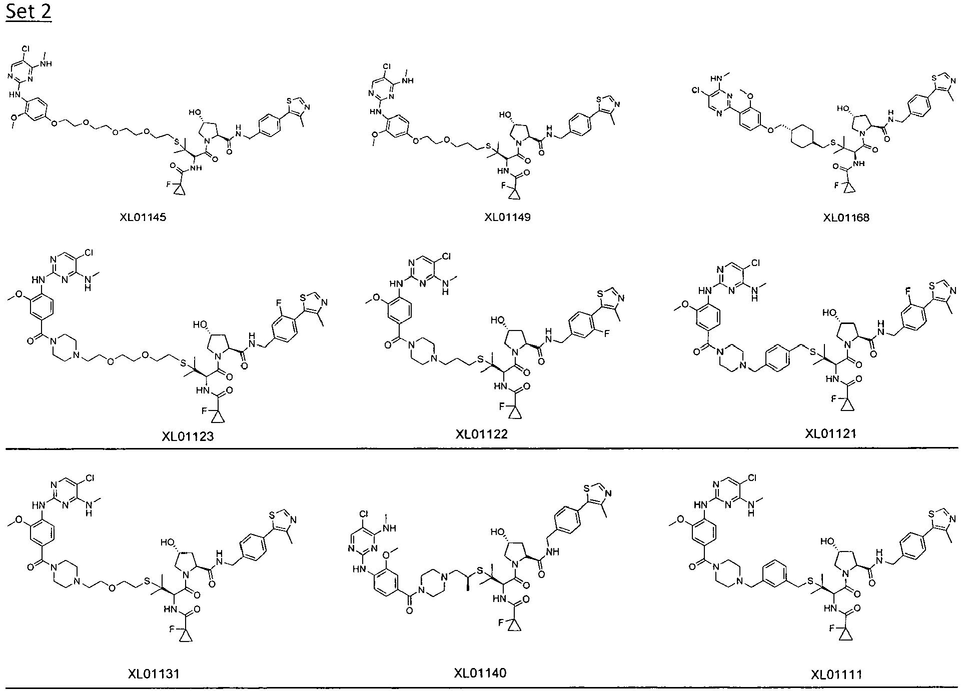

Western blot degradation assays for the LRRK2 protacs were performed with WT and PD-associated LRRK2 [G2019S] mouse embryonic fibroblasts (MEFs). The second-generation PROTACs were tested in parallel with the first generation PROTACs SD75, SD82, and SD100. The compounds were tested at 33 nM or 1 mM concentrations for 4 hours or 24 hours, running two biological replicates per condition and achieving quantifications representative of both replicates. Analysis by quantitative immunoblotting was performed with readouts of the total LRRK2 (antibody recognizes epitope in C-terminus of protein), RablO pThr73 (physiological substrate) and LRRK2 pSer935 (biomarker phosphorylation site).

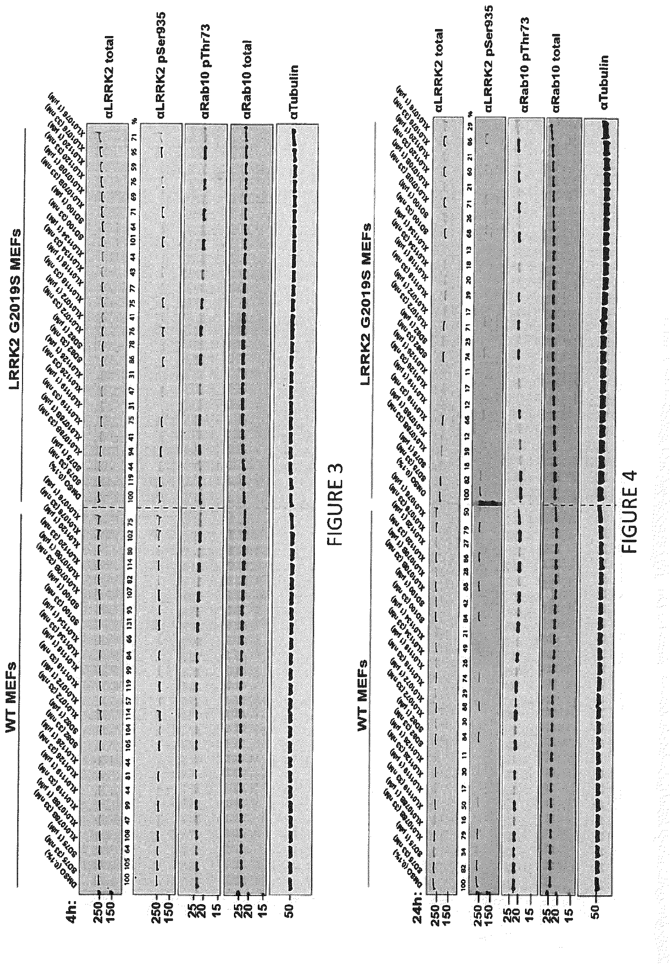

From the screening (FIGURE 3, 4, 5 and 6) of the second generation LRRK2 PROTACs, XL01126 and XL01134 were the top 2 compounds identified:

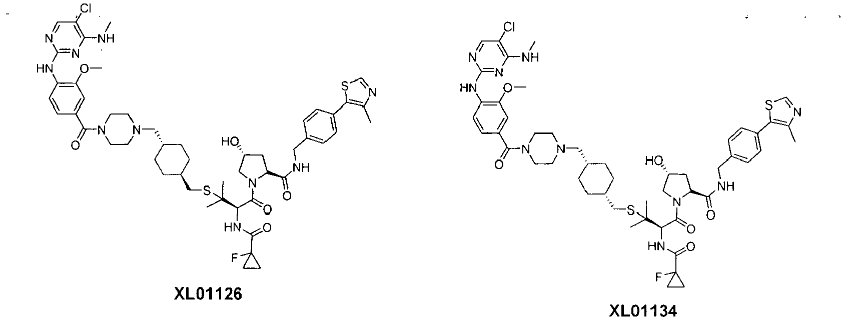

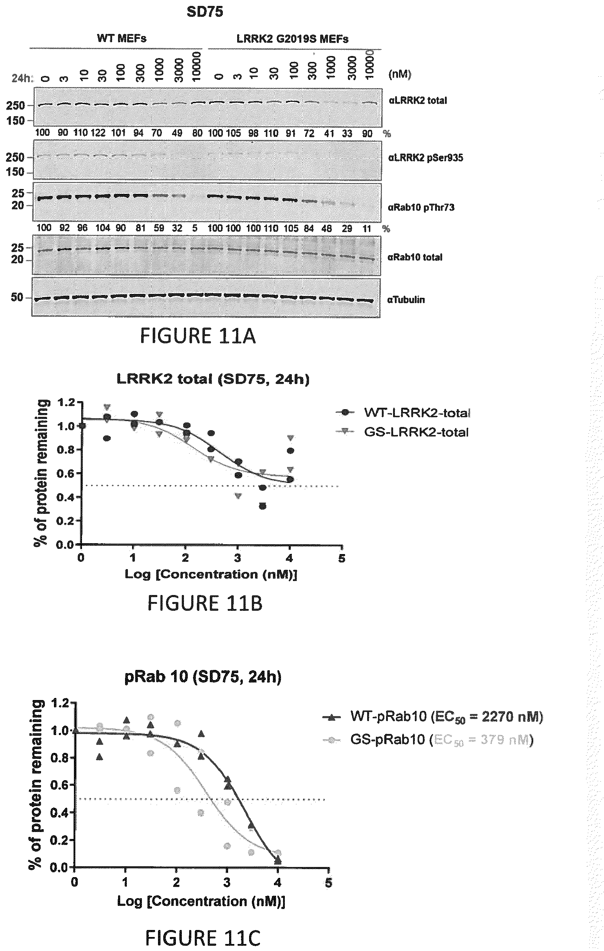

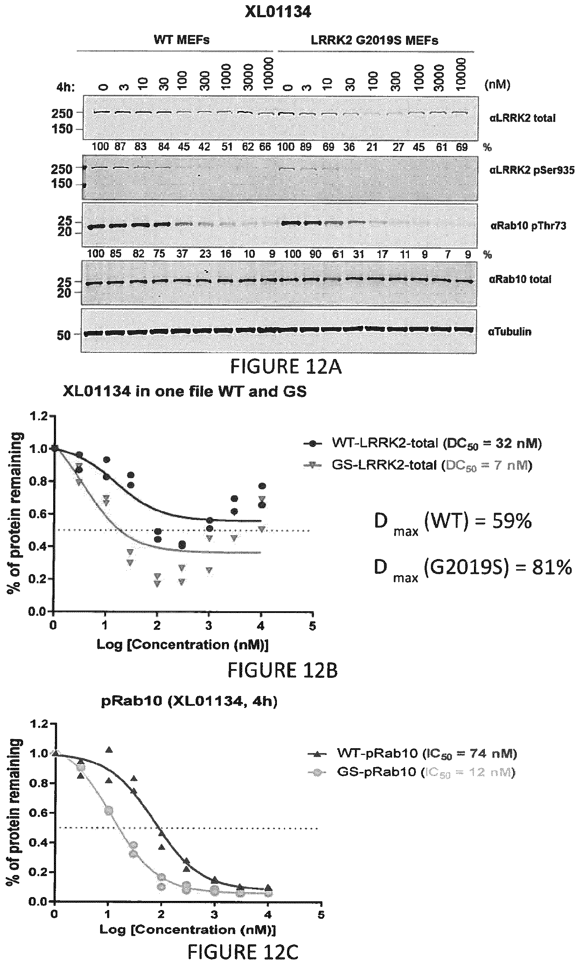

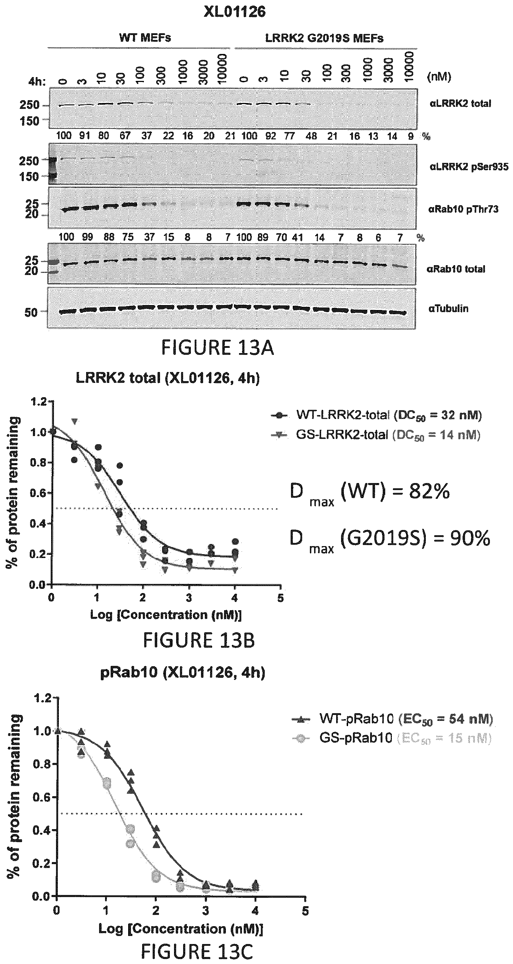

Therefore, a head-to-head comparison of SD75 vs XL01126 vs XL01134 in the dose-dependent assays showed that SD75 induced partial LRRK2 degradation (FIGUREs 11A-C), XL01134 induced LRRK2 degradation with "hook effect" (Douglass, et al., J Am Chem Soc. 2013; 135(16): 6092-6099.) (FIGUREs 12A-C), and XL01126 induced almost complete LRRK2 degradation (FIGUREs 13A-C).

**Table 1- Head-to-head degradation comparison of LRRK2 degraders XL01126 and XL01134 and SD75**

| | **XL01126 (4h)** | **XL01134 (4h)** | **SD75 (24 h)** |
|---|---|---|---|
| **WT MEFs LRRK2 DC₅₀ (nM)** | 32 | 32 | -- |
| **Dₘₐₓ on WT MEFs** | 82% | 58% | 50% |
| **G2019S MEFs LRRK2 DC₅₀(nM)** | 14 | 7 | -- |
| **Dₘₐₓ on G2019S MEFs** | 90% | 78% | 43% |
| **Selectivity index** | 2.3 | 4.6 | -- |

The results of Table 1 also show that XL01126 and XL01134 are more potent degraders than SD75.

**Table 2- Head-to-head comparison of pRablO dephosphorylation by XL01126, XL01134 and SD75**

| | **XL01126 (4h)** | **XL01134 (4h)** | **SD75 (24 h)** |
|---|---|---|---|
| **WT MEFs pRab10-dephosphrylation EC₅₀ (nM)** | 54 | 74 | 2270 |
| **G2019S MEFs-pRablO-dephosphorylation EC₅₀ (nM)** | 15 | 12 | 379 |
| **Selectivity Index** | 3.6 | 6.2 | 6.0 |

The results of Table 2 show that XL01126 and XL01134 are more potent than SD75 in dephosphorylating pRablO.

**Table 3- Head-to-head comparison of pSer935-LRRK2 dephosphorylation by XL01126, XL01134 and SD75**

| | **XL01126(4h)** | **XL01134 (4h)** | **SD75 (24 h)** |
|---|---|---|---|
| **WT MEFs pSer935 LRRK2-dephosphrylation EC₅₀ (nM)** | 18 | 35 | 745 |
| **G2019S MEFs-pSer935-dephosphorylation EC₅₀ (nM)** | 9 | 5 | 279 |
| **Selectivity Index** | 2 | 7 | 2.7 |

Table 3 shows that XL01126 and XL01134 are more potent than SD75 in dephosphorylating pSer935-LRRK2.

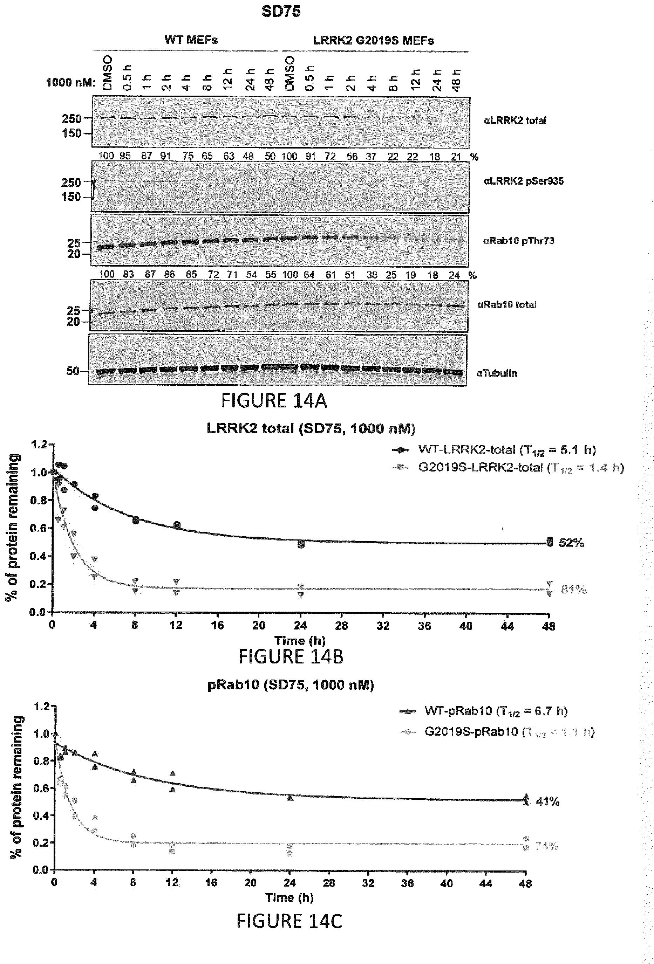

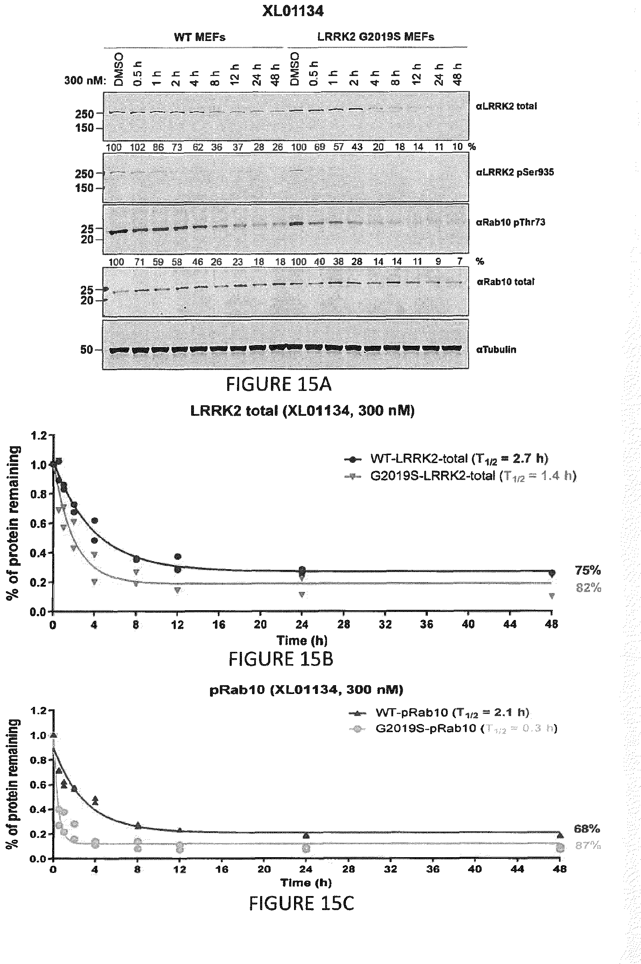

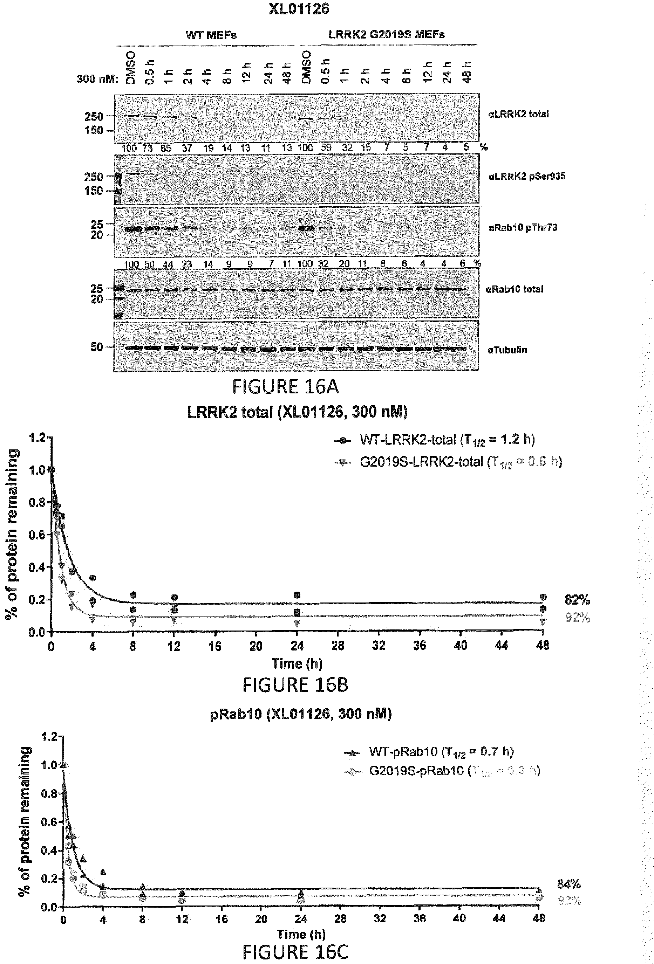

A side-by-side comparison of SD75, XL01134 and XL01126 in the time-dependent assay showed SD75 (FIGURE 14A-C), XL01134 (FIGURE 15A-C) and XL01126 (FIGURE 16A-C) all can induce time-dependent degradation of LRRK2, and time-dependent dephosphorylation of pRablO and LRRK2.

**Table 4- Summary of the dose response and time course experiments**

| | **T_{1/2} (h)** | | | **EC₅₀/DC₅₀ (nM)** | | |
|---|---|---|---|---|---|---|
| | **SD75** | **XL01134** | **XL01126** | **SD75** | **XL01134** | **XL01126** |
| **WT-LRRK2** | 5.1 | 2.7 | 1.2 | -- | 32 | 32 |
| **WT-pSer935-LRRK2** | 3.6 | 0.7 | 0.6 | 745 | 35 | 18 |
| **WT-pRab10** | 6.7 | 2.1 | 0.7 | 2270 | 74 | 54 |
| **GS-LRRK2** | 1.1 | 1.4 | 0.6 | | 7 | 14 |
| **GS-pSer935-LRRK2** | 1.4 | 0.3 | 0.3 | 279 | 5 | 9 |
| **GS-pRab10** | 0.7 | 0.3 | 0.3 | 379 | 12 | 15 |

The results show that
1) XL01126 is the most potent LRRK2 degrader and induced the fastest LRRK2 degradation among SD75 and XL01134.
2) XL01126 induced the most potent and fastest LRRK2 dephosphorylation and pRablO dephosphorylation comparing to SD75 and XL01134.

Further screening of compounds based on XL01126 was then performed and Cis-XL01126 was synthesized as a non-degrading control to dissect inhibition from degradation. Cis-XL01126 does not bind to VHL E3 ligase.

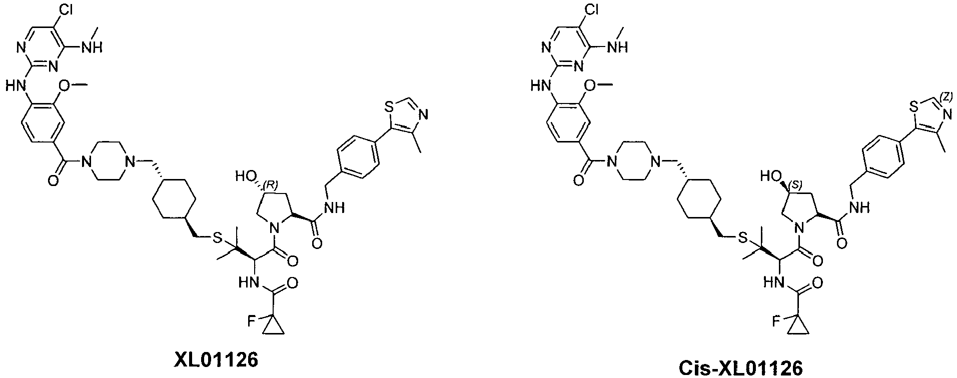

**Table 5 - binding affinity of VH101, Cis-XL01126, and XL01126 tested by fluorescence polarization assay**

| | **VH101** | **Cis-XL01126** | **XL01126** |
|---|---|---|---|
| **K, (µM)** | 0.116 - 0.350 | > 50 µM | 0.908 - 5.95 |

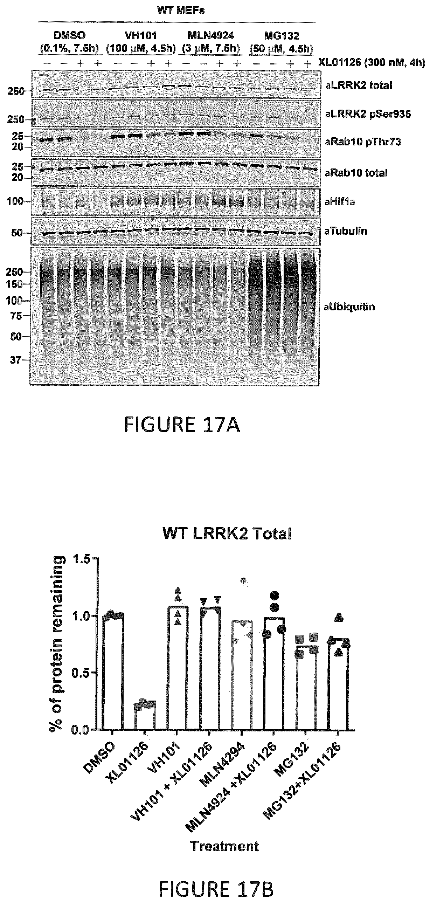

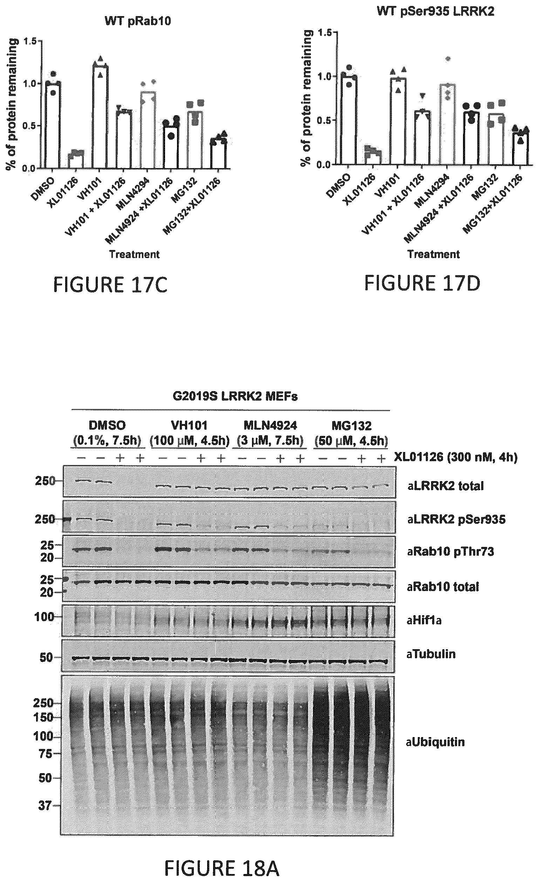

XL01126 induced LRRK2 degradation is dependent on the proteasome pathway (FIGURE 17A-D and FIGURE 18A-D).

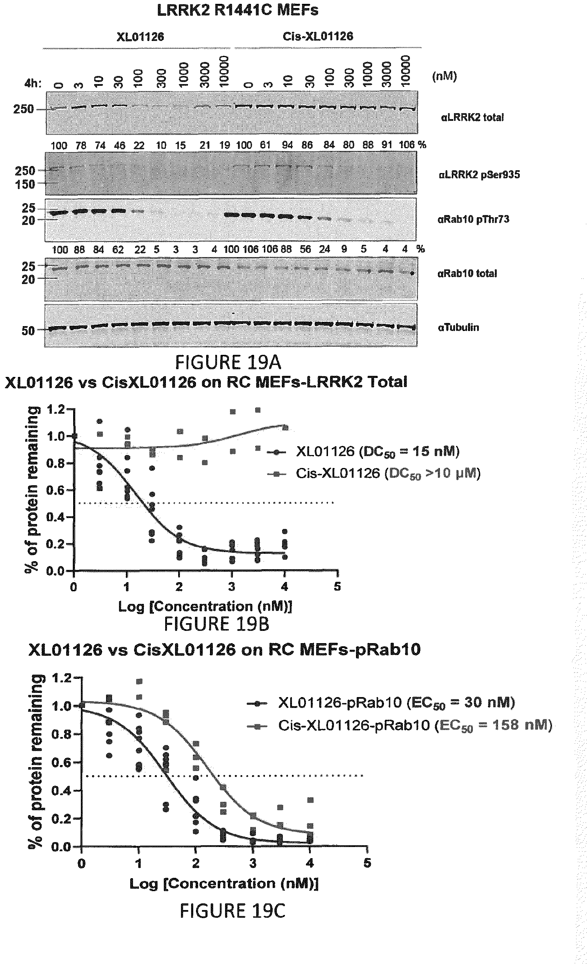

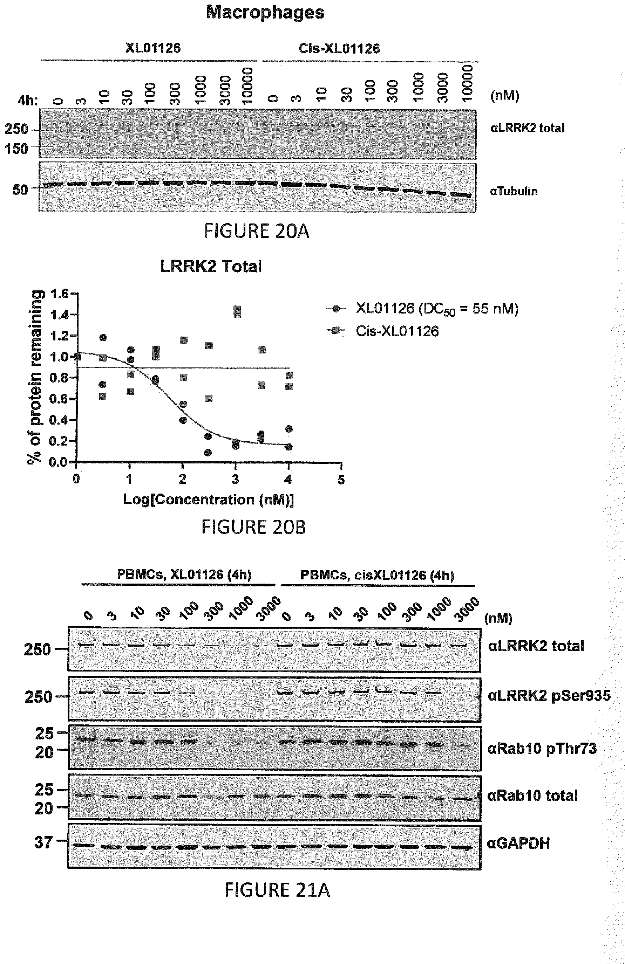

XL01126 degraded LRRK2 in LRRK2^{R1441C} MEFs (FIGURE 19A-C) and macrophage cells (FIGURE 20A-B) dose-dependently and also degraded LRRK2 in human PBMC cells dose- and time- dependently (FIGURE 21A-C, FIGURE 22A-C, FIGURE 23A-C).

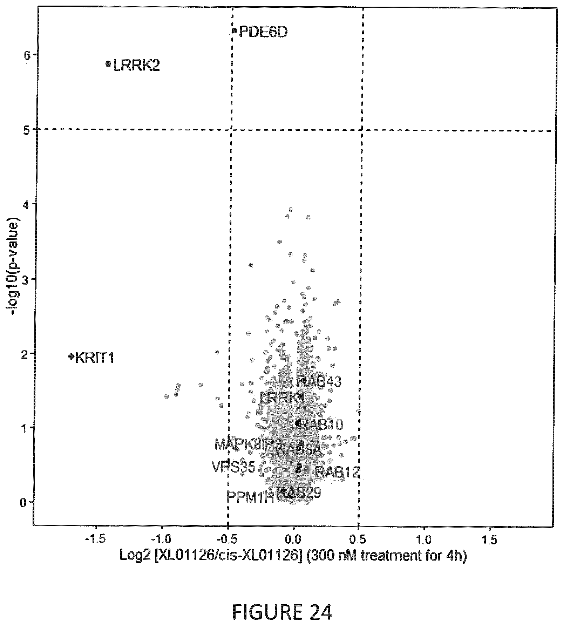

XL01126 induced selective LRRK2 degradation in WT MEFs as shown by the TMT-labelling proteomic study (FIGURE 24).

Table 6 shows that the solubilities of XL01126 in PBS and Fed State Simulated Intestinal Fluid (FeSSIP) are 0.55 µM and 26.05 µM separately. XL01126 can penetrant Caco-2 mono-cell layer. XL01126 is stable in mouse plasma and mouse hepatocytes with half-lives at 108.29 min and 314.33 min, respectively. XL01126 can be metabolized in mouse liver microsome with half-life at 3.65 min.

**Table 6. Physicochemical and in vitro ADME properties of XL01126**

| | **XL01126** |
|---|---|
| **Solubility in PBS (pH 7.4)** | 0.55 µM |
| **Solubility in FeSSIF (pH5.8)** | 26.05 µM |
| **Caco-2 Permeability** | A-B <0.74 × 10⁻⁶cm•s⁻¹ |
| | B-A < 1.43 × 10⁻⁶cm•s⁻¹ |
| **T_{1/2} in mouse plasma** | 108.29 min |
| **T_{1/2} in mouse liver microsome** | 3.65 min |
| **T_{1/2} in mouse hepatocytes** | 314.33 min |

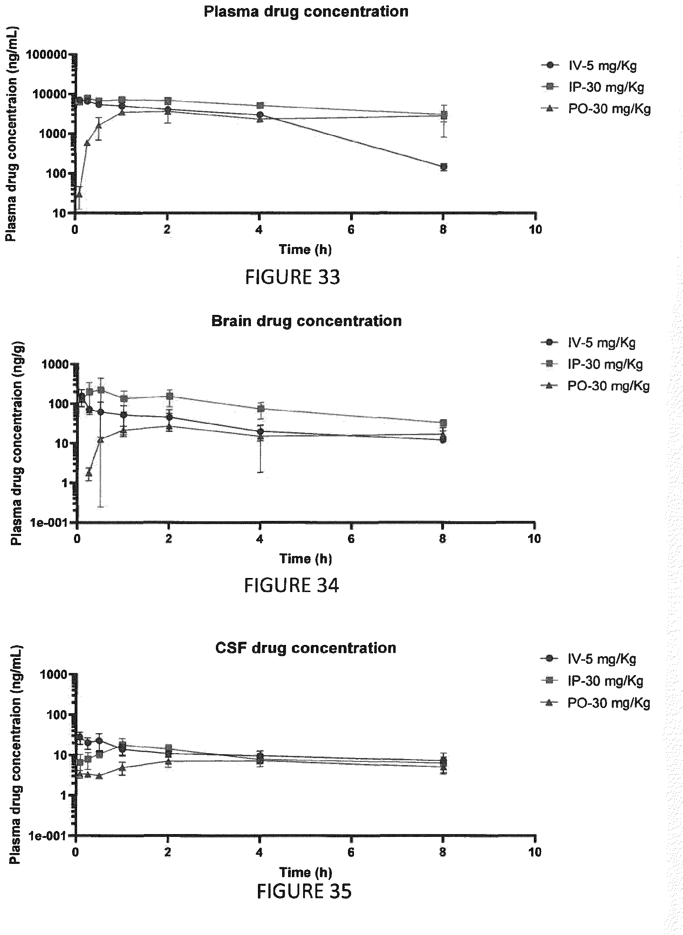

XL01126 exhibited striking in vivo pharmacokinetic profiles with 15% oral bioavailability and is capable of penetrating blood brain barrier (FIGURE 33, 34 and 35).

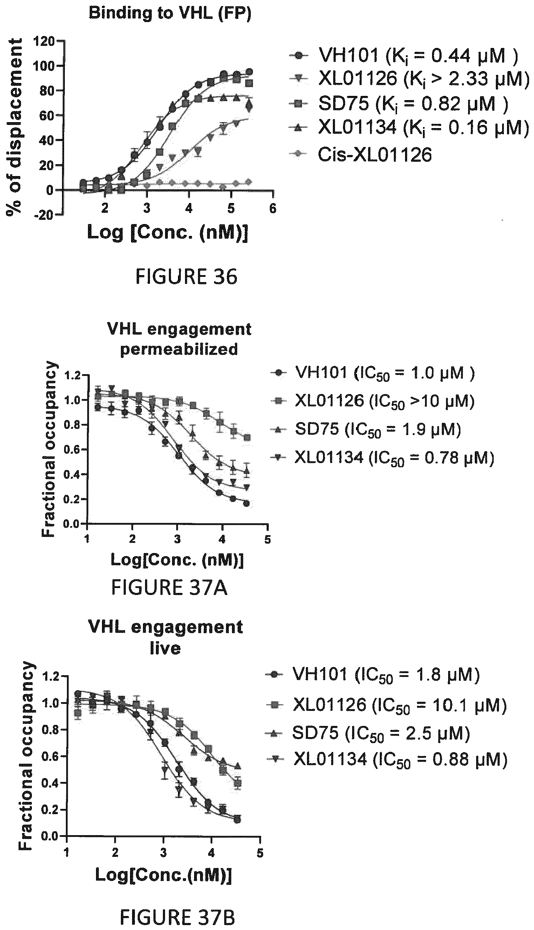

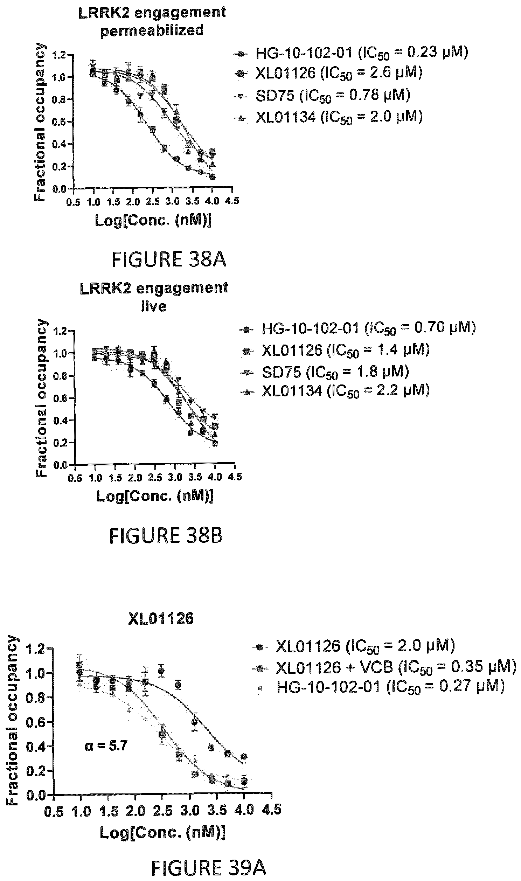

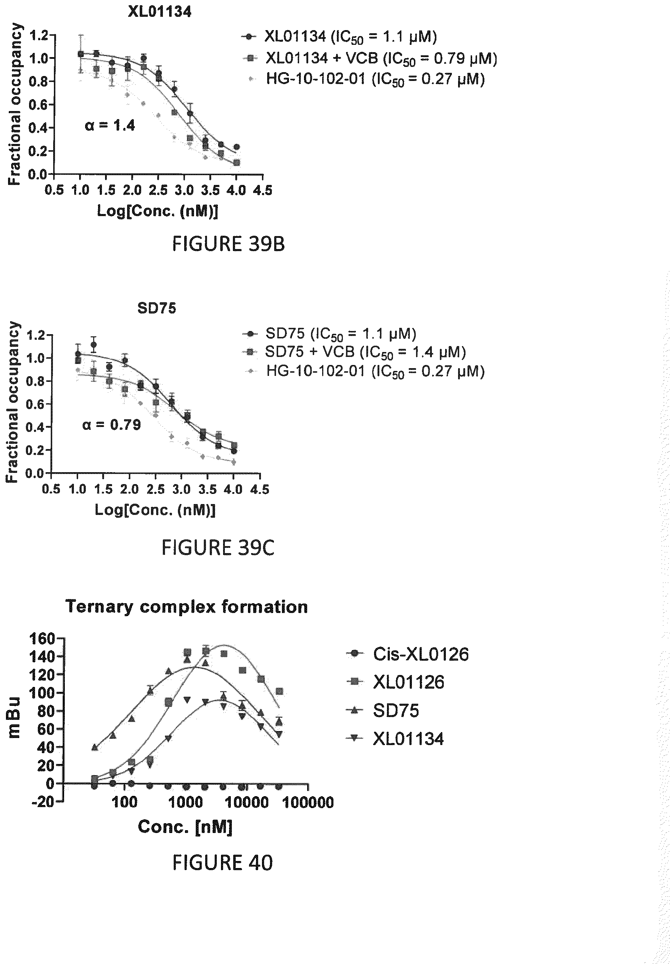

Although XL01126 has poor binary binding affinities to VHL (FIGURE 36, 37A, and 37B) and LRRK2 (FIGURE 38A and 38B), and is worse than XL01134 and SD75 at binding to VHL and LRRK2 in the permeabilized mode of VHL and LRRK2 target engagement assay, XL01126 is the most permeable among the testing PROTACs as evidenced by its highest IC50 ratio between permeabilized and live mode target engagement assay. XL01126 also forms higher and more cooperative ternary complex with VHL and LRRK2 than XL01134 and SD75 (FIGURE 39).

Given the favourable degradation profiles of XL01126 and XL01134, and excellent in vivo PK profiles of XL01126, further modification (the third generation) of LRRK2 PROTACs is based on XL01126 and XL01134:
XL02031, XL02038, and XL02017 are based on XL01126 with modifications on the piperidine moiety:

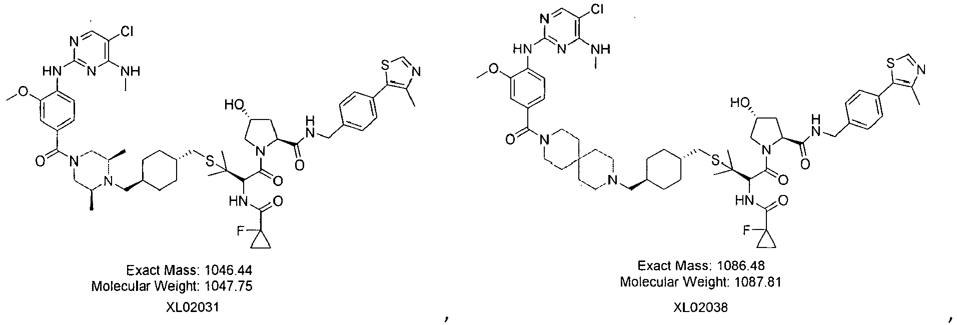

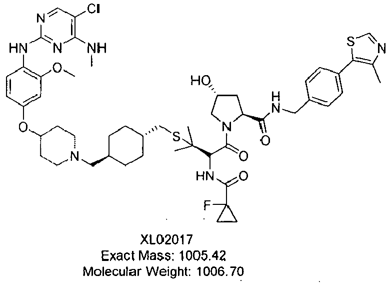

XL02058, XL02059, XL02060 and XL02063 are based on XL01134, with modifications on VHL ligand and linker:

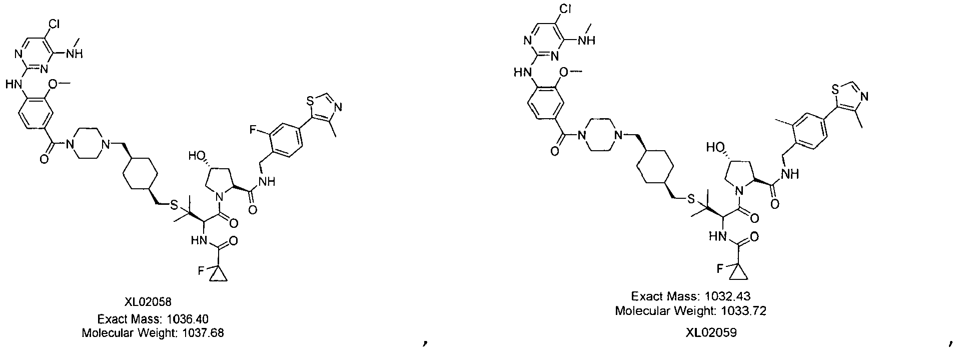

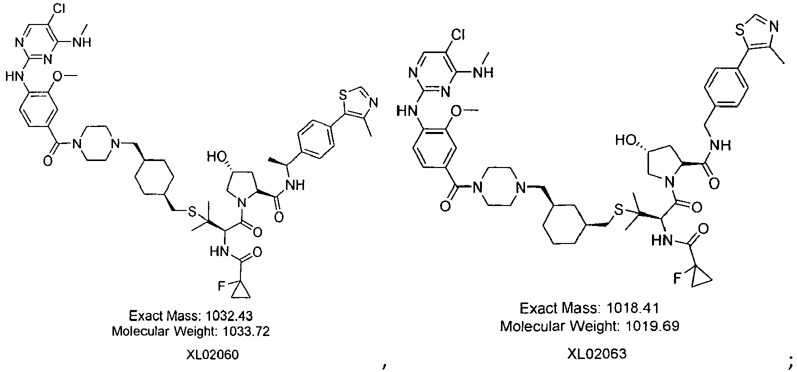

XL02047, XL02048, and XL02049 are based on XL01126 with modifications on VHL ligand; XL02067B, XL02068, and XL02077 have modifications in the linker:

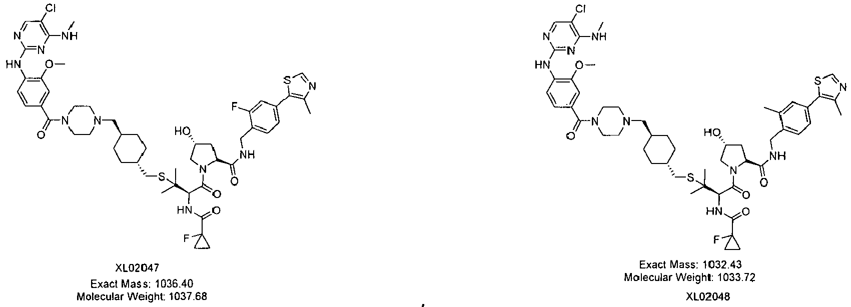

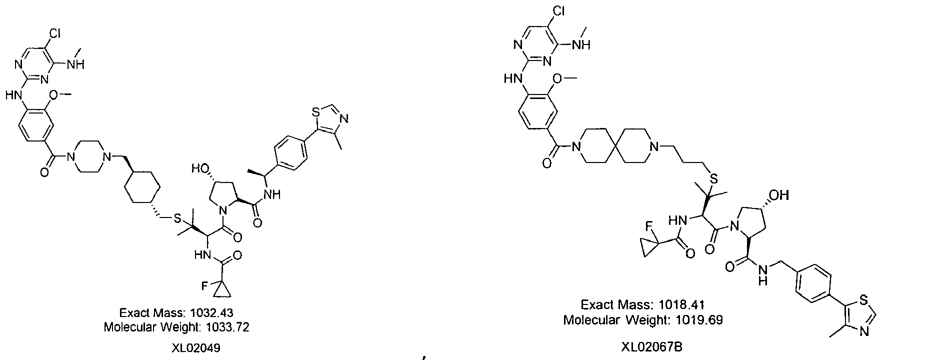

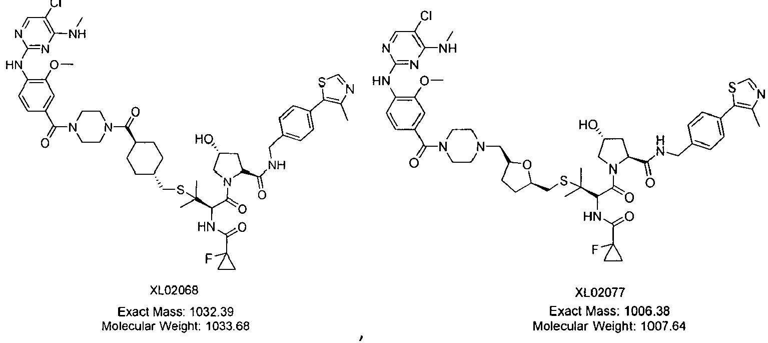

Most of the compounds from the third generation showed comparable degradation potency over XL01126 and XL01134.

### Results and Discussion

FIGURE 1 shows that after 33 nM/4h treatment, SD12, SD13, SD79, SD75, SD82, SD100, SD10, SD11, SD112, SD113, SD114 degraded 0-25% of LRRK2^{WT} and 0-60% of LRRK2^{G2019S}, and inhibited 0-25% of pRablO in WT MEFs and 0-60% of pRablO in LRRK2^{G2019S} MEFs (FIGURE1). After 1 µM/4h treatment, SD12, SD13, SD79, SD75, SD82, SD100, SD10, SD11, SD112, SD113, SD114 degraded 0-45% of LRRK2^{WT} and 0-75% of LRRK2^{G2019S} , and inhibited 18-90% of pRablO in WT MEFs and 30-95% of pRablO in LRRK2^{G2019S} MEFs (FIGURE 1).

As seen in FIGURE 2, after 33 nM/24h treatment, SD12, SD13, SD79, SD75, SD82, SD100, SD10, SD11, SD112, SD113, SD114 degraded 0-40% of LRRK2^{WT} and 0-70% of LRRK2^{G2019S} and inhibited 10-40% of pRablO in WT MEFs and 0-70% of pRablO in LRRK2^{G2019S} MEFs (FIGURE 2). After 1 µM/24h treatment, SD12, SD13, SD79, SD75, SD82, SD100, SD10, SD11, SD112, SD113, SD114 degraded 0-60% of LRRK2^{WT} and 0-85% of LRRK2^{G20195} , and inhibited 30-90% of pRablO in WT MEFs and 30-100% of pRablO in LRRK2^{G2019S} MEFs (FIGURE 2).

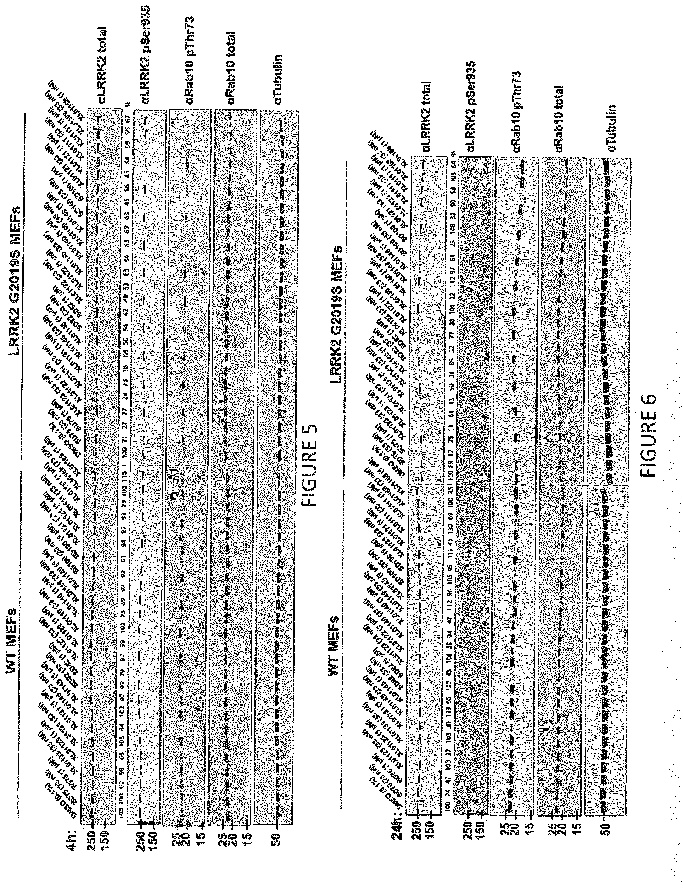

FIGURE 3 and FIGURE 5 show that after 33 nM/4h treatment, SD75, XL01078B, XL01119, XL01126, SD82, XL01072, XL01118, XL01134, SD100, XL01070B, XL01120, XL01076, XL01123, XL01131, XL01145, XL01122, XL01140, XL01149, XL01121, XL01111, XL01168 degraded 0-25% of LRRK2^{WT} and 0-60% of LRRK2^{G2019S}, and inhibited 0-40% of pRablO in WT MEFs and 0-70% of pRablO in LRRK2^{G2019S} MEFs (FIGURE 3 and FIGURE 5). After 1 µM/4h treatment, SD75, XL01078B, XL01119, XL01126, SD82, XL01072, XL01118, XL01134, SD100, XL01070B, XL01120, XL01076, XL01123, XL01131, XL01145, XL01122, XL01140, XL01149, XL01121, XL01111, XL01168 degraded 0-60% of LRRK2^{WT} and 10-80% of LRRK2^{G2019S}, and inhibited 10-95% of pRablO in WT MEFs and 20-100% of pRablO in LRRK2^{G2019S} MEFs (FIGURE3 andFIGURE5).

FIGURE 4 and FIGURE 6 show that after 33 nM/24h treatment, SD75, XL01078B, XL01119, XL01126, SD82, XL01072, XL01118, XL01134, SD100, XL01070B, XL01120, XL01076, XL01123, XL01131, XL01145, XL01122, XL01140, XL01149, XL01121, XL01111, XL01168 degraded 0-65% of LRRK2^{WT} and 0-80% of LRRK2^{G2019S}, and inhibited 0-60% of pRablO in WT MEFs and 0-90% of pRablO in LRRK2^{G2019S} MEFs (FIGURE 4 and FIGURE 6). After 1 µM/24h treatment, SD75, XL01078B, XL01119, XL01126, SD82, XL01072, XL01118, XL01134, SD100, XL01070B, XL01120, XL01076, XL01123, XL01131, XL01145, XL01122, XL01140, XL01149, XL01121, XL01111, XL01168 degraded 10-90% of LRRK2^{WT} and 20-90% of LRRK2^{G2019S} and inhibited 30-100% of pRablO in WT MEFs and 10-100% of pRablO in LRRK2^{G2019S} MEFs (FIGURE4 and FIGURE6).

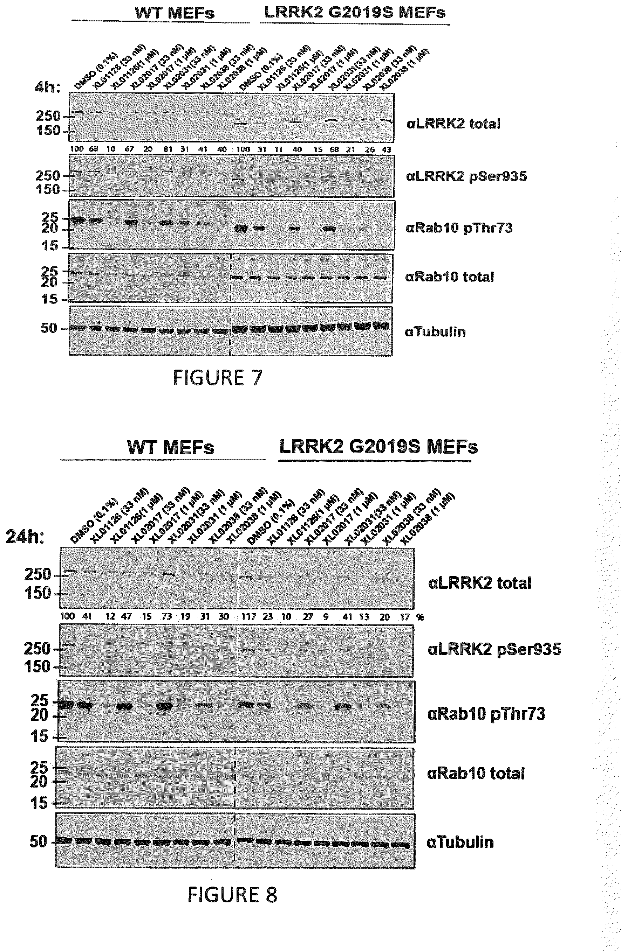

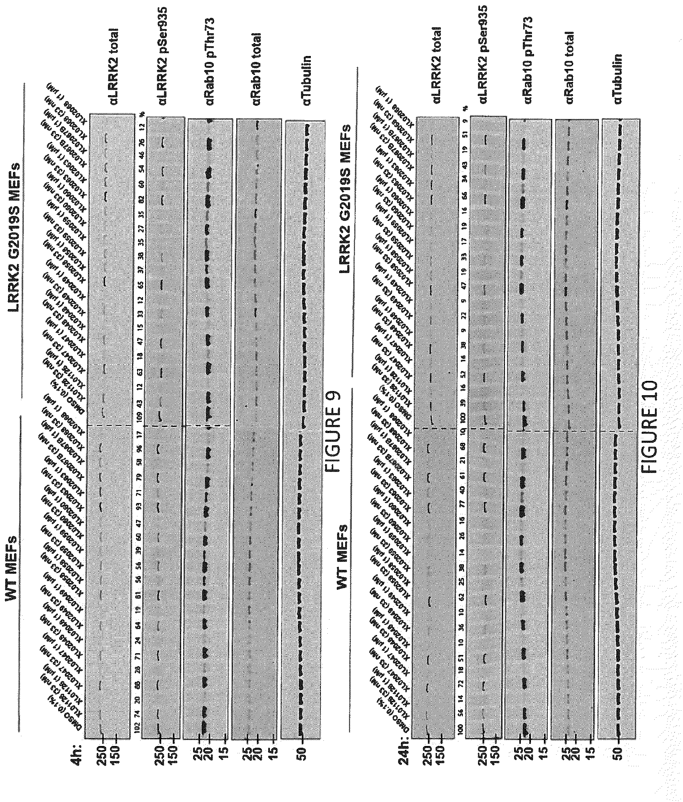

FIGURE 7 and FIGURE 9 show that after 33 nM/4h treatment, XL01126, XL02017, XL02031, XL02038, XL02047, XL02048, XL02049, XL02058, XL02059, XL02060, XL02063, XL02067B, XL02068 degraded 0-60% of LRRK2^{WT} and 0-70% of LRRK2^{G2019S}, and inhibited 0-70% of pRablO in WT MEFs and 0-90% of pRablO in LRRK2^{G2019S} MEFs (FIGURE 7 and FIGURE 9). After 1 µM/4h treatment, XL01126, XL02017, XL02031, XL02038, XL02047, XL02048, XL02049, XL02058, XL02059, XL02060, XL02063, XL02067B, XL02068 degraded 0-90% of LRRK2^{WT} and 40-90% of LRRK2^{G2019S}, and inhibited 80-100% of pRablO in WT MEFs and 10-100% of pRablO in LRRK2^{G2019S} MEFs (FIGURE 7 andFIGURE 9).

FIGURE 8 and FIGURE 10 show that after 33 nM/24h treatment, XL01126, XL02017, XL02031, XL02038, XL02047, XL02048, XL02049, XL02058, XL02059, XL02060, XL02063, XL02067B, XL02068 degraded 0-80% of LRRK2^{WT} and 20-90% of LRRK2^{G2019S}, and inhibited 0-80% of pRablO in WT MEFs and 30-90% of pRablO in LRRK2^{G2019S} MEFs (FIGURE 8 and FIGURE 10). After 1 µM/24h treatment, XL01126, XL02017, XL02031, XL02038, XL02047, XL02048, XL02049, XL02058, XL02059, XL02060, XL02063, XL02067B, XL02068 degraded 60-90% of LRRK2^{WT} and 60-95% of LRRK2^{G2019S}, and inhibited 80-100% of pRablO in WT MEFs and 80-100% of pRablO in LRRK2^{G2019S} MEFs (FIGURE 8 and FIGURE 10).

FIGURES 11A, 11B, 11C show that SD75 induced dose-dependent degradation of LRRK2^{WT} and LRRK2^{G2019S} in WT MEFs and LRRK2^{G2019S} MEFs separately, with D ₘₐₓ (WT) = 51% and D ₘₐₓ (G2019S) = 58% (FIGURE 11A and FIGURE 11B). SD75 induced dose-dependent dephosphorylation of RablO on WT MEFs and LRRK2^{G2019S} MEFs separately, with EC₅₀ (WT) = 2270 nM and EC₅₀ (G2019S) = 379 nM (FIGURE 11A and FIGURE 11C).

FIGURE 12A, 12B, 12C show that XL01134 induced dose-dependent degradation of LRRK2^{WT} and LRRK2^{G2019S}in WT MEFs and LRRK2^{G2019S} MEFs separately, with D ₘₐₓ (WT) = 59% DC₅₀ (WT) = 32 nM, D ₘₐₓ (G2019S) = 81% and DC₅₀ (G2019S) = 7 nM (FIGURE 12A and FIGURE 12B). XL01134 induced dose-dependent dephosphorylation of RablO on WT MEFs and LRRK2^{G2019S} MEFs separately, with EC₅₀ (WT) = 74 nM and EC₅₀ (G2019S) = 12 nM (FIGURE 12A and FIGURE 12C).

From FIGURES 13A, 13B, 13C it is seen that XL01126 induced dose-dependent degradation of LRRK2^{WT} and LRRK2^{G2019S} in WT MEFs and LRRK2^{G2019S} MEFs separately, with D ₘₐₓ (WT) = 82%, DC₅₀ (WT) = 32 nM, D ₘₐₓ (G2019S) = 90% and DC₅₀ (G2019S) = 14 nM (FIGURE 13A and FIGURE 13B). XL01126 induced dose-dependent dephosphorylation of RablO on WT MEFs and LRRK2^{G2019S} MEFs separately, with EC₅₀ (WT) = 54 nM and EC₅₀ (G2019S) = 15 nM ( FIGURE 13A and FIGURE 13C).

FIGURES 14A, 14B, 14C show that at 1000 nM, SD75 induced time-dependent degradation of LRRK2^{WT} and LRRK2^{G2019S} in WT MEFs and LRRK2^{G2019S} MEFs separately, with T_{1/2} (WT)= 5.1 h, Dₘₐₓ(WT) = 52%, T_{1/2} (G2019S) = 1.4 h, and Dₘₐₓ (G2019S) = 81% ( FIGURE 14A and FIGURE 14B). At 1000 nM, SD75 induced time-dependent dephosphorylation of RablO in WT MEFs and LRRK2^{G2019S} MEFs separately, with T_{1/2} (WT)= 6.7 h, Dₘₐₓ (WT) = 41%, T_{1/2} (G2019S) = 1.1 h, and Dₘₐₓ (G2019S) = 74% ( FIGURE 14A and FIGURE 14C).

As seen in FIGURES 15A, 15B, 15C, at 300 nM, XL01134 induced time-dependent degradation of LRRK2^{WT} and LRRK2^{G2019S} in WT MEFs and LRRK2^{G2019S} MEFs separately, with T_{1/2} (WT)= 2.7 h, Dₘₐₓ(WT) = 75 %, T_{1/2} (G2019S) = 1.4 h, and Dₘₐₓ (G2019S) = 82% (FIGURE 15A and FIGURE 15B). At 300 nM, XL01134 induced time-dependent dephosphorylation of RablO in WT MEFs and LRRK2^{G2019S} MEFs separately, with T_{1/2} (WT)= 2.1 h, Dₘₐₓ(WT) = 68%, T_{1/2} (G2019S) = 0.3 h, and Dₘₐₓ(G2019S) = 87% (FIGURE 15A and FIGURE 15C).

FIGURES 16A, 16B, 16C show that, at 300 nM, XL01126 induced time-dependent degradation of LRRK2^{WT} and LRRK2^{G2019S}in WT MEFs and LRRK2^{G2019S} MEFs separately, with T_{1/2} (WT)= 1.2 h, Dₘₐₓ(WT) = 82 %, T_{1/2} (G2019S) = 0.6 h, and Dₘₐₓ (G2019S) = 92% (FIGURE 16A and FIGURE 16B). At 300 nM, XL01126 induced time-dependent dephosphorylation of RablO in WT MEFs and LRRK2^{G2019S} MEFs separately, with T_{1/2} (WT)= 2.1 h, Dₘₐₓ(WT) = 84%, T_{1/2} (G2019S) = 0.3 h, and Dₘₐₓ(G2019S) = 92% (FIGURE 16A and FIGURE 16C).

FIGURES 17A, FIGURE 17B, FIGURE 17C, and FIG 17D show that the XL01126 induced degradation of LRRK2^{WT} total can be rescued by VHL Ligand (VH101), MLN4924, and MG132 pre-treatment. The inhibition of LRRK2^{WT} phosphorylation at Ser935 and the inhibition of phosphorylation of RablO at Thr73 can partially be rescued by VHL Ligand (VH101), MLN4924, and MG132 pre-treatment (FIGURE 17A, FIGURE 17B, FIGURE 17C, and FIGURE 17D).

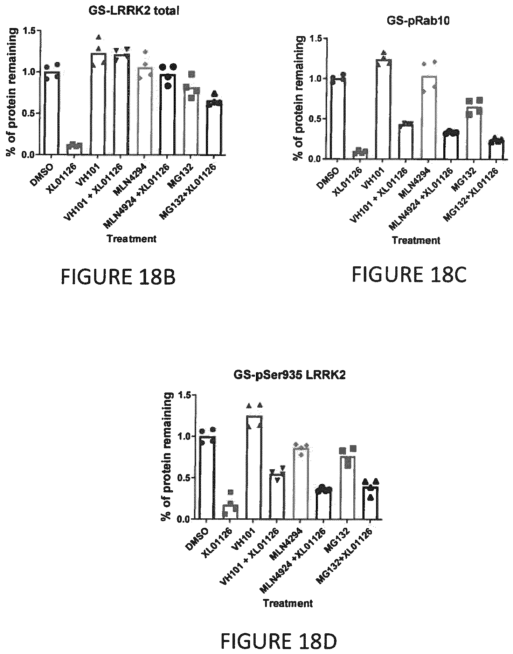

FIGURES 18A, FIGURE 18B, FIGURE 18C and FIGURE 18D demonstrate that XL01126 induced degradation of LRRK2^{G2019S} total can be rescued by VHL Ligand (VH101), MLN4924, and MG132 pre-treatment. The inhibition of LRRK2^{G2019S} phosphorylation at Ser935 and the inhibition of phosphorylation of RablO at Thr73 can partially be rescued by VHL Ligand (VH101), MLN4924, and MG132 pre-treatment (FIGURE 18A, FIGURE 18B, FIGURE 18C and FIGURE 18D).

FIGURES 19A, 19B, 19C show that XL01126 induced dose-dependent degradation of LRRK2^{R1441C} with DC₅₀ = 15 nM and Dₘₐₓ = 89% (FIGURE 19A and FIGURE 19B). XL01126 induced dose-dependent dephosphorylation of RablO in LRRK2^{R1441C} MEFs with EC₅₀ = 30 nM (FIGURE 19A and FIGURE 19C). CisXL01126 induced dose-dependent dephosphorylation of RablO in LRRK2^{R1441C} MEFs with EC₅₀ = 158 nM (FIGURE 19A and FIGURE 19C).

FIGURES 20A, 20B demonstrate that XL01126 induced dose-dependent degradation of LRRK2 in macrophages with DC₅₀ = 55 nM. Cis-XL01126 cannot degrade LRRK2 in macrophage cells (FIGURE 20A and 20B).

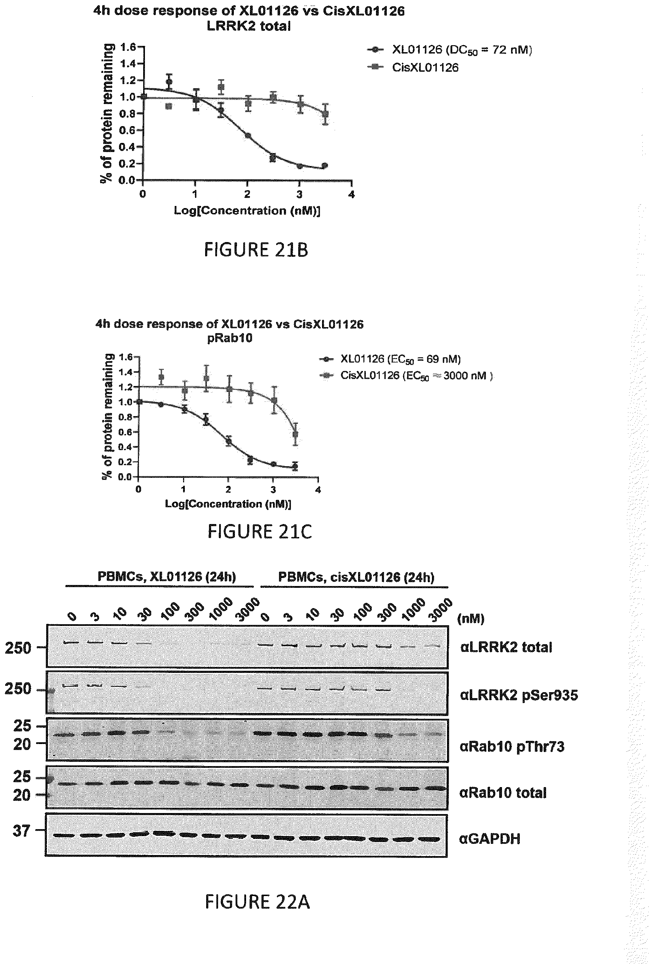

FIGURES21A, 21B, 21C show that after 4h treatment, XL01126 induced dose-dependent degradation of LRRK2 in PBMC with DC₅₀ = 72 nM and Dₘₐₓ = 88%. Cis-XL01126 cannot degrade LRRK2 in PBMC. (FIGURES 21A and 21B). After 4h treatment, XL01126 induced dose-dependent dephosphorylation of RablO in PBMC cells with EC₅₀ = 69 nM. Cis-XL01126 also dephosphorylated RablO in PBMC cells with EC₅₀ > 3 µM (FIGURE 21A and FIGURE 21C).

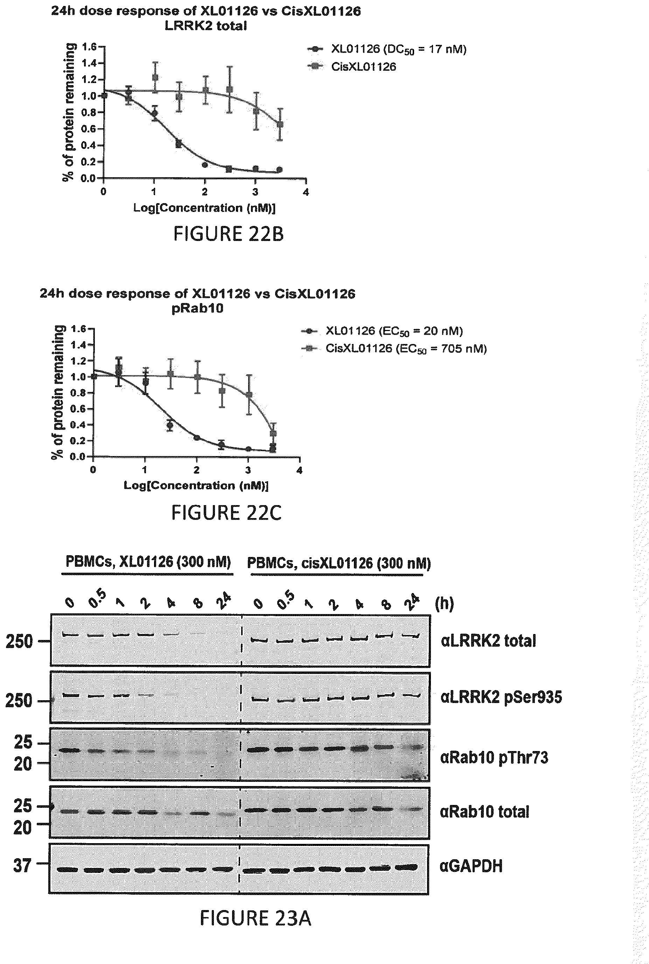

FIGURES 22A, 22B, 22C show that after 24h treatment, XL01126 induced dose-dependent degradation of LRRK2 in PBMC with DC₅₀ = 17 nM and Dₘₐₓ = 93%. Cis-XL01126 cannot degrade LRRK2 in PBMC. (FIGURE 22A and 22B). After 24h treatment, XL01126 induced dose-dependent dephosphorylation of RablO in PBMC cells with EC₅₀ = 20 nM. Cis-XL01126 also dephosphorylated RablO in PBMC cells with EC₅₀ = 705 nM (FIGURE 22A and FIGURE 22C).

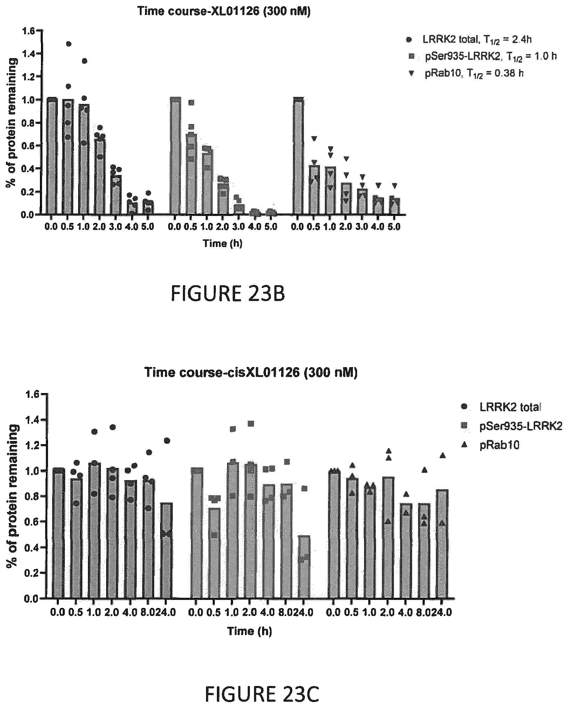

FIGURES 23A, 23B, 23C show that At 300 nM, XL01126 induced time-dependent degradation of LRRK2 in PBMC with T_{1/2} = 2.4 h and Dₘₐₓ = 92%. At 300 nM, XL01126 induced time-dependent dephosphorylation of LRRK2 and RablO in PBMC cells with T_{1/2} = 1h and 0.38 h separately (FIGURE 23A and FIGURE 23B). At 300 nM, Cis-XL01126 cannot degrade LRRK2, and induced little LRRK2 and RablO dephosphorylation (FIGURE 23A and FIGURE 23C).

FIGURE 24 shows that XL01126 selectively decreased intracellular LRRK2 protein level in the TMT-labelling proteomic study.

The data presented in Table1 shows that the solubilities of XL01126 in PBS and Fed State Simulated Intestinal Fluid (FeSSIP) are 0.55 µM and 26.05 µM separately (Table 6). XL01126 can penetrate Caco-2 mono-cell layer (Table 6). XL01126 is stable in mouse plasma and mouse hepatocytes with half-lives at 108.29 min and 314.33 min (Table 6), respectively. XL01126 can be metabolized in mouse liver microsome with half-life at 3.65 min (Table 6).

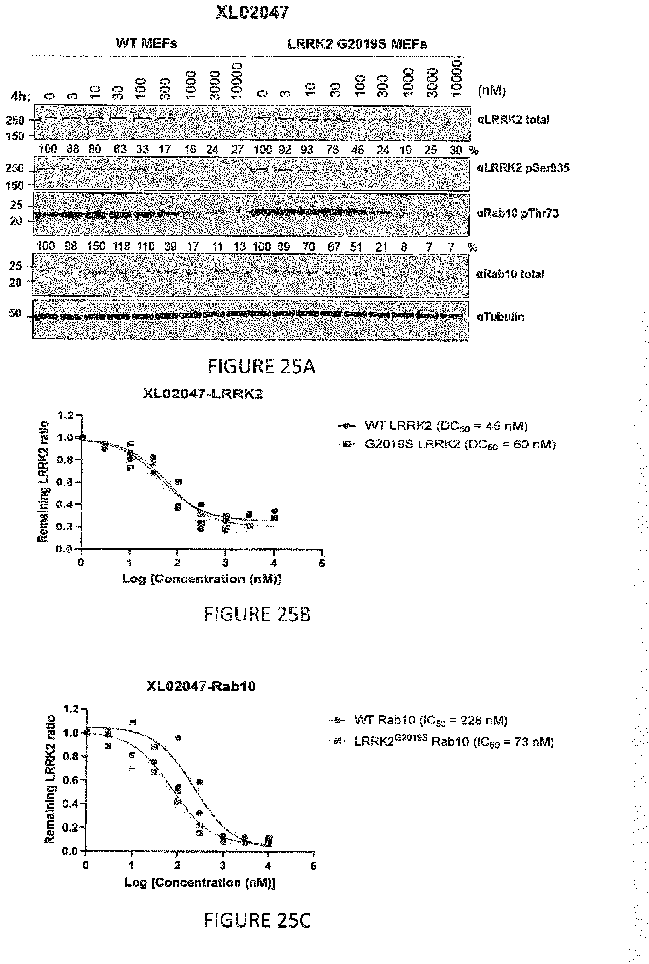

FIGURES 25A, 25B, 25C show that XL02047 induced dose-dependent degradation of LRRK2^{WT} and LRRK2^{G2019S} in WT MEFs and LRRK2^{G2019S} MEFs separately, with D ₘₐₓ (WT) = 78%, DC₅₀ (WT) = 45 nM, D ₘₐₓ (G2019S) = 79% and DC₅₀ (G2019S) = 60 nM (FIGURE 25A and FIGURE 25B). XL02047 induced dose-dependent dephosphorylation of RablO on WT MEFs and LRRK2^{G2019S} MEFs separately, with EC₅₀ (WT) = 228 nM and EC₅₀ (G2019S) = 73 nM (FIGURE 25A and FIGURE 25C).

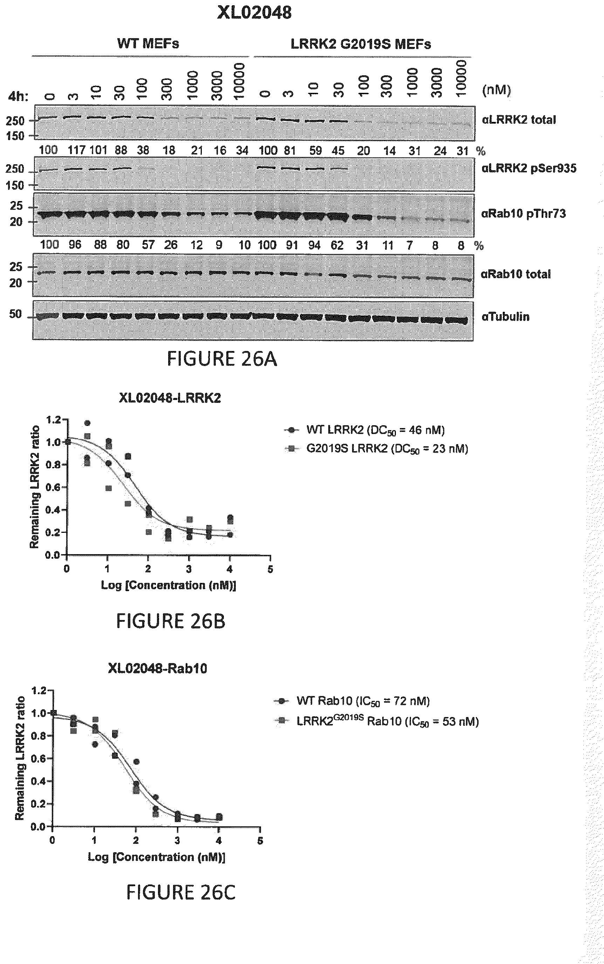

FIGURES 26A, 26B, 26C demonstrate that XL02048 induced dose-dependent degradation of LRRK2^{WT} and LRRK2^{G2019S} in WT MEFs and LRRK2^{G2019S} MEFs separately, with D ₘₐₓ (WT) = 81.5% DC₅₀ (WT) = 46 nM, D ₘₐₓ (G2019S) = 83.5% and DC₅₀ (G2019S) = 23 nM (FIGURE 26A and FIGURE 26B). XL02048 induced dose-dependent dephosphorylation of RablO on WT MEFs and LRRK2^{G2019S} MEFs separately, with EC₅₀ (WT) = 72 nM and EC₅₀ (G2019S) = 53 nM (FIGURE 26A and FIGURE 26C).

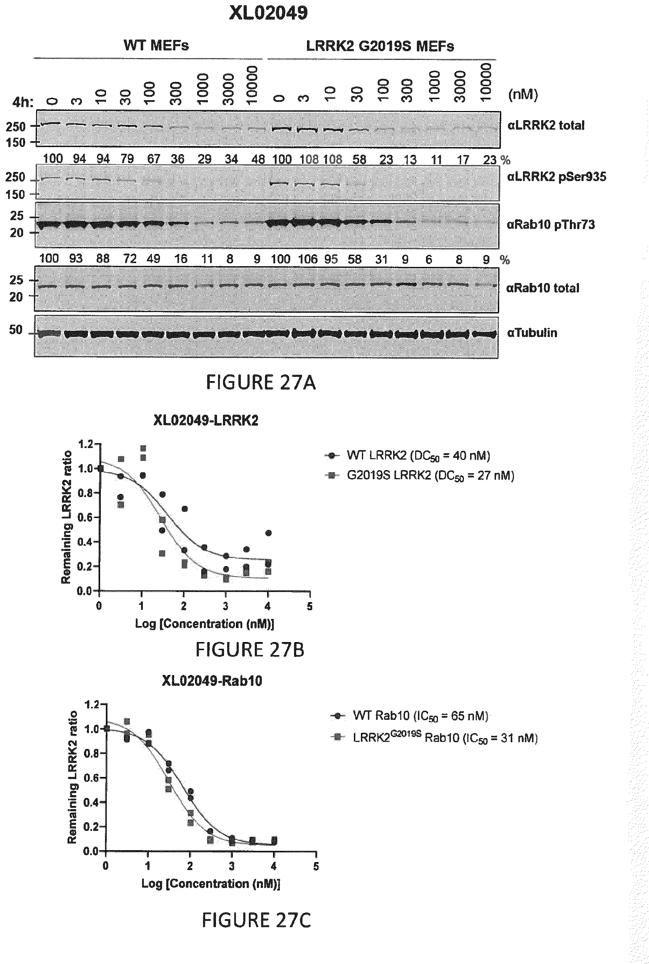

FIGURES 27A, 27B, 27C show that XL02049 induced dose-dependent degradation of LRRK2^{WT} and LRRK2^{G2019S} in WT MEFs and LRRK2^{G2019S} MEFs separately, with D ₘₐₓ (WT) = 76.5% DC₅₀ (WT) = 40 nM, D ₘₐₓ (G2019S) = 90% and DC₅₀ (G2019S) = 27 nM (FIGURE 27A and FIGURE 27B). XL02049 induced dose-dependent dephosphorylation of RablO on WT MEFs and LRRK2^{G2019S} MEFs separately, with EC₅₀ (WT) = 65 nM and EC₅₀ (G2019S) = 31 nM (FIGURE 27A and FIGURE 27C).

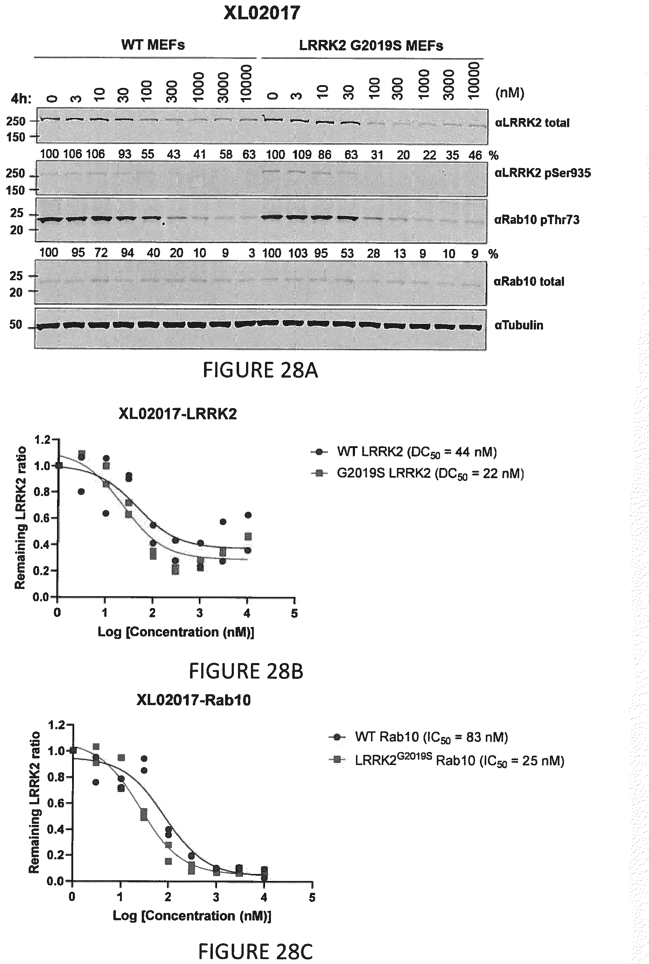

FIGURES 28A, 28B, 28C demonstrate that XL02017 induced dose-dependent degradation of LRRK2^{WT} and LRRK2^{G2019S} in WT MEFs and LRRK2^{G2019S} MEFs separately, with D ₘₐₓ (WT) = 68%, DC₅₀ (WT) = 44 nM, D ₘₐₓ (G2019S) = 79% and DC₅₀(G2019S) = 22 nM (FIGURE 28A and FIGURE 28B). XL02017 induced dose-dependent dephosphorylation of RablO on WT MEFs and LRRK2^{G2019S} MEFs separately, with EC₅₀ (WT) = 83 nM and EC₅₀ (G2019S) = 25 nM (FIGURE 28A and FIGURE 28C).

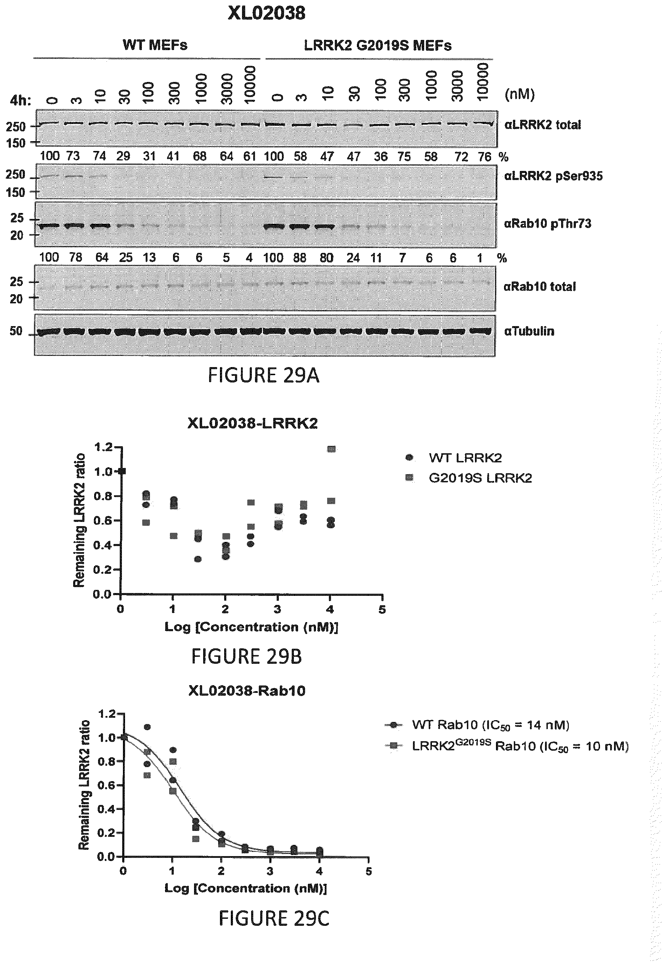

As shown in FIGURES 29A, 29B, 29C, XL02038 induced dose-dependent degradation of LRRK2^{WT} and LRRK2^{G2019S} in WT MEFs and LRRK2^{G2019S} MEFs separately, with D ₘₐₓ (WT) = 65%, and D ₘₐₓ (G2019S) = 79% (FIGURE 29A and FIGURE 29B). XL02038 induced dose-dependent dephosphorylation of RablO on WT MEFs and LRRK2^{G2019S} MEFs separately, with EC₅₀ (WT) = 14 nM and EC₅₀ (G2019S) = 10 nM (FIGURE 29A and FIGURE 29C).

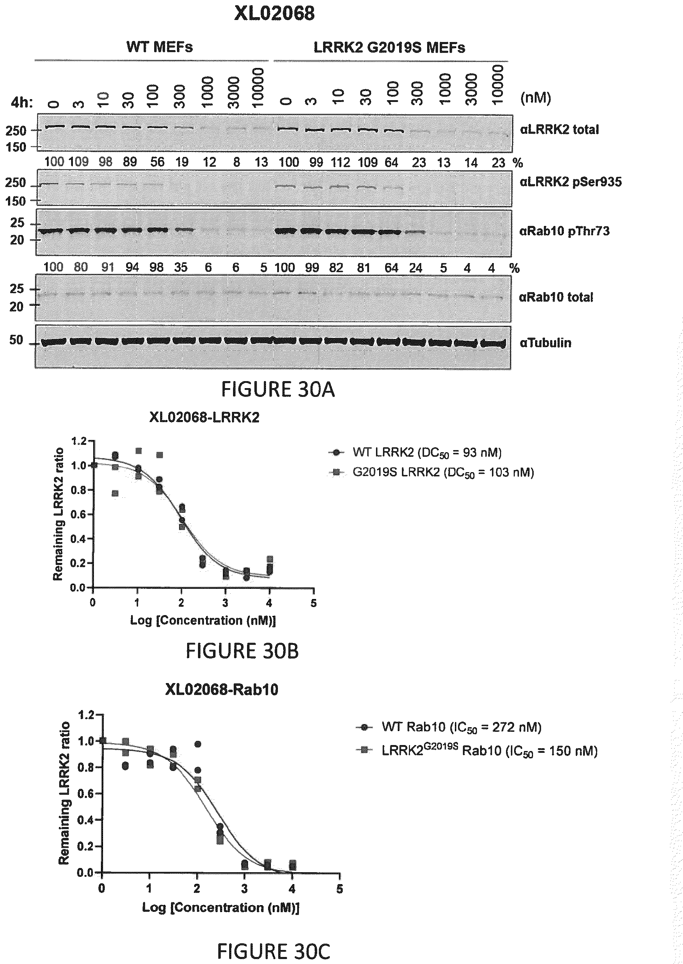

As shown in FIGURES 30A, 30B, 30C, XL02068 induced dose-dependent degradation of LRRK2^{WT} and LRRK2^{G2019S} in WT MEFs and LRRK2^{G2019S} MEFs separately, with D ₘₐₓ (WT) = 89% DC₅₀(WT) = 93 nM, D ₘₐₓ (G2019S) = 87% and DC₅₀(G2019S) = 103 nM (FIGURE 30A and FIGURE 30B). XL02068 induced dose-dependent dephosphorylation of RablO on WT MEFs and LRRK2^{G2019S} MEFs separately, with EC₅₀ (WT) = 272 nM and EC₅₀ (G2019S) = 150 nM (FIGURE 30A and FIGURE 30C).

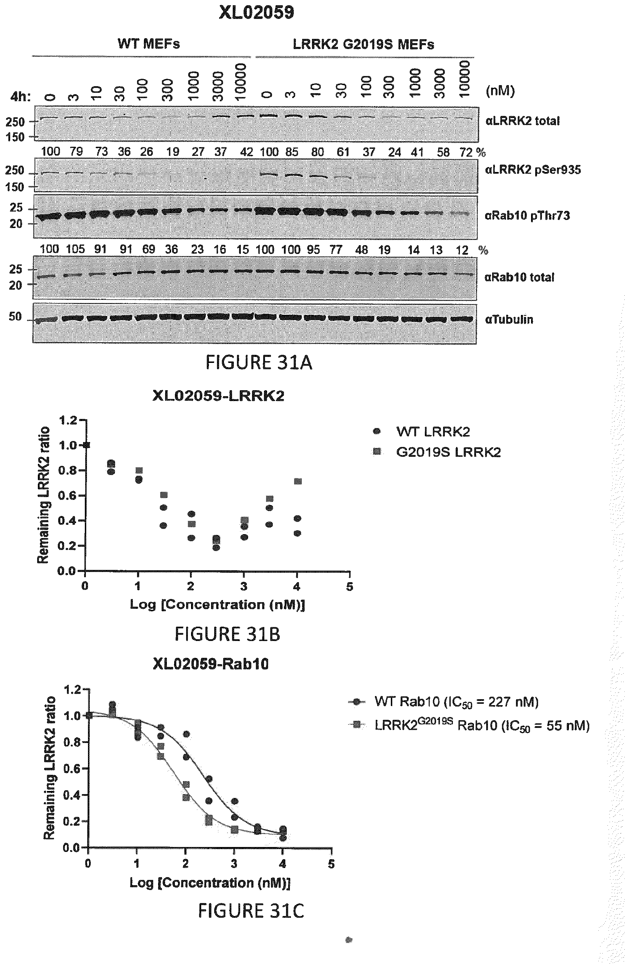

As shown in FIGURES31A, 31B, 31C, XL02059 induced dose-dependent degradation of LRRK2^{WT} and LRRK2^{G2019S} in WT MEFs and LRRK2^{G2019S} MEFs separately, with D ₘₐₓ (WT) = 77.5%, and D ₘₐₓ (G2019S) = 76% (FIGURE 31A and FIGURE 31B). XL02059 induced dose-dependent dephosphorylation of RablO on WT MEFs and LRRK2^{G2019S} MEFs separately, with EC₅₀ (WT) = 227 nM and EC₅₀ (G2019S) = 55 nM (FIGURE 31A and FIGURE 31C).

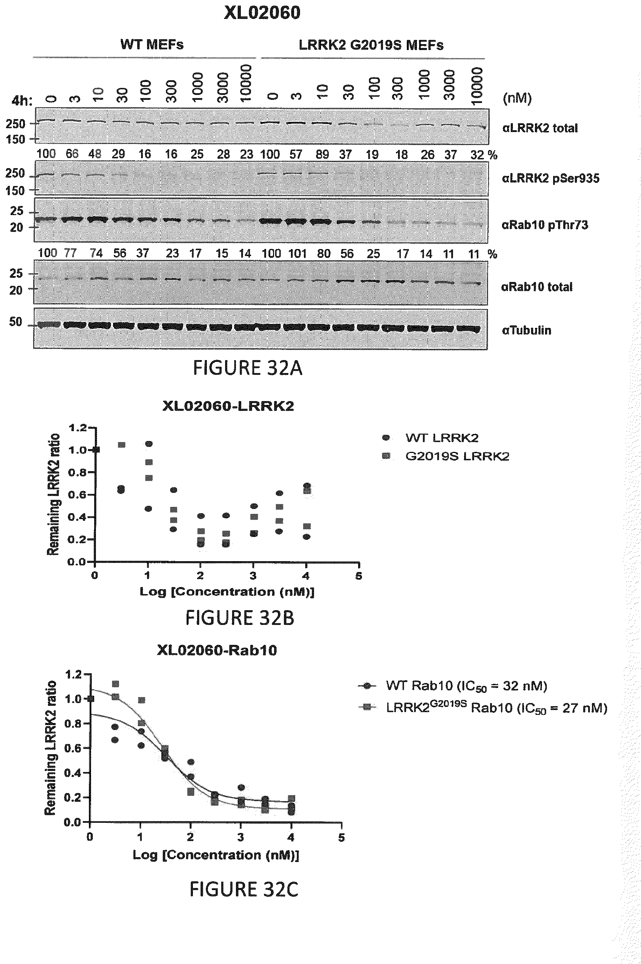

As seen in FIGURES 32A, 32B, 32C, XL02060 induced dose-dependent degradation of LRRK2^{WT} and LRRK2^{G2019S} in WT MEFs and LRRK2^{G2019S} MEFs separately, with D ₘₐₓ (WT) = 71 %, and D ₘₐₓ (G2019S) = 78.5% (FIGURE 32A and FIGURE 32B). XL02060 induced dose-dependent dephosphorylation of RablO on WT MEFs and LRRK2^{G2019S} MEFs separately, with EC₅₀ (WT) = 32 nM and EC₅₀ (G2019S) = 27 nM (FIGURE 32A and FIGURE 32C).

As seen in FIGURE 33, after mice were given 5 mg/Kg of XL01126 (single dose) through intravenous injection, the XL01126 in plasma has a clearance of 0.208 L/h/Kg, volume of distribution at 0.511 L/Kg, metabolic half-life at 1.52h, and area under the curves (AUC) of 23663 h^{∗}ng/mL and 23981 h^{∗}ng/mL for AUCₗₐₛₜ and AUC_{INF}, respectively (FIGURE 33). After given 30 mg/Kg (single dose) though intraperitoneal injection, the XL01126 plasma concentration reached its maximum (7700 ng/mL) at 0.25 h, with metabolic half-life at 5.2h and area under the curves (AUC) of 41434 h^{∗}ng/mL and 64068h^{∗}ng/mL for AUCₗₐₛₜ and AUC_{INF}, respectively. The bioavailability of XL01126 is 29.2% after intraperitoneal injection (FIGURE 33). After given 30 mg/Kg (single dose) via gavage, the XL01126 plasma concentration reached its maximum (3620 ng/mL) at 2 h, with metabolic half-life at 21.9 h and area under the curves (AUC) of 21337 h^{∗}ng/mL and 109271 h^{∗}ng/mL for AUCₗₐₛₜ and AUC_{INF}, respectively. The bioavailability of XL01126 is 15% after intraperitoneal injection (FIGURE 33).

As seen in FIGURE 34, after mice were given 5 mg/Kg (single dose) of XL01126 through intravenous injection, the XL01126 concentration in the brain tissue reached its maximum level (155 ng/mL) at 0.083h, with metabolic half-life at 3.67 h, and area under the curves (AUC) of 258 h^{∗}ng/mL and 349 h^{∗}ng/mL for AUCₗₐₛₜ and AUC_{INF}, respectively (FIGURE 34). After mice were given 30 mg/Kg of XL01126 (single dose) though intraperitoneal injection, the concentration of XL01126 in the brain tissue reached its maximum level (218 ng/mL) at 0.5 h, with metabolic half-life at 2.79 h and area under the curves (AUC) of 752 h^{∗}ng/mL and 884 h^{∗}ng/mL for AUCₗₐₛₜ and AUC_{INF}, respectively (FIGURE 34). After mice were given 30 mg/Kg of XL01126 (single dose) via gavage, the XL01126 brain tissue concentration reached its maximum (27 ng/mL) at 2 h, with metabolic half-life at 10.7 h and area under the curves (AUC) of 142 h^{∗}ng/mL and 402 h^{∗}ng/mL for AUCₗₐₛₜ and AUC_{INF}, respectively.

FIGURE 35 shows that after mice were given 5 mg/Kg of XL01126 (single dose) through intravenous injection, the XL01126 concentration in cerebrospinal fluid (CSF) reached its maximum concentration (27.4 ng/mL) at 0.083h, with metabolic half-life at 8.59 h, and area under the curves (AUC) of 107 h^{∗}ng/mL and 240 h^{∗}ng/mL for AUCₗₐₛₜ and AUC_{INF}, respectively (FIGURE 35). XL01126 also reaches CSF after given intraperitoneal (30 mg/Kg), and orally (30 mg/Kg).

Leucine Rich Repeat Kinase 2 (LRRK2) is one of the most promising disease-modifying targets for Parkinson's disease, yet current LRRK2 targeting strategy has been focused on kinase inhibitors with potential undesirable side-effects. Herein, we disclose the discovery of LRRK2 Proteolysis Targeting Chimeras (PROTAC) degraders, exemplified by compound XL01126, as an alternative LRRK2 targeting strategy. XL01126 induces fast and potent degradation of LRRK2 protein in multiple cell lines in a dose-dependent and time-dependent manner. Specifically, XL01126 is capable of reducing the LRRK2 protein level by 80-90% in WT MEFs, LRRK2^{G2019S} MEFs, LRRK2^{R1441C} MEFs, macrophagys, and PBMCs cells with DC₅₀s at 32 nM, 14 nM, 15 nM, 55 nM, and 17 nM, respectively. XL01126 has degradation half-lives of 1.2h, 0.6h and 2.4h on WT MEFs, LRRK2^{G2019S} MEFs, and PBMCs, respectively. XL01126 is orally bioavailable (F=15%) and can penetrant blood brain barrier after a single dose of XL01126 through intravenous (5 mg/Kg), intraperitoneal (30 mg/Kg), or oral dosing (30 mg/Kg) in mice. All these properties qualify XL01126 as a chemical probe to study LRRK2 biology *in vitro* and *in vivo* and offer a starting point and potential treatment for future PD drug development.

FIGURE 36 shows that VH101, XL01126, SD75, XL01134 and Cis-XL01126 displace a fluorescent tracer from VHL protein in a dose dependent manner with XL01134 (Kᵢ = 0.16 µM) showed the most potent displacement and therefore the highest binding affinity to VHL, followed by VH101 (Kᵢ = 0.44 µM), and SD75 (Kᵢ = 0.82 µM), while XL01126 (Kᵢ >2.33 µM) showed poor displacement (binding affinity to VHL) and Cis-XL01126 did not displace the fluorescent tracer.

FIGURE 37A shows that in the permeabilized HEK293 cells, VH101, XL01126, SD75, and XL01134 can displace VHL tracer from VHL protein in a dose dependent manner with XL01134 (IC₅₀ = 0.78 µM) showed the most potent displacement and therefore the highest binding affinity to VHL, followed by VH101 (IC₅₀ = 1.0 µM) , and SD75 (IC₅₀ = 1.9µM) , while XL01126 showed poor displacement (binding affinity to VHL).

FIGURE 37B shows that in the live HEK293 cells, VH101, XL01126, SD75, and XL01134 can displace VHL tracer from VHL protein in a dose dependent manner with XL01134 showed the most potent displacement (IC₅₀ = 0.88 µM) and therefore the highest binding affinity to VHL, followed by VH101 (IC₅₀ = 1.8 µM), and SD75 (IC₅₀ = 2.5 µM), while XL01126 (IC₅₀ = 10.1 µM) showed poor displacement (binding affinity to VHL). XL01126 has the highest IC₅₀ ratio (IC₅₀, ₚₑᵣ/IC_{50, live} > 0.99) between permeabilized and live mode VHL engagement and therefore the highest permeability among the testing PROTACs, followed by XL01134 (IC₅₀, ₚₑᵣ/IC_{50, live} = 0.89), and SD75 (IC₅₀, ₚₑᵣ/IC_{50, live} = 0.76).

FIGURE 38A shows that in the permeabilized HEK293 cells, HG-10-102-01, XL01126, SD75, and XL01134 can displace LRRK2 tracer from LRRK2 protein in a dose dependent manner withHG-10-102-01(IC₅₀ = 0.23 µM) showed the most potent displacement and therefore the highest binding affinity to LRRK2, followed by SD75 (IC₅₀ = 0.78 µM) , and XL01134 (IC₅₀ = 2.0 µM) , while XL01126 showed poor displacement.

FIGURE 38B shows that in the live HEK293 cells, HG-10-102-01, XL01126, SD75, and XL01134 can displace LRRK2 tracer from LRRK2 protein in a dose dependent manner with HG-10-102-01 showed the most potent displacement (IC₅₀ = 0.7 µM) and therefore the highest binding affinity to LRRK2, followed by XL01126 (IC₅₀ = 1.4 µM), SD75 (IC₅₀ = 1.8 µM), and XL01134 (IC₅₀ = 2.2µM). XL01126 has the highest IC₅₀ ratio (1.89) between permeabilized and live mode LRRK2 engagement and therefore the highest permeability among the testing PROTACs, followed by XL01134 (IC₅₀, ₚₑᵣ/IC_{50, live} = 0.91), and SD75 (IC₅₀, ₚₑᵣ/IC_{50, live} = 0.43).

FIGURE 39A shows that XL01126 engages with LRRK2 better in the presence of VCB protein than in the absence of VCB (IC₅₀ = 0.35 µM vs 2 µM), leading to a positive cooperativity (α) of 5.7.

FIGURE 39B shows that XL01134 engages with LRRK2 better in the presence of VCB protein than in the absence of VCB (IC₅₀ = 0.79 µM vs 1.1), leading to a positive cooperativity (α) of 1.4.

FIGURE 39C shows that SD75 engages with LRRK2 worse in the presence of VCB protein than in the absence of VCB (IC₅₀ = 1.4 µM vs 1.1), leading to a negative cooperativity (α) of 0.79.

FIGURE 40 shows that XL01126 can induce the highest ternary complex with VHL and LRRK2, followed by SD75 and XL01134. Cis-XL01126 cannot form ternary complex with VHL and LRRK2.

### Experimental

### Generation of mouse embryonic fibroblasts (MEFs)

Primary MEFs were generated as described in a previous study (Wiggin et al., Mol. Cell Biol. 2002 Apr;22(8):2871-81.). Briefly, the uterine horn was collected from adult female mice at day E12.5 and transferred to a 10 cm tissue culture dish containing cold PBS. Two forceps were used to tear the yolk sacs to isolate each embryo. Forceps were cleaned thoroughly with 70% ethanol between each embryo isolation. The embryos were culled, and a tissue piece was collected in a PCR tube for genotyping. The red tissue of the embryo, which grows to become the heart and liver, was removed. The remainder of the embryo was minced with a scalpel blade and incubated with 7.5 ml trypsin-EDTA solution for 10 minutes in a 37°C tissue culture CO₂ incubator. The dish was removed from the incubator and checked under a light microscope for single cells. 7.5 ml complete media was added to the trypsinised cells, and the cell suspension was transferred to a 15 ml Falcon tube, centrifuged at 1200 rpm for 5 minutes at room temperature. The trypsin was aspirated, the cell pellet was resuspended in 5 ml fresh complete media, and the cell suspension was plated in a 60 mm tissue culture dish and incubated in a 37°C, 5% CO₂ tissue culture incubator. The MEFs at this stage were considered as passage 0 and were passaged and expanded for experimental use once the genotype was confirmed by allelic sequencing and immunoblotting. MEFs were cultured in Dulbecco's modified Eagle medium (DMEM) containing 10% (v/v) fetal bovine serum (FBS), 2 mM L-glutamine, 100 U/ml penicillin, and 100 µg/ml streptomycin supplemented with 1X non-essential amino acids and 1 mM sodium pyruvate.

### Generation of bone marrow-derived macrophages (BMDMs)

Macrophages were cultured in complete media containing DMEM, 10% (v/v) heat inactive FBS, 20% (v/v) L929 preconditioned medium, 2.5% (v/v) HEPES, 2 mM L-glutamine, 100 U/ml penicillin and 100 µg/ml streptomycin, 2% sterile-filtered β-mercaptoethano), 1X non-essential amino acids and 1 mM sodium pyruvate. Bone marrow isolation and macrophage differentiation was modified from (Wells *et al.,* 2004), employing L929 preconditioned medium as the source of M-CSF for differentiation. Briefly, scissors and forceps were used to dissect femurs and tibiae from adult mice, and muscle tissue was carefully removed from bones. Clean femurs and tibiae were placed in a tissue culture dish containing complete media. The ends of each bone were cut with scissors to expose bone marrow. Bone marrow was flushed with a 25-gauge needle attached to a 10 ml syringe containing complete media. Media containing bone marrow was passed through a 70 µm cell strainer and precursor cells were plated on non-tissue culture treated 10 cm bacteriological plates containing 10 ml complete media. This was marked as day 0 of isolation. On day three post-isolation, macrophages were topped up with 5 ml fresh complete media. On day seven post-isolation, macrophages were rinsed once with PBS and incubated with Versene for 5 minutes in a 37°C 5% CO₂ tissue culture incubator. Macrophages were detached with cell scrapers and were centrifuged at 1200 rpm for 5 minutes at room temperature. The Versene was aspirated and the remaining cell pellet was resuspended in complete media. The cell suspension was counted, and cells were seeded for experimental analysis in a 6-well format, in tissue culture treated dishes at a final cell density of one million cells per 6-well.

### PBMC cells separation and treatment

PBMC cells were separated from human blood following existing protocol (dx.doi.org/10.17504/protocols.io.bnhxmb7n) and pelleted by centrifugation at 1000 *g* for 2 min. The supernatant was discarded and the PBMC pellet was resuspended in PBS containing 2% FBS for washing. The suspension was centrifuged at 1000 g for 2 min again and the PBMC pellet was resuspended in RPMI-1640 (Gibco) media supplemented with 10% FBS. The cells were then seeded into 6-well plates and treated with testing compounds at indicated concentrations and time period. After treatment, the cells were collected into 2-ml Eppendorf tube and centrifuged at 500 *g* for 2 min to pellet the cells, the supernatant was discarded, and the pellet was resuspended in 1 ml PBS and centrifuged at 500 g for 2 min again. The PBMC pellet was lysed with 60 µL of lysis buffer containing 50 mM Tris-HCl, pH 7.5, 1% (v/v) Triton X-100, 1 mM EGTA, 1 mM sodium orthovanadate, 50 mM NaF, 0.1% (v/v) 2-mercaptoethanol, 10 mM 2-glycerophosphate, 5 mM sodium pyrophosphate, 0.1 µg/ml mycrocystin-LR (Enzo Life Sciences), 270 mM sucrose, 0.5 mM DIFP (Sigma, Cat# D0879) in addition to Complete EDTA-free protease inhibitor cocktail (Sigma-Aldrich Cat # 11836170001). DIFP is highly toxic and must be prepared in a fume hood to a stock solution of 0.5 M in isopropanol. The lysed cells were then centrifuged at 1500 g for 15 min at 0 °C. The supernatants were collected for analysis by quantitative immunoblotting. For long term storage, the supernatant was flash frozen and stored at -80°C. Protein concentrations of cell lysates were determined using Pierce^{™} BCA Protein Assay Kit (ThermoFisher).

### Cell culture, treatment, and lysis

Culturing and passaging of adherent cell lines were carried out using aseptic technique in CL1 or CL2 (for PBMC isolation) biological safety cabinets. All cells were incubated in a 37°C incubator with 5% CO2. Cell lines were regularly tested for mycoplasma contamination. For western blot assay, the cells were seeded in 6-well plates. After treating with testing compounds at indicated concentrations and time period, the media was removed and the cells were washed with PBS and lysed in 100 µl ice-cold complete lysis buffer containing 50 mM Tris-HCl pH 7.4, 1 mM EGTA, 10 mM 2-glycerophosphate, 50 mM sodium fluoride, 5 mM sodium pyrophosphate, 270 mM sucrose, supplemented with 1 µg/ml microcystin-LR, 1 mM sodium orthovanadate, complete EDTA-free protease inhibitor cocktail (Roche) and 1% (v/v) Triton X-100. The cells were immediately placed on ice and were scraped and collected into 1.5 ml Eppendorf tubes. Cell lysates were incubated on ice for 10 minutes prior to centrifugation at 15,000 g at 4°C for 15 minutes. The cell pellet was discarded, and supernatant was collected for analysis by quantitative immunoblotting. For long term storage, the supernatant was flash frozen and stored at -80°C. Protein concentrations of cell lysates were determined using the Bradford assay.

### Quantitative immunoblotting

Cell lysates containing a quarterof a volume of 4X NuPAGE LDS sample buffer (NP0007) supplemented with 5% β-mercaptoethanol, were heated at 95°C for 5 minutes. 15 to 20 µg of samples were loaded onto pre-cast 4-12% Bis-Tris midi 20W or 26W gels (Thermofisher Scientific^{®}, Cat# WG1402BOX or WG1403BOX) and resolved at 130 V for 2 hours with NuPAGE MOPS SDS running buffer (Thermofisher Scientific^{®} Cat# NP0001-02). Proteins were electrophoretically transferred onto a 0.45 µm nitrocellulose membrane (GE Healthcare, Amersham Protran^{®} Supported 0.45 mm NC) at 90 V for 90 min on ice in transfer buffer (48 mM Tris base and 39 mM glycine supplemented with 20% methanol). The transferred membrane was blocked with 5% (w/v) skim milk powder dissolved in tris-buffered saline with tween (TBS-T) (50 mM Tris base, 150 mM sodium chloride (NaCl), 0.1% (v/v) Tween-20) at room temperature for 1 hour. Membranes were washed three times with TBS-T and were incubated in primary antibody overnight at 4°C. Prior to secondary antibody incubation, membranes were washed three times for 15 minutes with TBS-T. The membranes were incubated with secondary antibody for 1 hour at room temperature, protected from light. Thereafter, the membranes were washed with TBS-T three times with a 15-minute incubation for each wash, and protein bands were acquired via near-infrared fluorescent detection using the Odyssey CLx imaging system and quantified using Image Studio software. Graphs were generated using Graphpad Prism version 8 software.

### Antibodies

Monoclonal rabbit LRRK2 Ser935 (Cat# UDD2) was purified by MRC PPU Reagents and Services at the University of Dundee and was used at a final concentration of 1 µg/ml. Total LRRK2 (C-terminus) was from Antibodies Inc./Neuromab (Cat# 75-253) and was diluted 1:1000. The MJFF monoclonal rabbit RablO pThr73, which was characterized previously (Lis et al., Biochem J. 2018 Jan 2;475(1):1-22.) was purchased from Abcam Inc. (ab230261) and diluted 1:1000. Mouse monoclonal alpha-tubulin (#3873) was purchased from Cell Signaling Technology and used at 1:1000. The mouse monoclonal anti-RablO total antibody was purchased from Nanotools (#0680-100/Rab10-605B11) and was used at a final concentration of 1 µg/ml. Mouse monoclonal Hif-1α was purchased from R&D Systems (Cat# MAB1536) and was diluted 1:1000. Mouse monoclonal Ubiquitin was purchased from Biolegend (Cat# 646302) and was diluted 1:1000. Rabbit polyclonal PDE6D antibody was purchased from Novus Biologicals and was used at a final concentration of 1:500. All rabbit and mouse primary antibodies were diluted in 5% (w/v) bovine serum albumin (BSA) dissolved in TBS-T (50 mM Tris base, 150 mM sodium chloride (NaCl), 0.1% (v/v) Tween 20). Goat anti-mouse IRDye 800CW (#926-32210), goat anti-mouse IRDye 680LT (#926-68020), goat anti-rabbit IRDye 800CW (#926-32211), and goat anti-rabbit IRDye 680LT (#926-68021) IgG (H+L) secondary antibodies were from LI-COR and were diluted 1:10,000 in 5% (w/v) milk in TBS-T.

### Total proteome sample preparation and MS analysis

Wildtype MEFs were seeded in 10 cm tissue culture dishes, at a density of two million cells per dish. Cells were treated with 0.1% DMSO, 300 nM XL01126, or 300 nM cisXL01126 for 4 hours prior to harvest in 400 µl complete lysis buffer, supplemented with 1 µg/ml microcystin-LR, 1 mM sodium orthovanadate, complete EDTA-free protease inhibitor cocktail (Roche) and 1% (v/v) Triton X-100. Cell lysates were incubated on ice for 10 minutes, then underwent three rounds of high energy sonication for 15 cycles (30 seconds on, 30 seconds off) using the Diagenode Bioruptor. Cell lysates were centrifuged at 15,000 *g* at 4°C for 15 minutes. Cell pellet was discarded, and supernatant was collected for protein quantification using a BCA protein assay kit (Pierce #23225). 100 µg cell lysate employed for total proteomic analysis. Proteins in cell lysate were reduced with 0.1 M Tris(2-carboxyethyl)phosphine (TCEP) diluted in 300 mM triethylammonium bicarbonate (TEABC) to a final concentration of 10 mM. Samples were incubated on a Thermomixer for 30 minutes at 60°C at 800 rpm. Samples were incubated to room temperature and underwent alkylation with 0.4 M iodoacetamide (IAA) freshly dissolved in water. Samples were then incubated in the dark on a Thermomixer at room temperature for 30 minutes at 800 rpm. Alkylation was quenched with the addition of 0.1 M TCEP dissolved in 300 mM TEABC at a final concentration of 5 mM. Samples were incubated on a Thermomixer at room temperature for 20 minutes at 800 rpm. Sodium dodecyl sulfate (SDS) was added at a final concentration of 5% (w/v) from a 20% (w/v) stock. 12% (v/v) phosphoric acid was then added to a final concentration of 1.2% (v/v). Samples were diluted in 6 times the sample volume of S-trap wash buffer containing 90% (v/v) methanol diluted in 100 mM (v/v) TEAB pH 7.1.

### S trap cleanup and digestion

Samples underwent S-trap cleanup to remove detergents and other impurities with S-trap mini columns (PROTIFI Cat# MSPPC02-MINI-80) placed in 2 ml Eppendorfs. The protein mixtures were added to columns and centrifuged briefly (1000 *g* / 1 minute / RT). Columns were washed with 400 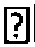 | S-trap buffer 4 times, centrifuging after each wash 1000 *g* / 1 minute / RT. Columns were placed in fresh 2 ml Eppendorfs and 100 µl of 5 µ g Trypsin/Lys-C freshly dissolved in 50 mM TEAB, pH 8.5 was added. Columns were centrifuged briefly (200 *g* / 1 minute / RT) and Trypsin/Lys-C mixture was pipetted back onto the column. 100 µ l 50 mM TEAB, pH 8.5 was added directly to the 2 ml Eppendorfs to cover any digested peptides remaining in the tube. The S-trap columns in 2 ml Eppendorfs were incubated at 47°C without shaking for 1.5 hours, then at RT overnight.

80 µl 50 mM TEAB was added to S-trap columns, which were centrifuged, and eluates were collected in new 1.5 ml Eppendorf tubes. 80 µl 0.2% (v/v) formic acid was added to columns, which were centrifuged, and second eluates were pooled with first eluates. 80 µ l 50% (v/v) acetonitrile diluted in 0.2% (v/v) formic acid was added to columns, which were centrifuged, and third eluates were pooled with previous eluates. 500 ng digested peptides were set aside to vacuum dry separately to verify that digestion efficiency was >90%. The remaining peptides were divided in half (50 µg peptides each tube) and vacuum dried and stored in -80°C prior to continuation with tandem mass tag (TMT) labeling.

### TMT labeling

800 µg TMT mass tag reagents were dissolved 80 µl 100% (v/v) anhydrous acetonitrile to obtain final concentrations of 10 µg/ µl. Resuspended TMT reagents were incubated at RT for 10 minutes, then vortexed and centrifuged briefly (2000 *g* / 2 minutes / RT). 50 µg lyophilized peptides were resuspended in 50 µl of a mixture containing 38 µl 50 mM TEAB and 8 µl 100% (v/v) anhydrous acetonitrile. Resuspended peptides were sonicated for 10 minutes, then centrifuged at 17,000 *g* for 10 minutes at RT. Peptides were transferred to fresh protein lo-bind 1.5 ml Eppendorf tubes. 20 µl of 10 µg/ µl TMT reagent were added to solubilized peptides, vortexed, centrifuged briefly (2,000 g / 1 minute / RT) and incubated on a Thermomixer for 2 hours at 800 rpm at RT. 50 µl of 50 mM TEAB was added to each reaction, followed by vortex, brief centrifugation (2,000 *g* / 1 minute / RT) and incubation on a Thermomixer at 800 rpm at RT for an additional 10 minutes. 5 µl of each TMT labeled sample was set aside, vacuum dried and injected on MS to confirm that labeling efficiency was >98%. The remaining reactions were stored in -80°C until labeling efficiency was verified. TMT samples thawed to RT and labeling reactions were quenched with the addition of 5 µl 5% (v/v) hydroxylamine (dissolved in water from a 50% (v/v) stock solution). Samples were incubated on a Thermomixer for 20 minutes at 800 rpm at RT. Quenched TMT labeled samples were pooled and proceeded with C18 stage-tip desalting.

### Pharmacokinetic (PK) studies

PK profiling was outsourced and undertaken by Shanghai ChemPartner Co., Ltd. All animal experiments performed were conducted in compliance with the Institutional Animal Care and Use Committee (IACUC) and the Office of Laboratory Animal Welfare (OLAW) guidelines. Six- to eight-week-old C57BL/6 male mice purchased from Jihui Laboratory Animal Co. LTD were used in the study. XL01126 was formulated in 10%HP-β-CD in 50mM Citrate buffer pH=3.0 at 1 mg/mL for IV injection and at 3 mg/mL for IP and PO injection. For IV injections, 5 mg/kg of XL01126 was administered into the tail vein of nine mice. For IP injections, 30 mg/kg of XL01126 was administered via intraperitoneal injection in mice. The animals were restrained manually at the designated time points (0.083, 0.25, 0.5, 1, 2, 4, and 8 h); approximately, 110 µL of blood sample was collected via facial vein into K2EDTA tubes. Three mice per time point were used, resulting in a total of 21 mice for each administration route. The blood sample was put on ice and centrifuged at 2000 *g* for 5 min to obtain the plasma sample within 15 min. The plasma, brain, CSF samples were stored at approximately -70 °C until analysis. A 30 µL aliquot of plasma was added with 200 µL of internal standard (Glipozode, 40 ng/mL) in MeCN with 5% citri. The mixture was then vortexed for 1 min and then centrifuged for 10 min at 5800 rpm. The supernatant (100 µL) was transferred to a new plate. The solvent (1 µL) was injected to LC-MS/MS. LC-MS/MS instrument used: SCIEX LC-MS/MS-49 (Triple Quad 6500+). Data was analyzed by WinNonLin and Microsoft Excel.

### Fluorescence polarization assay

FP competitive binding assays were performed following the method described previously (Bond et al, J. Med. Chem. 2021, 64 (20), 15477-15502.). All the measurements were taken on a PHERstar (BMG LABTECH) plate reader installed with a FP filter that sets excitation and emission wavelengths at 485 nm and 520 nM separately. Each well of 384-well plate (Coring 3575) contains 10 nM VCB protein, 5 nM FAM-lableled HIF-1α peptide (FAM-DEALAHypYIPMDDDFQLRSF, "JC9"), and decreasing concentrations of PROTACs (14 concentrations with 2-fold serial dilution starting from 250 µM) in FP assay buffer (100 mM Bis-Tris propane, 100 mM NaCl, 1 mM TCEP, pH 7) with a final DMSO concentration of 5%. The control wells containing the VCB and JC9 with no compound are set as the maximum signals (zero displacement). And the control wells containing JC9 in the absence of protein are set as the minimum signals. Control values were used to obtain the percentage of displacement which was graphed against Log [Compound]. Average IC₅₀ values were determined for each titration using nonlinear regression analysis with Prism (v.9.3.1). The Kᵢ values were back-calculated from the K_{d} of JC9 (1.5 nM -3.4 nM) and the fitted IC₅₀ values, as described previously.

### NanoBRET target engagement assay

For VHL and LRRK2 target engagement experiments in live and permeabilized cells, the HEK293 cells were transfected with VHL-NanoLuc fusion vector (Promega, N275A) or LRRK2-NanoLuc fusion vector (Promega, NV3401) following Promega's protocol and seeded into white 384-well plate (Corning3570) at a density of 6000 cells/well. To measure NanoBRET in permeabilized cells, the cells were treated with 50 µg/ mL digitonin (Sigma, D141), 125 nM VHL tracer/125 nM LRRK2 tracer, testing compounds at decreasing concentrations (12 concentrations with 2-fold serial dilution starting from 33 µM), and NanoBRET NanoGlo Substrate (Promega) at concentration recommended by the manufacturer's protocol. In the maximum signal control samples (DMSO control), DMSO was added instead of testing compounds. In the minimum signal control samples (no tracer control), DMSO and tracer dilution buffer were used to replace testing compounds and tracer separately. The filtered luminescence was measured within 10 min following addition of the substrate on a GloMax Discover microplate reader (Promega) or a PHERAstar (BMG LABTECH) plate reader equipped with a 450-nm bandpass filter (donor) and a 600-nm long pass filter (acceptor). To measure NanoBRET in live cells, the cells were treated with 250 nM VHL tracer/500 nM LRRK2 tracer, testing compounds at testing compounds at decreasing concentrations (12 concentrations with 2-fold serial dilution starting from 33 µM) and incubated at 37 °C in an incubator for 2 h. The plates were then cooled down and added with NanoBRET NanoGlo Substrate and Extracellular NanoLuc Inhibitor (Promega, N2160) before performing the same NanoBRET reading as the permeabilized mode on plate readers. NanoBRET ratio of each well was expressed in milliBRET according to the equation: mBRET = [(signal at 610 nM/signal at 450 nM) - (signal at 610 nMno tracer control/signal at 450 nMno tracer control)]×1000. The fractional occupancy was calculated according to the equation: fractional occupancy = (mBRETtesting compound -mBRETno tracer control)/(mBRETDMSO control - mBRETno tracer control).

### NanoBRET-based ternary binding and cooperativity assay

The HEK293 cells were transfected with LRRK2-NanoLuc fusion vector (Promega, NV3401) following Promega's protocol and seeded into white 384-well plate (Corning3570) at a density of 6000 cells/well. The cells were then treated with 50 µg/mL of digitonin, 125 nM of LRRK2 tracer, testing compounds at decreasing concentrations (11 concentrations with 2-fold serial dilution starting from 10 µM) or testing compounds and VCB mix (11 concentrations with 2-fold serial dilution starting from 10 µM compound for the compound. The first 6 concentrations of VCB start from 32 µM with 2-fold dilution, the last 5 concentrations of VCB keep at 1 µM), and NanoBRET NanoGlo Substrate (Promega) at concentration recommended by the manufacturer's protocol. In the maximum signal control samples (DMSO control), DMSO was added instead of testing compounds. In the minimum signal control samples (no tracer control), DMSO and LRRK2 tracer dilution buffer were used to replace testing compounds and LRRK2 tracer separately. The filtered luminescence was measured within 10 min following addition of the substrate on a PHERAstar (BMG LABTECH) plate reader equipped with a 450-nm bandpass filter (donor) and a 600-nm long pass filter (acceptor). The fractional occupancy was calculated according to the equation: fractional occupancy = (mBRETtesting compound -mBRETno tracer control)/(mBRETDMSO control - mBRETno tracer control).

### Bodipy 576/589 labeling of VCB

VCB was labeled with Bodipy 576/589 following protocol reported previously 76. Briefly, the VCB complex was mixed with Bodipy 576/589 NHS ester in a 20:1 molar ration and incubated at room temperature (protect from light) for 2h in reaction buffer (0.1 M sodium phosphate, 75 mM KOAc, 2 mM DTT, pH 7.4). The reaction was quenched by diluting 10 times with reaction buffer and the unreacted dye was removed with a PD-10 MiniTrap desalting column (GE Healthcare) equilibrated with 100 mM BisTris pH 7.0, 100 mM NaCl, 1 mM DTT, pH 7. The eluted labeled protein solution was collected and concentrated with Pierce Concentrator, 3K MWCO (Thermo scientific).

### NanoBRET Ternary complex formation assay

Hek293 cells were transfected with LRRK2-NanoLuc vector (Promega, NV3401) for 24h, harvested, and resuspended into OptiMEM media without phenol red (Life Technologies). The cells were then seeded into white 384-well plate (Corning3570) at a density of 6000 cells/well. Digitonin solution (final concentration 50 µg/mL), testing PROTACs at decreasing concentrations (11 concentrations with 2-fold serial dilution starting from 33 µM) or DMSO, and VCB protein labeled with Bodipy 576/589 (final concentration 0.5 µM) were added separately. Each well was added with NanoBRET NanoGlo Substrate (Promega, N2160) before performing the NanoBRET reading on PHERAstar (BMG LABTECH) plate reader equipped with a 450-nm bandpass filter (donor) and a 600-nm long pass filter (acceptor). NanoBRET ratio of each well was expressed in milliBRET according to the equation: mBRET = [(signal at 610 nM/signal at 450 nM) - (signal at 610 nM no tracer control/signal at 450 nM no tracer control)]×1000. The background signal as shown in the DMSO control samples was subtracted from each sample.

### Chemistry- Materials and Methods

Chemicals that are commercially available were purchased from Apollo Scientific, Sigma-Aldrich, Fluorochem, and Enamine and were used without further purification. All solvents use for reactions are anhydrous. LC-MS was carried out on Shimadzu HPLC/MS 2020 equipped with a Hypersil Gold column (1.9 µm 50 × 2.1 mm), photodiode array detector and ESI detector. The samples were eluted with a 3 min gradient of 5-95% acetonitrile in water containing 0.1% formic acid at a flow rate of 0.8 mL/min. Flash column chromatography was performed on Teledyne ISCO Combiflash Companion installed with disposable normal phase RediSep Rf columns (230-400 mesh, 40-63 mm; SiliCycle). Preparative HPLC purification was performed on Gilson Preparative HPLC system equipped with a Waters X-Bridge C18 column (100 mm × 19 mm and 5 µm particle size) using a gradient from 5 to 95% of acetonitrile in water containing 0.1% formic acid over 10 min at a flow rate of 25 mL/min. Compound characterization using NMR was performed either on a Bruker 500 Ultrashield or on a Bruker Ascend 400 spectrometer. The ¹H NMR and ¹³C NMR reference solvents used are *d*₁-CDCl₃ (δH = 7.26 ppm/δC = 77.16 ppm), *d₄*-CD₃OD (δH = 3.31 ppm/δC = 49.00 ppm) or *d₆-DMSO* (δH = 2.50 ppm/δC = 39.52 ppm) . Signal patterns are described as singlet (s), doublet (d), triplet (t), quartet (q), quintet (quint.), multiplet (m), broad (br), or a combination of the listed splitting patterns. The coupling constants (J) are measured in hertz (Hz). HRMS was performed on a Bruker MicroTOF II focus ESI Mass Spectrometer connected in parallel to a Dionex Ultimate 3000 RSLC system with diode array detector and a Waters XBridge C18 column (50 mm × 2.1, 3.5 µm particle size). All final compounds are >95% pure by HPLC.

### Synthesis

***General procedure 1** (Boc protection)* To a solution of benzyl amine compound (1 eq) and TEA (2 eq) in DCM was added Boc₂O (1.1 eq) at 0 °C, the resulting mixture was stirred at room temperature for 4h or overnight. DCM was added and the mixture was washed with 1N HCl aqueous, saturated NaHCO₃ aqueous, water and brine separately. The collected organic phase was then dried over sodium sulfate, filtered, and condensed under vacuum to afford the Boc protected compounds.

***General procedure 2** (Pd(OAc)₂ mediated coupling)* Under nitrogen protection, Pd(OAc)₂ (0.1 eq) was added to a mixture of bromide compound (1 eq), KOAc (2 eq), and 4-methylthiazole (2 eq) in DMA. The resulting mixture was heated to 110 °C and stirred at this temperature for overnight. The reaction mixture was then diluted with EtOAc and filtered through a pad of celite. The collected filtrate was washed with water and brine, dried over sodium sulfate, filtered, and condensed under vacuum to afford a residue which was purified by flash column chromatography on silica (0% -100% EtOAc in heptane) to afford the desired compounds.

***General procedure 3** (Boc deprotection and condensation with hydroxyl proline)* To a solution of Boc protected (1 eq) compound in DCM (same volume as 4N HCl in dioxane) was added 4N HCl in dioxane (8 eq). The resulting mixture was stirred at room temperature for 1h and then condensed to give a solid which was washed with diethyl ether and dried under vacuum to afford a HCl salt. The salt was suspended in DCM, followed by the addition of TEA (4 eq), (2*S*,4*R*)-1-(*tert*-Butoxycarbonyl)-4-hydroxypyrrolidine-2-carboxylic acid (1 eq) or (2*S*,4*S*)-1-(*tert*-butoxycarbonyl)-4-hydroxypyrrolidine-2-carboxylic acid (1 eq), and HATU (1.05 eq). After stirring at room temperature for 2h, the resulting mixture was diluted with DCM, washed with water and brine, dried over sodium sulfate, filtered, and condensed to give a residue which was purified by flash column chromatography on silica (0% -100% EtOAc in heptane) to afford the desired compounds.

***General procedure 4** (Boc deprotection and condensation with Fmoc-S-trityl-L-penicillamine)* To a stirring solution of Boc protected substrate (1 eq) in DCM was added 4N HCl in dioxane (same volume as DCM, 10 eq). After stirring at room temperature for 1h, the mixture was condensed under vacuum to afford a solid which was washed with diethyl ethyl to give light yellow solid as HCl salt quantitatively. The solid (1 eq) was dissolved in DMF, TEA (1.5 eq) was added to neutralize the HCl, followed by the addition of a mixed solution of Fmoc-*S*-trityl-L-penicillamine (1 eq), HATU (1.1 eq), and TEA (2 eq) in DMF dropwise at 0 °C. The resulting mixture was stirred at 0 °C for 2h then added with EtOAc and water. The water phase was extracted with EtOAc (3×) and the combined organic phase was washed with water, brine, dried over sodium sulfate, filtered and condensed to afford a residue which was purified by flash column chromatography on silica (0%-100% EtOAc in heptane) to afford the desired compound.

***General procedure 5** (Fmoc deprotection)* To a solution of Fmoc protected compound (1 eq) in DCM was added piperidine (5 eq) dropwise. The resulting mixture was stirred at room temperature for 2h then condensed under vacuum to give a residue which was purified by flash column chromatography on silica (0%-10% methanol in DCM with 0.7 M ammonia) to give the desired product.

***General procedure 6** (condensation with 1-fluorocyclopropane-1-carboxylic acid)* To a solution of amine compound (1 eq), TEA (2 eq), and HATU (1.05 eq) in DMF was added 1-fluorocyclopropane-1-carboxylic acid (1 eq). After stirring at room temperature overnight, the mixture was added with water, extracted with EtOAc (3x), and the combined organic phase was washed with water and brine separately, dried over sodium sulfate, filtered and condensed to give a residue which was purified by flash column chromatography on silica (0%-100% EtOAc in PE) to give the desired product.

***General procedure* 7** *(Trityl group deprotection)* To a solution of Trityl protected VHL ligand (1 eq) in DCM was added Triisopropyl silane (TIPS, 10 eq) and trifluoroacetic acid (10 eq) at 0 °C. After stirring at 0 °C for 10 min, the mixture was concentrated under vacuum to give a residue which was purified by flash column chromatography on silica (0%-10% methanol in DCM) to give desired thiol compound.

***General procedure 8*** (Substitution reaction) To a solution of tosylated or bromide compound (1 eq) in THF was added thiol compound (1 eq) and DBU (6 eq). After stirring at room temperature overnight, the reaction mixture was condensed to afford a residue which was purified on preparative HPLC under acidic condition (5-95 % CH₃CN in 0.1 % aq. HCO₂H) to give desired compound.

### Exemplary synthesis of Exemplary compound 1 (SD13)

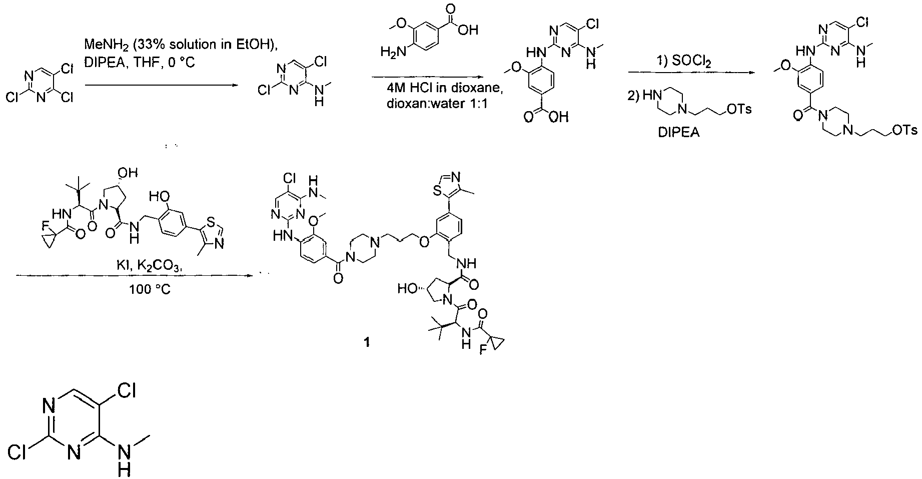

### 2,5-dichloro-N-methylpyrimidin-4-amine

To a stirred solution of 2,4,5-trichloropyrimide (5.8 g, 31.62 mmol) in anhydrous THF (100 mL) was added DIPEA (6.05 ml, 34.78 mmol), followed by methylamine (33% solution in ethanol, 4.33 mL, 34.78 mmol) dropwise at 0°C. The reaction mixture was stirred at 0°C for 1 hour, then warmed to room temperature (RT) and concentrated under reduced pressure. The crude residue was purified by flash column chromatography on silica gel (0%-50% EtOAc in Petroleum spirit) to give 2,5-dichloro-N-methylpyrimidin-4-amine (5.26 g, 29.54mmol, 93% yield) as white solid. ¹H NMR (500 MHz, CDCl3) δ 8.00 (s, 1H), 5.55 (s, 1H), 3.10 (d, *J* = 5.0 Hz, 3H); ¹³C NMR (126 MHz, CDCl3) δ 159.58, 158.77, 153.37, 113.49, 28.28; and LC-MS, ESI⁺, *m*/*z* 178 [M+H]⁺. 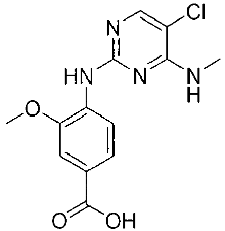 *4-((5-chloro-4-(methylamino)pyrimidin-2-yl)amino)-3-methoxybenzoic acid*

To a solution of 2,5-dichloro-N-methylpyrimidin-4-amine (4.39g, 24.65 mmol) in a mixture of dioxane and water (70 ml :70 ml) was added 4-amino-3-methoxybenzoic acid (4.13 g, 24.70 mmol) followed by 4N solution of HCl in dioxane (6.18 ml, 24.72 mmol) at room temperature. After refluxing the reaction mixture at 100°C overnight, the mixture was cooled down to precipitate white solid. The solids were filtered, washed with water, dried under vacuum to afford *4-((5-chloro-4-(methylamino)pyrimidin-2-yl)amino)-3-methoxybenzoic acid* as white solid (5.95 g, 19.32 mmol, 78% yield). ¹H NMR (400 MHz, DMSO-*d*₆) δ 12.64 (br s, 1H), 8.50 (d, J = 8.2 Hz, 1H), 8.02 (s, 1H), 7.93 (s, 1H), 7.59 (d, J = 8.2 Hz, 1H), 7.51 (s,1H), 7.47(m, 1H), 3.95 (s, 3H), 2.93 (d, J = 4.3 Hz, 3H). LC-MS, ESI⁺, m/z 309.08 [M+H]⁺. 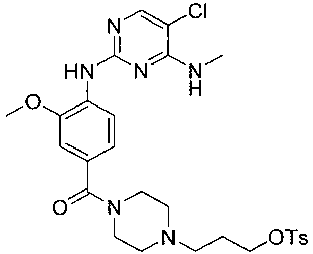 *3-(4-(4-((5-chloro-4-(methylamino)pyrimidin-2-yl)amino)-3-methoxybenzoyl)piperazin-1-yl)propyl 4-methylbenzenesulfonate*

A mixture of 4-((5-chloro-4-(methylamino)pyrimidin-2-yl)amino)-3-methoxybenzoic acid (220 mg, 0.7 mmol) and SOCl₂ (2 ml) was heated at 100 °C for 1h then cooled down and condensed to afford a residue which was dissolved in 2 ml DMF. 1.26 ml of the DMF solution was mixed with 3-(piperazin-1-yl)propyl 4-methylbenzenesulfonate (125 mg, 0.42 mmol) and DIPEA (0.22 ml, 1.26 mmol). The resulting mixture was stirred at room temperature overnight. After standard work up, *3-(4-(4-((5-chloro-4-(methylamino)pyrimidin-2-yl)amino)-3-methoxybenzoyl)piperazin-1-yl)propyl 4-methylbenzenesulfonate* was obtained and used to next step without further purification. 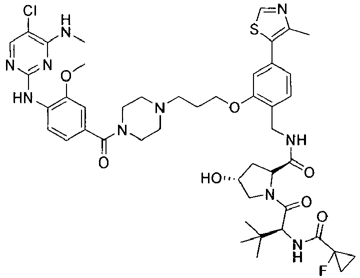 *(2R,4R)-N-(2-(3-(4-(4-((5-chloro-4-(methylamino)pyrimidin-2-yl)amino)-3-methoxybenzoyl)piperazin-1-yl)propoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (1, SD13)*

To a solution of 3-(4-(4-((5-chloro-4-(methylamino)pyrimidin-2-yl)amino)-3-methoxybenzoyl)piperazin-1-yl)propyl 4-methylbenzenesulfonate (7.36 mg, 0.0125 mmol) and (2*R*,4*R*)-1-((5)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (6.65 mg, 0.0125 mmol) (Farnaby et al., 2019) in DMF (0.2 ml) was added K₂CO₃ (5 mg, 0.036 mmol) and KI (0.2 mg, 0.0125 mmol). After stirring at 100 °C overnight, the resulting mixture was cooled down, filtered through 0.45 µm PTFE filter and purified with preparative HPLC under acidic condition (5-95 % CH₃CN in 0.1 % aq. HCO₂H) to give SD13 (2.95 mg, 25% yield). ¹H NMR (500 MHz, MeOD-*d*₄) δ 8.95 (s, 1H), 8.67 (d, *J* = 8.3 Hz, 1H), 7.95 (s, 1H), 7.55 (d, J = 7.7 Hz, 1H), 7.17 - 7.06 (m, 4H), 4.81 (s, 1H), 4.70 (t, *J* = 8.3 Hz, 1H), 4.59 (s, 1H), 4.52 (d, *J* = 2.5 Hz, 2H), 4.25 (t, J = 5.9 Hz, 2H), 4.06 (s, 3H), 3.93 (d, J = 11.1 Hz, 1H), 3.87 (dd, J = 11.0, 3.8 Hz, 1H), 3.83 - 3.66 (m, 4H), 3.14 (s, 3H), 2.79 (t, *J =* 7.3 Hz, 2H), 2.70 (s, 4H), 2.58 (s, 3H), 2.34 - 2.25 (m, 1H), 2.25 - 2.15 (m, 3H), 1.45 - 1.34 (m, 4H), 1.10 (s, 9H). HRMS (ESI⁺) *m*/*z,* calcd for C₄₆H₅₈CIFN₁₀O₇S:949.3961 [M + H]⁺, found 949.3944.

### Exemplary synthesis of Exemplary compound 2 (SD12)

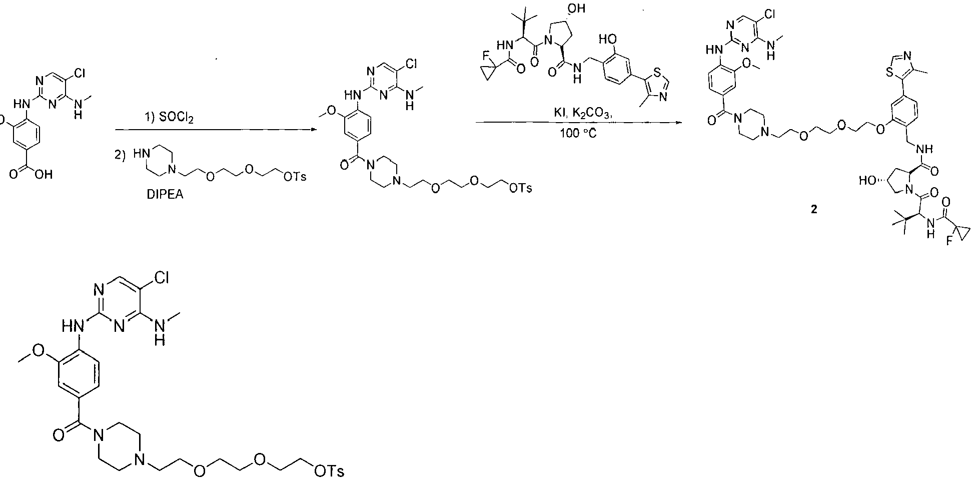

*2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate*

A mixture of 4-((5-chloro-4-(methylamino)pyrimidin-2-yl)amino)-3-methoxybenzoic acid (220 mg, 0.7 mmol) and SOCl₂ (2 ml) was heated at 100 °C for 1h then cooled down and condensed to afford a residue which was dissolved in 2 ml DMF. 0.96 ml of the DMF solution was mixed with 2-(2-(2-(piperazin-1-yl)ethoxy)ethoxy)ethyl 4-methylbenzenesulfonate (119 mg, 0.32 mmol) and DIPEA (0.18 ml, 1.0 mmol). The resulting mixture was stirred at room temperature overnight. After standard work up, *2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate* (167 mg, 36% yield for two steps) was obtained. ¹H NMR (400 MHz, CDCl3) δ 8.51 (d, J = 8.7 Hz, 1H), 7.87 (s, 1H), 7.78 - 7.71 (m, 2H), 7.59 (s, 1H), 7.29 (d, J = 8.0 Hz, 2H), 7.01 - 6.92 (m, 2H), 5.37 (q, J = 4.6 Hz, 1H), 4.14 -4.09 (m, 2H), 3.88 (s, 3H), 3.74 - 3.48 (m, 12H), 3.05 (d, J = 4.9 Hz, 3H), 2.57 (t, J = 5.7 Hz, 2H), 2.48 (s, 4H), 2.39 (s, 3H). ¹³C NMR (101 MHz, CDCl₃) δ 170.43, 158.57, 157.81, 152.62, 147.38, 144.82, 133.09, 131.34, 129.84, 127.94, 127.87, 120.19, 116.76, 109.57, 105.51, 70.81, 70.33, 69.24, 68.98, 68.72, 57.74, 55.88, 53.67, 28.08, 21.62. LC-MS, ESI⁺, m/z 663.3 [M+H]⁺. 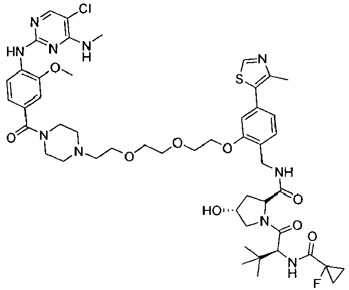 *(2R,4R)-N-(2-(2-(2-(2-(4-(4-((5-chloro-4-(methylamino)pyrimidin-2-yl)amino)-3-methoxybenzoyl)piperazin-1-yl)ethoxy)ethoxy)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (2, SD12)*

To a solution of 2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate (8.29 mg, 0.0125 mmol) and (2*R*,4*R*)-1-((*S*)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (6.65 mg, 0.0125 mmol) in DMF (0.2 ml) was added K₂CO₃ (5 mg, 0.036 mmol) and KI (0.2 mg, 0.0125 mmol). After stirring at 100 °C overnight, the resulting mixture was cooled down, filtered through 0.45 µm PTFE filter, and purified with preparative HPLC under acidic condition (5-95 % CH₃CN in 0.1 % aq. HCO₂H) to give SD12 (3.54 mg, 28% yield). ¹H NMR (500 MHz, MeOD-*d*₄) δ 8.93 (s, 1H), 8.64 (d, J = 8.3 Hz, 1H), 7.94 (s, 1H), 7.52 (d, *J* = 7.8 Hz, 1H), 7.12 (d, J = 1.4 Hz, 2H), 7.08 (ddd, *J* = 7.7, 4.0, 1.6 Hz, 2H), 4.82 (s, 1H), 4.71 (t, J = 8.3 Hz, 1H), 4.57 (dd, J = 4.1, 2.1 Hz, 1H), 4.56 - 4.44 (m, 2H), 4.37 - 4.24 (m, 2H), 4.04 (s, 3H), 3.99 (t, J = 4.5 Hz, 2H), 3.92 (d, J = 11.1 Hz, 1H), 3.88 (d, J = 3.7 Hz, 1H), 3.86 - 3.82 (m, 2H), 3.79 - 3.67 (m, 8H), 3.13 (s, 3H), 2.77 (t, J = 5.4 Hz, 2H), 2.70 (s, 4H), 2.56 (s, 3H), 2.31 (dd, J = 13.1, 7.7 Hz, 1H), 2.17 (ddd, J = 13.3, 9.0, 4.5 Hz, 1H), 1.45 - 1.36 (m, 4H), 1.11 (s, 9H). HRMS (ESI⁺) *m*/*z,* calcd for C₄₉H₆₄ClFN₁₀O₉S:1023.4329 [M + H]⁺, found 1023.4282.

### Exemplary synthesis of Exemplary compound 3 (SD79)

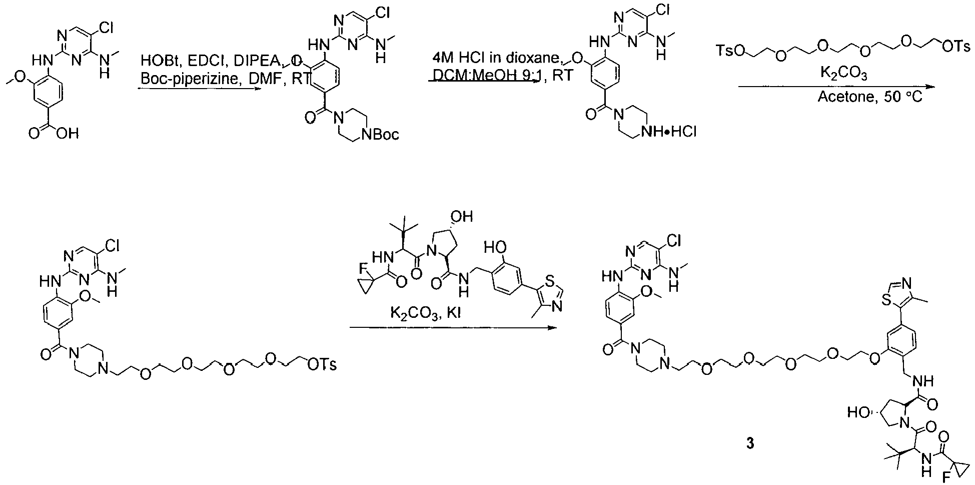

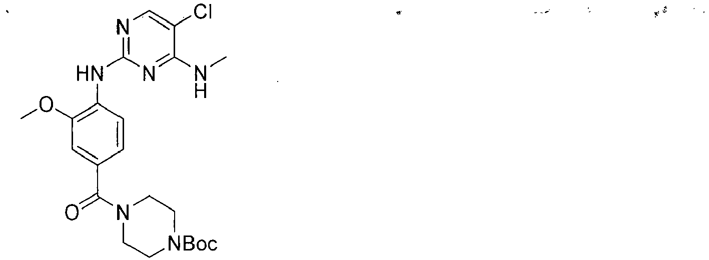

tert-butyl 4-(4-((5-chloro-4-(methylamino)pyrimidin-2-yl)amino)-3-methoxybenzoyl)piperazine-1-carboxylate

To a solution of 4-((5-chloro-4-(methylamino)pyrimidin-2-yl)amino)-3-methoxybenzoic acid (2.1 g, 6.08 mmol) in DMF (25 mL) was added HOBt (0.98 g, 7.29 mmol), EDCl (1.39 g, 7.29 mmol), 1-Boc-piperazine (1.19, 6.38 mmol), and DIPEA (4.23 mL, 24.33 mmol) separately at room temperature. The mixture was stirred at room temperature for 16 h, then diluted with water (50 mL) and extracted with EtOAc (200 mL). The organic layer was washed with brine (25 mL) and concentrated to give a residue which was purified by flash column chromatography on silica gel (0% to 100% of EtOAc in DCM) to give *tert*-butyl 4-(4-((5-chloro-4-(methylamino)pyrimidin-2-yl)amino)-3-methoxybenzoyl)piperazine-1-carboxylate as white solid (2.52 g, 5.28 mmol, 87%). ¹H NMR (500 MHz, CDCl3) δ8.58 (d, *J* = 8.05 Hz, 1H), 7.94 (s, 1H), 7.65 (s, 1H), 7.02 (m, 2H), 5.34 (m, 1H), 3.95 (s, 3H), 3.64 (br, s, 4H), 3.48 (br, s, 4H), 3.13 (d, J=4.8 Hz, 3H), 1.49 (s, 9H). ¹³C NMR (126 MHz, CDCl3) δ 170.93, 158.65, 157.84, 154.74, 152.73, 147.53, 131.69, 127.42, 120.28, 116.81, 109.61, 105.69, 80.42, 55.96, 44.06, 28.49, 28.21. LC-MS, ESI⁺, m/z 477.20 [M+H]⁺. 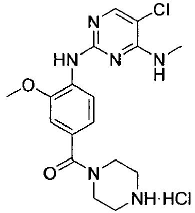 (4-((5-chloro-4-(methylamino)pyrimidin-2-yl)amino)-3-methoxyphenyl)(piperazin-1-yl)methanone HCl salt

To a solution of *tert*-butyl 4-(4-((5-chloro-4-(methylamino)pyrimidin-2-yl)amino)-3-methoxybenzoyl)piperazine-1-carboxylate (2.52 g, 5.28 mmol) in a mixture of DCM and MeOH 9:1 (30 ml) was added 4M solution of HCl in dioxane (5.28 ml, 21.12 mmol) at RT. Reaction mixture stirred overnight, diluted with Et₂O (200 ml), precipitate collected on glass filter, washed with Et₂O (100 ml) and dried overnight, (4-((5-chloro-4-(methylamino)pyrimidin-2-yl)amino)-3-methoxyphenyl)(piperazin-1-yl)methanone was obtained (2.13 g, 5.17 mmol, 98%) as a HCl salt. ¹H NMR (500 MHz, DMSO-*d*₆) δ 9.65 (s, 2H), 8.72 (s, 1H), 8.28 (s, 1H), 8.13 (d, *J* = 8.20 Hz, 1H), 7.20 (d, *J* = 1.70 Hz, 1H), 7.11 (dd, *J₁* = 1.70 Hz, *J₂* = 8.20 Hz,1H), 3.91 (s, 3H), 3.75 (br s, 4H), 3.15 (br s, 4H), 2.99 (d, *J* = 4.6 Hz, 3H). ¹³C NMR (126 MHz, DMSO-*d₆*) δ 169.28, 159.06, 152.3, 150.3, 127.80, 122.0, 120.02, 111.07, 105.06, 65.34, 56.67, 43.0, 29.0. LC-MS, ESI⁺, m/z 377.15 [M+H]⁺. 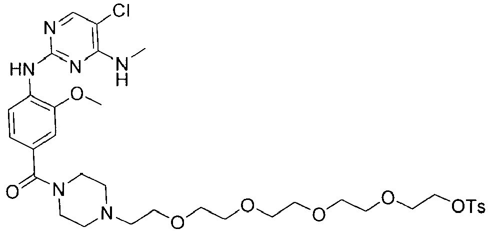 *14-(4-(4-((5-chloro-4-(methylamino)pyrimidin-2-yl)amino)-3-methoxybenzoyl)piperazin-1-yl)-3,6,9,12-tetraoxatetradecyl 4-methylbenzenesulfonate*

To a solution of (4-((5-chloro-4-(methylamino)pyrimidin-2-yl)amino)-3-methoxyphenyl)(piperazin-1-yl)methanone (40 mg, 0.097 mmol) in acetone (3 mL) was added pentaethylene glycol di(p-toluenesulfonate (159.3 mg, 0.292 mmol) and K₂CO₃ (67 mg, 0.49 mmol). After stirring at 50 °C overnight, the mixture was diluted with DCM, washed with water and brine, dried over sodium sulfate, filtered and condensed to afford a crude product which was purified via flash column chromatography on silica gel (0%-10% methanol in DCM) to give *14-(4-(4-((5-chloro-4-(methylamino)pyrimidin-2-yl)amino)-3-methoxybenzoyl)piperazin-1-yl)-3,6,9,12-tetraoxatetradecyl 4-methylbenzenesulfonate* (30 mg, 41% yield) as white solid. ¹H NMR (400 MHz, CDCl₃) δ 8.53 (d, *J* = 8.7 Hz, 1H), 7.90 (s, 1H), 7.78 (d, J = 8.2 Hz, 2H), 7.61 (s, 1H), 7.32 (d, J = 8.0 Hz, 2H), 7.02 - 6.96 (d, J = 7.0 Hz, 2H), 5.32 (q, J = 4.3 Hz, 1H), 4.19 - 4.11 (m, 2H), 3.91 (s, 3H), 3.77 - 3.50 (m, 20H), 3.09 (d, J = 4.8 Hz, 3H), 2.61 (t, J = 5.5 Hz, 2H), 2.52 (s, 4H), 2.43 (s, 3H); ¹³C NMR (101 MHz, CDCl₃) δ 170.53, 158.67, 157.92, 152.75, 147.50, 144.86, 133.24, 131.43, 129.92, 128.07, 127.99, 120.26, 116.85, 109.68, 105.61, 70.87, 70.76, 70.71, 70.65, 70.52, 69.32, 69.03, 68.81, 57.84, 55.98, 53.77, 28.18, 21.72. LC-MS, ESI⁻, m/z 749 [M-H]⁻. 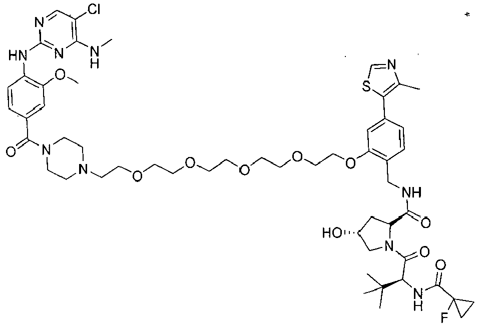 *(2R,4R)-N-(2-((14-(4-(4-((5-chloro-4-(methylamino)pyrimidin-2-yl)amino)-3-methoxybenzoy*/*)piperazin-1-yl)-3,6,9,12-tetraoxatetradecyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide **(3, SD79)***

To a solution of 14-(4-(4-((5-chloro-4-(methylamino)pyrimidin-2-yl)amino)-3-methoxybenzoyl)piperazin-1-yl)-3,6,9,12-tetraoxatetradecyl 4-methylbenzenesulfonate (9.39 mg, 0.0125 mmol) and (2*R*,4*R*)-1-((*S*)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (6.65 mg, 0.0125 mmol) in DMF (0.2 ml) was added K₂CO₃ (5 mg, 0.036 mmol) and KI (0.2 mg, 0.0125 mmol). After stirring at 100 °C overnight, the resulting mixture was cooled down, filtered through 0.45 µm PTFE filter, and purified with preparative HPLC under acidic condition (5-95 % CH₃CN in 0.1 % aq. HCO₂H) to give SD79 (2.24 mg, 16% yield). ¹H NMR (500 MHz, MeOD-*d*₄) δ 8.88 (s, 1H), 8.60 (d, *J* = 8.3 Hz, 1H), 7.86 (s, 1H), 7.48 (d, *J* = 7.6 Hz, 1H), 7.10 - 7.00 (m, 4H), 4.76 (s, 1H), 4.64 (t, J = 8.3 Hz, 1H), 4.54-4.38 (m, 3H), 4.26 -4.20 (m, 2H), 3.98 (s, 3H), 3.93 - 3.84 (m, 4H), 3.83 - 3.79 (m, 1H), 3.75 - 3.69 (m, 7H), 3.66 - 3.62 (m, 10H), 3.05 (s, 3H), 2.87 - 2.75 (m, 6H), 2.50 (s, 3H), 2.28 - 2.22 (m, 1H), 2.15 - 2.07 (m, 1H), 1.40 -1.30 (m, 4H), 1.05 (s, 9H). HRMS (ESI⁺) *m*/*z,* calcd for C₅₃H₇₂ClFN₁₀O₁₁S: 556.2460 [M + 2H]²⁺, found 556.2469.

### Exemplary synthesis of Exemplary compound 4 (SD82)

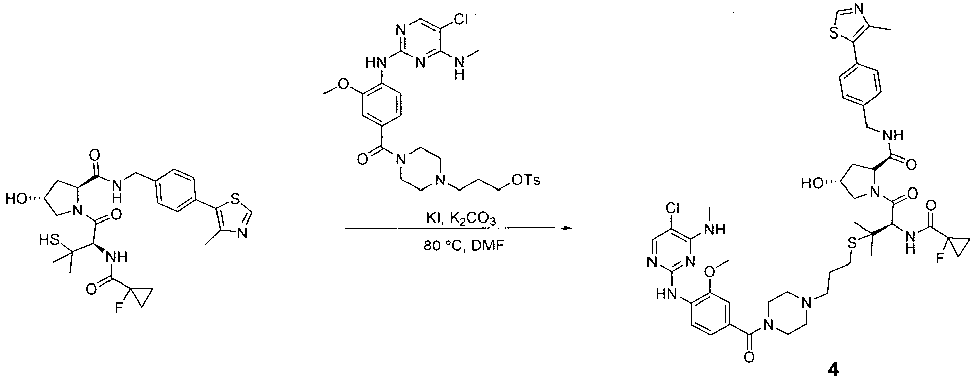

*(2R,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropane-1-corboxamido)-3-methylbutanoyl)-4-hydroxy-N-(4-(4-methylthiazol-5-yl)benzyl)pyrrolidine-2-corboxamide **(4, SD82)***

To a solution of 3-(4-(4-((5-chloro-4-(methylamino)pyrimidin-2-yl)amino)-3-methoxybenzoyl)piperazin-1-yl)propyl 4-methylbenzenesulfonate (7.36 mg, 0.0125 mmol) and (2*R*,4*R*)-1-((*R*)-2-(1-fluorocyclopropane-1-carboxamido)-3-mercapto-3-methylbutanoyl)-4-hydroxy-N-(4-(4-methylthiazol-5-yl)benzyl)pyrrolidine-2-carboxamide (Girardini et al., Bioorg. Med. Chem. 2019; 27(12):2466-2479.) (6.675 mg, 0.0125 mmol) in DMF (0.2 ml) was added K₂CO₃ (5 mg, 0.036 mmol) and KI (0.2 mg, 0.0125 mmol). After stirring at 80 °C overnight, the resulting mixture was cooled down and purified with preparative HPLC under acidic condition (10-80 % CH₃CN in 0.1 % aq. HCO₂H) to give SD82 (3.01 mg, 25% yield). ¹H NMR (500 MHz, MeOD-*d*₄) δ 8.87 (s, 1H), 8.60 (d, *J* = 8.3 Hz, 1H), 7.88 (s, 1H), 7.51 - 7.39 (m, 4H), 7.10 - 7.00 (m, 2H), 4.94 (s, 1H), 4.63 (t, J = 8.4 Hz, 1H), 4.58 (d, J = 15.6 Hz, 1H), 4.53 (s, 1H), 4.37 (d, *J* = 15.7 Hz, 1H), 3.98 (s, 3H), 3.94 - 3.86 (m, 2H), 3.78 - 3.62 (m, 4H), 3.06 (s, 3H), 2.76 - 2.55 (m, 8H), 2.49 (s, 3H), 2.29 (dd, J = 12.9, 7.8 Hz, 1H), 2.12 (ddd, J = 13.3, 9.2, 4.4 Hz, 1H), 1.81 - 1.72 (m, 2H), 1.46 (d, J = 10.8 Hz, 6H), 1.40 - 1.28 (m, 4H). HRMS (ESI⁺) *m*/*z,* calcd for C₄₅H₅₆ClFN₁₀O₆S₂: 951.3571 [M + H]⁺, found 951.3565.

### Exemplary synthesis of Exemplary compound 5 (SD75)

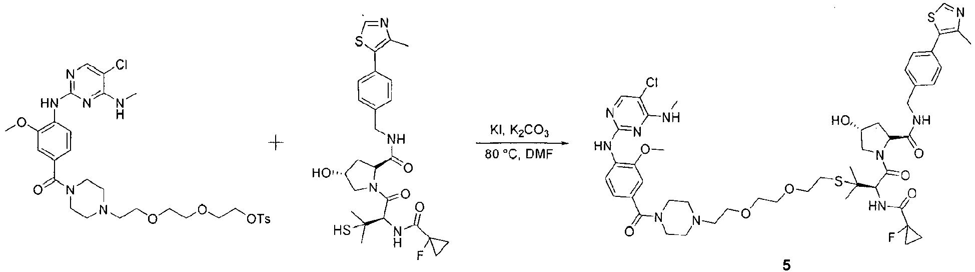

*(2R,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropone-1-carboxomido)-3-methylbutanoyl)-4-hydroxy-N-(4-(4-methylthiazol-5-yl)benzyl)pyrrolidine-2-carboxamide* (5, **SD75)**

To a solution of 2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate (8.29 mg, 0.0125 mmol) and (2*R*,4*R*)-1-((*R*)-2-(1-fluorocyclopropane-1-carboxamido)-3-mercapto-3-methylbutanoyl)-4-hydroxy-N-(4-(4-methylthiazol-5-yl)benzyl)pyrrolidine-2-carboxamide (6.68 mg, 0.0125 mmol) in DMF (0.2 ml) was added K₂CO₃ (5 mg, 0.036 mmol) and KI (0.2 mg, 0.0125 mmol). After stirring at 80 °C overnight, the resulting mixture was cooled down and purified with preparative HPLC under acidic condition (10-95 % CH₃CN in 0.1 % aq. HCO₂H) to give SD75 (2.12 mg, 17% yield). ¹H NMR (500 MHz, MeOD-*d*₄) δ 8.85 (s, 1H), 8.57 (d, J = 8.3 Hz, 1H), 7.84 (s, 1H), 7.45 - 7.36 (m, 4H), 7.06 - 6.99 (m, 2H), 4.91 (s, 1H), 4.58 (t, J = 8.4 Hz, 1H), 4.53 - 4.45 (m, 2H), 4.35 (d, J = 15.5 Hz, 1H), 3.96 - 3.92 (m, 3H), 3.87 (d, J = 2.6 Hz, 2H), 3.62 (t, J = 5.4 Hz, 3H), 3.59 - 3.50 (m, 6H), 3.34 (s, 3H), 3.03 (s, 3H), 2.76 (ddd, J = 12.7, 8.0, 4.9 Hz, 2H), 2.67 - 2.53 (m, 6H), 2.46 (s, 3H), 2.25 (ddd, J = 12.9, 7.7, 1.8 Hz, 1H), 2.08 (ddd, J = 13.3, 9.1, 4.5 Hz, 1H), 1.39 (d, *J* = 10.1 Hz, 6H), 1.36 -1.22 (m, 4H). HRMS (ESI⁺) *m*/*z,* calcd for C₄₈H₆₂ClFN₁₀O₈S₂:1025.3944 [M + H]⁺, found 1025.3915.

### Exemplary synthesis of Exemplary compound 6 (SD74)

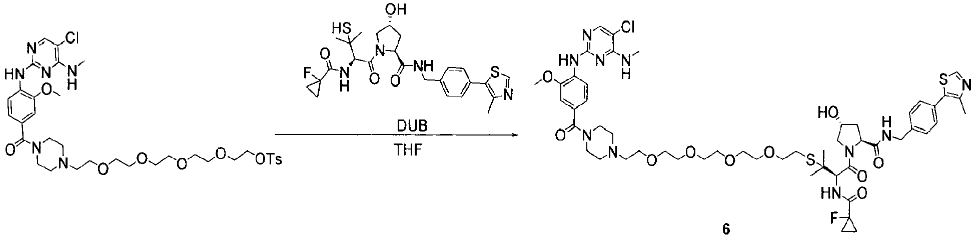

*(2S,4R)-1-((R)-1-(4-(4-((5-chloro-4-(methylamino)pyrimidin-2-yl)omino)-3-methoxybenzoyl)piperazin-1-yl)-17-(1-fluorocyclopropane-1-corboxamido)-16,16-dimethyl-3,6,9,12-tetraoxa-15-thiaoctadecon-18-oyl)-4-hydroxy-N-(4-(4-methylthiazol-5-yl)benzyl)pyrrolidine-2-carboxamide (6, SD74)*

SD74 was prepared following general procedure 8. 7.55 mg SD74 was obtained from 7.0 mg thiol compound, 51% yield. ¹H NMR (400 MHz, CDCl3) δ 8.67 (s, 1H), 8.54 (d, J = 8.7 Hz, 1H), 7.92 (s, 1H), 7.65 (s, 1H), 7.42 - 7.30 (m, 5H), 7.19 (d, J = 7.8 Hz, 1H), 7.03 - 6.96 (m, 2H), 5.39 - 5.27 (m, 1H), 4.85 (d, J = 8.2 Hz, 1H), 4.76 (t, J = 7.7 Hz, 1H), 4.59 - 4.33 (m, 3H), 3.92 (s, 3H), 3.91 - 3.82 (m, 2H), 3.82 - 3.64 (m, 5H), 3.65 - 3.44 (m, 15H), 3.10 (d, J = 4.8 Hz, 3H), 2.85 - 2.05 (m, 8H), 2.51 (s, 3H), 2.46 - 2.39 (m, 1H), 2.25 - 2.15 (m, 1H), 1.39 - 1.20 (m, 10H); ¹³C NMR (101 MHz, CDCl3) δ 170.98, 170.66, 170.25 (d, J = 20.48 Hz), 169.96, 158.74, 157.91, 152.74, 150.39, 148.65, 147.61, 138.31, 131.69, 131.10, 129.62, 129.08, 128.33, 126.03, 120.40, 116.97, 109.76, 105.72, 79.43, 70.72, 70.68, 70.63, 70.55, 70.25, 69.88, 68.36, 59.35, 57.69, 56.51, 56.06, 55.94, 53.61, 48.10, 43.19, 36.91, 28.72, 28.25, 25.97, 25.19, 21.48, 16.22, 14.00, 13.91; HRMS (ESI⁺) *m*/*z,* calcd for C₅₂H₇₀ClFN₁₀O₁₀S₂ : 1113.4463 [M + H]⁺, found 1113.4220.

### Exemplary synthesis of Exemplary compound 7 (SD100)

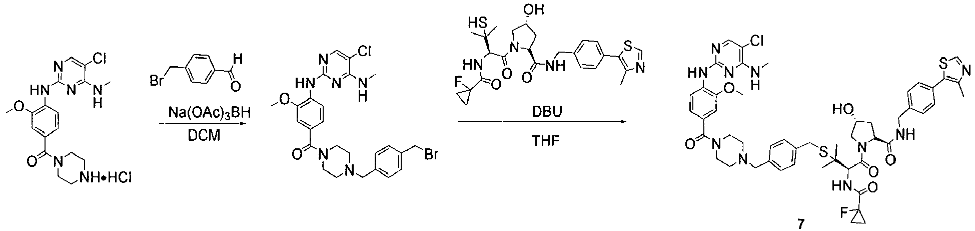

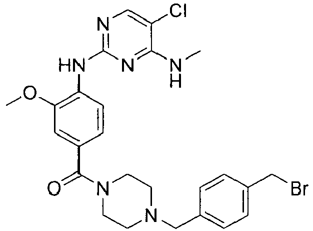

*(4-(4-(bromomethyl)benzyl)piperazin-1-yl)(4-((5-chloro-4-(methylamino)pyrimidin-2-yl)omino)-3-methoxyphenyl)methanone*

To a suspension of (4-((5-chloro-4-(methylamino)pyrimidin-2-yl)amino)-3-methoxyphenyl)(piperazin-1-yl)methanone HCl salt (80 mg, 0.19 mmol) and 4-(bromomethyl)benzaldehyde (38.5 mg, 0.19 mmol) in DCM ( 3 ml) was added Na(OAc)₃BH (123 mg, 0.58 mmol). After stirring at room temperature overnight, the mixture was diluted with DCM and washed with water and brine, dried over sodium sulfate, filtered and condensed to give a residue which was purified through flash column chromatography on silica gel (0% -10% methanol in DCM) to afford (4-(4-*(bromomethyl)benzyl)piperazin-1-yl)(4-((5-chloro-4-(methylamino)pyrimidin-2-yl)amino)-3-methoxyphenyl)methanone* (70 mg, 65% yield) as white solid. ¹H NMR (400 MHz, CDCl₃) δ 8.55 - 8.47 (m, 1H), 7.88 (s, 1H), 7.62 (s, 1H), 7.35 - 7.25 (m, 4H), 7.0 - 6.95 (m, 2H), 5.46 - 5.30 (m, 1H), 4.46 (s, 2H), 3.89 (s, 3H), 3.64 (s, 4H), 3.50 (s, 2H), 3.06 (d, *J* = 4.9 Hz, 3H), 2.44 (s, 4H). ¹³C NMR (100 MHz, CDCl₃) δ 170.50, 158.61, 157.83, 152.63, 147.45, 138.17, 136.87, 131.40, 129.50, 129.10, 127.88, 120.21, 116.83, 109.63, 105.55, 62.51, 55.92, 53.19, 33.37, 28.12, 14.25. LC-MS, ESI⁺, m/z 559 [M+H]⁺, 561 [M+H+2]⁺. 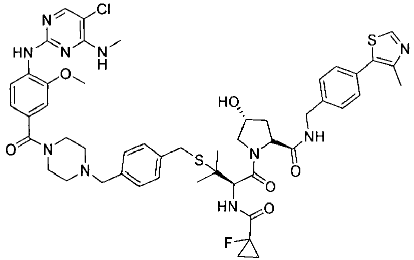 *(2R,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-carboxamido)-3-methylbutanoyl)-4-hydroxy-N-(4-(4-methylthiazol-5-yl)benzyl)pyrrolidine-2-carboxomide (7, SD100)*

To a solution of (4-(4-(bromomethyl)benzyl)piperazin-1-yl)(4-((5-chloro-4-(methylamino)pyrimidin-2-yl)amino)-3-methoxyphenyl)methanone (6 mg, 0.011 mmol) and (2*R*,4*R*)-1-((*R*)-2-(1-fluorocyclopropane-1-carboxamido)-3-mercapto-3-methylbutanoyl)-4-hydroxy-N-(4-(4-methylthiazol-5-yl)benzyl)pyrrolidine-2-carboxamide (6.0 mg, 0.011 mmol) in THF (1 ml) was added DBU (9.6 µl, 0.064 mmol). After stirring at room temperature overnight, the mixture was condensed under reduced pressure to give a residue which was purified through flash column chromatography on silica gel to give **SD100** (4.2 mg, 39% yield) as white solid. ¹H NMR (400 MHz, CDCl₃) δ 8.67 (s, 1H), 8.53 (d, J = 8.8 Hz, 1H), 7.92 (s, 1H), 7.64 (s, 1H), 7.33 (dt, J = 16.5, 5.2 Hz, 4H), 7.25 - 7.19 (m, 6H), 7.02 - 6.98 (m, 2H), 5.32 - 5.24 (m, 1H), 4.73 (t, J = 7.8 Hz, 1H), 4.63 (d, J = 7.5 Hz, 1H), 4.53 - 4.31 (m, 3H), 3.92 (s, 3H), 3.87 (d, J = 11.2 Hz, 1H), 3.81 - 3.54 (m, 7H), 3.48 (s, 2H), 3.10 (d, J = 4.9 Hz, 3H), 2.53 - 2.36 (m, 8H), 2.24 - 2.11 (m, 1H), 1.38 (s, 6H), 1.35 -1.25 (m, 4H). ¹³C NMR (101 MHz, CDCl₃) δ 170.65, 170.45, 170.16, 158.73, 157.96, 152.79, 150.41, 148.69, 147.58, 138.12, 136.98, 136.47, 131.69, 131.51, 131.21, 129.68, 129.08, 128.25, 128.00, 120.33, 116.94, 109.77, 105.67, 79.44, 77.37, 70.19, 62.70, 58.96, 56.51, 56.46, 56.03, 53.34, 48.77, 43.32, 36.59, 33.43, 28.23, 25.81, 25.49, 16.22, 14.06 (d, J = 11.94 Hz), 13.95 (d, J = 10.48 Hz). HRMS (ESI⁺) m/z, calcd for C₅₀H₅₉ClFN₁₀O₆S₂ 1013.3727 [M + H]⁺, found 1013.3849.

### Exemplary synthesis of Exemplary compound 8 (SD11)

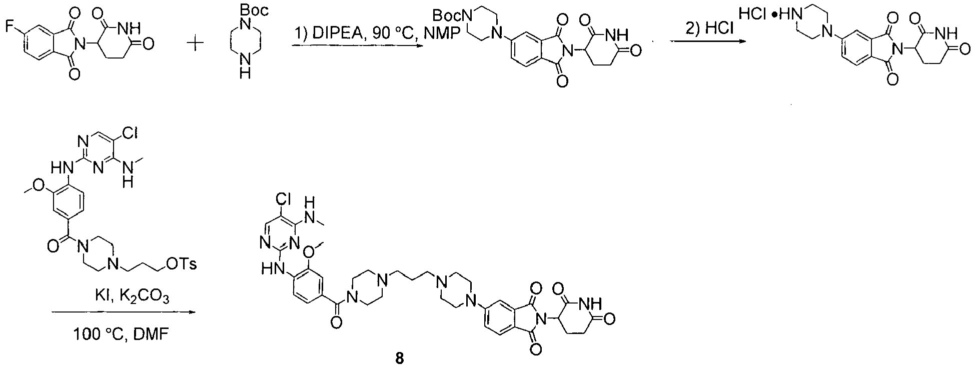

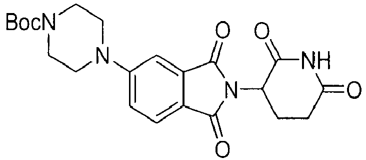

*tert-butyl 4-(2-(2,6-dioxopiperidin-3-yl)1-,3-dioxoisoindolin-5-yl)piperazine-1-carboxylate*

To a solution of 2-(2,6-dioxopiperidin-3-yl)-5-fluoroisoindoline-1,3-dione (644 mg, 2.33 mmol) and Boc-piperidine (868 mg, 4.66 mmol) in NMP (4.5 ml) was added DIPEA (0.324 ml, 1.86 mmol). After stirring at 90 °C overnight, the mixture was cooled down to room temperature, added with water and extracted with EtOAc (2x 20 ml). The combined organic phase was washed with brine, dried over MgSO₄, filtered and condensed under reduced pressure to afford a residue which was purified via flash column chromatography on silica gel (0-50% EtOAc in heptane) to give *tert-butyl 4-(2-(2,6-dioxopiperidin-3-yl)-1,3-dioxoisoindolin-5-yl)piperazine-1-carboxylate* (815 mg, 80% yield) as powder. ¹H NMR (500 MHz, DMSO-*d*₆) δ 11.04 (s, 1H), 7.69 (d, J = 8.5 Hz, 1H), 7.34 (d, J = 2.2 Hz, 1H), 7.24 (dd, J = 8.6, 2.3 Hz, 1H), 5.07 (dd, J = 12.8, 5.4 Hz, 1H), 3.47 (s, 8H), 2.94 - 2.82 (m, 1H), 2.63 - 2.52 (m, 2H), 2.05 - 1.99 (m, 1H), 1.43 (s, 9H). 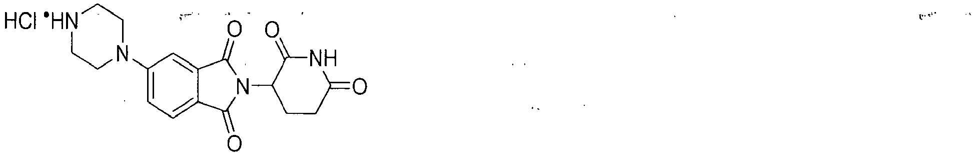 2-(2,6-dioxopiperidin-3-yl)-5-(piperazin-1-yl)isoindoline-1,3-dione, HCl salt

To a solution of *tert*-butyl 4-(2-(2,6-dioxopiperidin-3-yl)-1,3-dioxoisoindolin-5-yl)piperazine-1-carboxylate (50 mg, 0.11 mmol) in DCM (1 ml) was added with 4N HCl (1 ml). The mixture was stirred at room temperature for 1h and then condensed under reduced pressure to give the title compound as a residue which was used to next step without further purification. 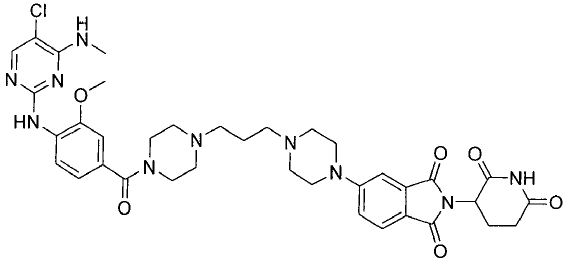 *5-(4-(3-(4-(4-((5-chloro-4-(methylamino)pyrimidin-2-yl)amino)-3-methoxybenzoyl)piperazin-1-yl)propyl)piperazin-1-yl)-2-(2,6-dioxopiperidin-3-yl)isoindoline-1,3-dione (8, SD11)*

To a solution of 3-(4-(4-((5-chloro-4-(methylamino)pyrimidin-2-yl)amino)-3-methoxybenzoyl)piperazin-1-yl)propyl 4-methylbenzenesulfonate (22 mg, 0.0375 mmol) and 2-(2,6-dioxopiperidin-3-yl)-5-(piperazin-1-yl)isoindoline-1,3-dione (14.2 mg, 0.0375 mmol) in DMF (0.4 ml) was added K₂CO₃ (15 mg, 0.11 mmol) and KI (0.6 mg, 0.00375 mmol). After stirring at 100 °C overnight, the resulting mixture was cooled down and purified with preparative HPLC under acidic condition (10-50 % CH₃CN in 0.1 % aq. HCO₂H) to give **SD11** (5.4 mg, 19% yield). ¹H NMR (400 MHz, MeOD-*d*₄) δ 8.60 (d, J = 8.2 Hz, 1H), 7.87 (s, 1H), 7.74 (dd, J = 14.8, 8.5 Hz, 1H), 7.45 (d, J = 21.7 Hz, 1H), 7.32 (dd, *J* = 21.5, 8.5 Hz, 1H), 7.15 - 7.01 (m, 2H), 5.22 - 5.03 (m, 1H), 3.98 (s, 3H), 3.82 - 3.67 (m, 5H),3.63 - 3.57 (m, 3H), 3.42 - 3.37 (m, 1H), 3.36 (s, 3H), 3.06 (s, 3H), 3.01 (s, 2H), 2.95 - 2.82 (m, 3H), 2.79 - 2.69 (m, 6H), 2.17 - 2.08 (m, 1H), 2.00 -1.77 (m, 2H). HRMS (ESI⁺) *m*/*z,* calcd for C₃₇H₄₃ClN₁₀O₆: 759.3128 [M + H]⁺, found 759.3135.

### Exemplary synthesis of Exemplary compound 9 (SD10)

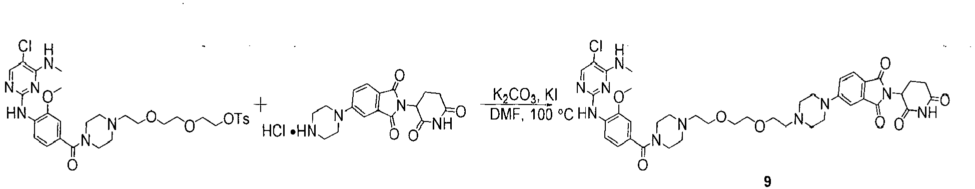

*5-(4-(2-(2-(2-(4-(4-((5-chloro-4-(methylamino)pyrimidin-2-yl)amino)-3-methoxybenzoyl)piperazin-1-yl)ethoxy)ethoxy)ethyl)piperazin-1-yl)-2-(2,6-dioxopiperidin-3-yl)isoindoline-1,3-dione **(9, SD10)***

To a solution of 2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate (24.9 mg, 0.0375 mmol) in DMF (0.5 ml) was added 2-(2,6-dioxopiperidin-3-yl)-5-(piperazin-1-yl)isoindoline-1,3-dione HCl salt (14.2 mg , 0.0375 mmol), K₂CO₃ (15 mg, 0.109 mmol), and KI (0.6 mg, 0.00375 mmol). The resulting mixture was heated to 100 °C and stirred at 100 °C overnight. The mixture was filtered through 0.45 µM filter and purified with preparative HPLC under acidic condition (10-85% CH₃CN in 0.1% aq HCO₂H) to give **SD10** (2.82 mg, 9% yield). ¹H NMR (400 MHz, MeOD-*d*₄) δ 8.63 - 8.49 (m, 1H), 7.84 (d, J = 15.7 Hz, 1H), 7.69 - 7.59 (m, 1H), 7.32 (d, J = 31.5 Hz, 1H), 7.19 (dd, J = 39.4, 8.5 Hz, 1H), 7.04 (dd, J = 18.7, 9.7 Hz, 2H), 5.12 - 5.04 (m, 1H), 3.97 (d, J = 4.2 Hz, 3H), 3.75 - 3.65 (m, 5H), 3.64 - 3.56 (m, 6H), 3.55 - 3.49 (m, 2H), 3.42 - 3.35 (m, 5H), 3.21 - 3.10 (m, 2H), 3.04 (d, J = 3.4 Hz, 3H), 2.96 - 2.81 (m, 2H), 2.71 - 2.58 (m, 10H). HRMS (ESI⁺) *m*/*z,* calcd for C₄₀H₄₉ClN₁₀O₈: 833.3496 [M + H]⁺, found 833.3521.

### Exemplary synthesis of Exemplary compound 10 (SD113)

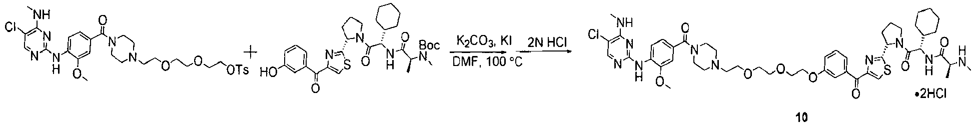

*(S)-N-((S)-2-((S)-2-(4-(3-(2-(2-(2-(4-(4-((5-chloro-4-(methylamino)pyrimidin-2-yl)amino)-3-methoxybenzoyl)piperazin-1-yl)ethoxy)ethoxy)ethoxy)benzoyl)thiazol-2-yl)pyrrolidin-1-yl)-1-cyclohexyl-2-oxoethyl)-2-(methylamino)propenamide HCl salt* **(10, SD113)**

To a solution of 2-(2-(2-(4-(4-((5-chloro-4-(methylamino)pyrimidin-2-yl)amino)-3-methoxybenzoyl)piperazin-1-yl)ethoxy)ethoxy)ethyl 4-methylbenzenesulfonate (8.29 mg, 0.0125 mmol) in DMF (0.2 ml) was added *tert*-butyl ((*S*)-1-(((*S*)-1-cyclohexyl-2-((*S*)-2-(4-(3-hydroxybenzoyl)thiazol-2-yl)pyrrolidin-1-yl)-2-oxoethyl)a mino)-1-oxopropan-2-yl)(methyl)carbamate (7.5 mg, 0.0125 mmol) (Shibata et al., 2018), K₂CO₃ (5 mg, 0.036 mmol), and KI (0.6 mg, 0.00375 mmol). The resulting mixture was heated to 100 °C and stirred at 100 °C overnight. The mixture was filtered through 0.45 µM filter and purified with preparative HPLC under acidic condition (10-85% CH₃CN in 0.1% aq HCO₂H) to give *tert*-butyl ((*S*)-1-(((*S*)-2-((*S*)-2-(4-(3-(2-(2-(2-(4-(4-((5-chloro-4-(methylamino)pyrimidin-2-yl)amino)-3-methoxybenzoyl)piperazin-1-yl)ethoxy)ethoxy)ethoxy)benzoyl)thiazol-2-yl)pyrrolidin-1-yl)-1-cyclohexyl-2-oxoethyl)amino)-1-oxopropan-2-yl)(methyl)carbamate (3 mg, 22% yield); HRMS (ESI⁺) *m*/*z,* calcd for C₅₄H₇₄ClN₁₀O₁₀S 1089.4993 [M + H]⁺, found 1089.5198. To a solution of *tert*-butyl ((*S*)-1-(((*S*)-2-((*S*)-2-(4-(3-(2-(2-(2-(4-(4-((5-chloro-4-(methylamino)pyrimidin-2-yl)amino)-3-methoxybenzoyl)piperazin-1-yl)ethoxy)ethoxy)ethoxy)benzoyl)thiazol-2-yl)pyrrolidin-1-yl)-1-cyclohexyl-2-oxoethyl)amino)-1-oxopropan-2-yl)(methyl)carbamate (3 mg, 0.0028 mmol) in 0.2 ml DCM was added 4N HCl solution in 1,4-dioxane (0.2 ml). The resulting mixture was stirred at room temperature for 2h, then condensed to afford **SD113** as HCl salt. ¹H NMR (500 MHz, DMSO-*d*₆) δ 8.48 (s, 1H), 8.39 (d, *J* = 8.3 Hz, 1H), 8.25 - 8.19 (m, 3H), 7.96 (s, 1H), 7.69 - 7.60 (m, 3H), 7.45 (t, *J* = 8.0 Hz, 1H), 7.34 (q, *J* = 4.3 Hz, 1H), 7.27 - 7.21 (m, 1H), 7.01 (d, J = 1.6 Hz, 1H), 6.96 (dd, J = 8.3, 1.6 Hz, 1H), 5.39 (dd, J = 8.0, 3.0 Hz, 1H), 4.55 - 4.44 (m, 1H), 4.18 - 4.15 (m, 2H), 3.89 (s, 3H), 3.82 - 3.76 (m, 4H), 3.62 - 3.58 (m, 4H), 3.55 - 3.51 (m, *J* = 7.6, 4.5 Hz, 6H), 3.30 (dd, J = 13.7, 6.8 Hz, 1H), 2.90 (d, J = 4.6 Hz, 3H), 2.49 (d, J = 5.9 Hz, 1H), 2.42 (s, 4H), 2.29 (s, 3H), 2.27 - 2.16 (m, 2H), 2.08 - 1.99 (m, 2H), 1.72 - 1.59 (m, 4H), 1.55 (d, *J* = 9.7 Hz, 2H), 1.19 *(d, J* = 6.9 Hz, 3H), 1.15 - 0.93 (m, 6H). HRMS (ESI⁺) *m*/*z,* calcd for C₄₉H₆₅ClN₁₀O₈S: 989.4468 [M + H]⁺, found 989.4537.

### Exemplary synthesis of Exemplary compound 11 (SD112)

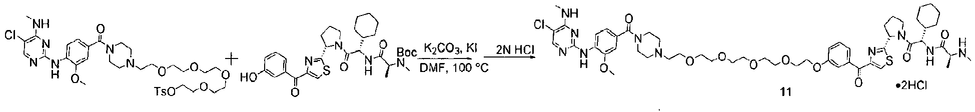

*(S)-N-((S)-2-((S)-2-(4-(3-((14-(4-(4-((5-chloro-4-(methylamino)pyrimidin-2-yl)omino)-3-methoxybenzoyl)piperazin-1-yl)-3,6,9,12-tetraoxatetradecyl)oxy)benzoyl)thiazol-2-yl)pyrrolidin-1-yl)-1-cyclohexyl-2-oxoethyl)-2-(methylamino)propenamide (11, SD112)*

To a solution of 14-(4-(4-((5-chloro-4-(methylamino)pyrimidin-2-yl)amino)-3-methoxybenzoyl)piperazin-1-yl)-3,6,9,12-tetraoxatetradecyl 4-methylbenzenesulfonate (9.4 mg, 0.0125 mmol) in DMF (0.2 ml) was added *tert*-butyl ((*S*)-1-(((*S*)-1-cyclohexyl-2-((*S*)-2-(4-(3-hydroxybenzoyl)thiazol-2-yl)pyrrolidin-1-yl)-2-oxoethyl)amino)-1-oxopropan-2-yl)(methyl)carbamate (7.5 mg, 0.0125 mmol), K₂CO₃ (5 mg, 0.036 mmol), and KI (0.6 mg, 0.00375 mmol). The resulting mixture was heated to 100 °C and stirred at 100 °C overnight. The mixture was filtered through 0.45 µM filter and purified with preparative HPLC under acidic condition (10-85% CH₃CN in 0.1% aq HCO₂H) to give tert-butyl ((*S*)-1-(((*S*)-2-((*S*)-2-(4-(3-(2-(2-(2-(4-(4-((5-chloro-4-(methylamino)pyrimidin-2-yl)amino)-3-methoxybenzoyl)piperazin-1-yl)ethoxy)ethoxy)ethoxy)benzoyl)thiazol-2-yl)pyrrolidin-1-yl)-1-cyclohexyl-2-oxoethyl)amino)-1-oxopropan-2-yl)(methyl)carbamate (3.8 mg, 26% yield); HRMS (ESI⁺) *m*/*z,* calcd for C₅₈H₈₂ClN₁₀O₁₂S 1177.5517 [M + H]⁺, found 1177.5747. To a solution of *tert*-butyl ((*S*)-1-(((*S*)-2-((*S*)-2-(4-(3-((14-(4-(4-((5-chloro-4-(methylamino)pyrimidin-2-yl)amino)-3-methoxybenzoyl)piperazin-1-yl)-3,6,9,12-tetraoxatetradecyl)oxy)benzoyl)thiazol-2-yl)pyrrolidin-1-yl)-1-cyclohexyl-2-oxoethyl)amino)-1-oxopropan-2-yl)(methyl)carbamate (3.8 mg, 0.0028 mmol) in 0.2 ml DCM was added 4N HCl solution in 1,4-dioxane (0.2 ml). The resulting mixture was stirred at room temperature for 2h, then condensed to afford **SD112** as HCl salt. ¹H NMR (500 MHz, DMSO- *d*₆) δ 8.49 (s, 1H), 8.40 (d, J = 8.3 Hz, 1H), 8.27 (s, 2H), 8.06 (d, J = 8.7 Hz, 1H), 7.96 (s, 1H), 7.70 - 7.60 (m, 3H), 7.46 (t, J = 8.0 Hz, 1H), 7.34 (q, J = 4.4 Hz, 1H), 7.29 - 7.21 (m, 1H), 7.02 (d, J = 1.6 Hz, 1H), 6.97 (dd, J = 8.3, 1.6 Hz, 1H), 5.39 (dd, J = 8.0, 2.9 Hz, 1H), 4.49 (dd, J = 8.5, 7.1 Hz, 1H), 4.18 - 4.13 (m, 2H), 3.89 (s, 3H), 3.82 - 3.78 (m, 2H), 3.77 - 3.74 (m, 2H), 3.60 - 3.58 (m, 2H), 3.55 - 3.52 (m, 2H), 3.52 - 3.46 (m, 15H), 3.12 (q, J = 6.8 Hz, 1H), 2.90 (d, J = 4.6 Hz, 3H), 2.48 (d, J = 5.8 Hz, 1H), 2.42 (s, 4H), 2.30 - 2.24 (m, 1H), 2.22 (s, 3H), 2.20 - 2.15 (m, 1H), 2.07 -1.99 (m, 2H), 1.74 - 1.61 (m, 4H), 1.55 (d, *J* = 10.2 Hz, 2H), 1.14 (d, J = 6.9 Hz, 3H), 1.12 - 0.92 (m, 6H); HRMS (ESI⁺) *m*/*z,* calcd for C₅₃H₇₃ClN₁₀O₁₀S: 1077.4993 [M + H]⁺, found 1077.5042.

### Exemplary synthesis of Exemplary compound 12 (SD114)

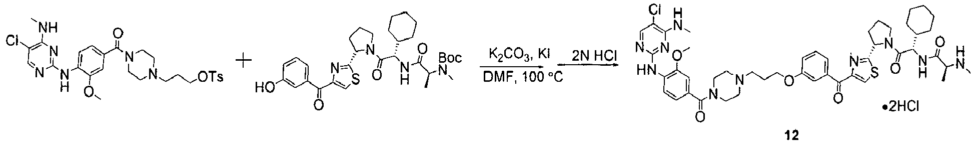

*(S)-N-((S)-2-((S)-2-(4-(3-(3-(4-(4-((5-chloro-4-(methylamino)pyrimidin-2-yl)omino)-3-methoxybenzoyl)piperazin-1-yl)propoxy)benzoyl)thiazol-2-yl)pyrrolidin-1-yl)-1-cyclohexyl-2-oxoethyl)-2-(methylamino)propenamide, HCl salt (**SD114**)*

To a solution of 3-(4-(4-((5-chloro-4-(methylamino)pyrimidin-2-yl)amino)-3-methoxybenzoyl)piperazin-1-yl)propyl 4-methylbenzenesulfonate (7.36 mg, 0.0125 mmol) in DMF (0.2 ml) was added tert-butyl ((*S*)-1-(((*S*)-1-cyclohexyl-2-((*S*)-2-(4-(3-hydroxybenzoyl)thiazol-2-yl)pyrrolidin-1-yl)-2-oxoethyl)amino)-1-oxopropan-2-yl)(methyl)carbamate (7.5 mg, 0.0125 mmol), K₂CO₃ (5 mg, 0.036 mmol), and KI (0.6 mg, 0.00375 mmol). The resulting mixture was heated to 100 °C and stirred at 100 °C overnight. The mixture was filtered through 0.45 µM filter and purified with preparative HPLC under acidic condition (10-85% CH₃CN in 0.1% aq HCO₂H) to give *tert*-butyl ((*S*)-1-(((*S*)-2-((*S*)-2-(4-(3-(3-(4-(4-((5-chloro-4-(methylamino)pyrimidin-2-yl)amino)-3-methoxybenzoyl)piperazin-1-yl)propoxy)benzoyl)thiazol-2-yl)pyrrolidin-1-yl)-1-cyclohexyl-2-oxoethyl)amino)-1-oxopropan-2-yl)(methyl)carbamate (4 mg, 32% yield); HRMS (ESI⁺) *m*/*z,* calcd for C₅₁H₆₇ClN₁₀O₈S 1015.462534 [M + H]⁺, found 1015.4770. To a solution of *tert*-butyl ((*S*)-1-(((*S*)-2-((*S*)-2-(4-(3-(3-(4-(4-((5-chloro-4-(methylamino)pyrimidin-2-yl)amino)-3-methoxybenzoyl)piperazin-1-yl)propoxy)benzoyl)thiazol-2-yl)pyrrolidin-1-yl)-1-cyclohexyl-2-oxoethyl)amino)-1-oxopropan-2-yl)(methyl)carbamate (4 mg, 0.0039 mmol) in 0.2 ml DCM was added 4N HCl solution in 1,4-dioxane (0.2 ml). The resulting mixture was stirred at room temperature for 2h, then condensed to afford SD114 as HCl salt. ¹H NMR (500 MHz, DMSO-*d*₆) δ 8.50 (s, 1H), 8.41 (d, *J* = 8.3 Hz, 1H), 8.06 (br, s, 1H), 7.97 (s, 1H), 7.73 - 7.63 (m, 3H), 7.51 - 7.41 (m, 1H), 7.35 (q, J = 4.3 Hz, 1H), 7.27 - 7.20 (m, 1H), 7.03 (d, J = 1.5 Hz, 1H), 6.98 (dd, J = 8.3, 1.6 Hz, 1H), 5.40 (dd, J = 7.9, 2.7 Hz, 1H), 4.52 -4.44 (m, 1H), 4.10 (t, *J* = 6.2 Hz, 2H), 3.90 (s, 3H), 3.85 - 3.77 (m, 4H), 3.52 - 3.47 (m, 4H), 2.91 (d, *J* = 4.6 Hz, 3H), 2.48 (d, *J* = 7.2 Hz, 1H), 2.46 - 2.35 (m, 4H), 2.31 - 2.15 (m, 4H), 2.14 - 2.08 (m, 1H), 2.08 - 2.00 (m, 2H), 1.98 -1.88 (m, 2H), 1.72 - 1.56 (m, 4H), 1.56 - 1.49 (m, 2H), 1.19 - 0.86 (m, 9H). HRMS (ESI⁺) m/z, calcd for C₄₆H₅₉ClN₁₀O₆S: 915.4101 [M + H]⁺, found 915.4190.

### Exemplary synthesis of Exemplary compound 13 (XL01078B)

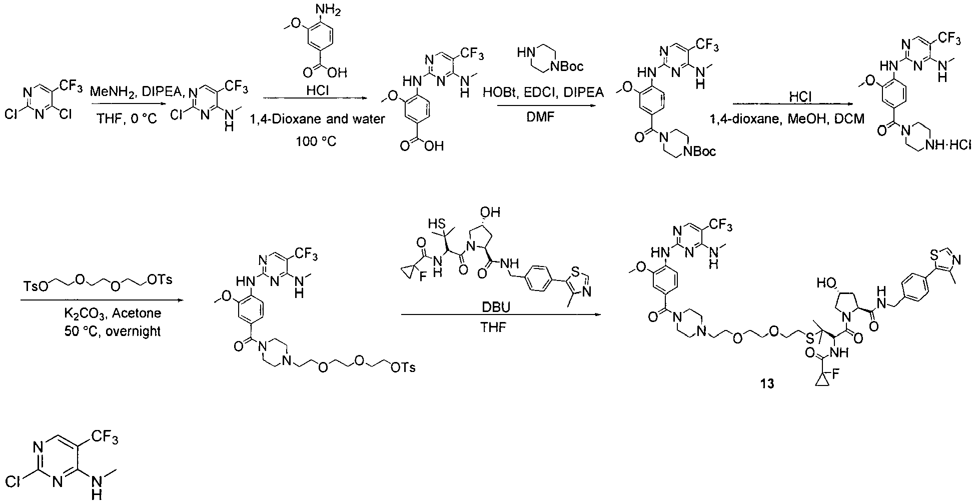

*2-chloro-N-methyl-5-(trifluoromethyl)pyrimidin-4-amine*

To a stirred solution of 2,4-dichloro-5-(trifluoromethyl)pyrimidine (2.6 g, 11.99 mmol) in anhydrous THF (50 mL) was added DIPEA (2.3 mL, 13.18 mmol) of DIPEA followed by methylamine (33% solution in ethanol, 1.64 mL, 13.18 mmol) dropwise at 0°C. The reaction mixture was stirred at 0°C for 1 hour, then warmed to room temperature and concentrated in vacuo. The crude residue was purified by flash column chromatography on silica gradient from 0% to 50% of EtOAc in Petroleum spirit to give *2-chloro-N-methyl-5-(trifluoromethyl)pyrimidin-4-amine* (1.36 g, 6.42 mmol, 53% yield) of. ¹H NMR (500 MHz, CDCl₃) δ 8.27 (d, J= 0.90 Hz, 1H), 5.51 (br s, 1H), 3.14 (d, *J* = 4.80 Hz, 3H); ¹⁹F NMR (471 MHz, CDCl₃) δ - 63.30; ¹³C NMR (126 MHz, CDCl3) δ 163.82, 159.75,154.91, 123.75 (q, *J* = 272 Hz), 106.05, 28.41. LC-MS, ESI⁺, m/z 212.02 [M+H]⁺. 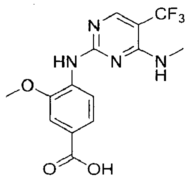 *3-methoxy-4-((4-(methylamino)-5-(trifluoromethyl)pyrimidin-2-yl)amino)benzoic acid*

To a solution of 2-chloro-N-methyl-5-(trifluoromethyl)pyrimidin-4-amine (1.0g, 4.72 mmol) in a mixture of dioxane and water (20 ml : 20 ml) was added 4-amino-3-methoxybenzoic acid (0.87g, 5.20 mmol) followed by 4M solution of HCl in dioxane (1.3 ml , 5.20 mmol). After refluxing at 100 °C for 2h, the mixture was cooled to room temperature to precipitate white solids. The solids were filtered, washed with water, dried under vacuum to afford the 3-methoxy-4-((4-(methylamino)-5-(trifluoromethyl)pyrimidin-2-yl)amino)benzoic acid as white solid (1.25 g, 77% yield). ¹H NMR (500 MHz, DMSO-*d*₆) δ 12.71 (br, 1H), 9.19 (s, 1H), 8.44 (s, 1H), 8.36 (d, J = 8.4 Hz, 1H), 8.07 (br, 1H), 7.63 (dd, *J* = 8.4,1.7 Hz, 1H), 7.57 (d, *J* = 1.7 Hz, 1H), 3.95 (s, 3H), 2.99 (d, *J* = 4.4 Hz, 3H). ¹⁹F NMR (471 MHz, DMSO-*d*₆) δ -61.32. ¹³C NMR (126 MHz, DMSO-*d*₆) δ 167.31, 158.41, 156.47, 149.50, 149.21, 131.41, 126.51, 124.00 (q, J = 270.37 Hz), 122.85, 120.39, 111.79, 99.33, 56.55, 29.19. LC-MS, ESI⁺, m/z 343 [M+H]⁺. 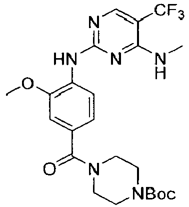 *tert-butyl4-(3-methoxy-4-((4-(methylamino)-5-(trifluoromethyl)pyrimidin-2-yl)amino)benzoyl)piperazine-1-carboxylate*

To a solution of 3-methoxy-4-((4-(methylamino)-5-(trifluoromethyl)pyrimidin-2-yl)amino)benzoic acid (0.98 g, 2.58 mmol) in DMF (15 mL) was added HOBt (0.42 g, 3.09 mmol), EDCl (0.59 g, 3.09 mmol), 1-Boc-piperazine (0.51g, 2.73 mmol), and DIPEA (1.8 mL, 10.32 mmol) at room temperature. After stirring at room temperature for 16 h, the mixture was diluted with water (30 mL) and extracted with EtOAc (100 mL). The organic layer was washed with brine (15 mL) and concentrated to give a residue which was purified by flash column chromatography on silica (0% to 100% of EtOAc in DCM) to give *tert-butyl4-(3-methoxy-4-((4-(methylamino)-5-(trifluoromethyl)pyrimidin-2-yl)amino)benzoyl)piperazine-1-carboxylate* (1.12 g, 2.19 mmol, 85% yield). ¹H NMR (500 MHz, CDCl₃) δ 8.60 (d, J=8.2 Hz, 1H), 8.21 (s, 1H), 7.85 (s, 1H), 7.04 (m, 2H), 5.26 (m, 1H), 3.97 (s, 3H), 3.65 (br s, 4H), 3.49 (br s, 4H), 3.14 (d, J = 4.7 Hz, 3H), 1.50 (s, 9H). ¹⁹F NMR (471 MHz, CDCl₃) δ - 61.52. ¹³C NMR (101 MHz, CDCl₃) δ 170.73, 162.62, 160.80, 159.70, 154.67 (q, J = 5.0 Hz), 147.93, 130.93, 128.54, 125.05 (q, J = 267 Hz), 120.13, 117.89, 109.73, 99.56, 80.43, 56.03, 43.93, 28.50, 28.45; LC-MS, ESI⁻, m/z 509.2 [M-H]⁻. 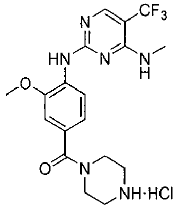 *(3-methoxy-4-((4-(methylamino)-5-(trifluoromethyl)pyrimidin-2-yl)amino)phenyl)(piperazin-1-yl)methanone HCl salt*

To a solution of *tert*-butyl4-(3-methoxy-4-((4-(methylamino)-5-(trifluoromethyl)pyrimidin-2-yl)amino)benzoyl)piperazine-1-carboxylate (1.12 g, 2.19 mmol) in a mixture of DCM and MeOH 9:1 (20 ml) was added 4N solution of HCl in dioxane (2.2 ml, 8.76 mmol) at room temperature. After stirring at room temperature overnight, the reaction mixture was diluted with Et₂O (100 ml) to precipitate white solid which was filtered, washed with Et₂O (50 ml) and air dried overnight to give *(3-methoxy-4-((4-(methylamino)-5-(trifluoromethyl)pyrimidin-2-yl)amino)phenyl)(piperazin-1-yl)methanone HCl salt* (0.97 g, 2.17 mmol, 99%) as a white powder. ¹H NMR (500 MHz, DMSO-*d*₆) δ 9.81 (s, 1H), 9.74 (s, 2H), 8.51 (s, 1H), 8.44 (s, 1H), 8.14 (d, *J* = 6.0 Hz, 1H), 7.21 (d, *J* = 1.6 Hz, 1H), 7.11 (dd, *J*=1.6 Hz, 8.20 Hz,1H), 3.91 (s, 3H), 3.76 (br s, 4H), 3.14 (br s, 4H), 2.99 (d, *J* = 4.4 Hz, 3H). ¹⁹F NMR (471 MHz, DMSO-*d*₆) δ - 61.54. ¹³C NMR (126 MHz, DMSO-*d*₆) δ 169.5, 158.2, 154.6, 150.4, 132.16, 127.53, 123.3 (q, *J* = 269 Hz), 119.97, 111.09, 99.4, 65.5, 56.7, 42.9, 29.7. LC-MS, ESI⁺, m/z 411 [M+H]+. 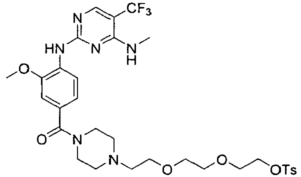 *2-(2-(2-(4-(3-methoxy-4-((4-(methylamino)-5-(trifluoromethyl)pyrimidin-2-y*/*)amino)benzoy*/*)piperazin-1-y*/*)ethoxy)ethoxy)ethyl 4-methylbenzenesulfonate*

To a solution of (3-methoxy-4-((4-(methylamino)-5-(trifluoromethyl)pyrimidin-2-yl)amino)phenyl)(piperazin-1-yl)methanone HCl salt (50 mg, 0.111 mmol) in acetone (3 ml) was added triethylene glycol ditosylate (205 mg, 0.448 mmol) and K₂CO₃ (124 mg, 0.896 mmol). After stirring at 50 °C overnight, the mixture was cooled down, diluted with DCM (10 ml), washed with water and brine, dried over sodium sulfate, filtered and condensed to afford a residue which was purified with flash column chromatography on silica gel to give *2-(2-(2-(4-(3-methoxy-4-((4-(methylomino)-5-(trifluoromethyl)pyrimidin-2-yl)amino)benzoyl)pipercizin-1-yl)ethoxy)ethoxy)ethyl 4-methylbenzenesulfonate* as white solid (36 mg, 46% yield). ¹H NMR (400 MHz, CDCl₃) δ 8.61 - 8.51 (m, 1H), 8.16 (d, *J* = 0.7 Hz, 1H), 7.86 - 7.73 (m, 3H), 7.31 (d, *J* = 8.0 Hz, 2H), 7.03 -6.98 (m, 2H), 5.29 - 5.24 (m, 1H), 4.16 - 4.11 (m, 2H), 3.91 (s, 3H), 3.80 - 3.47 (m, 12H), 3.09 (d, *J* = 4.7 Hz, 3H), 2.59 (t, *J* = 5.7 Hz, 2H), 2.51 (s, 4H), 2.41 (s, 3H); ¹³C NMR (101 MHz, CDCl₃) δ 170.32, 160.79, 159.67, 154.63 (q, *J* = 5.0 Hz), 147.84, 144.89, 133.19, 130.59, 129.91, 129.01, 128.03, 125.05 (d, *J* = 268 Hz), 120.11, 117.83, 109.73, 70.91, 70.42, 69.30, 69.08, 68.82, 57.82, 56.00, 53.74, 28.43, 21.69; LC-MS, ESI⁺, m/z 697 [M+H]⁺. 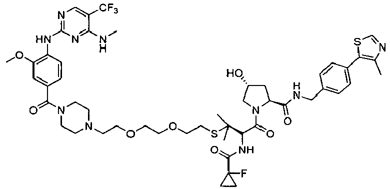 *(2S,4R)-1-((S)-2-(1-fluorocyclopropone-1-carboxamido)-3-((2-(2-(2-(4-(3-methoxy-4-((4-(methylamino)-5-(trifluoromethyl)pyrimidin-2-yl)amino)benzoyl)piperazin-1-yl)ethoxy)ethoxy)ethyl)thio)-3-methylbutanoyl)-4-hydroxy-N-(4-(4-methylthiazol-5-yl)benzyl)pyrrolidine-2-carboxamide (13, **XL01078B)***

**XL01078B** was prepared following general procedure 8. 3.6 mg **XL01078B** was obtained from 5 mg thiol compound, 36% yield. 1H NMR (500 MHz, CDCl₃) δ 8.68 (s, 1H), 8.55 (d, J = 8.8 Hz, 1H), 8.20 - 8.13 (m, 2H), 7.88 (s, 1H), 7.39 - 7.33 (m, 5H), 7.20 (dd, J = 8.3, 3.2 Hz, 1H), 7.03 - 6.98 (m, 2H), 5.25 (d, J = 3.6 Hz, 1H), 4.85 (d, J = 8.2 Hz, 1H), 4.76 (dd, J = 8.3, 6.3 Hz, 1H), 4.59 - 4.52 (m, 1H), 4.44 (qt, *J* = 12.8, 6.4 Hz, 2H), 3.93 (s, 3H), 3.91 - 3.87 (m, 2H), 3.72 (s, 4H), 3.66 - 3.57 (m, 4H), 3.56 - 3.52 (m, 4H), 3.50 - 3.46 (m, 2H), 3.11 (d, J = 4.7 Hz, 3H), 2.76 (ddd, J = 16.4, 12.4, 5.9 Hz, 2H), 2.69 (t, J = 5.4 Hz, 2H), 2.63 (s, 2H), 2.52 (s, 3H), 2.50 - 2.43 (m, 1H), 2.21 - 2.14 (m, 1H), 1.37 - 1.28 (m, 10H); ¹³C NMR (126 MHz, CDCl₃) δ 170.92, 170.51, 170.29 (d, *J*= 20.6 Hz), 170.01, 164.24, 160.75, 159.75, 154.57 (q, *J* = 4.8 Hz), 150.49, 148.60, 148.03, 138.24, 131.79, 131.12, 130.86, 129.66, 128.59, 128.33,125.05 (q, *J* = 270.5 Hz), 120.21, 118.03, 109.83, 79.20, 70.50, 70.44, 70.32, 69.77, 68.36, 59.27, 57.59, 56.28, 56.11, 55.97, 53.48, 48.06, 43.27, 36.62, 28.78, 28.52, 25.90, 25.34, 16.17, 14.00 (d, J = 9.9 Hz), 13.95 (d, J = 9.7 Hz); ¹⁹F NMR (471 MHz, CDCl3) δ -61.53, -197.74; HRMS (ESI⁺) m/z, calcd for C₄₉H₆₂F₄N₁₀O₈S₂: 1059.4202 [M + H]⁺, found 1059.3872.

### Exemplary synthesis of Exemplary compound 14 (XL01072)

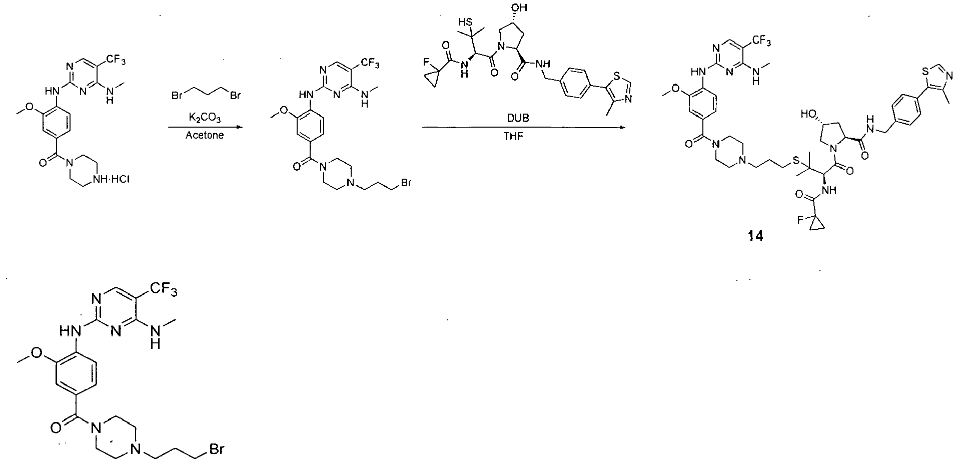

*(4-(3-bromopropyl)piperazin-1-yl)(3-methoxy-4-((4-(methylamino)-5-(trifluoromethyl)pyrimidin-2-yl)amino)phenyl)methanone*

To a solution of (3-methoxy-4-((4-(methylamino)-5-(trifluoromethyl)pyrimidin-2-yl)amino)phenyl)(piperazin-1-yl)methanone HCl salt (100 mg, 0.22 mmol) in acetone (2 ml) was added 1,3-dibromoproprane (181 mg, 0.90 mmol) and K₂CO₃ (93 mg, 0.67 mmol). After stirring at room temperature overnight, the resulting mixture was diluted with DCM, washed with water and brine separately, dried over sodium sulfate, filtered, and condensed to afford a residue which was purified with flash column chromatography on silica gel (0%-10% Methanol in DCM) to give *(4-(3-bromopropyl)piperazin-1-yl)(3-methoxy-4-((4-(methylamino)-5-(trifluoromethyl)pyrimidin-2-yl)amino)phenyl)methanone* as white solid (51 mg, 42% yield). ¹H NMR (400 MHz, CDCl₃) δ 8.63 - 8.49 (m, 1H), 8.16 (d, J = 0.7 Hz, 1H), 7.81 (s, 1H), 7.09 - 6.90 (m, 2H), 5.31 - 5.23 (m, 1H), 3.92 (s, 3H), 3.80 - 3.50 (m, 4H), 3.46 (t, *J* = 6.6 Hz, 2H), 3.09 (d, J = 4.7 Hz, 3H), 2.55 - 2.35 (m, 6H), 2.01 (p, J = 6.7 Hz, 2H); ¹³C NMR (101 MHz, CDCl3) δ 170.33, 160.80, 159.67, 154.65 (q, *J* = 4.9 Hz), 147.86, 130.64, 128.95, 125.05 (q, *J* = 268 Hz), 120.08, 117.84, 109.72, 99.61 (q, *J* = 31.65 Hz), 56.34, 56.00, 53.40, 31.60, 29.97, 28.42; LC-MS, ESI⁺, m/z 531 [M+H]⁺, 533 [M+H+2]⁺. 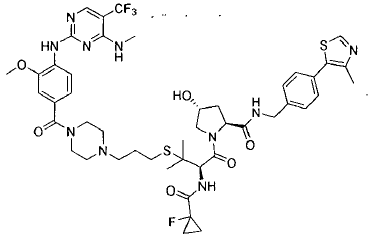 *(2S,4R)-1-((R)-2-(1-fluorocyclopropone-1-carboxamido)-3-((3-(4-(3-methoxy-4-((4-(methylamino)-5-(trifluoromethyl)pyrimidin-2-yl)amino)benzoyl)piperazin-1-yl)propyl)thio)-3-methylbutanoyl)-4-hydroxy-N-(4-(4-methylthiazol-5-yl)benzyl)pyrrolidine-2-carboxamide* (14, XL01072)

XL01072was prepared following general procedure 8. 6.1 mg XL01072was obtained from 7 mg thiol compound, 47% yield. ¹H NMR (400 MHz, DMSO-*d*₆) δ 8.96 (s, 1H), 8.54 (t, J = 5.9 Hz, 1H), 8.30 (d, J = 8.2 Hz, 1H), 8.19 (s, 1H), 8.05 (s, 1H), 7.47 - 7.34 (m, 5H), 7.22 - 7.16 (m, 1H), 7.03 (d, J = 1.7 Hz, 1H), 6.97 (dd, J = 8.2, 1.7 Hz, 1H), 5.19 (br, s, 1H), 4.79 (d, *J* = 9.2 Hz, 1H), 4.51 - 4.34 (m, 3H), 4.24 (dd, J = 15.7, 5.5 Hz, 1H), 3.89 (s, 3H), 3.74 (dd, J = 10.5, 4.0 Hz, 1H), 3.64 (d, *J* = 10.8 Hz, 1H), 3.58 - 3.42 (m, 4H), 2.92 (d, J = 4.4 Hz, 3H), 2.56 (t, J = 7.2 Hz, 2H), 2.44 (s, 3H), 2.37 - 2.26 (m, 6H), 2.13 - 2.04 (m, 1H), 1.97 - 1.86 (m, 1H), 1.62 - 1.51 (m, 2H), 1.44 - 1.32 (m, 8H), 1.25 - 1.18 (m, 2H); ¹³C NMR (101 MHz, DMSO-*d*₆) δ 171.44, 168.62, 168.05 (d, *²J_{C-F}* = 20.7 Hz, ), 167.79, 160.56, 158.42, 154.27 (d, J = 5.4 Hz), 151.32, 148.30, 147.71, 139.26, 131.04, 129.89, 129.72, 129.43, 128.69, 127.96, 127.46 (q, *J* = 267 Hz), 127.40, 119.34, 116.07, 109.84, 78.02 (d, *¹J_{C-F}* = 233.0 Hz, ), 68.78, 58.94, 56.59, 56.46, 55.99, 54.82, 52.61, 49.05, 41.61, 37.88, 27.99, 26.50, 26.45, 25.64, 24.19, 15.87, 12.93 (d, *²J_{C-F}* = 10.6 Hz, ), 12.78 (d, *²J_{C-F}* = 10.8 Hz, ); ¹⁹F NMR (471 MHz, CDCl₃) δ -61.50, -197.76. HRMS (ESI⁺) *m*/*z,* calcd for C₄₆H₅₆F₄N₁₀O₆S₂: 985.3835 [M + H]⁺, found 985.3813.

### Exemplary synthesis of Exemplary compound 15 (XL01070B)

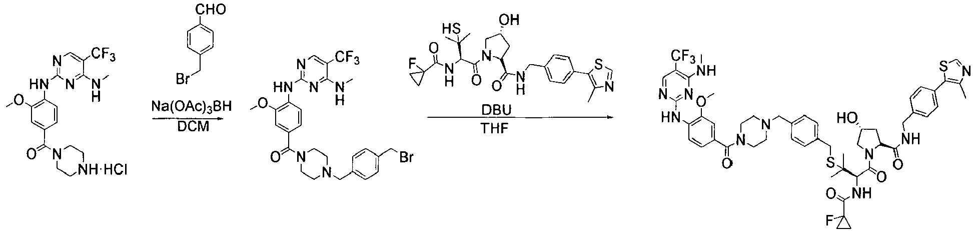

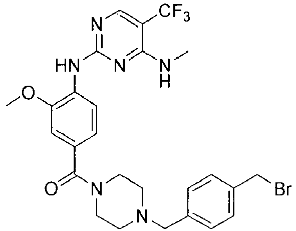

*(4-(4-(bromomethyl)benzyl)piperazin-1-yl)(3-methoxy-4-((4-(methylamino)-5-(trifluoromethyl)pyrimidin-2-yl)amino)phenyl)methanone*

Na(OAc)₃BH (114 mg, 0.54 mmol) was added to a mixture of 4-bromomethylbenzylaldehyde (36 mg, 0.18 mmol) and (3-methoxy-4-((4-(methylamino)-5-(trifluoromethyl)pyrimidin-2-yl)amino)phenyl)(piperazin-1-yl)methanone HCl salt (80 mg, 0.18 mmol) in DCM (5 ml). After stirring at room temperature for 3h, the resulting mixture was diluted with DCM, washed with water and brine separately, dried over sodium sulfate, filtered, and condensed under reduced pressure to afford a residue which was purified with flash column chromatography on silica gel (0%-10% Methanol in DCM) to give *(4-(4-(bromomethyl)benzyl)piperazin-1-yl)(3-methoxy-4-((4-(methylamino)-5-(trifluoromethyl)pyrimidin-2-yl)amino)phenyl)methanone* (70 mg, 66% yield). ¹H NMR (400 MHz, CDCl₃) δ 8.54 (d, J = 8.2 Hz, 1H), 8.17 (d, J = 0.8 Hz, 1H), 7.85 (s, 1H), 7.37 - 7.27 (m, 4H), 7.07 - 6.97 (m, 2H), 5.25 (d, J = 3.4 Hz, 1H), 4.49 (s, 2H), 3.92 (s, 3H), 3.66 (s, 4H), 3.54 (s, 2H), 3.10 (d, J = 4.7 Hz, 3H), 2.47 (s, 4H). ¹³C NMR (100 MHz, CDCl₃) δ 170.41, 160.80, 159.72, 154.63 (d, *J* = 5.2 Hz), 147.95, 137.04, 130.67, 129.64, 129.21, 127.7 (q, J = 261 Hz), 120.13, 117.94, 109.81, 99.48, 62.58, 56.05, 33.39, 31.01, 28.48, 20.85. LC-MS, ESI⁺, m/z 593 [M+H]⁺, 595 [M+H+2]⁺. 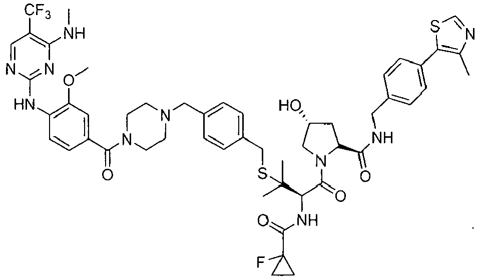 *(2R,4R)-1-((R)-2-(1-fluorocyclopropane-1-carboxamido)-3-((4-((4-(3-methoxy-4-((4-(methylamino)-5-(trifluoromethyl)pyrimidin-2-yl)omino)benzoyl)piperazin-1-yl)methyl)benzyl)thio)-3-methylbutanoyl)-4-hydroxy-N-(4-(4-methylthiazol-5-yl)benzyl)pyrrolidine-2-carboxamide (15, **XL01070B)***

***XL01070B*** was prepared following general procedure 8. 7.8 mg ***XL01070B*** was obtained from thiol compound, 57% yield. ¹H NMR (400 MHz, CDCl₃) δ 8.66 (s, 1H), 8.54 (d, J = 8.2 Hz, 1H), 8.18 (d, J = 0.7 Hz, 1H), 7.83 (s, 1H), 7.36 -7.28 (m, 4H), 7.26 - 7.18 (m, 6H), 7.03 - 6.97 (m, 2H), 5.26 - 5.19 (m, 1H), 4.73 (t, J = 7.8 Hz, 1H), 4.63 (d, J = 7.6 Hz, 1H), 4.54 - 4.33 (m, 3H), 3.93 (s, 3H), 3.87 (d, J = 11.3 Hz, 1H), 3.82 - 3.54 (m, 7H), 3.48 (s, 2H), 3.11 (d, J = 4.7 Hz, 3H), 2.52 - 2.37 (m, 8H), 2.21 - 2.11 (m, 1H), 1.41 - 1.24 (m, 10H); ¹³C NMR (101 MHz, CDCl₃) δ 170.67, 170.64, 170.42, 170.15, 160.84, 159.75, 154.69 (q, J = 4.9 Hz), 150.40, 148.68, 147.94, 138.12, 136.95, 136.48, 131.69, 131.20, 130.68, 129.67, 129.63, 129.08, 128.24, 127.73 (q, J = 262 Hz), 120.16, 117.92, 109.83, 78.40 (d, J = 207.5 Hz), 70.19, 62.69, 58.98, 56.53, 56.46, 56.07, 53.34, 48.81, 43.31, 36.63, 33.42, 28.49, 25.84, 25.47, 16.21, 14.04 (d, *J* = 10.3 Hz), 13.94 (d, 10.3 Hz); ¹⁹F NMR (471 MHz, CDCl₃) δ -61.49, -197.70; HRMS (ESI⁺) *m*/*z,* calcd for C₅₁H₅₈F₄N₁₀O₆S₂ 1047.3991 [M + H]⁺, found 1047.4052.

### Exemplary synthesis of Exemplary compound 16 (XL01119)

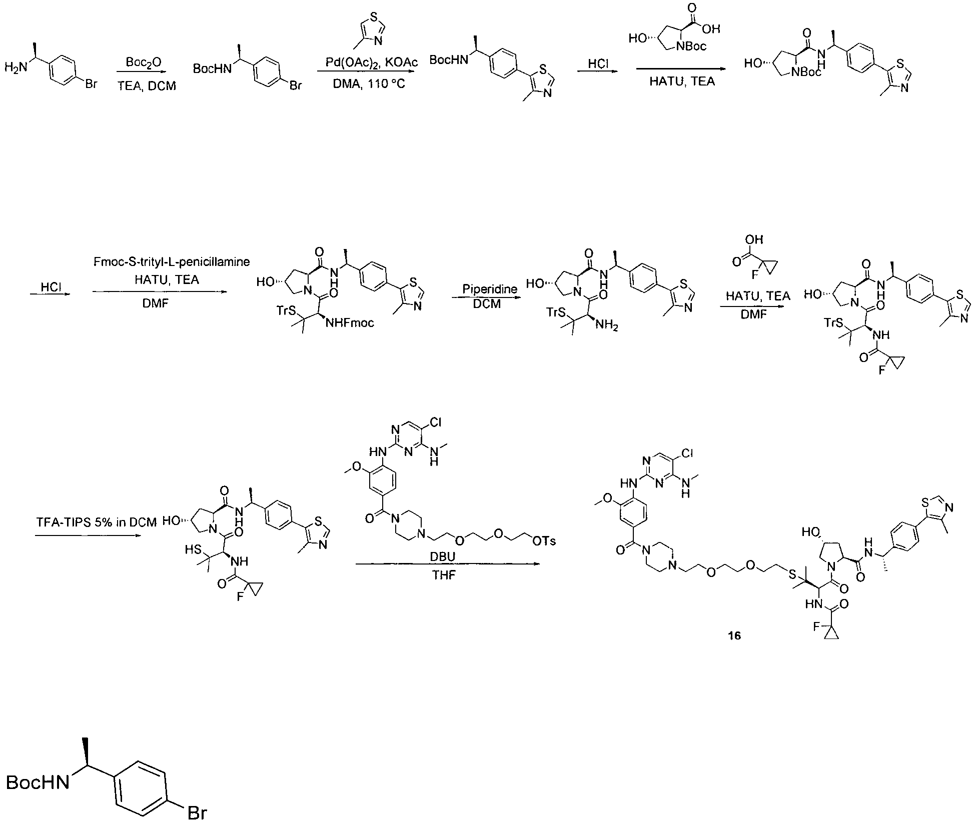

*tert-butyl (S)-(1-(4-bromophenyl)ethyl)carbamate* was prepared following general procedure 1. 3.6 g *tert-butyl (S)-(1-(4-bromophenyl)ethyl)carbamate* was obtained from 4.17 g amine compound 58% yield. ¹H NMR (400 MHz, CDCl₃) δ 7.51 - 7.44 (m, 2H), 7.20 (d, J = 8.4 Hz, 2H), 4.76 (s, 2H), 1.48 - 1.38 (m, 12H). 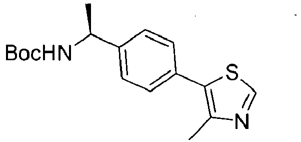 *tert-butyl (S)-(1-(4-(4-methylthiazol-5-yl)phenyl)ethyl)carbamate* was prepared following general procedure 2. 600 mg *tert-butyl (S)-(1-(4-(4-methylthiazol-5-yl)phenyl)ethyl)carbamate* was obtained from 1.03 g bromide compound, 55% yield. ¹H NMR (400 MHz, CDCl₃) δ 8.69 (s, 1H), 7.47 - 7.41 (m, 2H), 7.38 (d, J = 8.3 Hz, 2H), 4.83 (s, 2H), 2.56 (s, 3H), 1.50 (d, J = 6.6 Hz, 3H), 1.46 (s, 9H). 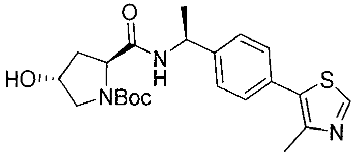 *tert-butyl (2S,4R)-4-hydroxy-2-(((S)-1-(4-(4-methylthiazol-5-yl)phenyl)ethyl)carbamoyl)pyrrolidine-1-carboxylate* was prepared following general proceure 3. 380 mg *tert-butyl (2S,4R)-4-hydroxy-2-(((S)-1-(4-(4-methylthiazol-5-yl)phenyl)ethyl)carbamoyl)pyrrolidine-1-carboxylate* was obtained from 337 mg *tert-butyl (S)-(1-(4-(4-methylthiazol-5-yl)phenyl)ethyl)carbamate,* 83% yield. ¹H NMR (400 MHz, CDCl3) δ 8.69 (s, 1H), 7.45 - 7.37 (m, 4H), 5.13 (s, 1H), 4.49 (d, J = 35.0 Hz, 2H), 3.75 - 3.35 (m, 2H), 2.55 (s, 3H), 1.94 (d, J = 3.8 Hz, 1H), 1.62 (s, 1H), 1.56 - 1.33 (m, 12H). 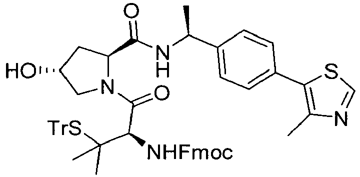 *(9H-fluoren-9-yl)methyl((R)-1-((2R,4R)-4-hydroxy-2-(((S)-1-(4-(4-methylthiazol-5-yl)phenyl)ethyl)carbamoyl)pyrrolidin-1-yl)-3-methyl-1-oxo-3-(tritylthio)butan-2-yl)carbamate* was prepared following general procedure 4. 190 mg *(9H-fluoren-9-yl)methyl ((R)-1-((2R,4R)-4-hydroxy-2-(((S)-1-(4-(4-methylthiazol-5-yl)phenyl)ethyl)carbamoyl)pyrrolidin-1-yl)-3-methyl-1-oxo-3-(tritylthio)butan-2-yl)carbamate* was obtained from 118 mg *tert-butyl (2S,4R)-4-hydroxy-2-(((S)-1-(4-(4-methylthiazol-5-yl)phenyl)ethyl)carbamoyl)pyrrolidine-1-carboxylate,* 75% yield. ¹H NMR (400 MHz, CDCl₃) δ 8.67 (s, 1H), 7.78 - 7.72 (m, 2H), 7.62 - 7.52 (m, 8H), 7.41 - 7.17 (m, 18H), 5.73 (d, J = 5.3 Hz, 1H), 5.00 (p, J = 7.0 Hz, 1H), 4.65 (t, J = 8.0 Hz, 1H), 4.42 - 4.15 (m, 4H), 3.56 (d, J = 5.3 Hz, 1H), 3.45 (d, J = 11.6 Hz, 1H), 3.17 (dd, *J* = 11.6, 3.5 Hz, 1H), 2.83 (br, s, 1H), 2.51 (s, 3H), 2.44 - 2.32 (m, 1H), 2.08 - 1.96 (m, 1H), 1.39 - 1.31 (m, 6H), 1.17 (s, 3H). ¹³C NMR (101 MHz, CDCl₃) δ 170.91, 169.57, 156.61, 150.35, 148.68, 144.60, 144.02, 143.58, 143.26, 141.41, 130.99, 130.04, 129.68, 128.03, 127.94, 127.28, 127.08, 126.58, 125.29, 125.13, 120.18, 70.25, 67.79, 58.61, 58.38, 56.62, 48.92, 47.14, 35.86, 26.12, 26.05, 22.34, 16.23. LC-MS, ESI⁺, *m*/*z* 927 [M+H]⁺. 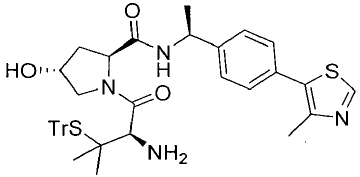 *(2R,4R)-1-((R)-2-omino-3-methyl-3-(tritylthio)butonoyl)-4-hydroxy-N-((S)-1-(4-(4-methylthiozol-5-yl)phenyl)ethyl)pyrrolidine-2-carboxamide* was prepared following general procedure 5. 80 mg *(2R,4R)-1-((R)-2-amino-3-methyl-3-(tritylthio)butanoyl)-4-hydroxy-N-((S)-1-(4-(4-methylthiazol-5-yl)phenyl)ethyl)pyrrolidine-2-carboxamide* was obtained from 150 mg Fmoc protected starting material, 70% yield. ¹H NMR (400 MHz, MeOD-*d*₄) δ 8.82 (s, 1H), 7.67 - 7.55 (m, 6H), 7.41 - 7.31 (m, 4H), 7.30- 7.23 (m, 6H), 7.23 - 7.14 (m, 3H), 4.92 (q, *J* = 7.0 Hz, 1H), 4.44 (t, *J* = 8.2 Hz, 1H), 4.33 - 4.23 (m, 1H), 3.13 (dd, *J* = 11.3, 4.1 Hz, 1H), 2.98 (d, *J* = 11.3 Hz, 1H), 2.64 (s, 1H), 2.43 (s, 3H), 2.13 - 2.04 (m, 1H), 1.86 - 1.78 (m, 1H), 1.40 (d, *J* = 7.0 Hz, 3H), 1.29 (s, 3H), 1.24 (s, 3H). ¹³C NMR (101 MHz, MeOD-*d*₄) δ 173.07, 172.65, 152.75, 149.07, 146.36, 145.48, 133.28, 131.47, 131.12, 130.43, 128.89, 127.84, 127.57, 70.55, 69.16, 60.62, 58.98, 58.61, 58.03, 38.68, 26.29, 25.39, 22.26, 15.83. LC-MS, ESI⁺, m/z 727.2 [M+Na⁺]. 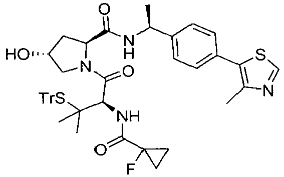 *(2R,4R)-1-((R)-2-(1-fluorocyclopropone-1-carboxamido)-3-methyl-3-(tritylthio)butanoyl)-4-hydroxy-N-((S)-1-(4-(4-methylthiazol-5-yl)phenyl)ethyl)pyrrolidine-2-carboxamide* was prepared following general procedure 6. 80 mg *(2R,4R)-1-((R)-2-(1-fluorocyclopropone-1-carboxamido)-3-methyl-3-(tritylthio)butanoyl)-4-hydroxy-N-((S)-1-(4-(4-methylthiazol-5-yl)phenyl)ethyl)pyrrolidine-2-carboxamide* was obtained from 80 mg amine compound, 89% yield. ¹H NMR (500 MHz, CDCl₃) δ 8.69 (s, 1H), 7.67 - 7.60 (m, 6H), 7.44 - 7.33 (m, 6H), 7.31 - 7.27 (m, 6H), 7.26 - 7.21 (m, 3H), 5.03 (p, *J* = 7.0 Hz, 1H), 4.65 (t, *J* = 8.1 Hz, 1H), 4.35 (s, 1H), 3.59 (d, *J* = 4.8 Hz, 1H), 3.49 (d, *J* = 11.5 Hz, 1H), 3.26 (d, *J* = 5.9 Hz, 1H), 3.19 (dd, *J* = 11.5, 3.7 Hz, 1H), 2.53 (s, 3H), 2.33 (ddd, *J* = 13.0, 8.1, 4.5 Hz, 1H), 2.11 - 1.97 (m, 1H), 1.41 - 1.32 (m, 8H), 1.29 - 1.20 (m, 5H). ¹⁹F NMR (471 MHz, CDCl₃) δ -197.26. ¹³C NMR (126 MHz, CDCl₃) δ 170.38 (*J* = 16.7 Hz), 170.11, 169.64, 150.34, 148.65, 144.60, 143.30, 131.77, 130.95, 129.94, 129.65, 128.02, 127.04, 126.60, 79.22, 70.25, 68.52, 58.64, 57.30, 56.78, 53.67, 48.90, 36.04, 26.16, 22.39, 16.21, 13.88 (d, *J* = 10.3 Hz), 13.71 (d, *J* = 10.3 Hz). LC-MS, ESI⁻, m/z 789 [M-H]⁻. 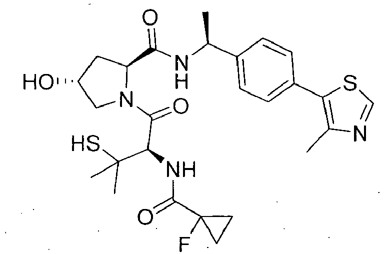 *(2S,4R)-1-((R)-2-(1-fluorocyclopropane-1-carboxamido)-3-mercapto-3-methylbutanoyl)-4-hydroxy-N-((S)-1-(4-(4-methylthiazol-5-yl)phenyl)ethyl)pyrrolidine-2-carboxamide* was prepared following general procedure 7. 25 mg *(2S,4R)-1-((R)-2-(1-fluorocyclopropane-1-carboxamido)-3-mercapto-3-methylbutanoyl)-4-hydroxy-N-((5)-1-(4-(4-methylthiazol-5-yl)phenyl)ethyl)pyrrolidine-2-carboxamide* was obtained from 40 mg trityl protected starting material, 90% yield. ¹H NMR (500 MHz, CDCl₃) δ 8.73 (s, 1H), 7.49 - 7.43 (m, 1H), 7.42 - 7.36 (m, 4H), 7.28 (d, *J* = 8.4 Hz, 1H), 5.10 (p, *J* = 7.0 Hz, 1H), 4.72 - 4.66 (m, 2H), 4.55 - 4.49 (m, 1H), 4.25 (s, 1H), 4.09 (d, *J* = 11.2 Hz, 1H), 3.76 (dd, *J* = 11.2, 3.9 Hz, 1H), 2.71 (s, 1H), 2.53 (s, 3H), 2.44 - 2.37 (m, 1H), 2.13 - 2.05 (m, 1H), 1.54 - 1.50 (m, 6H), 1.43 (s, 3H), 1.38 - 1.27 (m, 4H). ¹³C NMR (126 MHz, CDCl₃) δ 170.72 (d, *J* = 21.0 Hz), 170.43, 169.73, 150.70, 148.27, 143.35, 132.01, 130.85, 129.75, 126.66, 77.24 (d, *J* = 232.2 Hz), 70.17, 59.14, 57.85, 56.70, 49.10, 46.45, 36.51, 30.78, 28.82, 22.34, 15.97, 14.06 (d, *J* = 10.4 Hz), 14.05 (d, *J* = 10.9 Hz). ¹⁹F NMR (471 MHz, CDCl₃) δ -197.71. LC-MS, 549.5 [M+H]⁺. 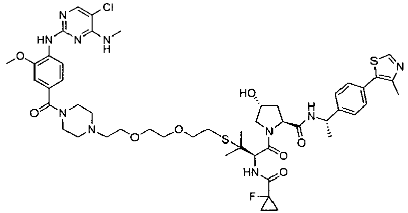 *(2S,4R)-1-((R)-3-((2-(2-(2-(4-(4-((5-chloro-4-(methylamino)pyrimidin-2-yl)amino)-3-methoxybenzoyl)piperazin-1-yl)ethoxy))ethyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide **(16, XL01119)***

**XL01119** was prepared following general procedure 8. 6.7 mg **XL01119** was obtained from 8 mg thiol starting material, 40% yield. ¹H NMR (500 MHz, CDCl₃) δ 8.66 (s, 1H), 8.54 (d, *J* = 8.7 Hz, 1H), 7.92 (s, 1H), 7.63 (s, 1H), 7.42 - 7.32 (m, 5H), 7.22 (dd, *J* = 8.2, 3.2 Hz, 1H), 7.03 - 6.98 (m, 2H), 5.30 (q, *J* = 4.5 Hz, 1H), 5.10 (p, *J* = 7.0 Hz, 1H), 4.88 (d, *J* = 8.1 Hz, 1H), 4.74 (dd, *J* = 8.4, 6.0 Hz, 1H), 4.54 (p, *J* = 4.4 Hz, 1H), 3.92 (s, 3H), 3.90 (d, *J* = 4.2 Hz, 2H), 3.72 - 3.55 (m, 12H), 3.10 (d, *J* = 4.9 Hz, 3H), 2.90 - 2.78 (m, 2H), 2.64 - 2.60 (m, 2H), 2.57 - 2.41 (m, 8H), 2.11 (ddd, *J* = 13.2, 8.5, 4.6 Hz, 1H), 1.48 (d, *J* = 7.0 Hz, 3H), 1.42 (s, 3H), 1.39 (s, 3H), 1.37 - 1.28 (m, 4H). ¹³C NMR (126 MHz, CDCl₃) δ 170.62, 170.39, 170.23, 169.83, 158.73, 157.96, 152.82, 150.34, 148.68, 147.58, 143.21, 131.79, 131.56, 131.02, 129.67, 127.88, 126.68, 120.36, 116.93, 109.75, 105.71, 79.22, 70.59, 70.53, 70.39, 69.72, 68.88, 59.21, 57.88, 56.22, 56.04, 53.80, 48.88, 47.98, 36.26, 28.92, 28.23, 25.97, 25.47, 22.26, 16.25, 14.04 (d, *J*= 10.3 Hz), 13.92 (d, *J* = 9.8 Hz). ¹⁹F NMR (471 MHz, CDCl₃) δ -197.73. HRMS (ESI⁺) m/z, calcd for C₄₉H₆₄ClFN₁₀O₈S₂: 1039.4095 [M + H]⁺, found 1039.3852.

### Exemplary synthesis of Exemplary compound 17 (XL01118)

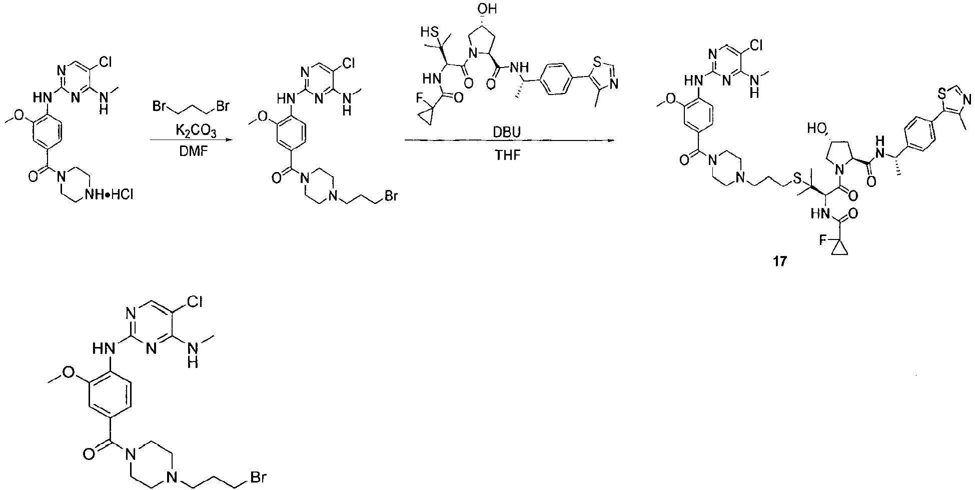

*(4-(3-bromopropyl)piperazin-1-yl)(4-((5-chloro-4-(methylcimino)pyrimidin-2-yl)amino)-3-methoxyphenyl)methanone*

To a solution of (4-((5-chloro-4-(methylamino)pyrimidin-2-yl)amino)-3-methoxyphenyl)(piperazin-1-yl)methanone HCl salt (50 mg, 0.12 mmol) in DMF(1 ml) was added K₂CO₃ (50 mg, 0.36 mmol) and 1,3-dibrompropane (98 mg, 0.48 mmol). After stirring at room temperature overnight, water was added and the resulting mixture was extracted with EtOAc (3x). The combined organic phase was washed with water and brine, dried over sodium sulfate, filtered and condensed under reduced pressure to afford *(4-(3-bromopropyl)piperazin-1-yl)(4-((5-chloro-4-(methylamino)pyrimidin-2-yl)amino)-3-methoxyphenyl)methanone* (30 mg, 50% yield) as white solid. ¹H NMR (400 MHz, CDCl₃) δ 8.59 - 8.47 (m, 1H), 7.91 (s, 1H), 7.62 (s, 1H), 7.03 - 6.97 (m, 2H), 5.36 - 5.26 (m, 1H), 3.91 (s, 3H), 3.77 - 3.53 (m, 4H), 3.47 (t, *J* = 6.6 Hz, 2H), 3.09 (d, *J* = 4.9 Hz, 3H), 2.56 - 2.36 (m, 6H), 2.02 (p, *J* = 6.7 Hz, 2H). ¹³C NMR (101 MHz, CDCl₃) δ 170.56, 158.68, 157.93, 152.77, 147.52, 131.49, 127.94, 120.28, 116.87, 109.69, 105.64, 56.38, 55.98, 53.45, 31.64, 30.00, 28.18. LC-MS, ESI⁺, m/z, 497, [M+H]⁺, 499 [M+H+2]⁺. 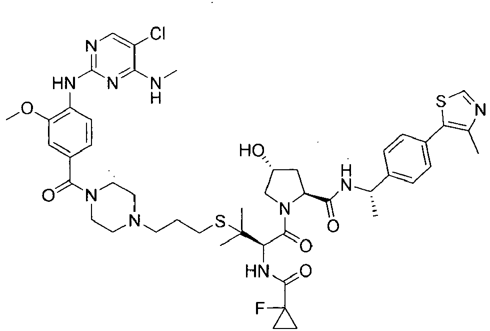 *(2S,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide **(17, XL01118)***

**XL01118** was prepared following general procedure 8. 3.1 mg **XL01118** was obtained from 8 mg thiol compound, 20% yield. ¹H NMR (500 MHz, CDCl₃) δ 8.69 (s, 1H), 8.57 (d, *J* = 8.2 Hz, 1H), 7.95 (s, 1H), 7.68 (s, 1H), 7.45 - 7.38 (m, 4H), 7.33 (d, *J* = 7.9 Hz, 1H), 7.32 - 7.28 (m, 1H), 7.06 - 6.98 (m, 2H), 5.32 (q, *J* = 4.5 Hz, 1H), 5.13 (p, *J* = 7.0 Hz, 1H), 4.82 - 4.76 (m, 2H), 4.56 (s, 1H), 4.09 (d, *J* = 11.3 Hz, 1H), 3.95 (s, 3H), 3.82 - 3.57 (m, 5H), 3.13 (d, *J* = 4.9 Hz, 3H), 2.71 - 2.59 (m, 2H), 2.55 (s, 3H), 2.54 - 2.40 (m, 8H), 2.21 - 2.13 (m, 1H), 1.85 - 1.72 (m, 2H), 1.52 (d, *J* = 6.9 Hz, 3H), 1.48 (s, 3H), 1.46 (s, 3H), 1.41 - 1.30 (m, 4H). ¹³C NMR (126 MHz, CDCl₃) δ 170.69 (d, *J* = 21.0 Hz), 170.66, 170.30, 169.64, 158.74, 157.94, 152.76, 150.41, 148.69, 147.62, 143.13, 131.74, 131.61, 131.09, 129.72, 127.82, 126.63, 120.35, 116.95, 109.77, 105.73, 79.24, 70.27, 58.97, 57.28, 56.64, 56.46, 56.04, 53.34, 48.94, 48.02, 36.28, 29.78, 28.24, 26.59, 26.26, 25.79, 22.41, 16.24, 14.15 (d,*J* = 10.4 Hz), 13.98 (d,*J* = 10.2 Hz). ¹⁹F NMR (471 MHz, CDCl₃) δ -197.76. HRMS (ESI⁺) m/z, calcd for e₄₆H₅₈ClFN₁₀O₆S₂: 965.3727 [M + H]⁺, found 965.4213.

### Exemplary synthesis of Exemplary compound 18 (XL01120)

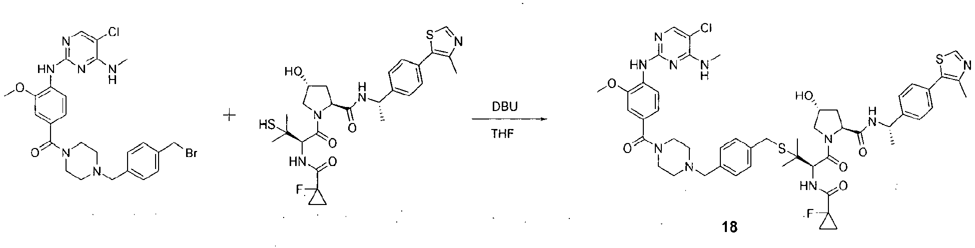

*(2S,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropone-1-carboxamido)-3-methylbutanoyl)-4-hydroxy-N-((S)-1-(4-(4-methylthiazol-5-yl)phenyl)ethyl)pyrrolidine-2-carboxamide* **(18, XL01120)**

**XL01120** was prepared following general procedure 8. 9.5 mg **XL01120** was obtained from 8 mg thiol compound, 65% yield. ¹H NMR (500 MHz, CDCl₃) δ 8.66 (s, 1H), 8.53 (d, *J* = 8.8 Hz, 1H), 7.91 (s, 1H), 7.62 (s, 1H), 7.38 (d, *J* = 8.3 Hz, 2H), 7.34 (d, *J* = 8.3 Hz, 2H), 7.31 - 7.23 (m, 6H), 7.02 - 6.97 (m, 2H), 5.30 *(q, J* = 4.7 Hz, 1H), 5.06 (p, *J* = 7.0 Hz, 1H), 4.71 (t, *J* = 7.8 Hz, 1H), 4.67 (d, *J* = 7.5 Hz, 1H), 4.48 - 4.42 (m, 1H), 3.91 (s, 3H), 3.88 (d, *J* = 11.3 Hz, 1H), 3.82 (q, *J* = 12.4 Hz, 2H), 3.75 - 3.54 (m, 5H), 3.49 (s, 2H), 3.09 (d, *J* = 4.9 Hz, 3H), 2.51 (s, 3H), 2.49 - 2.34 (m, 5H), 2.15 - 2.07 (m, 1H), 1.48 (d, *J* = 6.7 Hz, 6H), 1.40 (d, *J* = 6.9 Hz, 3H), 1.37 - 1.25 (m, 4H); ¹³C NMR (126 MHz, CDCl₃) δ 170.62, 170.33 (d, *J* = 19.0 Hz), 170.22, 169.75, 158.71, 157.95, 152.79, 150.36, 148.66, 147.55, 143.17, 137.08, 136.35, 131.75, 131.49, 131.03, 129.67, 129.60, 129.12, 128.00, 126.63, 120.33, 116.91, 109.75, 105.68, 78.28 (d, *J* = 231.5 Hz), 62.71, 59.06, 56.61, 56.52, 56.02, 53.33, 48.93, 48.85, 36.45, 33.48, 28.21, 25.92, 25.54, 22.30, 16.23, 14.01 (d, *J* = 10.2Hz), 13.94 (d, *J* = 10.2 Hz). ¹⁹F NMR (471 MHz, CDCl₃) δ -197.63. HRMS (ESI⁺) *m*/*z,* calcd for C₅₁H₆₀ClFN₁₀O₆S₂: 1027.3884 [M + H]⁺, found 1027.4164.

### Exemplary synthesis of Exemplary compound 19 (XL01126)

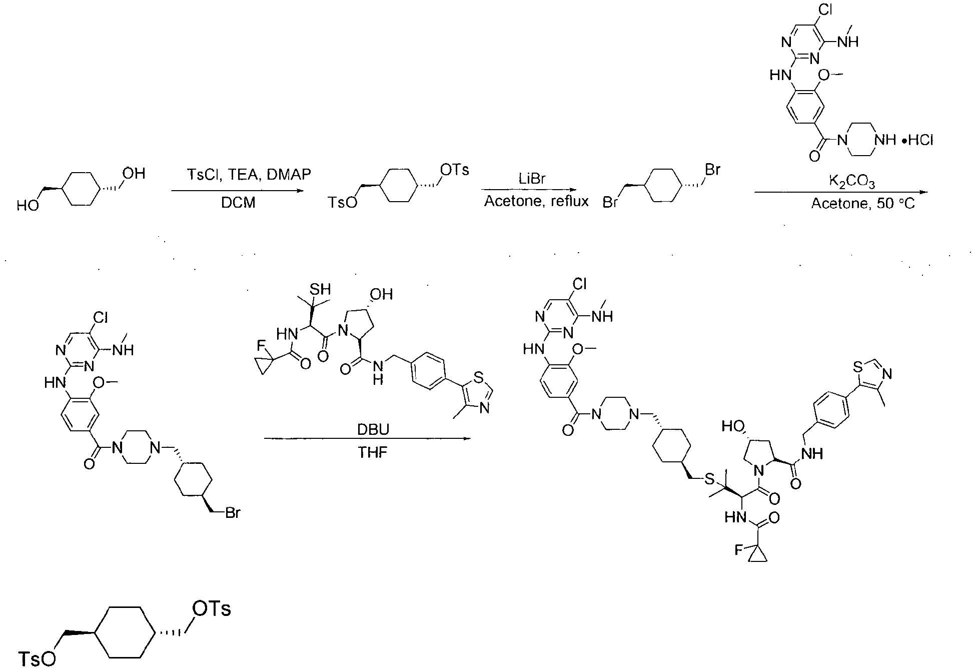

*trans-1,4-bis(p-toluenesulfonyloxymethyl)cyclohexone*

To a solution of trans-1,4-cyclohexanedimethanol (500 mg, 3.47 mmol) in DCM (10 ml) was added TEA (1.93 ml, 13.9 mmol), DMAP (42 mg, 0.34 mmol) and TsCl (1.655g, 8.68 mmol) separately at 0 °C. The resulting mixture was then warmed to room temperature and stirred at room temperature overnight. DCM was added, and the mixture was washed with water and brine, dried over sodium sulfate, filtered, condensed under reduced pressure to afford a residue which was purified through flash column chromatography on silica gel (0%-10% Methanol in DCM) to give *trans-1,4-bis(p-toluenesulfonyloxymethyl)cyclohexane(1.29* g, 82%yield) as white solid. ¹H NMR (400 MHz, CDCl₃) δ 7.76 (d, *J* = 8.3 Hz, 4H), 7.33 (d, *J* = 8.0 Hz, 4H), 3.80 (d, *J* = 6.3 Hz, 4H), 2.45 (s, 6H), 1.83 - 1.63 (m, 4H), 1.65 - 1.50 (m, 2H), 0.99 - 0.78 (m, 4H). ¹³C NMR (101 MHz, CDCl₃) δ 144.84, 133.31, 129.96, 128.01, 74.94, 37.15, 28.22, 21.76. LC-MS, ESI⁺, m/z 475 [M+Na]⁺. (Martin et al., J. Phys. Chem. C 2009, 113, 43, 18884-18890)

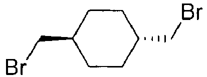

### trans-1,4-Bisbromomethyl-Cyclohexane

To a solution of trans-1,4-bis(p-toluenesulfonyloxymethyl)cyclohexane (125 mg, 0.28 mmol) in acetone (3 ml) was added LiBr (95 mg, 1.1 mmol). After stirring at reflux overnight, the mixture was cooled down, added with ethyl acetate, washed with water and brine, dried over sodium sulfate, filtered, and condensed under reduced pressure to give a residue which was purified with flash column chromatography on silica gel (0% to 20% ethyl acetate in heptane) to give
*trans-1,4-Bisbromomethyl-Cyclohexane* (61 mg, 82% yield) as white solid. ¹H NMR (400 MHz, CDCl₃) δ 3.28 (d, *J* = 6.3 Hz, 4H), 2.03 - 1.87 (m, 4H), 1.67 - 1.55 (m, 2H), 1.19 - 0.91 (m, 4H). ¹³C NMR (101 MHz, CDCl₃) δ 40.03, 39.91, 31.17. *(*Kuck et al., J. Chem. Soc., Perkin Trans. 2, 1990, 251-256) 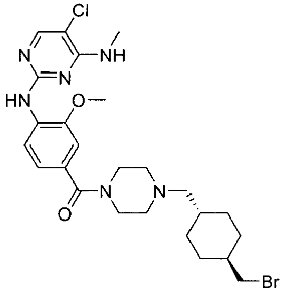 *(4-(((1r,4r)-4-(bromomethyl)cyclohexyl)methyl)piperazin-1-yl)(4-((5-chloro-4-(methylamino)pyrimidin-2-yl)amino)-3-methoxyphenyl)methanone*

To a solution of (4-((5-chloro-4-(methylamino)pyrimidin-2-yl)amino)-3-methoxyphenyl)(piperazin-1-yl)methanone HCl salt (25 mg, 0.06 mmol) in acetone (2 ml) was added trans-1,4-Bisbromomethyl-Cyclohexane (50 mg, 0.18 mmol) and K₂CO₃ (42 mg, 0.30 mmol). After stirring at 50 °C overnight, the mixture was cooled down to room temperature, diluted with DCM, washed with water and brine separately, dried over sodium sulfate, filtered, condensed under reduced pressure to afford a residue which was purified through flash column chromatography on silica gel (0%-10% Methanol in DCM) to yield *(4-(((1r,4r)-4-(bromomethyl)cyclohexyl)methyl)piperazin-1-yl)(4-((5-chloro-4-(methylamino)pyrimidin-2-yl)amino)-3-methoxyphenyl)methanone* as white solid (10 mg, 29% yield). ¹H NMR (400 MHz, CDCl₃) δ 8.54 (d, *J* = 8.8 Hz, 1H), 7.92 (s, 1H), 7.62 (s, 1H), 7.03 - 6.98 (m, 2H), 5.33 - 5.24 (m, 1H), 3.92 (s, 3H), 3.75 - 3.55 (s, 4H), 3.29 *(d, J* = 6.3 Hz, 2H), 3.11 (d, *J* = 4.9 Hz, 3H), 2.40 (s, 4H), 2.16 (d, *J* = 7.8 Hz, 2H), 1.95 - 1.81 (m, 4H), 1.71 - 1.58 (m, 1H), 1.50 - 1.38 (m, 1H), 1.07 - 0.85 (m, 4H). ¹³C NMR (101 MHz, CDCl₃) δ 170.53, 158.72, 157.99, 152.83, 147.56, 131.43, 128.18, 120.31, 116.92, 109.74, 105.66, 65.33, 56.01, 54.03, 40.61, 40.50, 35.07, 31.49, 31.25, 29.45, 28.22. LC-MS, ESI⁺, m/z 567.00 [M+H]⁺. 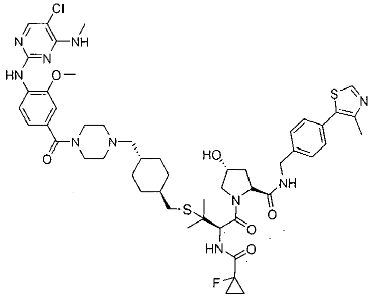 *(2R,4R)-1-((R)-3-((((1R,4R)-4-((4-(4-((5-chloro-4-(methylamino)pyrimidin-2-yl)omino)-3-methoxybenzoyl)piperazin-1-yl)methyl)cyclohexyi)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide **(19, XL01126)***

***XL01126*** was prepared following general procedure 8. 7.7 mg **XL01126** was obtained starting from 7.6 mg thiol compound, 53% yield. ¹H NMR (400 MHz, CDCl₃) δ 8.67 (s, 1H), 8.54 (d, *J* = 8.8 Hz, 1H), 7.92 (s, 1H), 7.62 (s, 1H), 7.41 - 7.30 (m, 5H), 7.22 (dd, *J* = 7.8, 3.3 Hz, 1H), 7.02 - 6.98 (m, 2H), 5.35 - 5.20 (m, 1H), 4.79 (t, *J* = 7.9 Hz, 1H), 4.72 (d, *J* = 7.7 Hz, 1H), 4.53 (s, 1H), 4.46 (d, *J* = 5.9 Hz, 2H), 4.06 (d, *J* = 11.3 Hz, 1H), 3.92 (s, 3H), 3.76 - 3.50 (m, 5H), 3.11 (d, *J* = 4.9 Hz, 3H), 2.72 (s, 1H), 2.52 (s, 3H), 2.52 - 2.45 (m, 1H), 2.44 - 2.31 (m, 6H), 2.28 - 2.19 (m, 1H), 2.11 (d, *J* = 7.1 Hz, 2H), 1.85 - 1.75 (m, 4H), 1.48 - 1.21 (m, 12H), 0.99 - 0.75 (m, 4H). ¹³C NMR (101 MHz, CDCl₃) δ 170.73, 170.64 (d, *J* = 24.8 Hz), 170.20, 158.73, 157.98, 152.82, 150.36, 148.69, 147.56, 138.18, 131.72, 131.45, 131.18, 129.66, 128.18, 120.30, 116.92, 109.74, 105.67, 78.4 (d, *J* = 261.9 Hz), 70.29, 65.41, 58.99, 56.67, 56.38, 56.02, 53.94, 47.66, 43.26, 38.54, 36.76, 35.41, 35.10, 32.83, 32.76, 31.48, 28.22, 25.79, 25.43, 16.27, 14.07 (d, *J* = 17.5 Hz), 14.0 (d, *J* = 17.4 Hz). ¹⁹F NMR (471 MHz, CDCl₃) δ -197.75. HRMS (ESI⁺) *m*/*z,* calcd for C₅₀H₆₄ClFN₁₀O₆S₂: 1019.4197 [M + H]⁺, found 1019.4173.

### Exemplary synthesis of Exemplary compound 20 (XL01134)

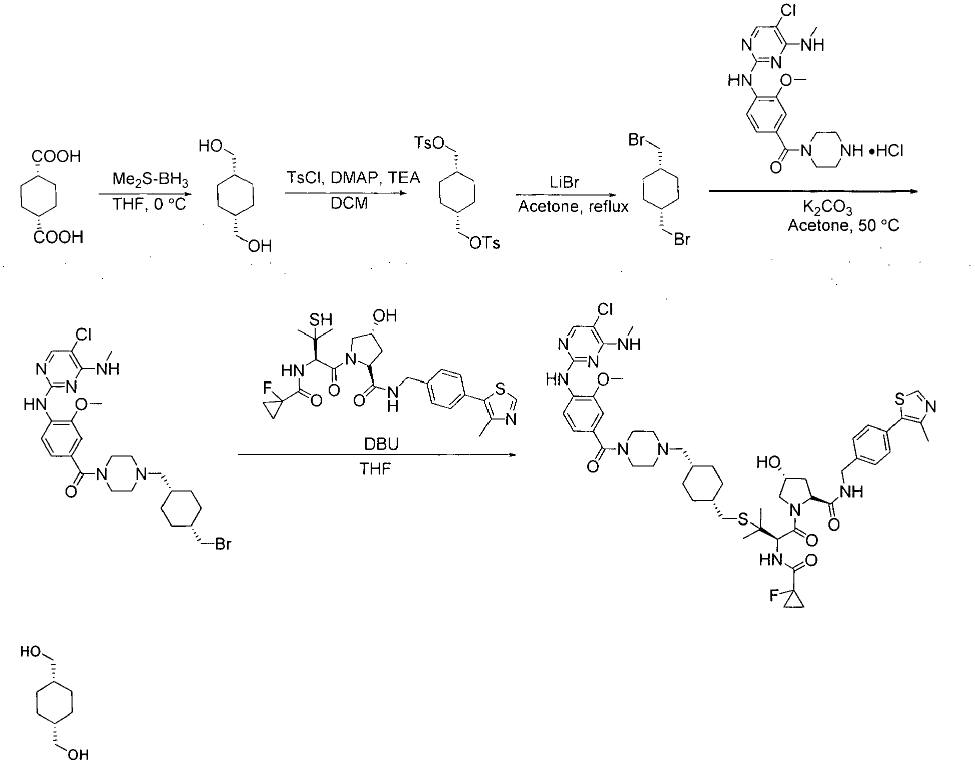

*cis-1,4-cyclohexanedimethanol*

To a solution of cis-cyclohexane-1,4-dicarboxylic acid (500 mg, 2.9 mmol) in THF (5 ml) was added Me₂S-BH₃ (2 M in THF, 5.8 ml, 11.6 mmol) at 0 °C. After stirring at 0 °C for 2h, the reaction mixture was quenched with methanol, then condensed under reduced pressure to afford a residue which was purified with flash column chromatography on silica gel to give *cis-1,4-cyclohexanedimethanol* (418 mg, 100% yield) as a colourless oil. ¹H NMR (400 MHz, CDCl₃) δ 3.54 (d, *J* = 6.9 Hz, 2H), 1.75 -1.63 (m, 2H), 1.60 - 1.50 (m, 2H), 1.46 - 1.37 (m, 2H). ¹³C NMR (101 MHz, CDCl₃) δ 66.23, 38.25, 25.44. 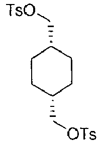 *cis-1,4-bis(p-toluenesulfonyloxymethyl)cyclohexone*

TsCl (437 mg, 2.3 mmol) was added to a mixture of *cis*-1,4-cyclohexanedimethanol (150 mg, 1.04 mmol), TEA (0.58 ml, 4.17 mmol), and DMAP (13 mg, 0.1 mmol) in DCM (20 ml) at 0 °C. The mixture was then warmed to room temperature and stirred for overnight. DCM was added, and the reaction mixture was washed with water and brine, dried over sodium sulfate, filtered, condensed to afford a residue which was purified through flash column chromatography on silica gel (0%-30% ethyl acetate in heptane) to give *cis-1,4-bis(p-toluenesulfonyloxymethyl)cyclohexane* (208 mg, 44% yield) as white solid. ¹H NMR (400 MHz, CDCl₃) δ 7.83 - 7.68 (m, 4H), 7.34 (d, *J* = 8.0 Hz, 4H), 3.85 (d, *J* = 7.1 Hz, 4H), 2.44 (s, 6H), 1.90 - 1.76 (m, 2H), 1.50 - 1.40 (m, 4H), 1.30 - 1.20 (m, 4H). ¹³C NMR (101 MHz, CDCl₃) δ 144.89, 133.19, 129.97, 127.91, 72.84, 34.52, 24.64, 21.71. LC-MS, ESI⁺, m/z 475 [M+Na]⁺. 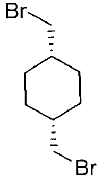 *cis-1,4-Bisbromomethyl-Cyclohexane*

To a solution of *cis*-1,4-bis(p-toluenesulfonyloxymethyl)cyclohexane (150 mg, 0.33 mmol) in acetone (5 ml) was added LiBr (358 mg, 1.32 mmol). After refluxing overnight, the reaction mixture was cooled down to room temperature, diluted with ethyl acetate, washed with water and brine separately, dried over sodium sulfate, filtered, condensed to afford a residue which was purified with flash column chromatography on silica gel (0%-30% ethyl acetate in heptane) to give *cis-1,4-Bisbromomethyl-Cyclohexane* (75 mg, 83% yield) as white solid. ¹H NMR (400 MHz, CDCl₃) δ 3.37 (d, *J* = 7.0 Hz, 4H), 1.92 - 1.80 (m, 2H), 1.71 - 1.58 (m, 4H), 1.57 - 1.45 (m, 4H). ¹³C NMR (101 MHz, CDCl₃) δ 38.20, 37.76, 27.17. 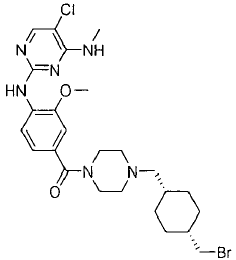 *(4-(((1s,4s)-4-(bromomethyl)cyclohexyl)methyl)piperazin-1-yl)(4-((5-chloro-4-(methylamino)pyrimidin-2-yl)amino)-3-methoxyphenyl)methanone*

To a solution of (4-((5-chloro-4-(methylamino)pyrimidin-2-yl)amino)-3-methoxyphenyl)(piperazin-1-yl)methanone HCl salt (25 mg, 0.06 mmol) in acetone (2 ml) was added *cis*-1,4-Bisbromomethyl-Cyclohexane (50 mg, 0.18 mmol) and K₂CO₃ (50 mg, 0.36 mmol). After stirring at 50 °C overnight, the mixture was cooled down to room temperature, diluted with DCM, washed with water and brine separately, dried over sodium sulfate, filtered, condensed under reduced pressure to afford a residue which was purified through flash column chromatography on silica gel (0%-10% Methanol in DCM) to yield *(4-(((1s,4s)-4-(bromomethyl)cyclohexyl)methyl)piperazin-1-yl)(4-((5-chloro-4-(methylamino)pyrimidin-2-yl)amino)-3-methoxyphenyl)methanone* (10 mg, 29% yield) as white solid. ¹H NMR (400 MHz, CDCl₃) δ 8.54 (d, *J* = 8.8 Hz, 1H), 7.92 (s, 1H), 7.62 (s, 1H), 7.00 (dd, *J* = 6.7, 1.7 Hz, 2H), 5.34 - 5.26 (m, 1H), 3.92 (s, 3H), 3.64 (s, 4H), 3.37 (d, *J* = 7.0 Hz, 2H), 3.10 (d, *J* = 4.9 Hz, 3H), 2.41 (s, 4H), 2.24 (d, *J* = 7.5 Hz, 2H), 1.89 - 1.81 (m, 1H), 1.79 - 1.67 (m, 1H), 1.68 - 1.32 (m, 8H). ¹³C NMR (101 MHz, CDCl₃) δ 170.53, 158.72, 157.98, 152.83, 147.55, 131.43, 128.18, 120.30, 116.91, 109.73, 105.66, 62.04, 56.01, 53.88, 38.99, 38.41, 31.92, 28.22, 27.43, 27.02. LC-MS, ESI⁺, m/z 565, [M+H]⁺, 567 [M+H+2]⁺. 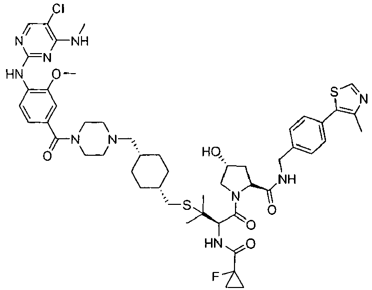 *(2R,4R)-1-((R)-3-((((1S,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide **(20, XL01134)***

***XL01134*** was prepared following general procedure 8. 9.3 mg **XL01134** was obtained starting from 8.0 mg thiol compound, 61% yield. ¹H NMR (500 MHz, CDCl₃) δ 8.67 (s, 1H), 8.54 (d, *J* = 8.6 Hz, 1H), 7.92 (s, 1H), 7.63 (s, 1H), 7.41 - 7.30 (m, 5H), 7.23 (dd, *J* = 7.6, 3.1 Hz, 1H), 7.03 - 6.96 (m, 2H), 5.29 (q, *J* = 4.7 Hz, 1H), 4.79 (t, *J* = 7.9 Hz, 1H), 4.73 (d, *J* = 7.7 Hz, 1H), 4.55 - 4.50 (m, 1H), 4.46 (d, *J* = 5.9 Hz, 2H), 4.06 (d, *J* = 11.1 Hz, 1H), 3.92 (s, 3H), 3.78 - 3.38 (m, 5H), 3.11 (d, *J* = 4.9 Hz, 3H), 2.68 (s, 1H), 2.54 - 2.29 (m, 10H), 2.26 - 2.14 (m, 3H), 1.68 (s, 1H), 1.59 -1.42 (m, 5H), 1.39-1.27 (m, 14H). ¹³C NMR (126 MHz, CDCl₃) δ 170.72, 170.55, 170.20, 158.73, 157.99, 152.82, 150.39, 148.70, 147.57, 138.19, 131.72, 131.46, 131.19, 129.67, 128.18, 120.31, 116.92, 109.75, 105.68, 79.22, 70.30, 62.34, 58.99, 56.64, 56.42, 56.03, 53.91, 47.67, 43.26, 36.76, 35.95, 32.98, 32.12, 28.69, 28.52, 28.22, 27.14, 25.80, 25.49, 16.25, 14.10 (d, *J*= 10.3 Hz), 13.95 (d, *J* = 10.3 Hz). ¹⁹F NMR (471 MHz, CDCl₃) δ -197.74. HRMS (ESI⁺) *m*/*z,* calcd for C₅₀H₆₄ClFN₁₀O₆S₂: 1019.4197 [M + H]⁺,found 1019.4526.

### Exemplary synthesis of Exemplary compound 21 (XL01076)

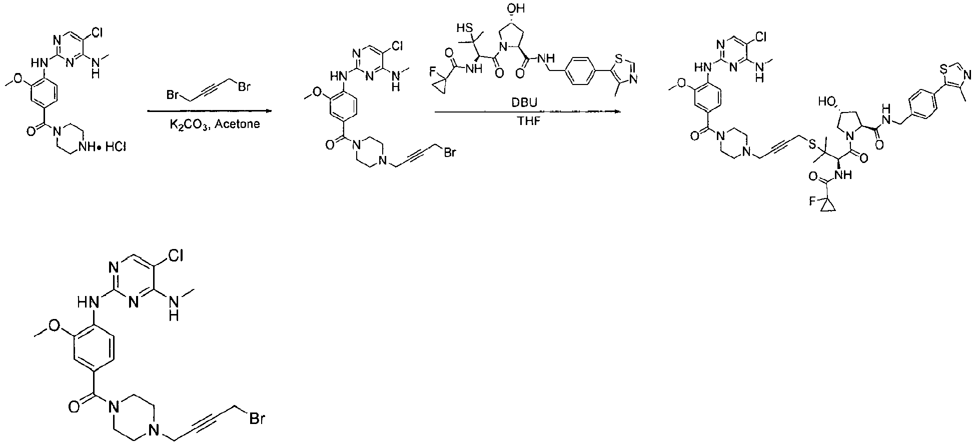

*(4-(4-bromobut-2-yn-1-yl)piperazin-1-yl)(4-((5-chloro-4-(methylamino)pyrimidin-2-yl)omino)-3-methoxyphenyl)methanone*

To a solution of (4-((5-chloro-4-(methylamino)pyrimidin-2-yl)amino)-3-methoxyphenyl)(piperazin-1-yl)methanone HCl salt (60 mg, 0.134 mmol) in acetone (2 ml) was added 1,4-dibromo-2-butyne (85.4 mg, 0.403 mmol) and K₂CO₃ (46.4 mg, 0.336 mmol). After stirring at room temperature for 3h, the resulting mixture was diluted with DCM, washed with water and brine separately, dried over sodium sulfate, filtered, and condensed to afford a residue which was purified on flash column chromatography on silica gel (0%-10% methanol in DCM) to give *(4-(4-bromobut-2-yn-1-yl)piperazin-1-yl)(4-((5-chloro-4-(methylamino)pyrimidin-2-yl)amino)-3-methoxyphenyl)methanone.* The product was unstable and was used to next step as an impure mixture. 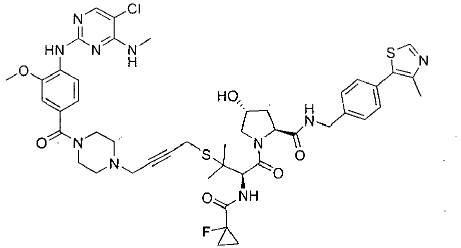 *(2S,4R)-1-((R)-3-((4-(4-(4-((5-chloro-4-(methylamino)pyrimidin-2-yl)amino)-3-methoxybenzoyl)piperazin-1-yl)but-2-yn-1-yl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(21, XL01076)***

*XL01076* was prepared following general procedure 8. 3.1 mg XL01076 was obtatined starting from 5 mg thiol starting material, 34% yield. ¹H NMR (500 MHz, CDCl₃) δ 8.68 (s, 1H), 8.53 (d, *J* = 8.7 Hz, 1H), 7.91 (s, 1H), 7.70 (s, 1H), 7.36 (dd, *J* = 20.1, 8.2 Hz, 4H), 7.25 - 7.17 (m, 2H), 7.03 - 6.97 (m, 2H), 5.36 - 5.27 (m, 1H), 4.91 (d, *J* = 8.0 Hz, 1H), 4.76 - 4.70 (m, 1H), 4.57 - 4.53 (m, 1H), 4.46 (ddd, *J* = 37.5, 15.0, 5.9 Hz, 2H), 4.00 (d, *J* = 11.1 Hz, 1H), 3.92 (s, 3H), 3.85 (dd, *J* = 11.0, 4.3 Hz, 1H), 3.69 (s, 4H), 3.43 - 3.36 (m, 1H), 3.34 - 3.28 (m, 3H), 3.10 (d, *J* = 4.9 Hz, 3H), 2.55 (s, 2H), 2.52 (s, 3H), 2.51 - 2.48 (m, 1H), 2.42 (s, 2H), 2.21 - 2.14 (m, 1H), 1.40 (s, 3H), 1.35 (s, 3H), 1.34 -1.27 (m, 4H); ¹³C NMR (126 MHz, CDCl₃) δ 170.74, 170.71, 170.52 (d, *J* = 20.7 Hz), 169.92, 162.81, 158.76, 157.85, 152.56, 150.53, 148.63, 147.70, 138.15, 131.75, 131.65, 131.20, 129.72, 128.31, 127.66, 120.38, 117.08, 109.82, 105.73, 81.99, 79.20, 77.99, 70.05, 59.17, 56.62, 56.26, 56.08, 52.20, 48.99, 47.36, 43.38, 36.48, 28.26, 25.64, 25.46, 17.53, 16.17, 14.08 (d, *J* = 11.06 Hz), 13.99 (d, *J* = 12.26 Hz); ¹⁹F NMR (471 MHz, CDCl₃) δ -197.70; HRMS (ESI⁺) *m*/*z,* calcd for C₄₆H₅₄ClFN₁₀O₆S₂: 961.3414 [M + H]⁺,found 961.3672.

### Exemplary synthesis of Exemplary compound 22 (XL01123)

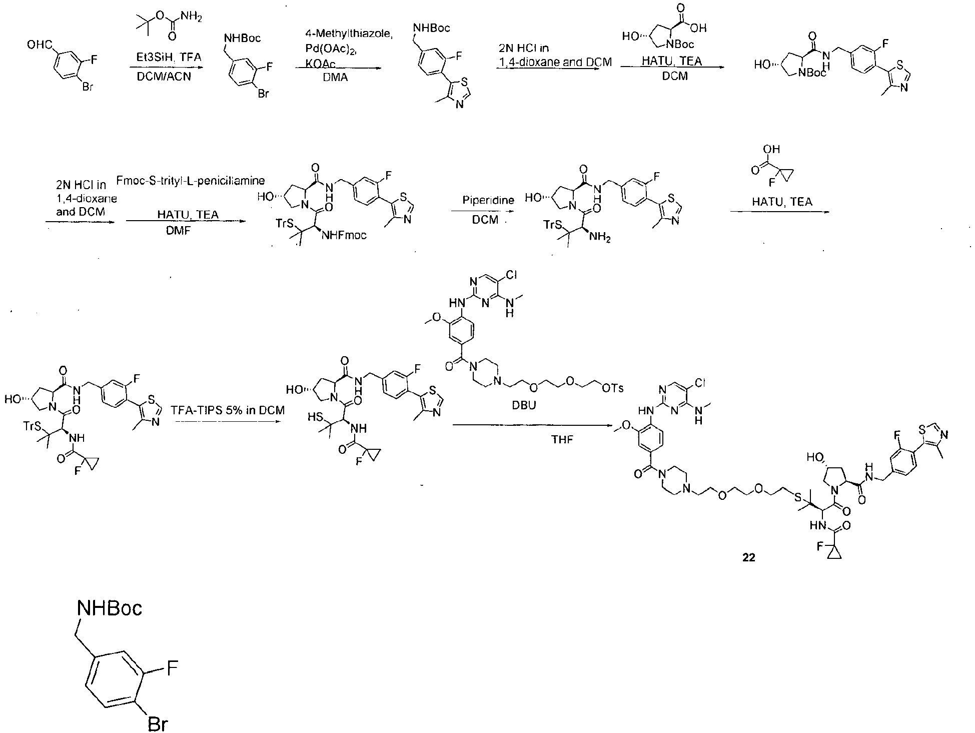

*tert-butyl (4-bromo-3-fluorobenzyl)carbamate*

Trifluoroacetic acid (0.754 ml, 9.9 mmol) was added to a solution of 4-bromo-3-fluorobenzaldehyde (1g, 4.9 mmol), *tert*-butyl carbamate (1.73 g, 14.8 mmol) and triethylsilane (1.72g, 14.8 mmol) in DCM and acetonitrile (10 ml DCM mixed with 30 ml acetonitrile). After stirring at room temperature overnight, the mixture was diluted with DCM, washed with water and brine separately, dried over sodium sulfate, filtered, and condensed to give a residue which was purified via flash column chromatography on silica gel (0%-10% ethyl acetate in heptane) to give *tert-butyl (4-bromo-3-fluorobenzyl)carbamate* (710 mg, 59% yield) as white solid. ¹H NMR (400 MHz, CDCl₃) δ 7.48 (dd, *J* = 8.1, 7.2 Hz, 1H), 7.05 (dd, *J* = 9.3, 1.9 Hz, 1H), 6.94 (dd, *J* = 8.2, 1.4 Hz, 1H), 4.91 (s, 1H), 4.26 (d, *J* = 5.9 Hz, 2H), 1.45 (s, 9H). ¹³C NMR (101 MHz, CDCl₃) δ 159.31 (d, *J* = 246.4 Hz), 155.93, 141.26 (d, *J* = 6.2 Hz), 133.70, 124.14, 115.47 (d, *J* = 22.7Hz), 107.65 (d, *J* = 20.8 Hz), 80.12, 43.89, 28.51. LC-MS, ESI⁺, m/z 247.7 [M-56+H]⁺, 249.7 [M+2-56+H]⁺. 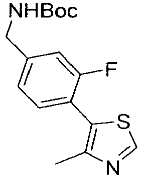 *tert-butyl (3-fluoro-4-(4-methylthiazol-5-yl)benzyl)carbamate was prepared following general procedure 2.* 550 mg *tert-butyl (3-fluoro-4-(4-methylthiazol-5-yl)benzyl)carbamate* was obtained from 865 mg *tert-butyl (4-bromo-3-fluorobenzyl)carbamate,* 60% yield.¹H NMR (400 MHz, CDCl₃) δ 8.73 (s, 1H), 7.33 - 7.27 (m, 1H), 7.14 - 7.05 (m, 2H), 5.14 (s, 1H), 4.33 (d, *J* = 5.8 Hz, 2H), 2.40 (d, *J* = 1.2 Hz, 3H), 1.45 (s, 9H). ¹³C NMR (100 MHz, CDCl₃) δ 159.79 (d, *J* = 248.2 Hz), 155.93, 151.66, 150.91, 142.40 (d, *J* = 7.12 Hz), 132.21 (d, *J* = 2.66 Hz), 124.36, 122.89 (d, *J* = 2.81 Hz), 118.30 (d, *J* = 15.67 Hz), 114.80 (d, *J* = 22.81 Hz), 79.85, 43.89, 28.38, 15.92 (d, *J* = 2.59 Hz). LC-MS, m/z 323.3 [M+H]⁺. 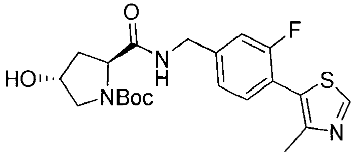 *tert-butyl (2S,4R)-2-((3-fluoro-4-(4-methylthiazol-5*-*yl)benzyl)carbamoyl)-4-hydroxypyrrolidine-1-carboxylate* was prepared following general procedure 3. 550 mg *tert-butyl (2S,4R)-2-((3-fluoro-4-(4-methylthiazol-5-yl)benzyl)carbamoyl)-4-hydroxypyrrolidine-1-carboxylate* was obtained starting from 502 mg *tert-butyl (3-fluoro-4-(4-methylthiazol-5-yl)benzyl)carbamate,* 81% yield. ¹H NMR (400 MHz, DMSO-*d*₆) δ 9.09 (s, 1H), 8.51 (t, *J* = 6.0 Hz, 1H), 7.47 - 7.37 (m, 1H), 7.34 - 7.18 (m, 2H), 5.02 - 4.94 (m, 1H), 4.40 - 4.14 (m, 4H), 3.49 - 3.37 (m, 1H), 3.31 (d, *J* = 10.8 Hz, 1H), 2.32 (s, 3H), 2.16 - 2.00 (m, 1H), 1.91 -1.82 (m, 1H), 1.41 (s, 3H), 1.26 (s, 6H). LC-MS, ESI⁺, m/z 436.5 [M+H]⁺. 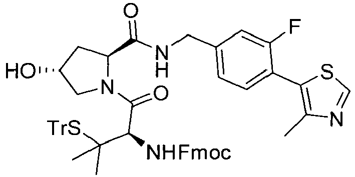 *(9H-fluoren-9-yl)methyl ((R)-1-((2R,4R)-2-((3-fluoro-4-(4-methylthiazol-5-yl)benzyl)carbamoyl)-4-hydroxypyrrolidin-1-yl)-3-methyl-1-oxo-3-(tritylthio)butan-2-yl)carbamate* was prepared following general procedure 4. 104 mg *(9H-fluoren-9-yl)methyl ((R)-1-((2R,4R)-2-((3-fluoro-4-(4-methylthiazol-5-yl)benzyl)carbamoyl)-4-hydroxypyrrolidin-1-yl)-3-methyl-1-oxo-3-(tritylthio)butan-2-yl)carbamate* was obtained from 119 mg *tert-butyl (2S,4R)-2-((3-fluoro-4-(4-methylthiazol-5-yl)benzyl)carbamoyl)-4-hydroxypyrrolidine-1-carboxylate,* 41% yield. ¹H NMR (400 MHz, CDCl₃) δ 8.79 (s, 1H), 7.77 - 7.72 (m, 2H), 7.61 - 7.47 (m, 8H), 7.40 - 7.33 (m, 3H), 7.26 - 7.15 (m, 12H), 7.07 - 6.96 (m, 2H), 5.64 (d, *J* = 5.8 Hz, 1H), 4.67 (t, *J* = 8.0 Hz, 1H), 4.45 - 4.24 (m, 4H), 4.23 - 4.13 (m, *J* = 18.5, 6.6 Hz, 2H), 3.68 (d, *J* = 5.9 Hz, 1H), 3.52 (d, *J* = 11.4 Hz, 1H), 3.31 (dd, *J* = 11.4, 3.6 Hz, 1H), 2.77 (s, 1H), 2.42 (s, 3H), 2.38 - 2.28 (m, 1H), 2.19 - 2.08 (m, 1H), 1.22 (s, 3H), 0.99 (s, 3H); ¹³C NMR (101 MHz, CDCl₃) δ 171.23, 170.53, 159.80 (d, *J* = 248 Hz), 156.52, 151.81, 150.99, 144.48, 143.93, 143.57, 141.70 (d, *J* = 7.32 Hz), 141.43, 141.38, 132.14 (d, *J* = 2.1 Hz), 129.97, 127.96, 127.26, 127.05, 125.23, 125.09, 124.59, 123.12 (d, *J* = 1.96 Hz), 120.17, 118.19 (d, *J* = 15.3 Hz), 115.05 (d, *J* = 22.7 Hz), 70.22, 68.70, 67.71, 59.07, 58.29, 56.81, 54.44, 47.09, 42.51, 36.90, 26.04, 25.45, 16.13 (d, *J* = 2.35 Hz). LC-MS, ESI⁺, m/z 931 [M+H]⁺. 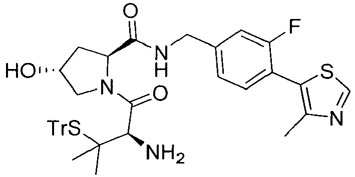 *(2R,4R)-1-((R)-2-amino-3-methyl-3-(tritylthio)butanoyl)-N-(3-fluoro-4-(4-methylthiazol-5-yl)benzyl)-4-hydroxypyrrolidine-2-carboxamide* was prepared following general procedure 5. 86 mg *(2R,4R)-1-((R)-2-amino-3-methyl-3-(tritylthio)butanoyl)-N-(3-fluoro-4-(4-methylthiazol-5-yl)benzyl)-4-hydroxypyrrolidine-2-carboxamide* was obtained from 150 mg Fmoc protected compound, 75% yield. ¹H NMR (400 MHz, MeOD-*d*₄) δ 8.96 (s, 1H), 7.62 - 7.53 (m, 6H), 7.35 - 7.23 (m, 7H), 7.22 - 7.15 (m, 5H), 4.45 (t, *J* = 8.2 Hz, 1H), 4.40 - 4.22 (m, 3H), 3.24 (dd, *J* = 11.2, 4.1 Hz, 1H), 3.06 (d, *J* = 11.2 Hz, 1H), 2.72 (s, 1H), 2.34 (s, 3H), 2.16 - 2.07 (m, 1H), 2.00 - 1.90 (m, 1H), 1.25 (s, 3H), 1.17 (s, 3H); ¹⁹F NMR (471 MHz, MeOD-*d*₄) δ -114.52. ¹³C NMR (126 MHz, CDCl₃) δ 172.43, 171.75, 159.82 (d, *J* = 249.9 Hz), 151.81, 151.01, 144.87, 141.79 (d, *J* = 7.2 Hz), 132.15 (d, *J* = 2.1 Hz), 130.03, 127.98, 126.95, 124.53, 122.95 (d, *J* = 2.9 Hz), 118.17 (d, *J* = 15.5 Hz), 114.86 (d, *J* = 23.1 Hz), 70.06, 68.29, 59.17, 58.32, 58.07, 56.94, 42.42, 37.30, 25.25, 24.74, 16.16 (d, *J* = 2.1 Hz); LC-MS, ESI⁺, m/z 731.6 [M+Na]⁺. 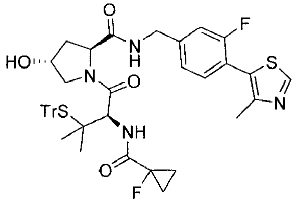 *(2 R,4R)-N-(3-fluoro-4-(4-methylthiazol- 5-yl)benzyl)-1-((R)-2-(1-fluorocyclopropane-1-carboxamido)-3-methyl-3-(tritylthio)butanoyl)-4-hydroxypyrrolidine-2-carboxamide* was prepared following general procedure 6. 77 mg *(2R,4R)-N-(3-fluoro-4-(4-methylthiazol-5-yl)benzyl)-1-((R)-2-(1-fluorocyclopropone-1-carboxamido)-3-methyl-3-(tritylthio)butanoyl)-4-hydroxypyrrolidine-2-carboxamide* was obtained from 85 mg amine compound , 81% yield. ¹H NMR (500 MHz, CDCl₃) δ 8.79 (s, 1H), 7.65 - 7.52 (m, 6H), 7.46 (t, *J* = 6.1 Hz, 1H), 7.34 - 7.18 (m, 11H), 7.03 (d, *J* = 9.2 Hz, 2H), 4.65 (t, *J* = 8.1 Hz, 1H), 4.38 (s, 1H), 4.26 (d, *J* = 6.1 Hz, 2H), 3.66 (d, *J* = 5.2 Hz, 1H), 3.50 (d, *J* = 11.4 Hz, 1H), 3.41 (d, *J* = 5.6 Hz, 1H), 3.31 (dd, *J* = 11.4, 3.8 Hz, 1H), 2.41 (d, *J* = 1.0 Hz, 3H), 2.27 - 2.20 (m, 1H), 2.16 - 2.10 (m, 1H), 1.41 - 1.20 (m, 7H), 1.10 (s, 3H); ¹³C NMR (126 MHz, CDCl₃) δ 171.17, 170.22 (d, *J* = 20.8 Hz), 170.14, 169.88, 159.78 (d, *J* = 250.0 Hz), 151.77, 150.98, 144.49, 141.68 (d, *J* = 7.7 Hz), 132.15, 129.88, 127.96, 127.01, 124.56, 123.16 (d, *J* = 3.1 Hz), 118.20 (d, *J* = 15.5 Hz), 115.08 (d, *J* = 23.1 Hz), 78.24 (d, *J* = 232.5Hz), 70.23, 68.53, 59.01, 57.08, 56.92, 54.09, 42.54, 36.90, 26.03, 25.64, 16.10 (d, *J* = 2.48 Hz), 13.72 (d, *J* = 10.0 Hz), 13.69 (d, *J* = 10.0 Hz). ¹⁹F NMR (471 MHz, CDCl₃) δ -112.01, -197.22. LC-MS, ESI⁻, m/z 793 [M-H]⁻. 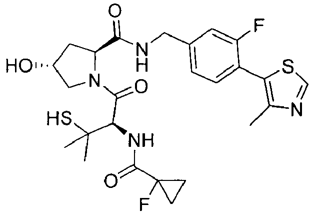 *(2S,4R)-N-(3-fluoro-4-(4-methylthiazol-5-yl)benzyl)-1-((R)-2-(1-fluorocyclopropane-1-carboxamido)-3-mercapto-3-methylbutanoyl)-4-hydroxypyrrolidine-2-carboxamide* was prepared following general procedure 7. 27 mg *(2S,4R)-N-(3-fluoro-4-(4-methylthiazol-5-yl)benzyl)-1-((R)-2-(1-fluorocyclopropane-1-carboxamido)-3-mercapto-3-methylbutanoyl)-4-hydroxypyrrolidine-2-carboxamide* was obtained from 40 mg trityl protected compound, 97%. ¹H NMR (500 MHz, CDCl₃) δ 9.04 (s, 1H), 7.53 (t, *J* = 5.7 Hz, 1H), 7.43 (dd, *J* = 8.5, 3.0 Hz, 1H), 7.29 - 7.24 (m, 1H), 7.20 - 7.13 (m, 2H), 5.67 (br, s, 1H), 4.76 -4.66 (m, 2H), 4.60 (dd, *J* = 15.5, 6.8 Hz, 1H), 4.56 - 4.52 (m, 1H), 4.32 (dd, *J* = 15.5, 5.3 Hz, 1H), 4.08 (d, *J* = 11.1 Hz, 1H), 3.81 (dd, *J* = 11.1, 3.7 Hz, 1H), 2.62 (s, 1H), 2.42 (s, 3H), 2.35 (ddd, *J* = 13.0, 8.4, 4.4 Hz, 1H), 2.24 - 2.16 (m, 1H), 1.43 (s, 3H), 1.38 (s, 3H), 1.36 -1.25 (m, 4H). ¹³C NMR (126 MHz, CDCl₃) δ 171.32, 170.6 (d, *J* = 20.7 Hz), 170.22, 159.8 (d, *J* = 251.0 Hz), 152.76, 149.28, 142.32 (d, *J* = 7.7 Hz), 132.14 (d, *J* = 2.3 Hz), 125.65, 123.55 (d, *J* = 4.4 Hz), 117.44 (d, *J* = 15.2 Hz), 115.38 (d, *J* = 22.9 Hz), 77.27 (d, *J* = 232.7 Hz), 70.26, 59.37, 57.81, 56.88, 46.55, 42.90, 37.12, 30.47, 28.83, 15.40, 14.05 (d, J = 10.5 Hz), 13.99 (d, *J* = 9.7 Hz).¹⁹F NMR (471 MHz, CDCl₃) δ -111.74, - 197.61. LC-MS, ESI⁺, m/z 553.4 [M+H]+. 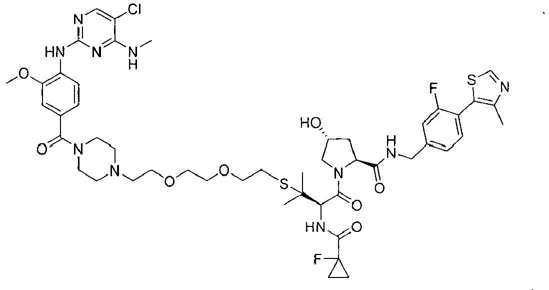 *(2S,4R)-1-((R)-3-((2-(2-(2-(4-(4-((5-chloro-4-(methylamino)pyrimidin-2-yl)amino)-3-methoxybenzoyl)piperazin-1-yl)ethoxy)ethoxy)ethyl)thio)-2-(1-fluorocyclopropane-1-carboxamido)-3-methylbutanoyl)-N-(3-fluoro-4-(4-methylthiazol-5-yl)benzyl)-4-hydroxypyrrolidine-2-carboxamide* **(22, XL01123)**

**XL01123** was prepared following general procedure 8. 5 mg **XL01123** obtained from 8 mg thiol compound, 40% yield. ¹H NMR (500 MHz, CDCl₃) δ 8.76 (s, 1H), 8.54 (d, *J* = 8.7 Hz, 1H), 7.92 (s, 1H), 7.63 (s, 1H), 7.44 (t, *J* = 5.9 Hz, 1H), 7.32 - 7.27 (m, 1H), 7.18 (dd, *J* = 8.3, 3.1 Hz, 1H), 7.15 - 7.11 (m, 2H), 7.02 - 6.98 (m, 2H), 5.32 - 5.27 (m, 1H), 4.87 (d, *J* = 8.1 Hz, 1H), 4.76 (dd, *J* = 8.4, 6.2 Hz, 1H), 4.58 - 4.52 (m, 1H), 4.50 - 4.40 (m, 2H), 3.92 (s, 3H), 3.91 - 3.88 (m, 2H), 3.80 - 3.45 (m, 13H), 3.10 (d, *J* = 4.9 Hz, 3H), 2.85 - 2.72 (m, 2H), 2.61 (s, 2H), 2.57 - 2.43 (m, 5H), 2.42 (d, *J* = 1.1 Hz, 3H), 2.21 - 2.14 (m, 1H), 1.36 (s, 3H), 1.34 (s, 3H), 1.33 - 1.27 (m, 4H); ¹³C NMR (126 MHz, CDCl₃) δ 171.05, 170.65, 170.21, 170.14, 163.36, 158.75, 157.93, 152.75, 151.85, 151.07, 147.62, 141.50, 132.40 (d, *J* = *2.5 Hz),* 131.61, 127.76, 124.51, 123.63 (*J* = *3.3 Hz),* 120.37, 118.59 (d, *J* = *15.7 Hz),* 116.99, 115.5 *(d, J* = *22.9 Hz),* 109.76, 105.71, 79.22, 70.56, 70.45, 70.22, 69.67, 68.65, 59.26, 57.77, 56.19, 56.06, 55.96, 53.63, 47.98, 42.88, 36.60, 28.89, 28.25, 26.00, 25.20, 16.13, 14.04 (d, *J* = 9.85 Hz), 13.96 (d, *J* = 9.84 Hz); HRMS (ESI⁺) *m*/*z,* calcd for C₄₈H₆₁ClF₂N₁₀O₈S₂: 1043.3845 [M + H]⁺, found 1043.3733.

### Exemplary synthesis of Exemplary compound 23 (XL01122)

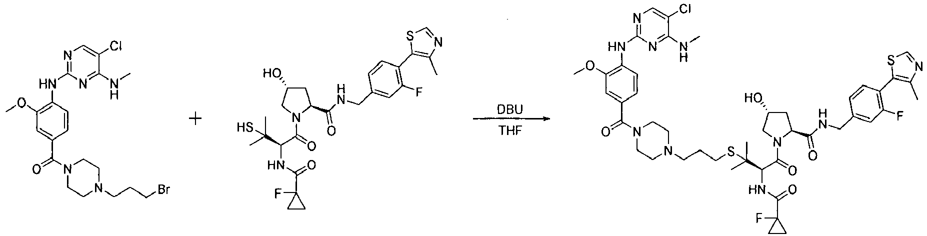

*(2S,4R)-1-((R)-3-((3-(4-(4-((5-chloro-4-(methylamino)pyrimidin-2-yl)amino)-3-methoxybenzoyl)piperazin-1-yl)propyl)thio)-2-(1-fluorocyclopropane-1-carboxamido)-3-methylbutanoyl)-N-(3-fluoro-4-(4-methylthiazol-5-yl)benzyl)-4-hydroxypyrrolidine-2-carboxamide* **(23, XL01122)**

**XL01122** was prepared following general procedure 8. 1.4 mg **XL01122** was obtained from 8 mg thiol compound, 10%. ¹H NMR (500 MHz, CDCl₃) δ 8.76 (s, 1H), 8.54 (d, *J* = 8.1 Hz, 1H), 7.93 (s, 1H), 7.65 (s, 1H), 7.36 - 7.28 (m, 2H), 7.23 (dd, *J* = 7.8, 3.2 Hz, 1H), 7.16 - 7.10 (m, 2H), 7.02 - 6.97 (m, 2H), 5.32 - 5.27 (m, 1H), 4.81 - 4.70 (m, 2H), 4.59 - 4.47 (m, 2H), 4.41 (dd, *J* = 15.3, 5.7 Hz, 1H), 4.08 (d, *J* = 11.4 Hz, 1H), 3.93 (s, 3H), 3.76 - 3.52 (m, 5H), 3.11 (d, *J* = 4.9 Hz, 3H), 2.62 - 2.47 (m, 4H), 2.47 - 2.36 (m, 9H), 2.26 - 2.20 (m, 1H), 1.74 - 1.64 (m, 2H), 1.37 (s, 3H), 1.36 (s, 3H), 1.34 - 1.26 (m, 4H); ¹³C NMR (126 MHz, CDCl₃) δ 170.81, 170.64, 170.61, 170.26, 158.75, 157.95, 152.78, 151.88, 151.12, 147.62, 141.46, 141.41, 132.45, 132.43, 131.61, 124.41, 123.55 (d, *J* = 3.0 Hz), 120.35, 116.95, 115.42 (d, *J* = 22.4 Hz), 109.77, 105.73, 79.24, 70.31, 59.00, 57.24, 56.69, 56.45, 56.05, 53.27, 47.98, 42.95, 36.62, 28.24, 26.17, 25.73, 25.53, 16.15, 14.15 (d, J = 10.6 Hz), 14.00 (d, *J* = 10.5 Hz); HRMS (ESI⁺) *m*/*z,* calcd for C₄₅H₅₅ClF₂N₁₀O₆S₂: 969.3477 [M + H]⁺, found 969.3772.

### Exemplary synthesis of Exemplary compound 24 (XL01121)

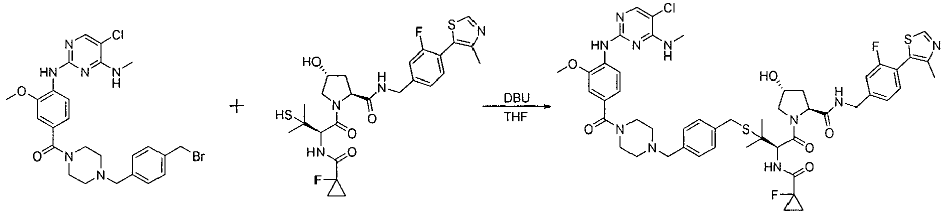

*(2S,4R)-1-((R)-3-((4-((4-(4-((5-chloro-4-(methylamino)pyrimidin-2-yl)amino)-3-methoxybenzoyl)piperazin-1-yl)methyl)benzyl)thio)-2-(1-fluorocyclopropane-1-carboxamido)-3-methylbutanoyl)-N-(3-fluoro-4-(4-methylthiazol-5-yl)benzyl)-4-hydroxypyrrolidine-2-carboxamide (**24**, **XL01121)***

**XL01121** was prepared following general procedure 8. 12.2 mg **XL01121** was obtained starting from 8 mg thiol compound, 83%. ¹H NMR (500 MHz, CDCl₃) δ 8.79 (s, 1H), 8.57 (d, *J* = 8.8 Hz, 1H), 7.95 (s, 1H), 7.66 (s, 1H), 7.41 (t, *J* = 6.0 Hz, 1H), 7.32 - 7.26 (m, 6H), 7.12 (d, *J* = 9.1 Hz, 2H), 7.06 - 7.00 (m, 2H), 5.34 (q, *J* = 4.7 Hz, 1H), 4.76 (t, *J* = 7.9 Hz, 1H), 4.66 (d*, J* = 7.5 Hz, 1H), 4.51 - 4.48 (m, 1H), 4.49 - 4.43 (m, 1H), 4.38 (dd, *J* = 15.4, 5.8 Hz, 1H), 3.95 (s, 3H), 3.90 (d, J = 11.2 Hz, 1H), 3.80 (dd, *J* = 12.5 Hz, 2H), 3.75 - 3.57 (m, 5H), 3.51 (s, 2H), 3.14 (d, *J* = 4.9 Hz, 3H), 2.56 - 2.38 (m, 8H), 2.24 - 2.16 (m, 1H), 1.47 (s, 3H), 1.43 (s, 3H), 1.39 - 1.29 (m, 4H); ¹³C NMR (126 MHz, CDCl₃) δ 171.04, 170.62, 170.46 (d, *J* = 20.9 Hz), 170.13, 159.86 (d, *J* = 250.1 Hz), 158.71, 157.95, 152.79, 151.82, 151.05, 147.54, 141.49 (d, *J* = 7.0 Hz), 137.04, 136.41, 132.33 (d, *J* = 2.4 Hz), 131.49, 129.60, 129.03, 127.99, 124.45, 123.39 (d, *J* = 3.5 Hz), 120.32, 118.51 (d, *J* = 15.7 Hz), 116.92, 115.30 (d, *J* = 23.3 Hz), 109.74, 105.67, 78.28 (d, *J* = 229.1 Hz), 70.17, 62.69, 59.15, 56.66, 56.47, 56.02, 53.30, 48.94, 42.84, 36.86, 33.39, 28.21, 25.75, 25.36, 16.11 (d, *J* = 2.8 Hz), 13.96 (d, J = 10.8 Hz), 13.94 (d, *J* = 10.9 Hz). HRMS (ESI⁺) *m*/*z,* calcd for C50H57ClF2N10O6S2: 1031.3633 [M + H]⁺, found 1031.3403.

### Exemplary synthesis of Exemplary compound 25 (XL01131)

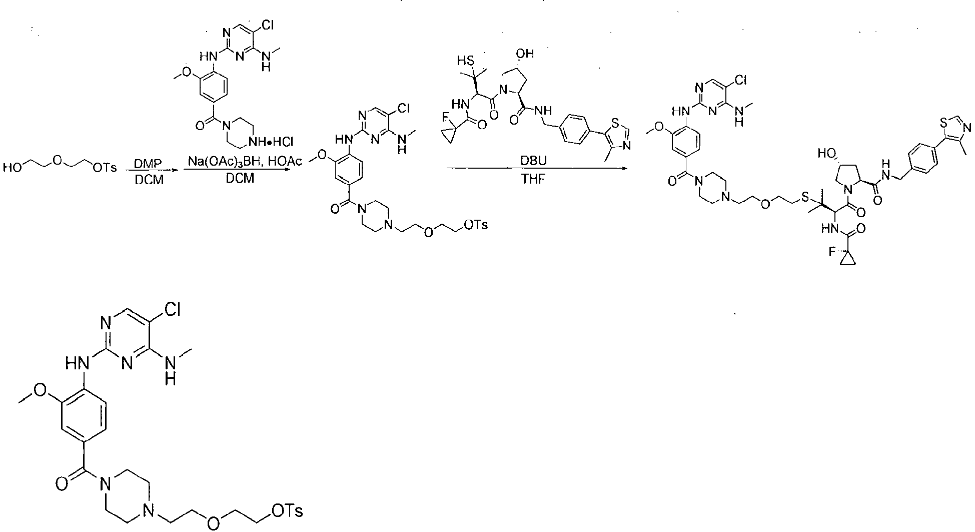

*2-(2-(4-(4-((5-chloro-4-(methylamino)pyrimidin-2-yl)amino)-3-methoxybenzoyl)piperazin-1-yl)ethoxy)ethyl 4-methylbenzenesulfonate*

To a solution of 2-(2-Hydroxyethoxy)ethyl 4-methylbenzenesulfonate (100 mg, 0.38 mmol) in DCM (3 ml) was added Dess-Martin Periodinane (195 mg, 0.46 mmol). After stirring at room temperature for 1h, the reaction mixture was diluted with DCM, washed with water and brine, dried over sodium sulfate, filtered, and condensed to afford a residue which was used to next step without further purification. Half of the residue from the last step was dissolved in DCM (4 ml), followed by addition of (4-((5-chloro-4-(methylamino)pyrimidin-2-yl)amino)-3-methoxyphenyl)(piperazin-1-yl)methanone HCl salt (63 mg, 0.15 mmol), Sodium triacetoxyborohydride (72 mg, 0.34 mmol), and HOAc (1 drop). After stirring at room temperature for 2h, the mixture was diluted with DCM, washed with water and brine, dried over sodium sulfate, filtered, condensed to afford a residue which was purified with flash column chromatography on silica gel (0%-10% methanol in DCM) to give *2-(2-(4-(4-((5-chloro-4-(methylamino)pyrimidin-2-yl)amino)-3-methoxybenzoyl)piperazin-1-yl)ethoxy)ethyl 4-methylbenzenesulfonate* (46 mg, 49% yield). ¹H NMR (400 MHz, CDCl₃) δ 8.54 (d, *J* = 8.9 Hz, 1H), 7.92 (s, 1H), 7.81 - 7.75 (m, 2H), 7.68 (s, 1H), 7.32 (d, *J* = 8.0 Hz, 2H), 7.03 - 6.95 (m, 2H), 5.34 - 5.29 (m, 1H), 4.17 - 4.13 (m, 2H), 3.92 (s, 3H), 3.74 - 3.58 (m, 8H), 3.14 - 3.07 (m, 3H), 2.64 (t, *J* = 5.4 Hz, 2H), 2.60 - 2.51 (m, 4H), 2.43 (s, 3H). ¹³C NMR (101 MHz, CDCl₃) δ 170.63, 158.74, 157.90, 152.68, 147.64, 145.03, 133.26, 131.59, 129.99, 128.07, 127.79, 120.34, 117.01, 109.76, 105.65, 69.23, 68.95, 68.73, 57.61, 56.04, 53.60, 28.24, 21.76. LC-MS, ESI⁺, m/z 619.35 [M+H]+. 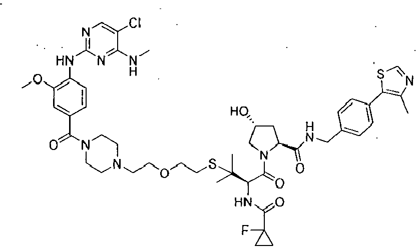 *(2S,4R)-1-((R)-3-((2-(2-(4-(4-((5-chloro-4-(methylamino)pyrimidin-2-yl)amino)-3-methoxybenzoyl)piperazin-1-y*/*)ethoxy)ethy*/*)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(25, XL01131)***

**XL01131** was prepared following general procedure 8. 5.9 mg **XL01131** was obtained starting from 8 mg thiol comound, 40% yield. ¹H NMR (500 MHz, CDCl₃) δ 8.70 (s, 1H), 8.55 (d, *J* = 8.5 Hz, 1H), 7.94 (s, 1H), 7.71 (s, 1H), 7.39 (dd, *J* = 20.4, 8.2 Hz, 4H), 7.31 (d, *J* = 5.9 Hz, 1H), 7.25 (dd, *J* = 8.1, 3.2 Hz, 1H), 7.05 - 6.99 (m, 2H), 5.37 - 5.31 (m, 1H), 4.82 (d, *J* = 8.1 Hz, 1H), 4.80 - 4.74 (m, 1H), 4.58 - 4.52 (m, 1H), 4.48 (d, *J* = 5.9 Hz, 2H), 3.99 (d, *J* = 12.1 Hz, 1H), 3.95 (s, 3H), 3.83 (dd, *J* = 10.9, 4.3 Hz, 1H), 3.79 - 3.65 (m, 4H), 3.61 (t, *J* = 5.4 Hz, 2H), 3.59 - 3.55 (m, 1H), 3.53 - 3.48 (m, 1H), 3.36 (s, 2H), 3.13 (d, *J* = 4.9 Hz, 3H), 2.80 - 2.70 (m, 2H), 2.68 (t, *J* = 5.4 Hz, 2H), 2.62 (s, 2H), 2.54 (s, 3H), 2.52 - 2.46 (m, 1H), 2.25 - 2.18 (m, 1H), 1.40 - 1.31 (m, 10H); ¹³C NMR (126 MHz, CDCl₃) δ 170.80, 170.69, 170.46 (d, *J* = 20.8 Hz), 169.99, 158.76, 157.87, 152.62, 150.48, 148.64, 147.69, 138.22, 131.73, 131.64, 131.17, 129.67, 128.27, 127.64, 120.36, 117.05, 109.80, 105.74, 79.22, 70.32, 70.04, 68.59, 59.18, 57.50, 56.49, 56.22, 56.06, 53.54, 48.16, 43.29, 36.66, 28.48, 28.25, 25.87, 25.52, 16.20, 14.06 (d, *J* = 10.6 Hz), 13.97 (d, *J* = 10.3 Hz). HRMS (ESI⁺) *m*/*z,* calcd for C₄₆H₅₈ClFN₁₀O₇S₂: 981.3676 [M + H]⁺, found 981.4273.

### Exemplary synthesis of Exemplary compound 26 (XL01140)

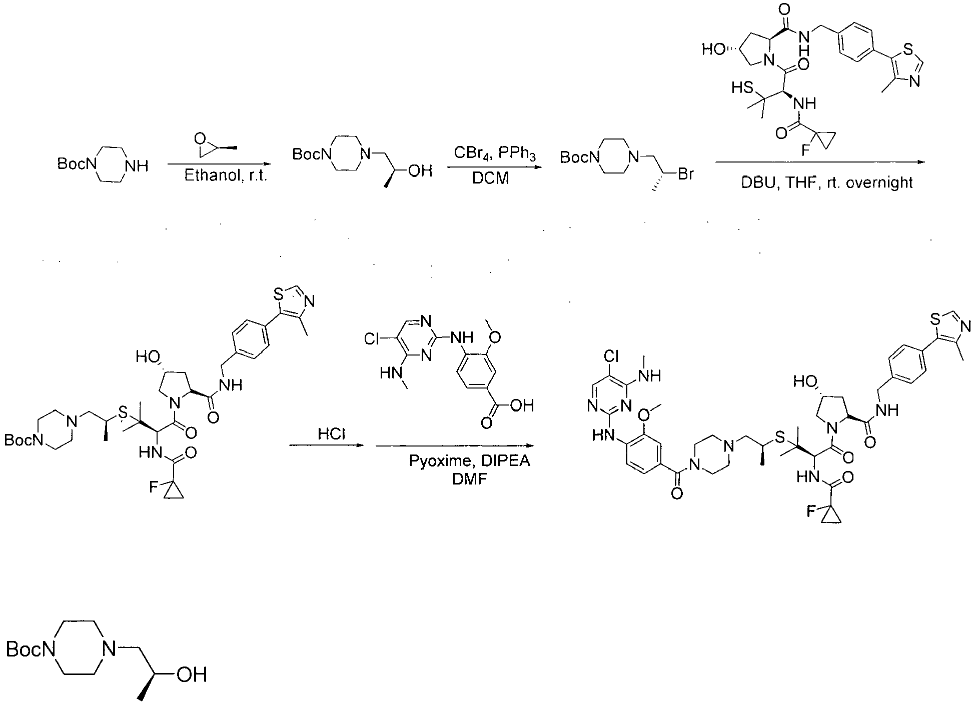

A mixture of *tert*-butyl piperazine-1-carboxylate (500 mg, 2.69 mmol), propylene oxide (234 mg, 4.0 mmol), and ethanol (3 ml) was stirred in a sealed microwave tube at room temperature overnight. The mixture was condensed under reduced pressure to afford a residue which was purified with flash column chromatography on silica gel (0%-10% methanol in DCM) to give *tert*-butyl (5)-4-(2-hydroxypropyl)piperazine-1-carboxylate (390 mg, 60% yield). ¹H NMR (400 MHz, CDCl₃) δ 3.93 - 3.73 (m, 1H), 3.48 - 3.34 (m, 4H), 3.30 (s, 1H), 2.66 - 2.53 (m, 2H), 2.36 - 2.17 (m, 4H), 1.43 (s, 9H), 1.11 (d, *J* = 6.1 Hz, 3H). ¹³C NMR (101 MHz, CDCl₃) δ 154.80, 79.80, 65.91, 62.45, 53.17, 43.81, 28.53, 20.09. LC-MS, ESI⁺, m/z 245.1 [M+H]+.

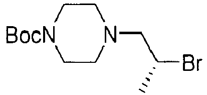

To a solution of *tert*-butyl (*S*)-4-(2-hydroxypropyl)piperazine-1-carboxylate (150 mg, 0.61 mmol) and CBr₄ (367 mg, 1.1 mmol) in DCM (6 ml) was added triphenyl phosphine (298 mg, 1.1 mmol) at 0 °C. After stirring at 0 °C for 1h, NH₄Cl aqueous was added and the mixture was extracted with DCM (3x).

The combined organic phase was washed with water and brine separately, dried over sodium sulfate, filtered, and condensed to afford a residue which was purified through flash column chromatography on silica gel (0%-50% ethyl acetate in heptane) to give *tert*-butyl (*R*)-4-(2-bromopropyl)piperazine-1-carboxylate (64 mg, 34% yield). ¹H NMR (500 MHz, CDCl₃) δ 4.21 - 4.04 (m, 1H), 3.42 (t, *J* = 5.0 Hz, 4H), 2.75 (dd, *J* = 13.2, 6.6 Hz, 1H), 2.54 (dd, *J* = 13.2, 7.5 Hz, 1H), 2.48 - 2.37 (m, 4H), 1.71 (d, *J* = 6.6 Hz, 3H), 1.45 (s, 9H). ¹³C NMR (126 MHz, CDCl₃) δ 154.88, 79.78, 67.00, 53.38, 46.62, 43.89, 28.58, 24.24. LC-MS, ESI⁺,m/z 306.9 [M+H]⁺, 308.8 [M+H+2]⁺. 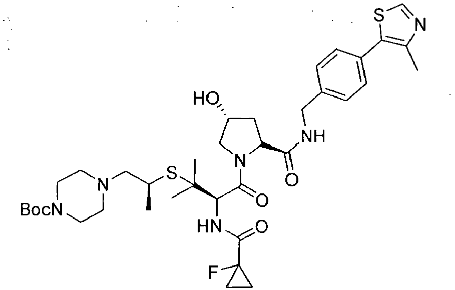 *tert-butyl 4-((S)-2-(((R)-3-(1-fluorocyclopropane-1-carboxamido)-4-((2R,4R)-4-hydroxy-2-((4-(4-methylthiazol-5-yl)benzyl)carbamoyl)pyrrolidin-1-yl)-2-methyl-4-oxobuton-2-y*/*)thio)propyl)piperazine-1-carboxylate* was prepared following general procedure 8. 16 mg *tert-butyl 4-((S)-2-(((R)-3-(1-fluorocyclopropane-1-carboxamido)-4-((2R,4R)-4-hydroxy-2-((4-(4-methylthiazol-5-y*/*)benzyl)carbamoyl)pyrrolidin-1-yl)-2-methyl-4-oxobutan-2-yl)thio)propyl)piperazine-1-carboxylate* was obtained from 20 mg thiol compound, 57% yield. ¹H NMR (500 MHz, CDCl₃) δ 8.67 (s, 1H), 7.40-7.32 (m, 4H), 7.30 - 7.26 (m, 1H), 4.75 (t, *J* = 7.9 Hz, 1H), 4.70 (d, *J* = 7.5 Hz, 1H), 4.53 (s, 1H), 4.45 (qd, *J* = 15.1, 6.0 Hz, 2H), 4.05 (d, *J* = 11.1 Hz, 1H), 3.70 (dd, *J* = 11.0, 3.6 Hz, 1H), 3.44 - 3.41 (m, 1H), 3.37 (s, 4H), 2.91 (s, 1H), 2.76 - 2.65 (m, 2H), 2.51 (s, 3H), 2.49 - 2.46 (m, 1H), 2.46 - 2.41 (m, 3H), 2.41 - 2.35 (m, 2H), 2.23 - 2.16 (m, 1H), 1.44 (s, 9H), 1.36 - 1.28 (m, 10H), 1.01 (d, *J* = 5.8 Hz, 3H); ¹³C NMR (126 MHz, CDCl₃) δ 170.76, 170.55 (d, *J* = 20.1 Hz), 170.10, 154.86, 150.40, 148.70, 138.16, 131.68, 131.21, 129.68, 128.14, 79.73, 79.20, 70.33, 67.00, 59.45, 59.09, 56.73, 56.26, 53.38, 48.26, 43.27, 36.77, 32.17, 28.59, 25.98, 25.24, 16.23, 14.53, 14.03 (d, *J* = 10.4 Hz), 13.91 (d, *J* = 10.4 Hz). LC-MS, ESI⁺, m/z 761.9 [M+H]⁺. 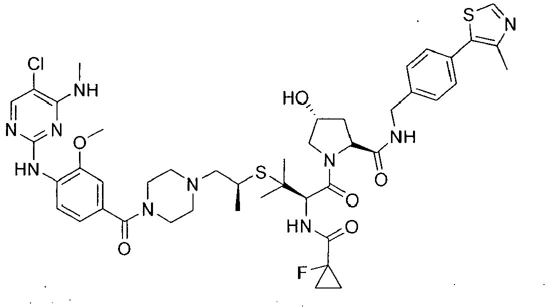 *(2R,4R)-1-((R)-3-(((S)-1-(4-(4-((5-chloro4-(methylamino)pyrimidin-2-yl)amino)-3-methoxybenzoy*/*)piperazin-1-yl)propan-2-y*/*)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(XL01140)***

To a stirring solution of *tert-butyl 4-((S)-2-(((R)-3-(1-fluorocyclopropane-1-carboxamido)-4-((2R,4R)-4-hydroxy-2-((4-(4-methylthiazol-5-yl)benzyl)corbamoyl)pyrrolidin-1-yl)-2-methyl-4-oxobuton-2-yl)thio)propyl)piperazine-1-carboxylate* (16 mg, 0.021 mmol) in DCM (1 mL) was added HCl (4N in 1,4-dioxane, 1 mL). After stirring at room temperature overnight, the mixture was concentrated to afford a solid which was washed with ethyl ether and used to next step. The solid was dissolved in DMF (1ml), 4-((5-chloro-4-(methylamino)pyrimidin-2-yl)amino)-3-methoxybenzoic acid (7 mg, 0.023 mmol), DIPEA (16 µL, 0.084 mmol), and PyOxim (12 mg, 0.023 mmol) was added separately. After stirring at room temperature for 2h, the reaction mixture was diluted with DCM (5ml), washed with water and brine, dried over sodium sulfate, filtered, and condensed to afford a residue which was purified via preparative HPLC under acidic condition (5-95 % CH₃CN in 0.1 % aq. HCO₂H) to give XL01140 (3.6 mg, 17% yield). ¹H NMR (500 MHz, CDCl₃) δ 8.67 (s, 1H), 8.54 (d, *J* = 8.2 Hz, 1H), 7.92 (s, 1H), 7.63 (s, 1H), 7.40 - 7.32 (m, 4H), 7.29 - 7.26 (m, 1H), 7.21 (t, *J* = 5.9 Hz, 1H), 7.02 - 6.96 (m, 2H), 5.33 - 5.25 (m, 1H), 4.75 (t, *J* = 8.0 Hz, 1H), 4.70 (d, *J* = 7.6 Hz, 1H), 4.55 (s, 1H), 4.46 (ddd, *J* = 38.2, 15.1, 5.9 Hz, 2H), 4.07 (d, *J* = 11.3 Hz, 1H), 3.92 (s, 3H), 3.73 - 3.54 (m, 5H), 3.11 (d, *J* = 4.9 Hz, 3H), 2.81 - 2.73 (m, 1H), 2.73 - 2.67 (m, 1H), 2.64 (s, 1H), 2.54 - 2.52 (m, 1H), 2.51 (s, 3H), 2.51 - 2.44 (m, 4H), 2.25 - 2.16 (m, 1H), 1.34 (d, *J* = 1.3 Hz, 6H), 1.33 - 1.27 (m, 4H), 1.03 (d, *J* = 6.4 Hz, 3H); ¹³C NMR (126 MHz, CDCl₃) δ 170.56, 170.50, 170.40, 170.00, 158.57, 157.81, 152.66, 150.26, 148.57, 147.42, 137.95, 131.48, 131.38, 131.12, 129.55, 128.01, 120.22, 116.73, 109.66, 105.56, 79.08, 70.21, 59.27, 58.83, 56.54, 56.15, 55.88, 48.28, 47.37, 43.17, 36.44, 32.06, 28.07, 25.91, 25.10, 16.09, 14.34, 13.96 (d, J = 10.4 Hz), 13.76 (d, J = 10.2 Hz). LC-MS, ESI⁻, m/z 949 [M-H]⁻. HRMS (ESI⁺) *m*/*z,* calcd for C₄₅H₅₆ClFN₁₀O₆S₂: 951.3571 [M + H]⁺, found 951.3586.

### Exemplary synthesis of Exemplary compound 27 (XL01111)

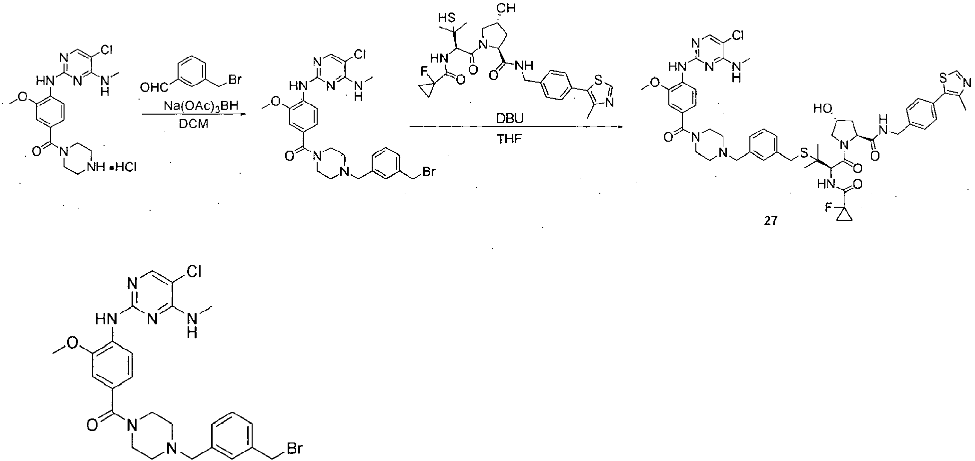

*(4-(3-(bromomethyl)benzyl)piperazin-1-yl)(4-((5-chloro-4-(methylamino)pyrimidin-2-yl)cimino)-3-methoxyphenyl)methanone*

To a suspension of (4-((5-chloro-4-(methylamino)pyrimidin-2-yl)amino)-3-methoxyphenyl)(piperazin-1-yl)methanone HCl salt (80 mg, 0.19 mmol) and 3-bromomethyl benzylaldehyde (38.5 mg, 0.19 mmol) in DCM ( 4 mL) was added sodium triacetoxyborohydride (123 mg, 0.58 mmol). After stirring at room temperature overnight, the mixture was diluted with DCM, washed with water and brine, dried over sodium sulfate, filtered, and condensed under reduced pressure to afford a residue which was purified with flash column chromatography on silica gel (0%-10% methanol in DCM) to give (4-(3-*(bromomethyl)benzyl)piperazin-1-yl)(4-((5-chloro-4-(methylamino)pyrimidin-2-yl)omino)-3-methoxyphenyl)methanone* (51 mg, 46% yield). ¹H NMR (400 MHz, CDCl₃) δ 8.46 (d, J = 8.8 Hz, 1H), 7.84 (s, 1H), 7.56 (s, 1H), 7.28 (s, 1H), 7.23 - 7.14 (m, 3H), 6.95 - 6.90 (m, 2H), 5.30 - 5.22 (m, 1H), 4.41 (s, 2H), 3.84 (s, 3H), 3.67 - 3.51 (m, 4H), 3.46 (s, 2H), 3.02 (d, *J* = 4.9 Hz, 3H), 2.39 (s, 4H). ¹³C NMR (100 MHz, CDCl₃) δ 170.59, 158.68, 157.92, 152.74, 147.53, 138.53, 138.06, 131.47, 129.74, 129.27, 128.93, 128.11, 127.97, 120.28, 116.89, 109.70, 105.62, 62.71, 55.99, 53.26, 33.57, 28.19. LC-MS, ESI⁺, m/z 559.2 [M+H]⁺, 561.2 [M+H+2]⁺. 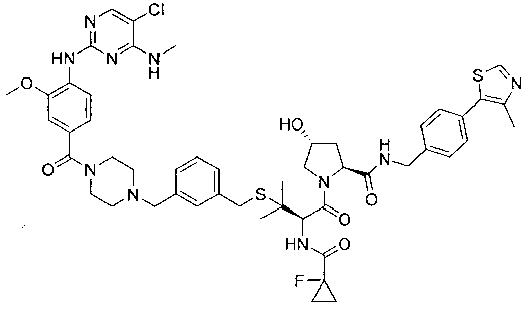 *(2S,4R)-1-((R)-3-((3-((4-(4-((5-chloro-4-(methylamino)pyrimidin-2-yl)amino)-3-methoxybenzoyl)piperazin-1-yl)methyl)benzyl)thio)-2-(l-fluorocyclopropcine-1-carboxamido)-3-methylbutanoyl)-4-hydroxy-N-(4-(4-methylthiazol-5-yl)benzyl)pyrrolidine-2-carboxamide **(XL01111)***

**XL01111** was prepared following general procedure 8. 7.5 mg **XL01111** was obtained from 10 mg thiol compound, 42%. ¹H NMR (400 MHz, CDCl₃) δ 8.66 (s, 1H), 8.53 (d, *J* = 8.4 Hz, 1H), 7.91 (s, 1H), 7.62 (s, 1H), 7.34 - 7.26 (m, 5H), 7.26 - 7.15 (m, 5H), 7.02 - 6.96 (m, 2H), 5.37 - 5.23 (m, 1H), 4.84 - 4.68 (m, 2H), 4.50 - 4.32 (m, 3H), 3.96 - 3.88 (m, 4H), 3.82 - 3.71 (m, 3H), 3.64 (s, 4H), 3.51 (d, *J* = 13.0 Hz, 1H), 3.44 (d, *J* = 13.0 Hz, 1H), 3.10 (d, *J* = 4.9 Hz, 3H), 2.55 - 2.36 (m, 8H), 2.23 - 2.11 (m, 1H), 1.41 - 1.25 (m, 10H).¹³C NMR (101 MHz, CDCl₃) δ 170.70, 170.63,170.50 (d, *J* = 20.8 Hz), 170.04, 158.72, 157.95, 152.79, 150.38, 148.68, 147.57, 138.21, 138.10, 137.31, 131.67, 131.53, 131.16, 129.81, 129.65, 128.86, 128.41, 128.20, 127.92, 120.33, 116.91, 109.75, 105.70, 78.40 (d, *J* = 209.1 Hz), 69.99, 62.89, 59.09, 56.43, 56.25, 56.03, 48.98, 43.25, 36.67, 33.60, 28.22, 25.73, 25.63, 16.22, 14.07 (d, *J* = 9.3 Hz), 13. 95 (d, *J* = 8.9 Hz). ¹⁹F NMR (471 MHz, CDCl₃) δ -197.75. HRMS (ESI⁺) *m*/*z,* calcd for C₅₀H₅₈ClFN₁₀O₆S₂:1013.3727 [M + H]⁺, found 1013.3893.

### Exemplary synthesis of Exemplary compound 28 (XL01145)

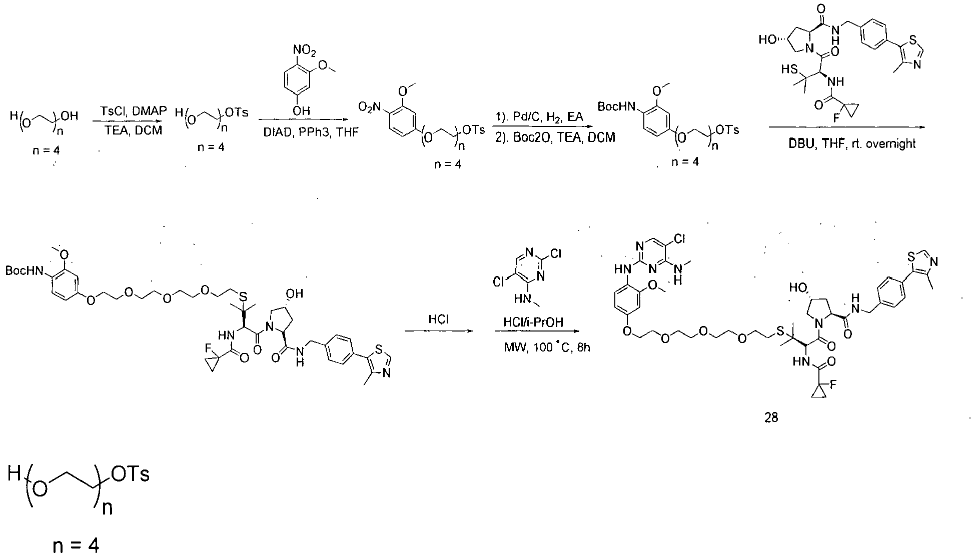

*2-(2-(2-(2-hydroxyethoxy)ethoxy)ethoxy)ethyl 4-methylbenzenesulfonate*

To a solution of tetraethylene glycol (6.1 g, 31.5 mmol) in DCM (100 mL) was added TEA (2.92 mL, 20.98 mmol), DMAP (64 mg, 0.52 mmol), and TsCl (2g, 10.5 mmol) at 0 °C. After stirring at room temperature overnight, the mixture was diluted with DCM, washed with water and brine, dried over sodium sulfate, filtered, condensed to afford a residue which was purified through flash column chromatography on silica gel (0%-100% ethyl acetate in heptane) to give 2-(2-(2-(2-*hydroxyethoxy)ethoxy)ethoxy)ethyl 4-methylbenzenesulfonate* (2.4 g, 66% yield) as white solid. ¹H NMR (400 MHz, CDCl₃) δ 7.79 (d, *J* = 8.0 Hz, 2H), 7.33 (d, *J* = 8.0 Hz, 2H), 4.18 -4.14 (m, 2H), 3.74 - 3.53 (m, 14H), 2.44 (s, 3H). 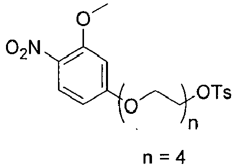 *2-(2-(2-(2-(3-methoxy-4-nitrophenoxy)ethoxy)ethoxy)ethoxy)ethyl 4-methylbenzenesulfonate*

Triphenyl phosphine (275 mg, 1.05 mmol) was added to a solution of 3-methoxy-4-nitrophenol (111 mg, 0.658 mmol), 2-(2-(2-(2-hydroxyethoxy)ethoxy)ethoxy)ethyl 4-methylbenzenesulfonate (229 mg, 0.658 mmol), and Diisopropyl azodicarboxylate (199 mg, 0.987 mmol) in THF (5 ml) under nitrogen protection. After stirring at room temperature overnight, the mixture was diluted with ethyl acetate, washed with NaOH_{aq}, 1N HCl_{aq} and brine separately, dried over sodium sulfate, filtered, and condensed under reduced pressure to give a residue which was purified through flash column chromatography on silica gel (0%-100% ethyl acetate in heptane) to afford *2-(2-(2-(2-(3-methoxy-4-nitrophenoxy)ethoxy)ethoxy)ethoxy)ethyl4-methylbenzenesulfonate* (85 mg, 24%). ¹H NMR (400 MHz, CDCl₃) δ 7.97 (d, *J* = 9.1 Hz, 1H), 7.83 - 7.73 (m, 2H), 7.33 (d, *J* = 8.0 Hz, 2H), 6.59 (d, *J* = 2.5 Hz, 1H), 6.51 (dd, *J* = 9.1, 2.5 Hz, 1H), 4.21 (dd, *J* = 5.3, 4.1 Hz, 2H), 4.15 (dd, *J* = 5.4, 4.3 Hz, 2H), 3.93 (s, 3H), 3.88 (dd, *J* = 5.3, 4.1 Hz, 2H), 3.74 - 3.64 (m, 6H), 3.63 - 3.56 (m, 4H), 2.44 (s, 3H). ¹³C NMR (101 MHz, CDCl₃) δ 164.21, 155.80, 144.97, 133.25, 129.98, 128.51, 128.10, 105.49, 100.57, 71.03, 70.95, 70.88, 70.73, 69.61, 69.37, 68.89, 68.35, 56.66, 21.76. LC-MS, ESI⁺, m/z 500 [M+H]⁺. 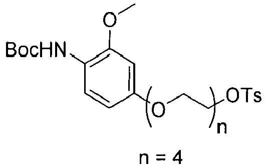 *2-(2-(2-(2-(4-((tert-butoxycarbonyl)amino)-3-methoxyphenoxy)ethoxy)ethoxy)ethoxy)ethyl 4-methylbenzenesulfonate*

To a solution of 2-(2-(2-(2-(3-methoxy-4-nitrophenoxy)ethoxy)ethoxy)ethoxy)ethyl 4-methylbenzenesulfonate (85 mg, 0.17 mmol) in ethyl acetate (3 ml) was added 10% palladium charcoal (10 mg) under nitrogen protection. The nitrogen was then replaced with hydrogen and the resulting mixture was stirred under hydrogen pressure (hydrogen balloon) overnight. The mixture was filtered through a pad of celite and concentrated to afford a residue which was dissolved in DCM, followed by addition of TEA (0.047 ml, 0.46 mmol) and Di-*tert*-butyl decarbonate (37 mg, 0.17 mmol). After stirring at room temperature for 2h, the resulting mixture was concentrated and purified via flash column chromatography on silica gel (0%-100% ethyl acetate in heptane) to give 2-(2-(2-(2-(4-*((tert-butoxycarbonyl)amino)-3-methoxyphenoxy)ethoxy)ethoxy)ethoxy)ethyl 4-methylbenzenesulfonate* (58 mg, 60% yield for two steps). ¹H NMR (400 MHz, CDCl₃) δ 7.88 (d, J = 7.8 Hz, 1H), 7.83 - 7.71 (m, 2H), 7.32 (d, J = 8.0 Hz, 2H), 6.81 (s, 1H), 6.50 (d, J = 2.6 Hz, 1H), 6.44 (dd, J *=* 8.8, 2.6 Hz, 1H), 4.17 - 4.12 (m, 2H), 4.11 - 4.02 (m, 2H), 3.86 - 3.77 (m, 5H), 3.71 - 3.66 (m, 4H), 3.65 - 3.61 (m, 2H), 3.60 - 3.55 (m, 4H), 2.43 (s, 3H), 1.51 (s, 9H). ¹³C NMR (101 MHz, CDCl₃) δ 154.81, 153.12, 148.97, 144.87, 133.26, 129.93, 128.08, 121.94, 119.07, 104.95, 99.64, 80.13, 70.89, 70.81, 70.70, 69.94, 69.35, 68.83, 67.94, 55.79, 28.51, 21.72. LC-MS, ESI⁺,m/z 470.15 [M+H]⁺. 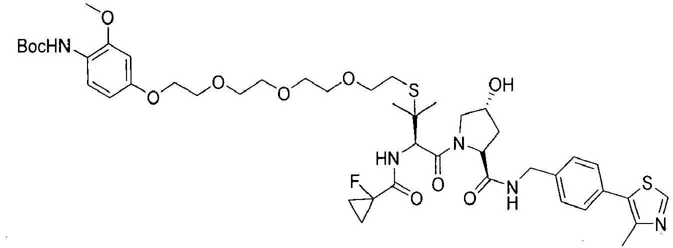 *tert-butyl (4-(((R)-1-(l-fluorocyclopropyl)-3-((2R,4R)-4-hydroxy-2-((4-(4-methylthiazol-5-yl)benzy*/*)carbamoyl)pyrrolidine-1-carbonyl)-4,4-dimethyl-1-oxo-8,11,14-trioxa-5-thia-2-azahexadecan-16-yl)oxy)-2-methoxyphenyl)carbamate*

To a solution of 2-(2-(2-(2-(4-((*tert*-butoxycarbonyl)amino)-3-methoxyphenoxy)ethoxy)ethoxy)ethoxy)ethyl 4-methylbenzenesulfonate (29 mg, 0.05 mmol) and (2R,4R)-1-((R)-2-(1-fluorocyclopropane-1-carboxamido)-3-mercapto-3-methylbutanoyl)-4-hydroxy-N-(4-(4-methylthiazol-5-yl)benzyl)pyrrolidine-2-carboxamide (27 mg, 0.05 mmol) in THF (2 ml) was added 1,8-Diazabicyclo[5.4.0]undec-7-ene (0.072 mL, 0.5 mmol). After stirring at room temperature overnight, the mixture was concentrated and purified through flash column chromatography on silica gel (0%-10% methanol in DCM) to yield *tert-butyl (4-(((R)-1-(1-fluorocyclopropyl)-3-((2R,4R)-4-hydroxy-2-((4-(4-methylthiazol-5-yl)benzy*/*)carbamoyl)pyrrolidine-1-carbonyl)-4,4-dimethyl-1-oxo-8,11,14-trioxa-5-thia-2-azahexodecon-16-yl)oxy)-2-methoxyphenyl)carbomate*(36 mg, 77% yield). ¹H NMR (500 MHz, CDCl₃) δ 8.59 (s, 1H), 7.81 (s, 1H), 7.34 - 7.23 (m, 5H), 7.14 (dd, J = 8.1, 3.2 Hz, 1H), 6.75 (s, 1H), 6.42 (d, J = 2.6 Hz, 1H), 6.38 (dd, J = 8.8, 2.6 Hz, 1H), 4.74 (d, J = 8.0 Hz, 1H), 4.68 (t, J = 7.8 Hz, 1H), 4.47 - 4.30 (m, 3H), 4.06 - 3.98 (m, 2H), 3.87 (d, J = 11.2 Hz, 1H), 3.80 - 3.71 (m, 6H), 3.65 - 3.59 (m, 2H), 3.57 - 3.55 (m, 2H), 3.54 - 3.41 (m, 6H), 3.14 (s, 1H), 2.80 - 2.56 (m, 2H), 2.44 (s, 3H), 2.33 (ddd, *J* = 7.2, 6.0, 3.3 Hz, 1H), 2.19 - 2.05 (m, 1H), 1.43 (s, 9H), 1.29 -1.19 (m, 10H); ¹³C NMR (126 MHz, CDCl₃) δ 170.81, 170.22 (d, *J* = 20.68 Hz), 169.81, 154.67, 153.04, 150.24, 148.91, 148.49, 138.13, 131.60, 130.95, 129.46, 128.13, 121.82, 119.06, 104.83, 99.53, 80.05, 78.12 (d, J = 232.46 Hz), 70.78, 70.59, 70.53, 70.40, 70.24, 69.89, 69.82, 67.80, 59.15, 56.43, 56.00, 55.68, 47.96, 43.03, 36.81, 28.45, 28.39, 25.75, 25.20, 16.07, 13.83 (d, J = 10.6 Hz), 13.81 (d, *J* = 10.5 Hz). LC-MS, ESI⁺, m/z 933 [M+H]⁺. 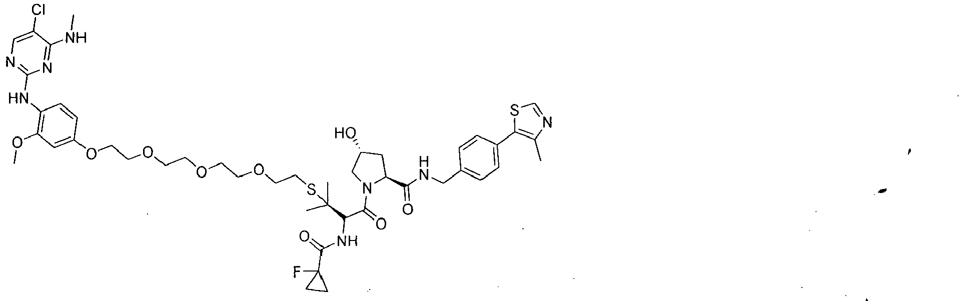 *(2S,4R)-1-((S)-1-(4-((5-chloro-4-(methylamino)pyrimidin-2-yl)amino)-3-methoxyphenoxy)-14-(1-fluorocyclopropane-1-carboxamido)-13,13-dimethyl-3,6,9-trioxa-12-thiapentadecan-15-oyl)-4-hydroxy-N-(4-(4-methylthiazol-5-yl)benzyl)pyrrolidine-2-carboxamide **(28, XL01145)***

To a solution of *tert*-butyl (4-(((*R*)-1-(1-fluorocyclopropyl)-3-((2*R*,4*R*)-4-hydroxy-2-((4-(4-methylthiazol-5-yl)benzyl)carbamoyl)pyrrolidine-1-carbonyl)-4,4-dimethyl-1-oxo-8,11,14-trioxa-5-thia-2-azahexadecan-16-yl)oxy)-2-methoxyphenyl)carbamate (36 mg, 0.039 mmol) in DCM (1 mL) was added HCl (4N in 1,4-dioxane, 1 mL). After stirring at room temperature overnight, the mixture was condensed and dissolved in isopropanol (1.5 ml). 2,5-dichloro-N-methylpyrimidin-4-amine (8 mg, 0.045 mmol) and HCl (4N in 1,4-dioxane, one drop) were added and the resulting mixture was heated in microwave at 100 °C for 8h. Removal of the solvent under reduced pressure give a residue which was purified through preparation HPLC under acidic condition (5-95 % CH₃CN in 0.1 % aq. HCO₂H) to give **XL01145** (8.3 mg, 22% yield for two steps). ¹H NMR (400 MHz, CDCl₃) δ 8.67 (s, 1H), 8.25 (d, *J* = 8.8 Hz, 1H), 7.85 (s, 1H), 7.41 - 7.29 (m, 6H), 7.19 (dd, J = 8.0, 3.2 Hz, 1H), 6.54 (d, J = 2.6 Hz, 1H), 6.49 (dd, J = 8.9, 2.7 Hz, 1H), 5.27 - 5.20 (m, 1H), 4.81 (d, J = 7.8 Hz, 1H), 4.78 - 4.73 (m, 1H), 4.55 - 4.50 (m, 1H), 4.50 - 4.38 (m, 2H), 4.14 - 4.08 (m, 2H), 3.95 (d, *J* = 11.2 Hz, 1H), 3.87 - 3.78 (m, 6H), 3.74 - 3.67 (m, 2H), 3.67 - 3.62 (m, 2H), 3.62 - 3.46 (m, 6H), 3.07 (d, J = 4.9 Hz, 3H), 2.83 - 2.67 (m, 2H), 2.51 (s, 3H), 2.48 - 2.40 (m, 1H), 2.26 - 2.14 (m, 1H), 1.38 - 1.23 (m, 10H); ¹³C NMR (101 MHz, CDCl₃) δ 170.83, 170.43 (d, *J* = 20.6 Hz), 170.06, 158.69, 158.37, 154.30, 152.65, 150.41, 149.52, 148.65, 138.26, 131.76, 131.14, 129.64, 128.33, 123.53, 119.70, 104.83, 104.62, 99.76, 78.40 (d, J = 209.1 Hz), 70.97, 70.77, 70.70, 70.58, 70.35, 70.03, 68.03, 59.19, 56.46, 56.17, 55.92, 47.94, 43.23, 36.80, 28.64, 28.13, 25.96, 25.27, 16.22, 14.05 (d, J = 9.6 Hz), 13.95 (d, J = 9.5 Hz); HRMS (ESI⁺) *m*/*z,* calcd for C₄₅ₛH₅₈ClFN₈O₉S₂:973.3513 [M + H]⁺, found 973.3903.

### Exemplary synthesis of Exemplary compound 29 (XL01149)

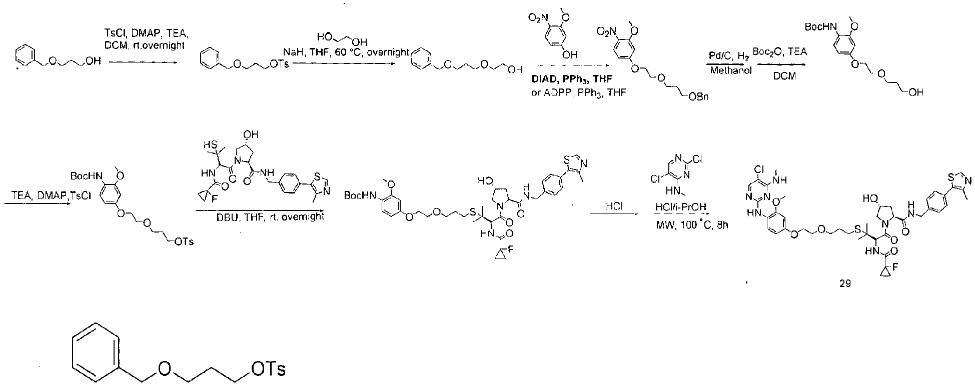

*3-(benzyloxy)propyl 4-methylbenzenesulfonate*

TsCI (867 mg, 4.54 mmol) was added to a solution of 3-(benzyloxy)propan-1-ol (720 mg, 4.33 mmol), TEA (0.904 mL, 6.5 mmol), and DMAP (26 mg, 0.21 mmol) in DCM (10 mL) at 0 °C. After stirring at room temperature overnight, the mixture was diluted with DCM (20 mL), washed with water and brine, dried over sodium sulfate, filtered, and condensed under reduced pressure to give a residue which was purified through flash column chromatography on silica gel (0%-50% ethyl acetate in heptane) to yield *3-(benzyloxy)propyl 4-methylbenzenesulfonate* (750 mg, 54% yield). ¹H NMR (400 MHz, CDCl₃) δ 7.85 - 7.77 (m, 2H), 7.39 - 7.24 (m, 7H), 4.43 (s, 2H), 4.20 (t, J = 6.1 Hz, 2H), 3.53 (t, J = 6.1 Hz, 2H), 2.45 (s, 3H), 1.97 (p, J = 6.1 Hz, 2H). 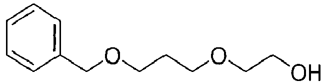 *2-(3-(benzyloxy)propoxy)ethan-1-ol*

NaH (60% in mineral oil, 206 mg, 5.16 mmol) was added to a solution of ethylene glycol (1.453 g, 23.4 mmol) in THF (20 mL) at 0 °C. The resulting mixture was heated to reflux and stirred at reflux overnight. The reaction mixture was then cooled down in ice bath and NH₄Cl_{aq} was added dropwise to quench the reaction. The resulting mixture was extracted with ethyl acetate (3x 30 mL), the combined organic phase was washed with water and brine, dried over sodium sulfate, filtered, and condensed to afford a residue which was purified through flash column chromatography on silica gel (0%-10% methanol in DCM) to give *2-(3-(benzyloxy)propoxy)ethan-1-ol* (534 mg, 54% yield). ¹H NMR (400 MHz, CDCl₃) δ 7.43 - 7.18 (m, 5H), 4.53 (s, 2H), 3.75 - 3.69 (m, 2H), 3.68 - 3.49 (m, 6H), 2.26 (t, J = 5.8 Hz, 1H), 1.93 (p, J = 6.3 Hz, 2H). ¹³C NMR (101 MHz, CDCl₃) δ 138.57, 128.46, 127.74, 127.67, 73.08, 71.99, 68.34, 67.32, 61.90, 30.12. LC-MS, ESI⁺, m/z 233 [M+Na]⁺. 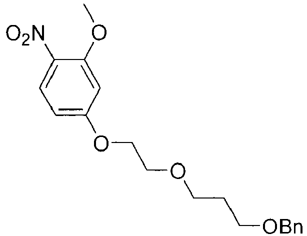 *4-(2-(3-(benzyloxy)propoxy)ethoxy)-2-methoxy-1-nitrobenzene*

To a solution of 3-methoxy-4-nitrophenol (81 mg, 0.48 mmol), 2-(3-(benzyloxy)propoxy)ethan-1-ol (100 mg, 0.48 mmol), and triphenyl phosphine (312 mg, 1.19 mmol) in THF was added Diisopropyl azodicarboxylate (192 mg, 0.95 mmol) at 0 °C. The resulting reaction mixture was stirred at room temperature with nitrogen protection overnight. Ethyl acetate was added. The resulting mixture was washed with 1 N NaOH_{aq}, water, and brine separately, then dried over sodium sulfate, filtered, and condensed to give a residue which was purified through flash column chromatography on silica gel (0%-100% ethyl acetate in heptane) to afford *4-(2-(3-(benzyloxy)propoxy)ethoxy)-2-methoxy-1-nitrobenzene* (80 mg, 38% yield). ¹H NMR (400 MHz, CDCl₃) δ 7.97 (d, J = 9.1 Hz, 1H), 7.40 - 7.23 (m, 5H), 6.58 (d, *J* = 2.5 Hz, 1H), 6.50 (dd, J = 9.1, 2.5 Hz, 1H), 4.49 (s, 2H), 4.17 (dd, J = 5.3, 4.1 Hz, 2H), 3.91 (s, 3H), 3.80 (dd, J = 5.3, 4.1 Hz, 2H), 3.65 (t, J = 6.3 Hz, 2H), 3.57 (t, *J* = 6.3 Hz, 2H), 1.92 (p, J = 6.3 Hz, 2H). ¹³C NMR (101 MHz, CDCl₃) δ 164.21, 155.74, 138.64, 128.48, 127.68, 105.37, 100.60, 73.11, 69.12, 68.71, 68.28, 67.19, 56.59, 30.18. LC-MS, ESI⁺, m/z 361.9 [M+H]⁺. 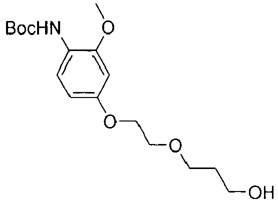 *tert-butyl (4-(2-(3-hydroxypropoxy)ethoxy)-2-methoxyphenyl)carbamate*

To a solution of 4-(2-(3-(benzyloxy)propoxy)ethoxy)-2-methoxy-1-nitrobenzene (80 mg, 0.22 mmol) in methanol (3 mL) was added 10% Palladium charcoal (8 mg) under nitrogen protection. The nitrogen was evacuated and backfilled with hydrogen and the resulting mixture was stirred under positive hydrogen pressure (hydrogen balloon) overnight. The mixture was then filtered through a pad of celite, and the filtrate was condensed to afford a residue which was dissolved in DCM (3 ml), followed by addition of TEA (0.092 mL, 0.66 mmol) and Di-*tert*-butyl decarbonate (48 mg, 0.22 mmol). After stirring at room temperature overnight, the mixture was diluted with DCM, washed with 1 M HCl_{aq}, water and brine separately, dried over sodium sulfate, filtered, and condensed under reduced pressure to give a residue which was purified through flash column chromatography on silica gel (0%-10% methanol in DCM) to yield *tert-butyl (4-(2-(3-hydroxypropoxy)ethoxy)-2-methoxyphenyl)carbamate* (14.3 mg, 19% yield). ¹H NMR (400 MHz, CDCl₃) δ 7.89 (d, J = 7.9 Hz, 1H), 6.82 (s, 1H), 6.50 (d, J = 2.6 Hz, 1H), 6.45 (dd, J = 8.8, 2.6 Hz, 1H), 4.11 - 4.06 (m, 2H), 3.82 (s, 3H), 3.81 - 3.76 (m, 4H), 3.72 (t, *J* = 5.8 Hz, 2H), 2.29 (t, J = 5.5 Hz, 1H), 1.93 - 1.81 (m, 2H), 1.51 (s, 9H). ¹³C NMR (101 MHz, CDCl₃) δ 154.74, 153.15, 149.04, 122.09, 119.11, 104.90, 99.76, 80.18, 70.65, 69.68, 67.82, 61.97, 55.83, 32.15, 28.54. LC-MS, ESI⁺, m/z 364.1 [M+Na]⁺. 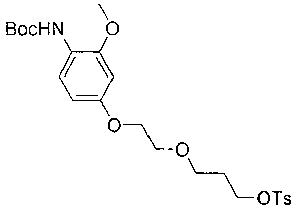 *3-(2-(4-((tert-butoxycarbonyl)amino)-3-methoxyphenoxy)ethoxy)propyl 4-methylbenzenesulfonate*

To a solution of *tert*-butyl (4-(2-(3-hydroxypropoxy)ethoxy)-2-methoxyphenyl)carbamate (33 mg, 0.10 mmol), TEA (0.027 mL, 0.19 mmol) and DMAP (1 mg, 0.008 mmol) in DCM (2 mL) was added TsCl (20 mg, 0.1 mmol). After stirring at room temperature overnight, the mixture was diluted with DCM, washed with water and brine, dried over sodium sulfate, filtered, and condensed under reduced pressure to afford a residue which was purified through flash column chromatography on silica gel (0%-10% methanol in DCM) to give *3-(2-(4-((tert-butoxycarbonyl)amino)-3-methoxyphenoxy)ethoxy)propyl 4-methylbenzenesulfonate* (30 mg, 62% yield). ¹H NMR (400 MHz, CDCl₃) δ 7.89 (s, 1H), 7.82 - 7.70 (m, 2H), 7.31 (d, J = 8.0 Hz, 2H), 6.83 (s, 1H), 6.50 (d, J = 2.6 Hz, 1H), 6.42 (dd, *J* = 8.9, 2.6 Hz, 1H), 4.15 (t, J = 6.1 Hz, 2H), 4.00 (dd, J = 5.4, 4.2 Hz, 2H), 3.82 (s, 3H), 3.68 (dd, J = 5.4, 4.2 Hz, 2H), 3.55 (t, J = 6.1 Hz, 2H), 2.42 (s, 3H), 1.93 (p, J = 6.1 Hz, 2H), 1.51 (s, 9H). ¹³C NMR (101 MHz, CDCl₃) δ 154.75, 153.12, 148.99, 144.82, 133.33, 129.94, 128.02, 122.03, 119.04, 104.82, 99.69, 80.15, 69.62, 67.81, 67.76, 66.89, 55.81, 29.45, 28.52, 21.71. LC-MS, ESI⁺, m/z 496.1 [M+H]⁺. 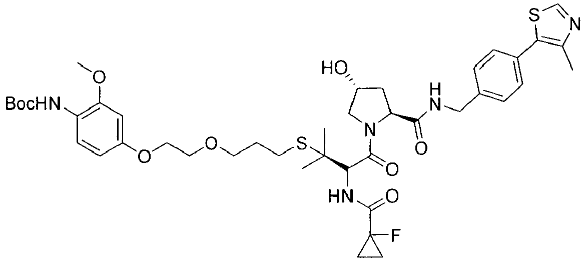 *tert-butyl (4-(2-(3-(((S)-3-(1-fluorocyclopropone-1-carboxamido)-4-((2S,4R)-4-hydroxy-2-((4-(4-methylthiazol-5-yl)benzyl)carbamoyl)pyrrolidin-1-y*/*)-2-methyl-4-oxobutan-2-yl)thio)propoxy)ethoxy)-2-methoxyphenyl)carbamate* was prepared following general procedure 8. 30 mg *tert-butyl (4-(2-(3-(((S)-3-(1-fluorocyclopropane-1-carboxamido)-4-((2S,4R)-4-hydroxy-2-((4-(4-methylthiazol-5-yl)benzyl)carbamoyl)pyrrolidin-1-yl)-2-methyl-4-oxobutan-2-yl)thio)propoxy)ethoxy)-2-methoxyphenyl)carbamate* was obtained from 27 mg thiol compound, 70% yield. ¹H NMR (500 MHz, CDCl₃) δ 8.65 (s, 1H), 7.87 (d, J = 6.0 Hz, 1H), 7.38 - 7.29 (m, 5H), 7.20 (dd, J = 8.0, 3.2 Hz, 1H), 6.81 (s, 1H), 6.48 (d, J = 2.6 Hz, 1H), 6.44 (dd, J = 8.8, 2.6 Hz, 1H), 4.81 - 4.73 (m, 2H), 4.44 (d, *J* = 5.8 Hz, 3H), 4.07 - 4.02 (m, 2H), 3.99 - 3.94 (m, 1H), 3.80 (s, 3H), 3.74 - 3.69 (m, 3H), 3.57 - 3.47 (m, 2H), 3.03 (s, 1H), 2.68 - 2.60 (m, 1H), 2.60 - 2.53 (m, 1H), 2.50 (s, 3H), 2.42 - 2.35 (m, 1H), 2.24 - 2.16 (m, 1H), 1.79 - 1.68 (m, 2H), 1.50 (s, 9H), 1.36 - 1.24 (m, 10H). ¹⁹F NMR (471 MHz, CDCl₃) δ -197.69. ¹³C NMR (126 MHz, CDCl₃) δ 170.88, 170.42 (d, *J* = 20.9 Hz), 170.04, 154.83, 153.21, 150.37, 149.15, 148.63, 138.21, 131.72, 131.11, 129.61, 128.23, 122.00, 119.32, 104.91, 99.67, 78.27 (d, *J* = 231.44 Hz), 70.11, 69.81, 69.55, 67.95, 59.18, 56.64, 56.14, 55.82, 48.15, 43.18, 36.95, 29.62, 28.53, 25.88, 25.16, 25.11, 16.21, 14.00 (d, J = 9.08 Hz), 13.93 (d, *J* = 9.17 Hz). LC-MS, ESI⁺, m/z 858.95 [M+H]⁺. 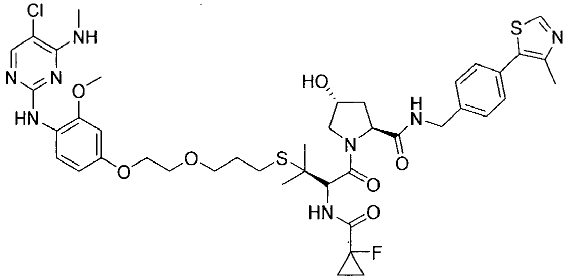 *(2S,4R)-1-((S)-3-((3-(2-(4-((5-chloro-4-(methylamino)pyrimidin-2-yl)amino)-3-methoxyphenoxy)ethoxy)propyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide (29, **XL01149)***

To a solution of *tert-butyl (4-(2-(3-(((S)-3-(1-fluorocyclopropane-1-carboxamido)-4-((2S,4R)-4-hydroxy-2-((4-(4-methylthiazol-5-yl)benzyl)carbamoyl)pyrrolidin-1-yl)-2-methyl-4-oxobutan-2-yl)thio)propoxy)ethoxy)-2-methoxyphenyl)carbamate* (30 mg, 0.035 mmol) in DCM (1 mL) was added HCl (4N in 1,4-dioxane, 1 mL). After stirring at room temperature overnight, the mixture was condensed under reduced pressure to give a solid which was dissolved in iso-propanol, followed by addition of 2,5-dichloro-N-methylpyrimidin-4-amine (7 mg, 3.9 mmol). After heating in a microwave reactor at 100 °C for 8h, the mixture was diluted with DCM, washed with water and brine, dried over sodium sulfate, filtered, and condensed to afford a residue which was purified with preparative HPLC under acidic condition (5-95 % CH₃CN in 0.1 % aq. HCO₂H) to yield **XL01149** (7.5 mg, 24% for two steps). ¹H NMR (500 MHz, CDCl₃) δ 8.66 (s, 1H), 8.22 (d, J = 8.8 Hz, 1H), 7.85 (s, 1H), 7.40 - 7.30 (m, 5H), 7.28 (s, 1H), 7.19 (dd, *J* = 7.9, 3.1 Hz, 1H), 6.54 (d, *J* = 2.6 Hz, 1H), 6.49 (dd, *J* = 8.8, 2.6 Hz, 1H), 5.24 (d, J = 4.6 Hz, 1H), 4.81 - 4.74 (m, 2H), 4.46 (d, J = 5.9 Hz, 2H), 4.39 (s, 1H), 4.12 - 4.04 (m, 2H), 3.97 (d, J = 11.2 Hz, 1H), 3.84 (s, 3H), 3.78 - 3.66 (m, 3H), 3.60 - 3.44 (m, 2H), 3.07 (d, J = 4.9 Hz, 3H), 2.71 - 2.63 (m, 1H), 2.61 - 2.54 (m, 1H), 2.51 (s, 3H), 2.44 - 2.39 (m, 1H), 2.25 - 2.15 (m, 1H), 1.82 - 1.68 (m, 2H), 1.38 - 1.27 (m, 10H); ¹³C NMR (126 MHz, CDCl₃) δ 170.79, 170.51 (d, *J* = 20.9 Hz), 170.15, 163.12, 158.70, 158.42, 154.41, 152.62, 150.41, 149.76, 148.65, 138.22, 131.75, 131.16, 129.66, 128.30, 123.51, 120.03, 104.77, 99.73, 78.26 (d, *J* = 242.0 Hz), 70.09, 69.82, 69.63, 68.06, 59.09, 56.57, 56.12, 55.92, 48.01, 43.24, 36.85, 29.65, 28.11, 25.96, 25.16, 25.07, 16.21, 14.07 (d, J = 10.67 Hz), 13.95 (d, *J* = 10.67 Hz); ¹⁹F NMR (471 MHz, CDCl₃) δ -197.75; HRMS (ESI⁺) m/z, calcd for C₄₂H₅₂ClFN₈O₇S₂: 899.3146 [M + H]⁺, found 899.2639.

### Exemplary synthesis of Exemplary compound 30 (XL01168)

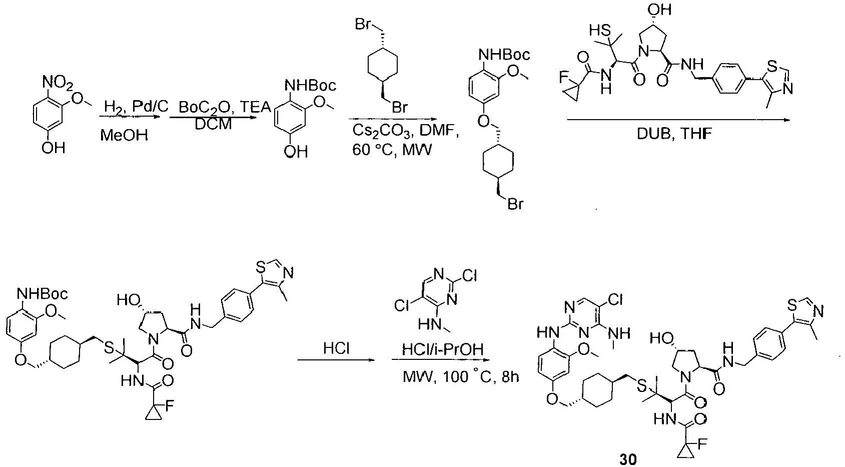

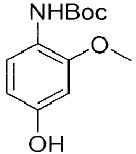

*tert-butyl (4-hydroxy-2-methoxyphenyl)carbamate*

To a solution of 3-methoxyl-4-nitrophenol (1 g, 5.9 mmol) in methanol (10 ml) was added 10% Palladium charcoal (100 mg) under nitrogen protection. The resulting mixture was evacuated and backfilled with hydrogen by attaching a hydrogen balloon. After stirring at room temperature overnight, the mixture was filtered through a pad of celite, and the filtrate was condensed to afford a residue which was dissolved in DCM (20 mL). Followed by addition of TEA (1.65 mL, 11.8 mmol) and BoC₂O (1.29 g, 5.9 mmol), the mixture was stirred at room temperature overnight. DCM (30 mL) was added and the resulting mixture was washed with 1N HCl_{aq}, water, and brine separately, then dried over sodium sulfate, filtered, and condensed to give a residue which was purified through flash column chromatography on silica gel (0%-10% methanol in DCM) to give *tert-butyl (4-hydroxy-2-methoxyphenyl)carbamate* (200 mg, 14% yield for two steps). ¹H NMR (500 MHz, CDCl₃) δ 7.70 (s, 1H), 6.76 (s, 1H), 6.40 (d, J = 2.6 Hz, 1H), 6.35 (dd, J = 8.7, 2.6 Hz, 1H), 6.17 (s, 1H), 3.76 (s, 3H), 1.51 (s, 9H). ¹³C NMR (126 MHz, CDCl₃) δ 153.68, 152.43, 149.63, 120.66, 120.37, 107.04, 99.27, 80.44, 55.70, 28.52. LC-MS, ESI⁻, m/z 238 [M-H]⁻. 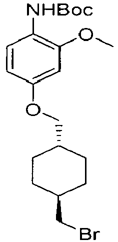 *tert-butyl(4-(((1r,4r)-4-(bromomethyl)cyclohexyl)methoxy)-2-methoxyphenyl)carbamate*

In a microwave tube was added *tert-*butyl (4-hydroxy-2-methoxyphenyl)carbamate (20 mg, 0.084 mmol), trans-1,4-bis(bromomethyl)cyclohexane (68 mg, 0.25 mmol), DMF (1 ml), and Cs₂CO₃ (82 mg, 0.25 mmol). After heating in microwave at 60 °C for 30 min, the mixture was cooled down and added with water. The resulting mixture was then extracted with ethyl acetate (3x), the combined organic phase was washed with water and brine, dried over sodium sulfate, filtered, and condensed to afford a residue which was purified through flash column chromatography on silica gel to give *tert-butyl (4-(((1r,4r)-4-(bromomethyl)cyclohexyl)methoxy)-2-methoxyphenyl)carbamate* (15 mg, 42% yield). ¹H NMR (400 MHz, CDCl₃) δ 7.81 (d, *J* = 7.5 Hz, 1H), 6.73 (s, 1H), 6.39-6.32 (m, 2H), 3.76 (s, 3H), 3.67 (d, J = 6.3 Hz, 2H), 3.23 (d, J = 6.3 Hz, 2H), 1.95 - 1.83 (m, 4H), 1.72 - 1.62 (m, 1H), 1.62 -1.52 (m, 1H), 1.44 (s, 9H), 1.10 - 0.92 (m, 4H). ¹³C NMR (101 MHz, CDCl₃) δ 155.30, 153.18, 149.05, 121.68, 119.24, 104.84, 99.37, 73.60, 55.80, 40.41, 40.30, 37.78, 31.22, 29.40, 28.56. LC-MS, ESI⁺, m/z 450 [M+Na]⁺, 452 [M+Na+2]⁺. 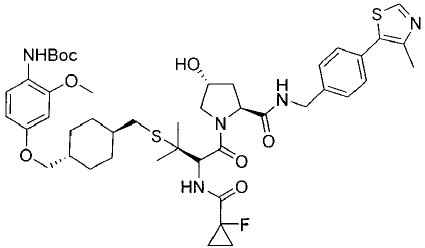 *tert*-butyl (4-(((1S,4R)-4-((((S)-3-(1-fluorocyclopropane-1-carboxamido)-4-((2*S*,4*R*)-4-hydroxy-2-((4-(4-methylthiazol-5-yl)benzyl]carbamoyl)pyrrolidin-1-yl)-2-methyl-4-oxobutan-2-yl)thio)methyl)cyclohexyl)methoxy)-2-methoxyphenyl)carbamate was prepared following general procedure 8. 25 mg *tert*-butyl (4-(((1S,4R)-4-((((S)-3-(1-fluorocyclopropane-1-carboxamido)-4-((2*S*,4*R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methoxy)-2-methoxyphenyl)carbamate obtained from 18 mg thiol compound, 81% yield. ¹H NMR (500 MHz, CDCl₃) δ 8.65 (s, 1H), 7.87 (s, 1H), 7.40 - 7.32 (m, 5H), 7.24 (dd, J = 8.0, 3.4 Hz, 1H), 6.80 (s, 1H), 6.46 - 6.39 (m, 2H), 4.79 (t, J = 7.9 Hz, 1H), 4.74 (d, J = 7.8 Hz, 1H), 4.53 (s, 1H), 4.46 (d, J = 5.9 Hz, 2H), 4.05 (d, *J* = 11.3 Hz, 1H), 3.82 (s, 3H), 3.73 (dd, J = 11.2, 3.9 Hz, 1H), 3.69 (d, *J* = 6.3 Hz, 2H), 2.90 (d, *J* = 4.4 Hz, 1H), 2.51 (s, 3H), 2.50 - 2.34 (m, 3H), 2.28 - 2.19 (m, 1H), 1.87 (t, *J* = 10.3 Hz, 4H), 1.71- 1.65 (m, 1H), 1.51 (s, 9H), 1.41-1.28 (m, 11H), 1.08 -0.90 (m, 4H). ¹⁹F NMR (471 MHz, CDCl₃) δ -197.70. ¹³C NMR (126 MHz, CDCl₃) δ 170.83, 170.60 (d, J = 20.7 Hz), 170.14, 155.28, 153.17, 150.39, 149.07, 148.67, 138.18, 131.71, 131.17, 129.65, 128.15, 121.67, 119.25, 104.83, 99.35, 78.28 (d, J = 232.0 Hz), 73.64, 70.27, 59.10, 56.73, 56.36, 55.80, 47.80, 43.24, 38.33, 37.80, 36.88, 35.37, 32.51, 32.46, 29.62, 28.55, 25.78, 25.47, 16.24, 14.05 (d, J = 11.0 Hz), 13.96 (d, J = 11.0 Hz). LC-MS, 882 [M+H]⁺. 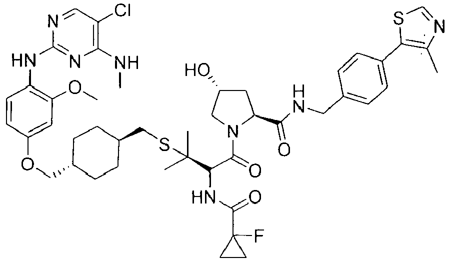 *(2S,4R)-1-((5)-3-((((1R,4S)-4-((4-((5-chloro-4-(methylamino)pyrimidin-2-y*/*)amino)-3-methoxyphenoxy)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(30, XL01168)***

*tert*-butyl (4-(((1*S*,4*R*)-4-((((*S*)-3-(1-fluorocyclopropane-1-carboxamido)-4-((2*S*,4*R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methoxy)-2-methoxyphenyl)carbamate (25 mg, 0.028 mmol) was dissolved in DCM (1 ml), followed by addition of HCl (4N HCl in 1,4-dioxane, 1 mL). After stirring at room temperature overnight, the mixture was condensed under reduced pressure to give a solid which was used to next step directly without further purification. The solid from previous step was dissolved in iso-propanol (1 mL), followed by addition of 2,5-dichloro-N-methylpyrimidin-4-amine (4.8 mg, 0.027 mmol) and HCl (4N in 1,4-dioxane, 0.006 ml). The resulting mixture was heated in microwave at 100 °C for 9h, cooled down, diluted with water and extracted with DCM (3x). The combined organic phase was washed with water and brine, dried over sodium sulfate, filtered, condensed to give a residue which was purified through preparation HPLC under acidic condition (5-95 % CH₃CN in 0.1 % aq. HCO₂H) to yield **XL01168** (10 mg, 26% yield for two steps). ¹H NMR (500 MHz, CDCl₃) δ 8.65 (s, 1H), 8.26 (d, J = 8.8 Hz, 1H), 7.87 (s, 1H), 7.41 - 7.31 (m, 5H), 7.25 - 7.21 (m, 2H), 6.56 - 6.35 (m, 2H), 5.24 - 5.18 (m, 1H), 4.80 (t, J = 7.9 Hz, 1H), 4.74 (d, J = 7.8 Hz, 1H), 4.54 (s, 1H), 4.46 (d, *J* = 5.9 Hz, 2H), 4.07 (d, *J* = 11.3 Hz, 1H), 3.86 (s, 3H), 3.75 - 3.70 (m, 3H), 3.08 (d, J = 4.9 Hz, 3H), 2.52 (s, 3H), 2.51 - 2.45 (m, 1H), 2.45-2.34 (m, 2H), 2.27 - 2.20 (m, 1H), 1.88 (t, *J* = 12.6 Hz, 4H), 1.76 - 1.64 (m, 1H), 1.40 - 1.25 (m, 11H), 1.08 -0.91 (m, 4H); ¹³C NMR (126 MHz, CDCl₃) δ 170.75, 170.66 (d, *J* = 23.3 Hz), 170.21, 158.66, 158.49, 154.72, 152.89, 150.40, 149.46, 148.69, 138.17, 131.71, 131.20, 129.68, 128.18, 123.27, 119.69, 104.64, 104.56, 99.41, 78.3 (d, *J* = 234.4 Hz), 73.71, 70.30, 59.02, 56.69, 56.39, 55.88, 47.71, 43.27, 38.34, 37.82, 36.79, 35.39, 32.54, 32.48, 29.65, 28.11, 25.81, 25.45, 16.25, 14.09 (d, J = 10.3 Hz), 13.96 (d, *J* = 10.3 Hz); LC-MS, ESI⁻, m/z 921 [M-H]⁻. HRMS (ESI⁺) m/z, calcd for C₄₅H₅₆ClFN₈O₆S₂:923.3509 [M + H]⁺, found 923.3479.

### Exemplary synthesis of Exemplary compound 31 (XL02017)

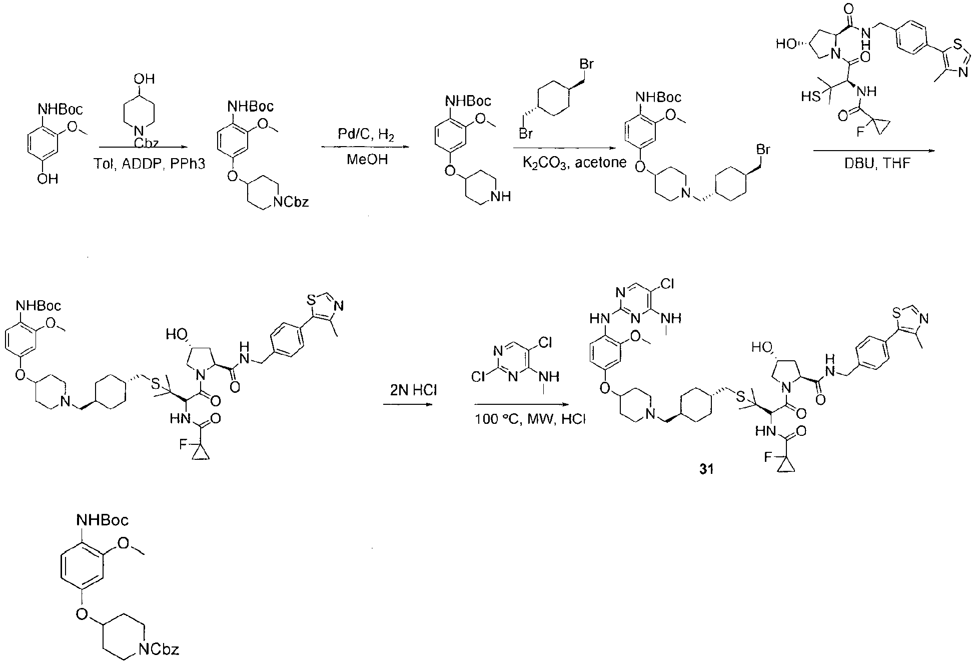

*Benzyl 4-(4-((tert-butoxycarbonyl)amino)-3-methoxyphenoxy)piperidine-1-carboxylate*

To a solution of *tert-butyl* (4-hydroxy-2-methoxyphenyl)carbamate (300 mg, 1.26 mmol), benzyl 4-hydroxypiperidine-1-carboxylate (354 mg, 1.5 mmol), and triphenyl phosphine (724 mg, 2.76 mmol) in toluene (10 ml) was added 1,1'-(Azodicarbonyl)dipiperidine (633 mg, 2.5 mmol). After stirring at room temperature with nitrogen protection overnight, the reaction mixture was diluted with ethyl acetate, washed with water and brine, dried over sodium sulfate, filtered, condensed to afford a residue which purified through flash column chromatography on silica gel (0%-50% ethyl acetate in heptane) to yield *Benzyl 4-(4-((tert-butoxycarbonyl)amino)-3-methoxyphenoxy)piperidine-1-carboxylate* (450 mg, 79% yield). ¹H NMR (400 MHz, CDCl₃) δ 7.91 (d, J = 8.3 Hz, 1H), 7.39 - 7.28 (m, 5H), 6.84 (s, 1H), 6.55-6.37 (m, 2H), 5.15 (s, 2H), 4.46 - 4.36 (m, 1H), 3.82 (s, 3H), 3.80-3.68 (m, 2H), 3.49 - 3.40 (m, 2H), 1.99 - 1.83 (m, 2H), 1.83 - 1.71 (m, 2H), 1.52 (s, 9H). ¹³C NMR (101 MHz, CDCl₃) δ 155.40, 153.09, 152.91, 149.10, 136.97, 128.59, 128.08, 127.96, 122.23, 119.16, 106.95, 101.14, 80.17, 72.56, 67.21, 55.77, 40.89, 30.59, 28.49. LC-MS, ESI⁺, m/z 479.2 [M+Na]+. 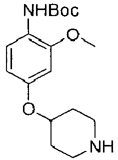 *tert-butyl (2-methoxy-4-(piperidin-4-yloxy)phenyl)carbamate*

To a solution of benzyl 4-(4-((*tert*-butoxycarbonyl)amino)-3-methoxyphenoxy)piperidine-1-carboxylate (450 mg, 0.99 mmol) in methanol (15 ml) was added 10% palladium charcoal (50 mg) under nitrogen protection. The resulting mixture was stirred at room temperature overnight under positive hydrogen pressure created by a balloon of hydrogen gas, then filtered through a pad of celite, and the filtrate was condensed and purified through flash column chromatography on silica gel (0%-10% methanol in DCM with 0.7 M ammonia) to give *tert-butyl (2-methoxy-4-(piperidin-4-yloxy)phenyl)carbamate* (210 mg, 66%). ¹H NMR (400 MHz, CDCl₃) δ 7.87 (d, J = 7.9 Hz, 1H), 6.82 (s, 1H), 6.49 - 6.43 (m, 2H), 4.35 - 4.18 (m, 1H), 3.82 (s, 3H), 3.20 - 2.98 (m, 2H), 2.70 (ddd, J = 12.6, 9.4, 3.1 Hz, 2H), 2.05 - 1.90 (m, 2H), 1.71 - 1.57 (m, 3H), 1.50 (s, 9H); ¹³C NMR (101 MHz, CDCl₃) δ 153.24, 153.14, 149.09, 121.94, 119.18, 106.99, 101.19, 80.13, 74.25, 55.78, 44.18, 32.70, 28.51. LC-MS, ESI⁺, m/z 323 [M+H]⁺. 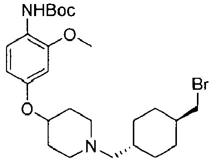 *tert-butyl (4-((1-(((1R,4R)-4-(bromomethyl)cyclohexyl)methyl)piperidin-4-yl)oxy)-2-methoxyphenyl)carbamate*

To a solution of tert-butyl (2-methoxy-4-(piperidin-4-yloxy)phenyl)carbamate (56 mg, 0.17 mmol) and tans-1,4-bis(bromomethyl)cyclohexane (147 mg, 0.54 mmol) in acetone (3 ml) was added K₂CO₃ (126 mg, 0.91 mmol). After stirring at 60 °C overnight, the reaction mixture was cooled down, diluted with water, and extracted with ethyl acetate (3×). The combined organic phase was washed with water and brine, dried over sodium sulfate, filtered, and condensed to give a residue which was purified through flash column chromatography on silica gel (0%-10% methanol in DCM) to yield *tert-butyl (4-((1-(((1R,4R)-4-(bromomethyl)cyclohexyl)methyl)piperidin-4-yl)oxy)-2-methoxyphenyl)carbamate* (41 mg, 45% yield). ¹H NMR (400 MHz, CDCl₃) δ 7.87 (d, J = 7.8 Hz, 1H), 6.81 (s, 1H), 6.48 - 6.43 (m, 2H), 4.21 (tt, J = 7.8, 3.7 Hz, 1H), 3.82 (s, 3H), 3.28 (d, *J* = 6.3 Hz, 2H), 2.73 - 2.64 (m, 2H), 2.23 - 2.17 (m, 2H), 2.14 (d, *J* = 7.1 Hz, 2H), 1.98 - 1.85 (m, 5H), 1.82 - 1.71 (m, 2H), 1.65 - 1.54 (m, 1H), 1.51 (s, 9H), 1.48 - 1.37 (m, 1H), 1.09 - 0.85 (m, 4H); ¹³C NMR (101 MHz, CDCl₃) δ 153.44, 153.17, 149.09, 121.88, 119.19, 107.03, 101.20, 80.14, 73.95, 65.39, 55.80, 51.50, 40.72, 40.53, 35.51, 31.57, 31.38, 31.23, 28.54. LC-MS, ESI⁺, m/z 511 [M+H]⁺, 513 [M+H+2]⁺. 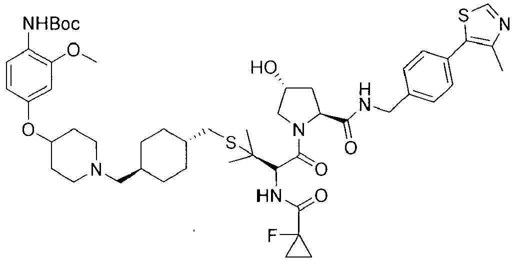 tert-butyl (4-((1-(((1S,4r)-4-((((S)-3-(1-fluorocyclopropane-1-carboxamido)-4-((*2S*,*4R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)piperidin-4-yl)oxy)-2-methoxyphenyl)carbamate *tert*-butyl (4-((1-(((1S,4r)-4-((((S)-3-(1-fluorocyclopropane-1-carboxamido)-4-((*2S,4R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)piperidin-4-yl)oxy)-2-methoxyphenyl)carbamate was prepared following general procedure 8. 27 mg tert-butyl (4-((1-(((1S,4r)-4-((((S)-3-(1-fluorocyclopropane-1-carboxamido)-4-((*2S,4R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)piperidin-4-yl)oxy)-2-methoxyphenyl)carbamate was obtained from 21 mg thiol compound, 72% yield. ¹H NMR (400 MHz, CDCl₃) δ 8.66 (s, 1H), 7.89 (d, J = 7.4 Hz, 1H), 7.41 - 7.31 (m, 5H), 7.23 (dd, J = 8.1, 3.3 Hz, 1H), 6.82 (s, 1H), 6.48 - 6.42 (m, 2H), 4.80 - 4.71 (m, 2H), 4.53 (s, 1H), 4.44 (d, J = 6.0 Hz, 2H), 4.40 (d, J = 12.3 Hz, 1H), 3.99 (d, J = 11.2 Hz, 1H), 3.82 (s, 3H), 3.74 (dd, J = 11.1, 4.0 Hz, 1H), 2.87-2.60 (m, 4H), 2.51 (s, 3H), 2.46 - 2.18 (m, 8H), 2.01 - 1.80 (m, 6H), 1.50 (s, 9H), 1.38 - 1.21 (m, 12H), 0.91 (d, J = 8.8 Hz, 4H); ¹³C NMR (101 MHz, CDCl₃) δ 170.95, 170.44 (d, J = 20.62 Hz), 169.99, 153.13, 152.71, 150.39, 149.15, 148.61, 138.21, 131.75, 131.06, 129.60, 128.14, 122.37, 119.30, 107.15, 100.80, 80.29, 78.26 (d, J = 230.6 Hz), 70.11, 64.73, 59.23, 56.71, 56.24, 55.85, 50.36, 47.98, 43.17, 38.05, 36.98, 35.27, 34.47, 32.46, 32.40, 31.64, 28.51, 25.74, 25.45, 16.25, 14.01, 13.90. LC-MS, ESI⁺, m/z 966 [M+H]⁺. 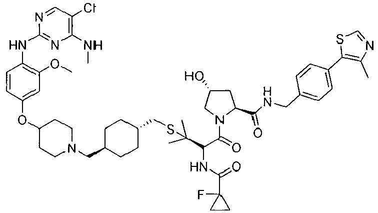 *(2S,4R)-1-((S)-3-((((1R,4S)-4-((4-(4-((5-chloro-4-(methylamino)pyrimidin-2-yl)omino)-3-methoxyphenoxy)piperidin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(31, XL02017)***

To a solution of *tert*-butyl (4-((1-(((1*S*,4*R*)-4-((((*S*)-3-(1-fluorocyclopropane-1-carboxamido)-4-((*2S,4R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)piperidin-4-yl)oxy)-2-methoxyphenyl)carbamate (27 mg, 0.028 mmol) in DCM (1 mL) was added HCl (4N in 1,4-dioxane, 1 mL). After stirring at room temperature overnight, the mixture was condensed and used to the next step without further purification. The condensed residue (18.6 mg, 0.02 mmol) from last step was dissolved in iso-propanol (1 mL) in a microwave tube, followed by addition of 2,5-dichloro-N-methylpyrimidin-4-amine (3.7 mg, 0.02 mmol) and HCl (4N HCl in 1,4-dioxane, one drop). After heating in the microwave reactor at 100 °C for 8h, the reaction mixture was condensed and purified through preparation HPLC under acidic condition (5-95 % CH₃CN in 0.1 % aq. HCO₂H) to give **XL02017** (2.8 mg, 13% yield two steps). ¹H NMR (500 MHz, CDCl₃) δ 8.67 (s, 1H), 8.42 (s, 1H), 8.33 - 8.26 (m, 1H), 7.87 (s, 1H), 7.40 - 7.30 (m, 5H), 7.21 (dd, J = 7.9, 3.2 Hz, 1H), 6.51 -6.47 (m, 2H), 5.23 (q, *J* = 4.8 Hz, 1H), 4.83 - 4.72 (m, 2H), 4.54 (s, 1H), 4.46 (d, J = 5.9 Hz, 2H), 4.41 (s, 1H), 4.02 (d, J = 11.4 Hz, 1H), 3.86 (s, 3H), 3.74 (dd, J = 11.1, 4.1 Hz, 1H), 3.08 (d, J = 4.9 Hz, 3H), 2.92 - 2.72 (m,4H), 2.53 (s, 3H), 2.51 - 2.46 (m, 4H), 2.38 (ddd, J = 18.0, 11.5, 6.9 Hz, 4H), 2.25 - 2.16 (m, 3H), 2.02 - 1.93 (m, 2H), 1.88 - 1.79 (m, 3H), 1.60 (s, 1H), 1.36 - 1.28 (m, 10H), 0.94 (t, J = 10.2 Hz, 4H); HRMS (ESI⁺) *m*/*z,* calcd for C₅₀H₆₅ClFN₉O₆S₂: 1006.4244 [M + H]⁺, found 1006.4302.

### Exemplary synthesis of Exemplary compound 32 (XL02031)

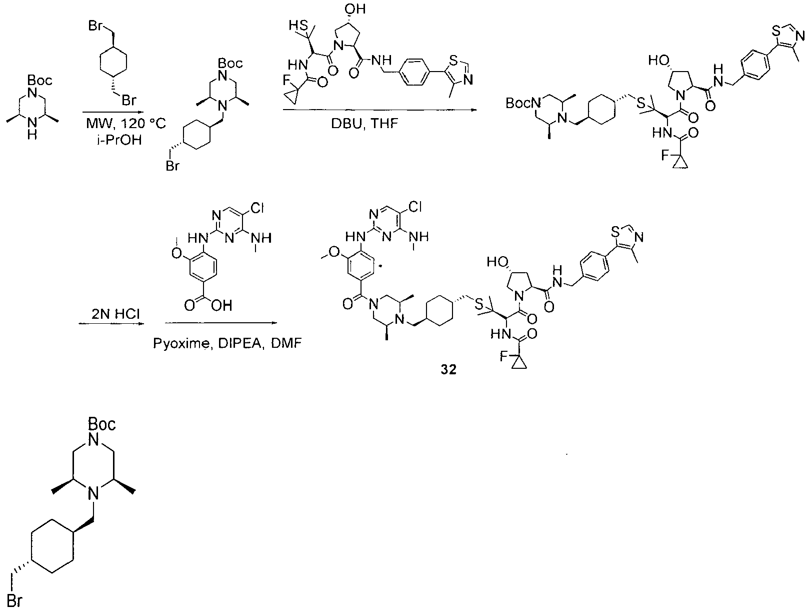

*tert-butyl(3S,5R)-4-(((1R,4R)-4-(bromomethyl)cyclohexyl)methyl)-3,5-dimethylpiperazine-1-carboxylate*

To a solution of *tert*-butyl (3*S*,5*R*)-3,5-dimethylpiperazine-1-carboxylate (34 mg, 0.16 mmol) and trans-1,4-bis(bromomethyl)cyclohexane (130 mg, 0.48 mmol) in isopropanol (1 mL) was added N,N-Diisopropylethylamine (0.083 mL, 0.48 mmol). After stirring at 120 °C in a microwave reactor for 6h, the mixture was cooled down and condensed to afford a residue which was purified through flash column chromatography on silica gel (0%-100% ethyl acetate in heptane) to yield *tert-butyl (3S,5R)-4-(((1R,4R)-4-(bromomethyl)cyclohexyl)methyl)-3,5-dimethylpiperazine-1-carboxylate* (11 mg, 17% yield). ¹H NMR (400 MHz, CDCl₃) δ 3.74 (s, 2H), 3.27 (d, *J* = 6.3 Hz, 2H), 2.60 (t, J = 11.3 Hz, 2H), 2.50 - 2.38 (m, 2H), 2.32 (d, J = 6.6 Hz, 2H), 1.95 - 1.84 (m, 4H), 1.66 - 1.50 (m, 1H), 1.45 (s, 9H), 1.39 - 1.27 (m, 1H), 1.08 - 0.71 (m, 10H). ¹³C NMR (101 MHz, CDCl₃) δ 154.65, 79.62, 57.17, 56.69, 50.15, 40.62, 40.43, 38.39, 31.74, 31.68, 28.61, 18.81. LC-MS, ESI⁺, m/z 403 [M+H]⁺. 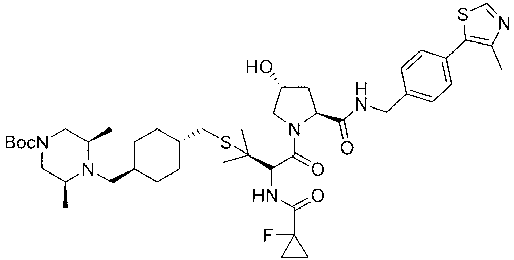 *tert-butyl (3R,5S)-4-(((1S,4R)-4-((((S)-3-(1-fluorocyclopropane-1-carboxamido)-4-((2S,4R)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)-3,5-dimethylpiperazine-1-carboxylate* was prepared following general procedure 8. 15 mg *tert-butyl (3R,5S)-4-(((1S,4R)-4-((((S)-3-(1-fluorocyclopropane-1-carboxamido)-4-((2S,4R)-4-hydroxy-2-((4-(4-methylthiazol-5-yl)benzyl)carbamoyl)pyrrolidin-1-yl)-2-methyl-4-oxobutan-2*-*yl)thio)methyl)cyclohexyl)methyl)-3,5-dimethylpiperazine-1-carboxylate* was obtained from 15 mg thiol compound, 62.5% yield. ¹H NMR (500 MHz, CDCl₃) δ 8.67 (s, 1H), 7.40 - 7.32 (m, 5H), 7.22 (dd, J = 7.7, 3.1 Hz, 1H), 4.79 (t, J = 7.9 Hz, 1H), 4.72 (d, J = 7.7 Hz, 1H), 4.52 (s, 1H), 4.45 (d, J = 5.9 Hz, 2H), 4.05 (d, J = 11.3 Hz, 1H), 3.82 - 3.62 (br, s, 1H), 3.72 (dd, J = 11.2, 3.9 Hz, 2H), 2.78 (s, 1H), 2.64 - 2.54 (m, 2H), 2.52 (s, 3H), 2.50 - 2.30 (m, 5H), 2.30 - 2.20 (m, 3H), 1.81 (d, *J* = 10.0 Hz, 4H), 1.45 (s, 9H), 1.37 -1.26 (m, 12H), 1.01 (d, J = 6.2 Hz, 6H), 0.96 - 0.84 (m, 2H), 0.84 - 0.72 (m, 2H). ¹³C NMR (126 MHz, CDCl₃) δ 170.78, 170.62 (d, J = 16.6 Hz), 170.18, 154.65, 150.37, 148.68, 138.18, 131.72, 131.17, 129.66, 128.18, 79.65, 79.20, 70.28, 59.03, 57.25, 56.69, 56.35, 50.46, 49.70, 47.71, 43.26, 38.44, 36.82, 35.41, 33.07, 33.01, 31.91, 28.61, 25.79, 25.40, 18.80, 16.26, 14.07 (d, J = 8.4 Hz), 13.95 (d, *J* = 8.4 Hz). LC-MS, ESI⁺, m/z 857.9 [M+H]⁺. 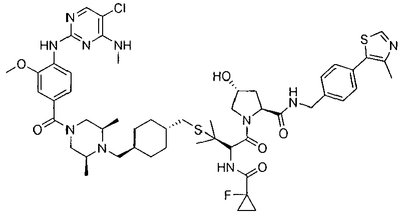 *(2S,4R)-1-((S)-3-((((1R,4S)-4-(((2R,6S)-4-(4-((5-chloro-4-(methylamino)pyrimidin-2-yl)amino)-3-methoxybenzoyl)-2,6-dimethylpiperazin-1-yl)methyl)cyclohexyl)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide (32, **XL02031)***

To a solution of *tert*-butyl (3*R*,5*S*)-4-(((1*S*,4*R*)-4-((((*S*)-3-(1-fluorocyclopropane-1-carboxamido)-4-((2*S*,4*R*)-4-hydroxy-2-((4-(4-methylthiazol-5-yl)benzyl)carbamoyl)pyrrolidin-1-yl)-2-methyl-4-oxobutan-2-yl)thio)methyl)cyclohexyl)methyl)-3,5-dimethylpiperazine-1-carboxylate (15 mg, 0.018 mmol) in DCM (1 mL) was added HCl (4N in 1,4-dioxane, 1 mL). After stirring at room temperature overnight, the mixture was condensed and used to next step without further purification. The residue from previous step was dissolved in DMF (1 mL), followed by addition of 4-((5-chloro-4-(methylamino)pyrimidin-2-yl)amino)-3-methoxybenzoic acid (6 mg, 0.019 mmol), N,N-Diisopropylethylamine (0.012 mL, 0.070 mmol) and Pyoxim (11 mg, 0.02 mmol). The resulting mixture was stirred at room temperature overnight, filtered through 0.45 µM filter and injected into preparative HPLC for purification under acidic condition (5-95 % CH₃CN in 0.1 % aq. HCO₂H) to give **XL02031** (2 mg, 11% yield for two steps). ¹H NMR (500 MHz, CDCl₃) δ 8.67 (s, 1H), 8.54 (d, *J* = 8.2 Hz, 1H), 7.93 (s, 1H), 7.65 (s, 1H), 7.40 - 7.31 (m, 5H), 7.22 (dd, J = 7.7, 3.2 Hz, 1H), 7.03 - 6.98 (m, 2H), 5.29 (q, J = 4.8 Hz, 1H), 4.80 (t, J = 7.9 Hz, 1H), 4.72 (d, J = 7.7 Hz, 1H), 4.54 (s, 1H), 4.46 (d, *J* = 5.9 Hz, 2H), 4.07 (d, *J* = 11.3 Hz, 1H), 3.93 (s, 3H), 3.72 (dd, J = 11.2, 3.9 Hz, 1H), 3.11 (d, J = 4.9 Hz, 3H), 2.80 (s, 2H), 2.57 - 2.44 (m, 6H), 2.41 - 2.30 (m, 4H), 2.27 - 2.20 (m, 1H), 1.86 - 1.74 (m, 6H), 1.36 - 1.28 (m, 12H), 1.14 - 0.86 (m, 8H), 0.80 (dd, J = 23.0, 11.6 Hz, 2H). ¹⁹F NMR (471 MHz, CDCl₃) δ -197.79; HRMS (ESI⁺) m/z, calcd for C₅₂H₆₈ClFN₁₀O₆S₂: 1047.4510 [M + H]⁺, found 1047.4608.

### Exemplary synthesis of Exemplary compound 33 (XL02038)

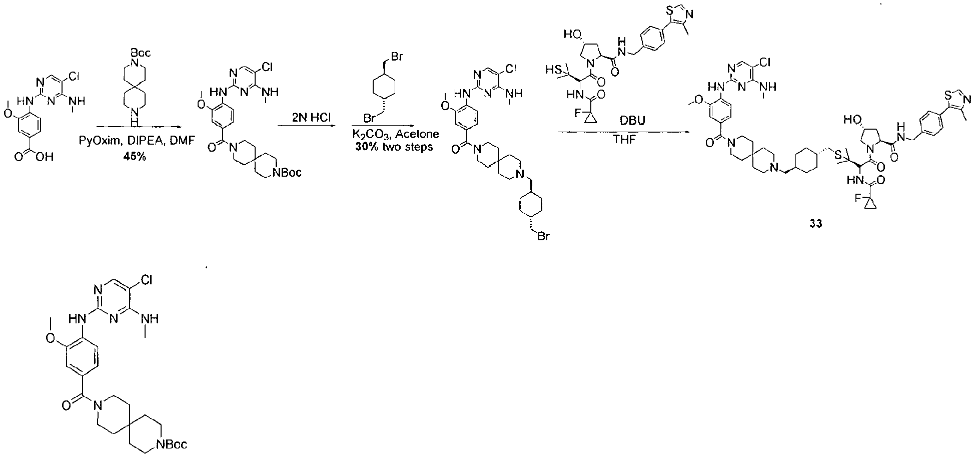

*tert-butyl 9-(4-((5-chloro-4-(methylamino)pyrimidin-2-yl)amino)-3-methoxybenzoyl)-3,9-diazaspiro[5.5] undecane-3-carboxylate*

To a solution of 4-((5-chloro-4-(methylamino)pyrimidin-2-yl)amino)-3-methoxybenzoic acid (100 mg, 0.32 mmol), *tert*-butyl 3,9-diazaspiro[5.5]undecane-3-carboxylate (83 mg, 0.33 mmol), and N,N-diisopropylethylamine (0.113 ml, 0.65 mmol) in DMF (5 mL) was added Pyoxim (171 mg, 0.32 mmol). After stirring at room temperature for 2h, the reaction mixture was diluted with ethyl acetate, washed with water and brine, dried over sodium sulfate, filtered, and condensed to afford a residue which was purified through flash column chromatography on silica gel (0%-10% methanol in DCM) to give *tert-butyl 9-(4-((5-chloro-4-(methylamino)pyrimidin-2-yl)amino)-3-methoxybenzoyl)-3,9-diazaspiro[5.5]undecane-3-carboxylate* (80 mg, 45% yield). ¹H NMR (400 MHz, CDCl₃) δ 8.53 (d, *J* = 8.7 Hz, 1H), 7.91 (s, 1H), 7.62 (s, 1H), 7.06 - 6.92 (m, 2H), 5.32 (q, *J* = 4.8 Hz, 1H), 3.91 (s, 3H), 3.59 (s, 4H), 3.43 - 3.36 (m, 4H), 3.09 (d, J = 4.9 Hz, 3H), 1.56 - 1.46 (m, 8H), 1.45 (s, 9H). ¹³C NMR (101 MHz, CDCl₃) δ 170.70, 158.69, 157.95, 155.06, 152.78, 147.53, 131.37, 128.36, 120.05, 116.91, 109.57, 105.62, 79.58, 55.99, 39.43, 35.41, 30.59, 28.59, 28.19. LC-MS, ESI⁺, 545 [M+H]⁺. 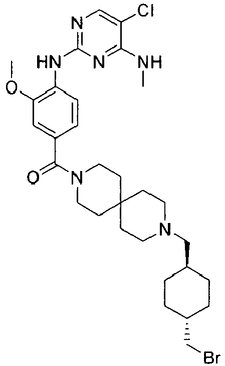 *(9-(((1r,4r)-4-(bromomethyl)cyclohexyl)methyl)-3,9-diazaspiro[5.5]undecan-3-yl)(4-((5-chloro-4-(methylamino)pyrimidin-2-yl)amino)-3-methoxyphenyl)methanone*

To a solution of *tert*-butyl 9-(4-((5-chloro-4-(methylamino)pyrimidin-2-yl)amino)-3-methoxybenzoyl)-3,9-diazaspiro[5.5]undecane-3-carboxylate (70 mg, 0.128 mmol) in DCM (1 mL) was added HCl (4N HCl in 1,4-dioxane, 1 mL). After stirring at room temperature overnight, the mixture was condensed and used to next step. The condensed residue was dissolved in acetone, followed by addition of trans-1,4-bis(bromomethyl)cyclohexane (44 mg, 0.163 mmol) and K₂CO₃ (37 mg, 0.27 mmol). The resulting mixture was stirred at 50 °C for overnight, then cooled down to room temperature. Water was added and the mixture was extracted with ethyl acetate (3x). The combined organic phase was washed with water and brine, dried over sodium sulfate, filtered, and condensed to give the crude product as a residue which was purified through flash column chromatography on silica gel (0%-10% methanol in DCM) to give *(9-(((1r,4r)-4-(bromomethyl)cyclohexyl)methyl)-3,9-diazaspiro[5.5]undecan-3-yl)(4-((5-chloro-4-(methylamino)pyrimidin-2-yl)amino)-3-methoxyphenyl)methanone* (12 mg, 35% yield). ¹H NMR (400 MHz, CDCl₃) δ 8.52 (d, J = 8.7 Hz, 1H), 7.92 (s, 1H), 7.62 (s, 1H), 7.00 (td, J = 4.3, 1.7 Hz, 2H), 5.32 - 5.26 (m, 1H), 3.92 (s, 3H), 3.73 - 3.41 (m, 4H), 3.28 (d, J = 6.2 Hz, 2H), 3.10 (d, *J* = 4.9 Hz, 3H), 2.36 (s, 4H), 2.14 (d, *J* = 6.4 Hz, 2H), 1.98 - 1.78 (m, 4H), 1.64 - 1.36 (m, 10H), 1.08 - 0.85 (m, 4H). ¹³C NMR (101 MHz, CDCl₃) δ 170.66, 158.72, 158.00, 152.83, 147.54, 131.29, 128.60, 120.06, 116.94, 109.59, 105.60, 65.79, 56.01, 49.82, 40.69, 40.46, 35.68, 35.28, 31.54, 31.46, 30.25, 28.22. LC-MS, ESI⁺, 633 [M+H]⁺. 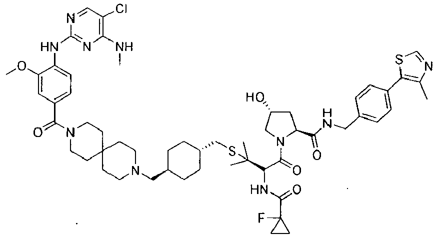 *(2S,4R)-1-((S)-3-((((1R,4S)-4-((9-(4-((5-chloro-4-(methylamino)pyrimidin-2-yl)amino)-3-methoxybenzoyl)-3,9-diazaspiro[5.5]undecan-3-yl)methyl)cyclohexy*/*)methyl)thio)-2-(1-fluorocyclopropone-1-carboxamido)-3-methylbutanoyl)-4-hydroxy-N-(4-(4-methylthiazol-5-yl)benzyl)pyrrolidine-2-carboxamide(**33**, **XL02038**)*

**XL02038** was prepared following general procedure 8. 6.7 mg **XL02038** was obtained from 10 mg thiol compound, 33% yield. ¹H NMR (500 MHz, CDCl₃) δ 8.59 (s, 1H), 8.45 (d, *J* = 8.7 Hz, 1H), 7.84 (s, 1H), 7.55 (s, 1H), 7.34 - 7.23 (m, 5H), 7.14 (dd, *J* = 7.8, 3.0 Hz, 1H), 6.94 - 6.88 (m, 2H), 5.22 (q, J = 4.8 Hz, 1H), 4.77 -4.63 (m, 2H), 4.45 (s, 1H), 4.37 (d, J = 5.9 Hz, 2H), 3.94 (d, J = 11.1 Hz, 1H), 3.84 (s, 3H), 3.65 (dd, *J* = 11.1, 3.9 Hz, 1H), 3.50 (s, 4H), 3.02 (d, J = 4.9 Hz, 3H), 2.55 - 2.45 (br, 6H), 2.44 (s, 3H), 2.42 - 2.35 (m, 2H), 2.34 - 2.27 (m, 2H), 2.25 - 2.19 (m, 2H), 2.18 - 2.11 (m, 1H), 1.80 - 1.65 (m, 4H), 1.65 - 1.50 (m, 4H), 1.43 (s, 4H), 1.32-1.13 (m, 11H), 0.93-0.74 (m, 4H); ¹³C NMR (126 MHz, CDCl₃) δ 170.83, 170.74, 170.58 (d, *J* = 20.7 Hz), 170.13, 158.73, 157.97, 152.79, 150.39, 148.66, 147.58, 138.20, 131.76, 131.42, 131.14, 129.65, 128.34, 128.20, 120.09, 116.96, 109.61, 105.66, 79.20, 77.41, 77.16, 76.91, 70.15, 64.86, 59.09, 56.63, 56.29, 56.02, 49.31, 47.77, 43.25, 38.16, 36.78, 35.39, 34.65, 34.37, 32.65, 32.62, 31.56, 30.00, 28.22, 25.80, 25.41, 16.26, 14.07 (d, *J* = 10.6 Hz), 13.95 (d, J = 10.5 Hz). ¹⁹F NMR (471 MHz, CDCl₃) δ -197.76; HRMS (ESI⁺) m/z, calcd for C55H72ClFN10O6S2: 1087.4823 [M + H]⁺, found 1087.4601.

### Exemplary synthesis of Exemplary compound 34 (XL02047)

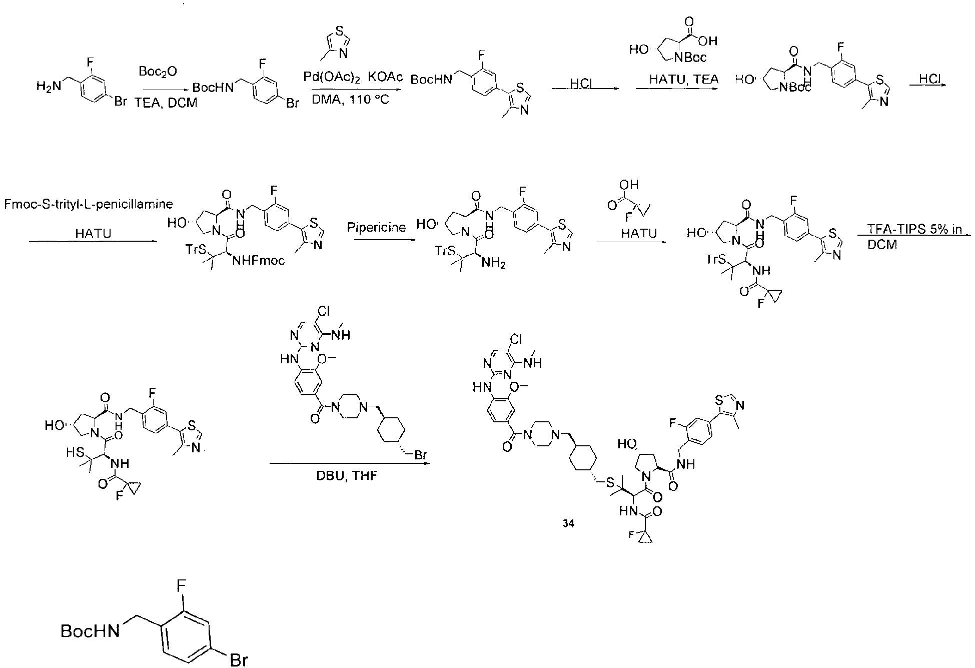

*tert-butyl (4-bromo-2-fluorobenzyl)carbamate* was prepared following general procedure 1. 6.1g *tert-butyl (4-bromo-2-fluorobenzyl)carbamate* was obtained from 5 g amine compound, 83% yield. ¹H NMR (400 MHz, CDCl₃) δ 7.26 (ddd, J = 16.8, 9.3, 2.0 Hz, 3H), 4.92 (s, 1H), 4.32 (d, J = 5.7 Hz, 2H), 1.46 (s, 9H). 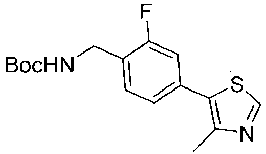 *tert-butyl (2-fluoro-4-(4-methylthiazol-5-yl)benzyl)carbamate* was prepared following general procedure 2. 3.4g *tert-butyl (2-fluoro-4-(4-methylthiazol-5-yl)benzyl)carbamate* was obtained from 4 g *tert-*butyl (4-bromo-2-fluorobenzyl)carbamate, 80% yield. ¹H NMR (500 MHz, CDCl₃) δ 8.66 (s, 1H), 7.38 (t, J = 7.8 Hz, 1H), 7.17 (dd, J = 7.9, 1.6 Hz, 1H), 7.11 (dd, J = 10.8, 1.6 Hz, 1H), 5.08 (s, 1H), 4.37 (d, *J* = 5.1 Hz, 2H), 2.51 (s, 3H), 1.44 (s, 9H); ¹³C NMR (126 MHz, CDCl₃) δ 160.81 (d, *J* = 247.6 Hz), 155.95, 152.78, 150.74, 149.25, 133.25 (d, J = 8.8 Hz), 130.18, 125.93 (d, J = 14.9 Hz), 125.30, 116.21 (d, J = 23 Hz), 79.91, 38.55, 28.53, 16.26. LC-MS, ESI⁺, 543 [M+H]⁺. 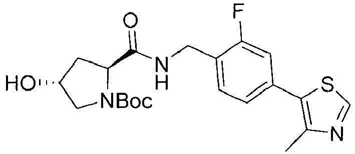 *tert-butyl (2S,4R)-2-((2-fluoro-4-(4-methylthiazol-5-yl)benzyl)carbamoyl)-4-hydroxypyrrolidine-1-carboxylate* was prepared following general procedure 3. 1.8 g *tert-butyl (2S,4R)-2-((2-fluoro-4-(4-methylthiazol-5-yl)benzyl)carbamoyl)-4-hydroxypyrrolidine-1-carboxylate* was obtained from 1.48 g tert-butyl (2-fluoro-4-(4-methylthiazol-5-yl)benzyl)carbamate, 90% yield. ¹H NMR (500 MHz, CDCl₃) δ 8.69 (s, 1H), 7.40 (s, 1H), 7.15 (dd, *J* = 22.4, 9.2 Hz, 2H), 4.60 - 4.30 (m, 4H), 3.70 - 3.30 (m, 2H), 2.52 (s, 3H), 2.35 - 1.95 (m, 2H), 1.50 - 1.25 (m, 9H); ¹⁹F NMR (471 MHz, CDCl₃) δ -118.01. LC-MS, ESI⁺, m/z 436.1 [M+H]⁺. 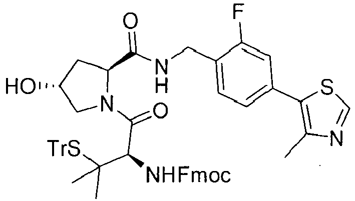 *(9H-fluoren-9-yl)methyl ((R)-1-((2R,4R)-2-((2-fluoro-4-(4-methylthiazol-5-yl)benzyl)carbamoyl)-4-hydroxypyrrolidin-1-yl)-3-methyl-1-oxo-3-(tritylthio)butan-2-yl)carbamate* was prepared following general procedure 4. 170 mg *(9H-fluoren-9-yl)methyl ((R)-1-((2R,4R)-2-((2-fluoro-4-(4-methylthiazol-5-yl)benzyl)carbamoyl)-4-hydroxypyrrolidin-1-yl)-3-methyl-1-oxo-3-(tritylthio)buton-2-yl)carbamate* was obtained from 119 mg tert-butyl (2*S*,4*R*)-2-((2-fluoro-4-(4-methylthiazol-5-yl)benzyl)carbamoyl)-4-hydroxypyrrolidine-1-carboxylate, 67% yield. ¹H NMR (500 MHz, CDCl₃) δ 8.70 (s, 1H), 7.73 (t, *J* = 7.2 Hz, 2H), 7.58 (d, J = 7.5 Hz, 1H), 7.53 (d, *J* = 7.2 Hz, 7H), 7.41 (t, J = 5.9 Hz, 1H), 7.39 - 7.34 (m, 3H), 7.24 - 7.15 (m, 11H), 7.06 (t, J = 8.5 Hz, 2H), 5.70 (d, *J* = 5.6 Hz, 1H), 4.64 (t, J = 8.0 Hz, 1H), 4.38 - 4.29 (m, 3H), 4.28 - 4.21 (m, 2H), 4.21 - 4.16 (m, 1H), 3.59 (d, J = 5.6 Hz, 1H), 3.45 (d, *J* = 11.3 Hz, 1H), 3.27 - 3.16 (m, 2H), 2.51 (s, 3H), 2.28 - 2.20 (m, 1H), 2.12 - 2.02 (m, 1H), 1.23 (s, 3H), 1.03 (s, 3H); ¹³C NMR (126 MHz, CDCl₃) δ 171.13, 170.49, 160.47 (d, J = 248 Hz), 156.47, 150.75, 149.11, 144.49, 143.93, 143.55, 141.40, 141.35, 132.88 (d, *J* = 7.5 Hz), 130.62, 129.94, 127.93, 127.23, 127.21, 127.00, 125.16 (d, J = 11.3 Hz), 125.07, 120.14, 115.95 (d, J = 22.8 Hz), 70.18, 68.63, 67.65, 58.88, 58.24, 56.70, 54.34, 47.08, 37.01 (d, J = 3.15 Hz), 36.68, 25.96, 25.49, 16.24; ¹⁹F NMR (471 MHz, CDCl₃) δ -117.64. LC-MS, ESI⁺, m/z 931 [M+H]⁺. 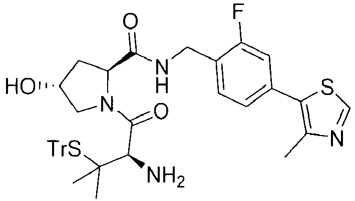 *(2R,4R)-1-((R)-2-amino-3-methyl-3-(tritylthio)butanoyl)-N-(2-fluoro-4-(4-methylthiazol-5-yl)benzyl)-4-hydroxypyrrolidine-2-carboxamide* was prepared following general procedure 5. 105 mg *(2R,4R)-1-((R)-2-amino-3-methyl-3-(tritylthio)butanoyl)-N-(2-fluoro-4-(4-methylthiazol-5-yl)benzyl)-4-hydroxypyrrolidine-2-carboxamide* was obtained from 150 mg Fmoc protected compound, 92% yield. ¹H NMR (500 MHz, CDCl₃) δ 8.71 (s, 1H), 7.58 (d, *J* = 7.5 Hz, 7H), 7.39 (t, J = 6.0 Hz, 2H), 7.29 (s, 1H), 7.27 (d, J = 7.9 Hz, 5H), 7.23 - 7.18 (m, 4H), 7.11 (dd, *J* = 7.9, 1.6 Hz, 1H), 7.06 (dd, *J* = 10.7, 1.6 Hz, 1H), 4.85 (br, s, 1H), 4.58 (t, *J* = 8.1 Hz, 1H), 4.34 (s, 1H), 4.30 (d, J = 6.2 Hz, 2H), 3.15 (dd, J = 11.2, 3.7 Hz, 1H), 3.10 (d, J = 11.3 Hz, 1H), 2.79 (s, 1H), 2.53 (s, 3H), 2.18 - 2.05 (m, 2H), 1.23 (s, 3H), 1.06 (s, 3H); ¹³C NMR (126 MHz, CDCl₃) δ 172.15, 171.77, 160.38 (d, J = 248 Hz), 150.72, 149.09, 144.86, 132.85 (d, J = 8.7 Hz), 130.58, 129.98, 129.87 (d, J = 4.7 Hz), 127.91, 126.89, 125.24 (d, J = 15.2 Hz), 125.11 (d, J = 2.9 Hz), 116.92 (d, J = 23.0 Hz), 69.85, 68.28, 59.10, 58.27, 58.11, 56.96, 37.37, 36.90 (d, *J* = 3.5 Hz), 25.32, 24.61, 16.21; ¹⁹F NMR (471 MHz, CDCl₃) δ -117.81; LC-MS, ESI⁺, m/z 731 [M+Na]⁺. 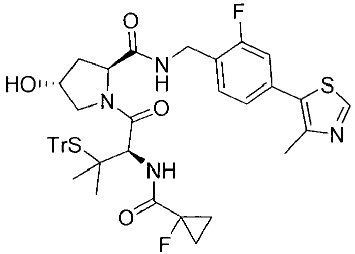 *(2R,4R)-N-(2-fluoro-4-(4-methylthiazol-5-yl)benzyl)-1-((R)-2-(1-fluorocyclopropane-1-carboxamido)-3-methyl-3-(tritylthio)butanoyl)-4-hydroxypyrrolidine-2-carboxamide* was prepared following general procedure 6. 71 mg *(2R,4R)-N-(2-fluoro-4-(4-methylthiazol-5-yl)benzyl)-1-((R)-2-(1-fluorocyclopropane-1-carboxamido)-3-methyl-3-(tritylthio)butanoyl)-4-hydroxypyrrolidine- 2-carboxamide* was obtained from 105 mg amine compound, 60% yield. ¹H NMR (500 MHz, CDCl₃) δ 8.73 (s, 1H), 7.61 - 7.53 (m, 6H), 7.44 - 7.34 (m, 2H), 7.28 - 7.18 (m, 10H), 7.12 - 7.06 (m, 2H), 4.65 (t, J = 8.1 Hz, 1H), 4.38 (s, 1H), 4.33 (t, J = 5.4 Hz, 1H), 3.62 (d, J = 5.1 Hz, 1H), 3.49 (d, J = 11.5 Hz, 1H), 3.43 (d, J = 5.5 Hz, 1H), 3.25 (dd, *J* = 11.4, 3.7 Hz, 1H), 2.54 (s, 3H), 2.29 (ddd, J = 12.9, 8.2, 4.5 Hz, 1H), 2.14 -2.05 (m, 2H), 1.42 -1.33 (m, 2H), 1.32 -1.25 (m, 5H), 1.08 (s, 3H); ¹³C NMR (126 MHz, CDCl₃) δ 171.0, 170.27 (d, *J* = 20.90 Hz), 169.87, 160.51 (d, J = 248 Hz), 150.75, 149.15, 144.51, 132.96 (d, *J* = 8.63 Hz), 130.62, 130.15 (d, J = 4.56 Hz), 129.87, 127.96, 127.02, 125.16 (d, *J* = 14.67 Hz), 125.17 (d, J = 3 Hz), 116.01 (d, J = 23.00 Hz), 78.27 (d, *J* = 231.37 Hz), 70.24, 68.50, 58.78, 57.05, 56.79, 53.94, 37.05 (d, *J* = 3.97 Hz), 36.59, 25.97, 25.72, 16.26, 13.79 (d, J = 10.9 Hz), 13.70 (d, J = 10.4 Hz); ¹⁹F NMR (471 MHz, CDCl₃) δ -117.70, -197.28; LC-MS, ESI⁻, 793 [M-H]⁻. 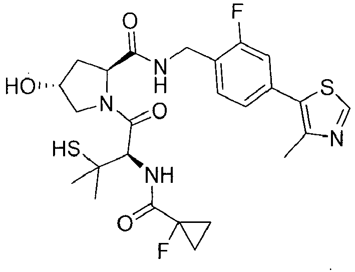 *(2S,4R)-N-(2-fluoro-4-(4-methylthiazol-5-y*/*)benzyl)-1-((R)-2-(1-fluorocyclopropane-1-carboxamido)-3-mercapto-3-methylbutanoyl)-4-hydroxypyrrolidine-2-carboxamide* was prepared following general procedure 7. 39 mg *(2S,4R)-N-(2-fluoro-4-(4-methylthiazol-5-yl)benzyl)-1-((R)-2-(1-fluorocyclopropane-1-carboxamido)-3-mercapto-3-methylbutanoyl)-4-hydroxypyrrolidine-2-carboxamide* was obtained from 71 mg trityl protected compound, 80% yield. ¹H NMR (500 MHz, CDCl₃) δ 8.71 (s, 1H), 7.47 -7.40 (m, 2H), 7.30 (t, *J* = 5.9 Hz, 1H), 7.15 (dd, J = 7.9,1.7 Hz, 1H), 7.11 (dd, J = 10.6, 1.6 Hz, 1H), 4.71 - 4.64 (m, 2H), 4.58 - 4.49 (m, 2H), 4.42 (dd, J = 15.2, 5.5 Hz, 1H), 4.06 (d, J = 11.3 Hz, 1H), 3.75 (dd, J = 11.2, 3.9 Hz, 1H), 3.31 (s, 1H), 2.59 (d, J = 0.7 Hz, 1H), 2.52 (s, 3H), 2.40 (ddd, J = 12.9, 7.9, 4.6 Hz, 1H), 2.18 - 2.09 (m, 1H), 1.39 - 1.25 (m, 10H). ¹³C NMR (126 MHz, CDCl₃) δ 170.92, 170.69 (d, J = 21.2 Hz), 170.23, 160.74 (d, J = 248.1 Hz), 150.99, 149.04, 133.26 (d, J = 8.0 Hz), 130.66, 130.55 *(d, J* = 4.9 Hz), 125.35 (d, J = 3.2 Hz), 125.01 (d, *J* = 15.1 Hz), 116.27 (d, J = 22.8 Hz), 78.20 (d, J = 232.5 Hz), 70.20, 59.10, 57.75, 56.71, 46.39, 37.46 (d, J = 3.8 Hz), 36.69, 30.46, 28.71, 16.15, 14.07, 13.99. ¹⁹F NMR (471 MHz, CDCl₃) δ -117.66, -197.71. LC-MS, ESI⁺, 553.2 [M+H]⁺. 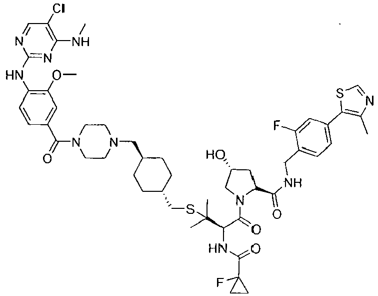 *(2R,4R)-1-(^{®}-3-((((1r,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-N-(2-fluoro-4-(4-methylthiazol-5-yl)benzyl)-4-hydroxypyrrolidine-2-carboxamide **(34, XL02047)***

**XL01047** was prepared following general procedure 8.12 mg **XL02047** was obtained from 10 mg thiol compound, 65% yield. ¹H NMR (400 MHz, CDCl₃) δ 8.70 (s, 1H), 8.56 (d, J = 8.8 Hz, 1H), 7.94 (s, 1H), 7.65 (s, 1H), 7.48 - 7.36 (m, 2H), 7.26 (dd, J = 7.8, 3.3 Hz, 1H), 7.19 (dd, J = 7.9, 1.7 Hz, 1H), 7.13 (dd, J = 10.7, 1.7 Hz, 1H), 7.04 - 7.00 (m, 2H), 5.32 (q, J = 4.4 Hz, 1H), 4.85 - 4.70 (m, 2H), 4.61 - 4.45 (m, 3H), 4.07 (d, *J* = 11.4 Hz, 1H), 3.94 (s, 3H), 3.80 - 3.47 (m, 5H), 3.13 (d, *J* = 4.9 Hz, 3H), 2.55 (s, 3H), 2.51 - 2.34 (m, 7H), 2.30 - 2.19 (m, 1H), 2.14 (d, J = 7.0 Hz, 2H), 1.91 - 1.80 (m, 4H), 1.44 - 1.30 (m, 12H), 1.02 -0.78 (m, 4H); ¹³C NMR (101 MHz, CDCl₃) δ 170.96, 170.61 (d, *J* = 20.3 Hz), 170.54, 170.16, 160.69 (d, *J* = 248.5 Hz), 158.72, 157.96, 152.80, 150.78, 149.22, 147.55, 133.27 (d, *J* = 8.5 Hz), 131.44, 130.55, 130.47 (d, J = 4.7 Hz), 128.12, 125.34 (d, J = 3.0 Hz), 125.04 (d, *J* = 15.0 Hz), 120.29, 116.92, 116.14 (d, J = 22.8 Hz), 109.73, 105.66, 78.39 (d, *J* = 208.4 Hz), 70.25, 65.40, 59.00, 56.69, 56.32, 56.01, 53.94, 47.71, 38.54, 37.16 (d, J = 3.7 Hz), 36.77, 35.42, 35.08, 32.80, 32.75, 31.48, 28.22, 25.68, 25.40, 16.31, 14.06 (d, J = 10.9 Hz), 13.95 (d, J = 10.8 Hz); ¹⁹F NMR (471 MHz, CDCl₃) δ -117.87, -197.73; HRMS (ESI⁺) *m*/*z,* calcd for C₅₀H₆₃ClF₂N₁₀O₆S₂: 1037.4102 [M + H]⁺, found 1037.4098.

### Exemplary synthesis of Exemplary compound 35 (XL02048)

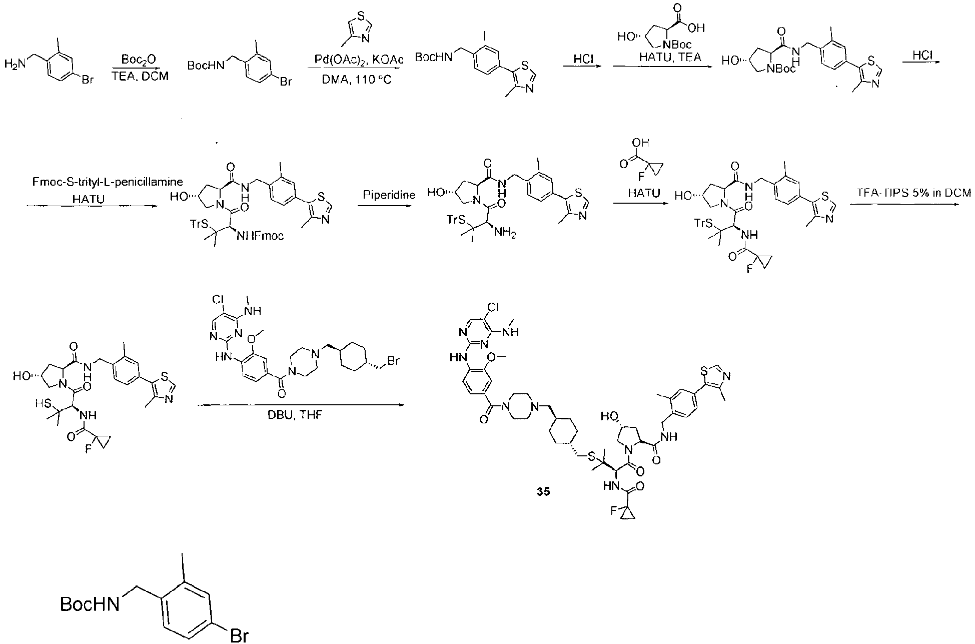

*tert-butyl (4-bromo-2-methylbenzyl)carbamate* was prepared following general procedure 1. 5.7g *tert-butyl (4-bromo-2-methylbenzyl)carbamate* was obtained from 4 g amine compound, 95% yield. ¹H NMR (400 MHz, CDCl₃) δ 7.43 - 7.27 (m, 2H), 7.10 (d, J = 7.9 Hz, 1H), 4.71 (s, 1H), 4.25 (d, J = 5.4 Hz, 2H), 2.29 (s, 3H), 1.45 (s, 9H). ¹³C NMR (101 MHz, CDCl₃) δ 155.79, 138.49, 135.83, 133.30, 129.6.3, 129.25, 121.31, 42.35, 28.54, 27.58, 18.88. LC-MS, ESI⁺, m/z 246 [M-56+H]⁺. 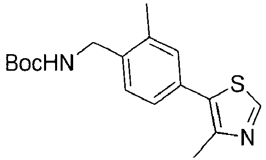 *tert-butyl (2-methyl-4-(4-methylthiazol-5-yl)benzyl)carbamate* was prepared following general procedure 2. 2.35g *tert-butyl (2-methyl-4-(4-methylthiazol-5-yl)benzyl)carbamate* was obtained from 3 g bromide compound, 70% yield. ¹H NMR (400 MHz, CDCl₃) δ 8.62 (s, 1H), 7.28 (d, *J* = 7.7 Hz, 1H), 7.24 - 7.20 (m, 2H), 4.92 (s, 1H), 4.32 (d, *J* = 5.3 Hz, 2H), 2.50 (s, 3H), 2.34 (s, 3H), 1.45 (s, 9H); ¹³C NMR (101 MHz, CDCl₃) δ 155.85, 150.19, 148.50, 136.66, 131.69, 131.30, 131.10, 128.21, 127.03, 79.62, 42.47, 28.49, 19.03, 16.14. LC-MS, ESI⁺, m/z 319.2 [M+H]⁺. 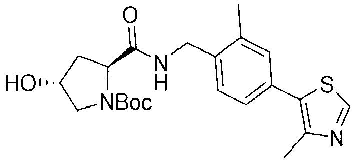 *tert-butyl (2S,4R)-4-hydroxy-2-((2-methyl-4-(4-methylthiazol-5-yl)benzyl)carbamoyl)pyrrolidine-1 - carboxylate* was prepared following general procedure 3. 1.2g *tert-butyl (2S,4R)-4-hydroxy-2-((2-methyl-4-(4-methylthiazol-5-yl)benzyl)carbamoyl)pyrrolidine-1-carboxylate* was obtained from 1.18 g tert-butyl (2-methyl-4-(4-methylthiazol-5-yl)benzyl)carbamate, 75% yield. ¹H NMR (400 MHz, MeOD) δ 8.87 (s, 1H), 7.49 - 7.35 (m, 1H), 7.34 - 7.17 (m, 2H), 4.53 (d, J = 14.9 Hz, 1H), 4.45 - 4.31 (m, 3H), 3.65-3.46 (m, 2H), 2.48 (s, 3H), 2.43 (s, 2H), 2.39 (s, 1H), 2.32 - 2.18 (m, 1H), 2.12 - 2.00 (m, 1H), 1.49 (s, 3H), 1.37 (s, 6H). LC-MS, ESI⁺, m/z 432 [M+H]⁺. 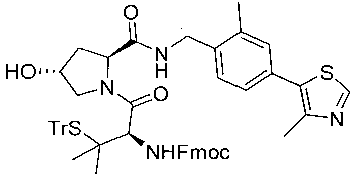 *(9H-fluoren-9-yl)methyl ((R)-1-((2R,4R)-4-hydroxy-2-((2-methyl-4-(4-methylthiazol-5-yl)benzyl)carbamoyl)pyrrolidin-1-yl)-3-methyl-1-oxo-3-(tritylthio)butan-2-yl)carbamate* was prepared following general procedure 4. 310 mg *(9H-fluoren-9-yl)methyl ((R)-1-((2R,4R)-4-hydroxy-2-((2-methyl-4-(4-methylthiazol-5-yl)benzyl)carbamoyl)pyrrolidin-1-yl)-3-methyl-1-oxo-3-(tritylthio)butan-2-yl)carbamate* was obtained from 176 mg *tert-butyl (2S,4R)-4-hydroxy-2-((2-methyl-4-(4-methylthiazol-5-yl)benzyl)carbamoyl)pyrrolidine-1-carboxylate,* 82% yield. ¹H NMR (400 MHz, CDCl₃) δ 8.60 (s, 1H), 7.70 - 7.61 (m, 2H), 7.48 (d, *J* = 7.5 Hz, 1H), 7.46 - 7.39 (m, 7H), 7.30 - 7.24 (m, 2H), 7.20 - 7.16 (m, 3H), 7.16 - 7.02 (m, 12H), 5.59 (d, J = 5.8 Hz, 1H), 4.57 (t, *J* = 7.9 Hz, 1H), 4.32-4.12 (m, 4H), 4.14 - 3.99 (m, 2H), 3.57 (d, *J* = 5.8 Hz, 1H), 3.39 (d, *J* = 11.2 Hz, 1H), 3.22 (dd, J = 11.3, 3.6 Hz, 1H), 3.06 (d, J = 4.1 Hz, 1H), 2.43 (s, 3H), 2.24 - 2.15 (m, 1H), 2.13 (s, 3H), 2.07 - 1.97 (m, 1H), 1.10 (s, 3H), 0.89 (s, 3H). ¹³C NMR (101 MHz, CDCl₃) δ 170.81, 170.49, 156.47, 150.27, 148.45, 144.50, 143.94, 143.56, 141.41, 141.36, 136.48, 136.05, 131.89, 131.13, 130.89, 129.94, 128.21, 127.93, 127.23, 127.05, 127.00, 125.21, 125.08, 120.14, 70.17, 68.63, 67.64, 58.93, 58.29, 56.71, 54.38, 47.10, 41.01, 36.78, 26.00, 25.37, 19.10, 16.24. LC-MS, ESI⁺, m/z 927 [M+H]⁺. 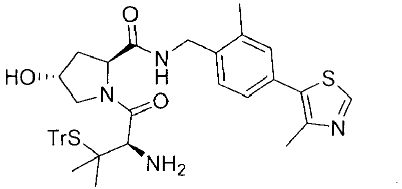 *(2R,4R)-1-((R)-2-amino-3-methyl-3-(tritylthio)butanoyl)-4-hydroxy-N-(2-methyl-4-(4-methylthiazol-5-yl)benzyl)pyrrolidine-2-carboxamide* was prepared following general procedure 5. 135 mg *(2R,4R)-1-((R)-2-amino-3-methyl-3-(tritylthio)butanoyl)-4-hydroxy-N-(2-methyl-4-(4-methylthiazol-5-yl)benzyl)pyrrolidine-2-carboxamide* was obtained from 250 mg Fmoc protected compound, 71% yield. ¹H NMR (400 MHz, CDCl₃) δ 8.69 (s, 1H), 7.63 - 7.52 (m, 6H), 7.32 - 7.15 (m, 15H), 4.75 (s, 1H), 4.61 (t, *J* = 8.1 Hz, 1H), 4.34 (s, 1H), 4.28-4.15 (m, 2H), 3.18 (dd, *J* = 11.3, 3.6 Hz, 1H), 3.12 (d, *J* = 11.4 Hz, 1H), 2.80 (s, 1H), 2.52 (s, 3H), 2.20 (s, 3H), 2.18 - 2.10 (m, 1H), 2.10 - 2.03 (m, 1H), 1.21 (s, 3H), 1.04 (s, 3H). ¹³C NMR (101 MHz, CDCl₃) δ 172.24, 171.48, 150.24, 148.46, 144.90, 136.41, 136.12, 131.85, 131.11, 130.86, 130.01, 127.98, 127.94, 127.04, 126.91, 69.94, 68.28, 59.21, 58.31, 58.13, 57.03, 40.97, 37.46, 25.23, 24.70, 19.04, 16.23. LC-MS, ESI⁺, m/z 727 [M+Na]⁺. 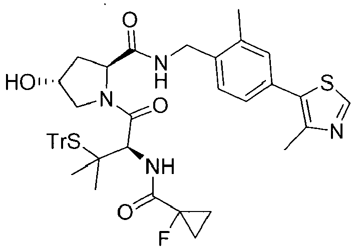 *(2R,4R)-1-((R)-2-(1-fluorocyclopropane-1-carboxamido)-3-methyl-3-(tritylthio)butanoyl)-4-hydroxy-N-(2-methyl-4-(4-methylthiazol-5-yl)benzyl)pyrrolidine-2-carboxamide* was prepared following general procedure 6. 79.5 mg *(2R,4R)-1-((R)-2-(1-fluorocyclopropane-1-carboxamido)-3-methyl-3-(tritylthio)butanoyl)-4-hydroxy-N-(2-methyl-4-(4-methylthiazol-5-yl)benzyl)pyrrolidine-2-carboxamide* was obtained from 102 mg amine compound, 70% yield. ¹H NMR (500 MHz, CDCl₃) δ 8.69 (s, 1H), 7.56 - 7.49 (m, 6H), 7.25 - 7.13 (m, 15H), 4.64 (t, J = 8.1 Hz, 1H), 4.37 (s, 1H), 4.29 (dd, *J* = 15.2, 5.7 Hz, 1H), 4.20 (dd, *J* = 15.2, 5.9 Hz, 1H), 3.67 (d, *J* = 5.3 Hz, 1H), 3.50 (d, *J* = 11.5 Hz, 1H), 3.27 (dd, J = 11.4, 3.7 Hz, 2H), 2.52 (s, 3H), 2.36 - 2.29 (m, 1H), 2.24 (s, 3H), 2.14 - 2.07 (m, 1H), 1.38 - 1.30 (m, 2H), 1.28 - 1.23 (m, 2H), 1.20 (s, 3H), 1.01 (s, 3H); ¹³C NMR (126 MHz, CDCl₃) δ 170.67, 170.35 (d, J = 21.0 Hz), 169.92, 150.29, 148.53, 144.53, 136.60, 136.03, 131.88, 131.23, 131.04, 129.90, 128.47, 127.98, 127.12, 127.03, 78.28 (d, J = 231.89 Hz), 70.25, 68.54, 58.75, 57.09, 56.77, 53.91, 41.12, 36.60, 26.00, 25.66, 19.15, 16.26, 13.88 (d, *J* = 13.4 Hz), 13.88 (d, *J* = 13.4 Hz); ¹⁹F NMR (471 MHz, CDCl₃) δ -197.34; LC-MS, ESI⁻, 789 [M-H]⁻. 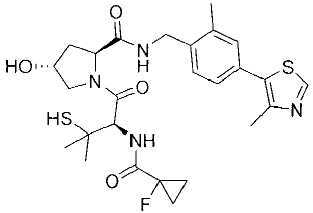 *(2R,4R)-1-((R)-2-(1-fluorocyclopropane-1-carboxamido)-3-mercapto-3-methylbutanoyl)-4-hydroxy-N-(2-methyl-4-(4-methylthiazol-5-yl)benzyl)pyrrolidine-2-carboxamide* was prepared following general procedure 7. 40 mg *(2R,4R)-1-((R)-2-(1-fluorocyclopropane-1-carboxamido)-3-mercapto-3-methylbutanoyl)-4-hydroxy-N-(2-methyl-4-(4-methylthiazol-5-yl)benzyl)pyrrolidine-2-carboxamide* was obtained from 79.5 mg trityl protected compound, 73% yield. ¹H NMR (500 MHz, CDCl₃) δ 8.69 (s, 1H), 7.42 (d, *J* = 6.1 Hz, 1H), 7.36-7.30 (m, 1H), 7.24 - 7.19 (m, 2H), 7.14 (t, *J* = 4.8 Hz, 1H), 4.70-4.64 (m, 2H), 4.52 (s, 1H), 4.47 (dd, J = 15.0, 5.9 Hz, 1H), 4.39 (dd, J = 15.0, 5.4 Hz, 1H), 4.06 (d, J = 11.0 Hz, 1H), 3.77 (dd, *J* = 11.1, 3.6 Hz, 1H), 3.45 (s, 1H), 2.52 (s, 1H), 2.50 (s, 3H), 2.39 (ddd, J = 12.9, 7.9, 4.6 Hz, 1H), 2.34 (s, 3H), 2.19 - 2.11 (m, 1H), 1.40 - 1.26 (m, 10H); ¹³C NMR (126 MHz, CDCl₃) δ 170.72, 170.57, 170.14, 150.56, 148.29, 136.89, 135.90, 131.94, 131.43, 131.20, 128.82, 127.20, 78.21 (d, J = 232.6 Hz), 70.20, 59.23, 57.77, 56.75, 46.40, 41.50, 36.93, 30.50, 28.62, 19.21, 16.07, 14.02 (d, *J* = 10.8 Hz), 14.00 (d, J = 10.4 Hz); ¹⁹F NMR (471 MHz, CDCl₃) δ -197.64; LC-MS, ESI⁺, 549.3 [M+H]⁺. 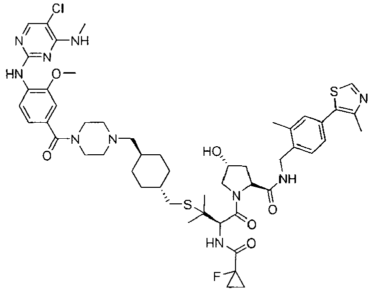 *(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(2-methyl-4-(4-methylthiazol-5-yl)benzyl)pyrrolidine-2-carboxamide* **(35, XL02048)**

**XL02048** was prepared following general procedure 8. 12.6 mg **XL02048** was obtained from 10 mg thiol compound, 69% yield. ¹H NMR (400 MHz, CDCl₃) δ 8.65 (s, 1H), 8.53 (d, *J* = 8.8 Hz, 1H), 7.91 (s, 1H), 7.62 (s, 1H), 7.30 (d, J = 8.4 Hz, 1H), 7.24 -7.16 (m, 4H), 7.02 - 6.97 (m, 2H), 5.31 (q, *J* = 4.4 Hz, 1H), 4.77 (t, *J* = 7.9 Hz, 1H), 4.72 (d, J = 7.8 Hz, 1H), 4.51 (s, 1H), 4.43 (tt, *J* = 15.0, 7.4 Hz, 2H), 4.04 (d, J = 11.3 Hz, 1H), 3.92 (s, 3H), 3.76 - 3.52 (m, 5H), 3.10 (d, J = 4.9 Hz, 3H), 2.51 (s, 3H), 2.48 - 2.32 (m, 10H), 2.23 (dd, J = 12.2, 10.2 Hz, 1H), 2.11 (d, J = 7.0 Hz, 2H), 1.79 (d, *J* = 9.9 Hz, 4H), 1.45 - 1.25 (m, 12H), 0.98-0.75 (m, 4H). ¹³C NMR (101 MHz, CDCl₃) δ 170.70, 170.54, 170.47, 170.11, 158.71, 157.95, 152.79, 150.29, 148.55, 147.55, 136.86, 135.88, 131.79, 131.44, 131.36, 131.27, 128.72, 128.10, 127.13, 120.29, 116.91, 109.72, 105.65, 79.41, 70.22, 65.38, 59.11, 56.71, 56.28, 56.01, 53.91, 47.70, 41.39, 38.54, 36.94, 35.41, 35.07, 32.79, 32.74, 31.47, 28.21, 25.68, 25.38, 19.26, 16.27, 14.04 (d, J = 10.1Hz), 13.94 (d, J = 9.9 Hz); ¹⁹F NMR (471 MHz, CDCl₃) δ -197.70; HRMS (ESI⁺) m/z, calcd for C₅₁H₆₆ClFN₁₀O₆S₂: 1033.4353 [M + H]⁺, found 1033.4349.

### Exemplary synthesis of Exemplary compound 36 (XL02049)

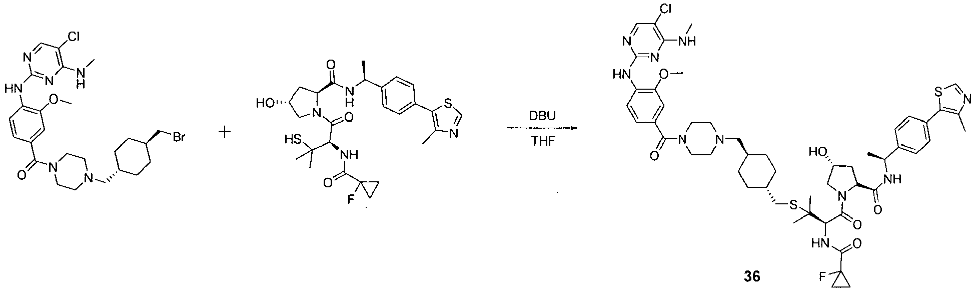

*(2R,4R)-1-((R)-3-((((1R,4R)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-((S)-1-(4-(4-methylthiazol-5-yl)phenyl)ethyl)pyrrolidine-2-carboxamide* (36, **XL02049)**

**XL02049** was prepared following general procedure 8. 8.95 mg **XL02049** was obtained from 10 mg thiol compound, 49% yield. ¹H NMR (400 MHz, CDCl₃) δ 8.66 (s, 1H), 8.53 (d, J = 8.8 Hz, 1H), 7.92 (s, 1H), 7.63 (s, 1H), 7.43 - 7.34 (m, 5H), 7.31 - 7.27 (m, 1H), 7.03 - 6.97 (m, 2H), 5.30 (q, J = 4.6 Hz, 1H), 5.10 (p, J = 6.9 Hz, 1H), 4.82 - 4.71 (m, 2H), 4.51 (s, 1H), 4.07 (d, J = 11.3 Hz, 1H), 3.92 (s, 3H), 3.78 - 3.52 (m, 5H), 3.10 (d, *J* = 4.9 Hz, 3H), 2.79 (s, 1H), 2.53 (s, 3H), 2.51 - 2.30 (m, 7H), 2.20 - 2.10 (m, 3H), 1.92 -1.78 (m, 4H), 1.49 (d, *J* = 6.9 Hz, 3H), 1.42 (d, *J* = 4.8 Hz, 6H), 1.39 - 1.23 (m, 6H), 1.03 - 0.83 (m, 4H); ¹³C NMR (101 MHz, CDCl₃) δ 170.70 (d, J = 20.9 Hz), 170.55, 170.35, 169.74, 158.73, 157.97, 152.81, 150.37, 148.68, 147.56, 143.17, 131.77, 131.45, 131.04, 129.68, 128.13, 126.65, 120.30, 116.92, 109.74, 105.68, 79.44, 70.25, 65.43, 58.98, 56.66, 56.47, 56.02, 53.97, 48.88, 47.62, 38.61, 36.41, 35.50, 35.12, 32.84, 31.53, 28.22, 25.82, 25.74, 22.33, 16.24, 14.11 (d, J = 10.3 Hz), 13.96 (d, J = 10.2 Hz). ¹⁹F NMR (471 MHz, CDCl₃) δ -197.73. HRMS (ESI⁺) m/z, calcd for C₅₁H₆₆ClFN₁₀O₆S₂: 1033.4353 [M + H]⁺, found 1033.4120.

### Exemplary synthesis of Exemplary compound 37 (XL02058)

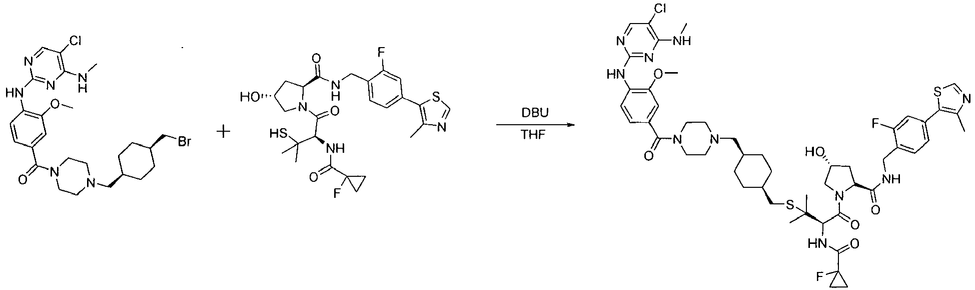

*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-N-(2-fluoro-4-(4-methylthiazol-5-yl)benzyl)-4-hydroxypyrrolidine-2-carboxamide (37, **XL02058)***

**XL02058** was prepared following general procedure 8. 16.99 mg **XL02058** was obtained from 10 mg thiol compound, 71% yield. ¹H NMR (500 MHz, CDCl₃) δ 8.68 (s, 1H), 8.53 (d, J = 8.7 Hz, 1H), 7.92 (s, 1H), 7.62 (s, 1H), 7.42 (t, J = 7.9 Hz, 1H), 7.37 (t, J = 5.8 Hz, 1H), 7.24 (dd, J = 7.7, 3.2 Hz, 1H), 7.16 (d, J = 7.9 Hz, 1H), 7.10 (d, J = 10.6 Hz, 1H), 7.02 - 6.98 (m, 2H), 5.30 (q, *J* = 4.1 Hz, 1H), 4.87 - 4.66 (m, 2H), 4.57 - 4.46 (m, 3H), 4.04 (d, *J* = 11.2 Hz, 1H), 3.92 (s, 3H), 3.77 - 3.49 (m, 5H), 3.10 (d, *J* = 4.9 Hz, 3H), 2.94 (s, 1H), 2.52 (s, 3H), 2.50 - 2.31 (m, 7H), 2.25 - 2.17 (m, 2H), 1.85 - 1.65 (m, 3H), 1.56 - 1.45 (m, 4H), 1.38 - 1.28 (m, 13H), 0.97 -0.78 (m, 1H); ¹³C NMR (126 MHz, CDCl₃) δ 170.97, 170.59 (d, *J* = 20.7 Hz), 170.55, 170.14, 160.69 (d, *J* = 248.1 Hz), 158.72, 157.96, 152.80, 150.79, 149.22, 147.56, 133.6 (d, J = 8.7 Hz), 131.45, 130.55, 130.44 (d, J = 4.5 Hz), 128.11, 125.34 (d, *J* = 2.9 Hz), 125.04 (d, *J* = 15.1 Hz), 120.30, 116.92, 116.13 (d, *J* = 22.8 Hz), 109.73, 105.67, 79.20, 70.24, 62.32, 59.03, 56.67, 56.36, 56.02, 53.86, 47.75, 37.16 (d, *J* = 3.7 Hz), 36.80, 35.96, 33.00, 32.09, 28.68, 28.47, 28.21, 27.13 (d, J = 3.7 Hz), 25.69, 25.47, 16.29, 14.05 (d, J = 11.1 Hz), 13.95 (d, J = 10.6 Hz). ¹⁹F NMR (471 MHz, CDCl₃) δ -117.88, -197.71. HRMS (ESI⁺) *m*/*z,* calcd for C₅₀H₆₃ClF₂N₁₀O₆S₂: 1037.4102 [M + H]⁺, found 1037.3870.

### Exemplary synthesis of Exemplary compound 38 (XL02059)

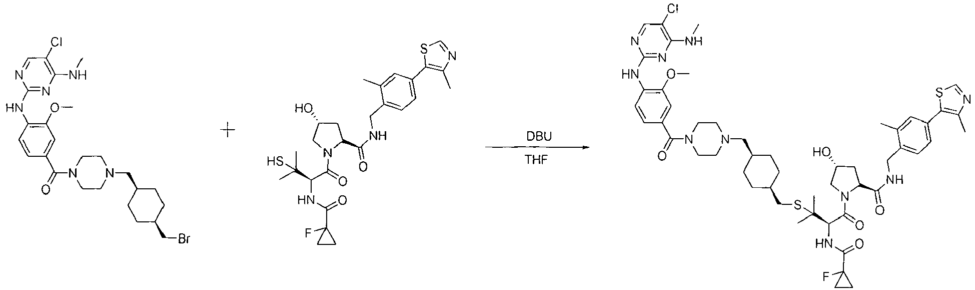

*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-(2-methyl-4-(4-methylthiazol-5-yl)benzyl)pyrrolidine-2-carboxamide* **(38, *XL02059)***

**XL02059** was prepared following general procedure 8. 14.29 mg **XL02059** was obtained from 10 mg thiol compound, 59% yield. ¹H NMR (400 MHz, CDCl₃) δ 8.66 (s, 1H), 8.53 (d, *J* = 8.8 Hz, 1H), 7.92 (s, 1H), 7.62 (s, 1H), 7.30 (d, *J* = 8.4 Hz, 1H), 7.25 - 7.16 (m, 4H), 7.02 - 6.98 (m, 2H), 5.31 (q, *J* = 4.5 Hz, 1H), 4.84 - 4.70 (m, 2H), 4.56 - 4.36 (m, 3H), 4.03 (d, *J* = 11.3 Hz, 1H), 3.92 (s, 3H), 3.75 - 3.52 (m, 5H), 3.10 (d, *J* = 4.9 Hz, 3H), 3.00 (s, 1H), 2.51 (s, 3H), 2.48 - 2.36 (m, 6H), 2.35 (s, 3H), 2.28 - 2.16 (m, 3H), 1.90 - 1.75 (m, 2H), 1.68 (s, 1H), 1.55 - 1.44 (m, 4H), 1.34 - 1.27 (m, 13H), 0.95 - 0.75 (m, 1H); ¹³C NMR (126 MHz, CDCl₃) δ 170.71, 170.56 (d, *J* = 20.8 Hz), 170.55, 170.08, 158.71, 157.95, 152.78, 150.31, 148.54, 147.55, 136.85, 135.89, 131.79, 131.45, 131.36, 131.26, 128.70, 128.09, 127.13, 120.29, 116.91, 109.72, 105.66, 78.26 (d, *J* = 231.8 Hz,), 70.21, 62.33, 59.13, 56.70, 56.32, 56.01, 53.86, 47.73, 41.37, 36.96, 35.93, 32.98, 32.09, 28.68, 28.47, 28.21, 27.12, 25.67, 25.45, 19.26, 16.24, 14.03 (d, *J* = 11.0 Hz), 13.95 (d, *J* = 10.8 Hz). ¹⁹F NMR (471 MHz, CDCl₃) δ -197.69. HRMS (ESI⁺) *m*/*z,* calcd for C₅₁H₆₆ClFN₁₀O₆S₂: 1033.4353 [M + H]⁺, found 1033.4125.

### Exemplary synthesis of Exemplary compound 39 (XL02060)

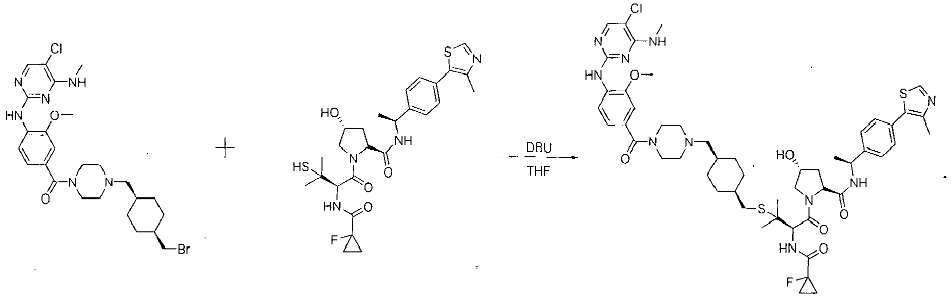

*(2R,4R)-1-((R)-3-((((1s,4S)-4-((4-(4-((5-chloro-4-(methylamino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutanoyl)-4-hydroxy-N-((S)-1-(4-(4-methylthiazol-5-yl)phenyl)ethyl)pyrrolidine-2-carboxamide **(39, XL02060)***

**XL02060** was prepared following general procedure 8. 17.22 mg **XL02060** was obtained from 10 mg thiol compound, 71% yield. ¹H NMR (400 MHz, CDCl₃) δ 8.66 (s, 1H), 8.53 (d, *J* = 8.8 Hz, 1H), 7.92 (s, 1H), 7.62 (s, 1H), 7.44 - 7.34 (m, 5H), 7.29 (dd, *J* = 7.6, 3.2 Hz, 1H), 7.02 - 6.97 (m, 2H), 5.30 (q, *J* = 4.7 Hz, 1H), 5.10 (p, *J* = 7.0 Hz, 1H), 4.80 - 4.70 (m, 2H), 4.50 (s, 1H), 4.09 - 4.00 (m, 1H), 3.92 (s, 3H), 3.78 - 3.49 (m, 5H), 3.10 (d, *J* = 4.9 Hz, 3H), 2.95 (s, 1H), 2.56 - 2.49 (m, 5H), 2.47 - 2.33 (m, 5H), 2.23 (d, *J* = 7.4 Hz, 2H), 2.20 - 2.11 (m, 1H), 1.90 - 1.70 (m, 2H), 1.65 - 1.50 (m, 4H), 1.48 (d, *J* = 6.9 Hz, 3H), 1.45 - 1.30 (m, 13H), 1.03 - 0.79 (m, 1H); ¹³C NMR (101 MHz, CDCl₃) δ 170.74, 170.55, 170.27, 169.79, 158.72, 157.96, 152.80, 150.36, 148.66, 147.55, 143.20, 131.77, 131.45, 131.01, 129.67, 128.11, 126.64, 120.30, 116.91, 109.73, 105.67, 79.43, 70.22, 62.37, 59.04, 56.66, 56.49, 56.01, 53.88, 48.86, 47.70, 36.51, 35.99, 33.05, 32.14, 28.70, 28.56, 28.21, 27.16, 25.81, 22.35, 16.23, 14.06 (d, *J* = 10.8 Hz), 13.96 (d, *J* = 10.7 Hz); ¹⁹F NMR (471 MHz, CDCl₃) δ -197.68; HRMS (ESI⁺) *m*/*z,* calcd for C₅₁H₆₆ClFN₁₀O₆S₂: 1033.4353 [M + H]⁺, found 1033.4323.

### Exemplary synthesis of Exemplary compound 40 (XL02063)

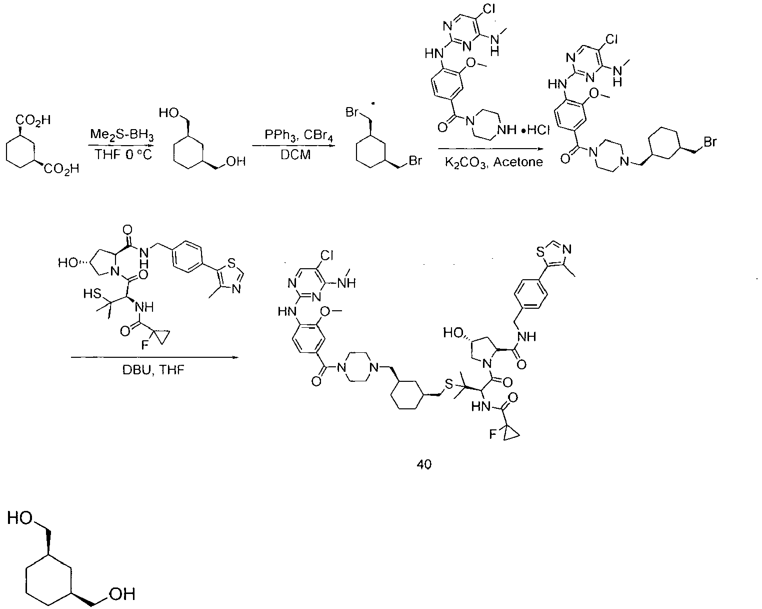

*(cis-cyclohexone-1,3-diyl)dimethanol*

To a solution of Cis-cyclohexane-1,3-dicarboxylic acid (500 mg, 2.9 mmol) in THF (10 mL) was added Me₂S-BH₃ (3.2 ml, 6.38 mmol) dropwise at 0 °C. After stirring at 0 °C for 2-3h, the reaction was quenched with methanol slowly and then added with water. The mixture was extracted with DCM (3x), the combined organic phase was washed with water and brine, dried over sodium sulfate, filtered, condensed to afford a residue which was purified through flash column chromatography on silica gel (0%-10% methanol in DCM) to give *(cis-cyclohexane-1,3-diyl)dimethanol* (250 mg, 60% yield). ¹H NMR (400 MHz, CDCl₃) δ 3.54 - 3.37 (m, 4H), 2.04 (s, 2H), 1.93 - 1.69 (m, 4H), 1.62 - 1.42 (m, 2H), 1.30 (qt, *J* = 12.2, 3.2 Hz, 1H), 0.87 (qd, *J* = 12.6, 3.4 Hz, 2H), 0.63 (q, *J* = 12.1 Hz, 1H). ¹³C NMR (101 MHz, CDCl₃) δ 77.48, 77.16, 76.84, 68.74, 40.20, 32.70, 29.59, 25.39. 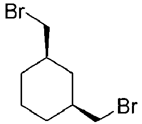 *cis-1,3-bis(bromomethyl)cyclohexane*

CBr₄ (690 mg, 2.08 mmol) was dissolved in DCM (5 mL) and added dropwise to a solution of (cis-cyclohexane-1,3-diyl)dimethanol (100 mg, 0.69 mmol) and triphenyl phosphine (636 mg, 2.43 mmol) in DCM (6 mL) at 0 °C. The resulting mixture was slowly warmed to room temperature and stirred at room temperature overnight. Water was added and the mixture was extracted with DCM (3x). The combined organic phase was washed with water and brine, dried over sodium sulfate, filtered, condensed to give a residue which was purified through flash column chromatography on silica gel (0%-50% ethyl acetate in heptane) to afford *cis-1,3-bis(bromomethyl)cyclohexane* (140 mg, 75% yield). ¹H NMR (400 MHz, CDCl₃) δ 3.37 - 3.22 (m, 4H), 2.11 - 1.97 (m, 1H), 1.93 - 1.79 (m, 3H), 1.76 - 1.61 (m, 2H), 1.41 - 1.22 (m, 1H), 1.04 - 0.88 (m, 2H), 0.79 (dd, *J* = 24.3, 12.0 Hz, 1H). ¹³C NMR (101 MHz, CDCl₃) δ 40.13, 39.79, 36.76, 31.38, 25.36. 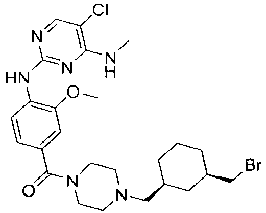 *(4-(((1S,3R)-3-(bromomethyl)cyclohexyl)methyl)piperazin-1-yl)(4-((5-chloro-4-(methylamino)pyrimidin-2-yl)amino)-3-methoxyphenyl)methanone*

To a solution of (4-((5-chloro-4-(methylamino)pyrimidin-2-yl)amino)-3-methoxyphenyl)(piperazin-1-yl)methanone (25 mg, 0.06 mmol) and cis-1,3-bis(bromomethyl)cyclohexane (50 mg, 0.185 mmol) in acetone was added K₂CO₃ (42 mg, 0.3mmol). After stirring at 50 °C overnight, the mixture was cooled down to room temperature and diluted with water. The mixture was then extracted with DCM (3x) and the combined organic phase was washed with water and brine, dried over sodium sulfate, filtered, and condensed to afford a residue which was purified through flash column chromatography on silica gel (0%-10% methanol in DCM) to give *(4-(((1S,3R)-3-(bromomethyl)cyclohexyl)methyl)piperazin-1-yl)(4-((5-chloro-4-(methylamino)pyrimidin-2-yl)amino)-3-methoxyphenyl)methanone* (6 mg, 17% yield). ¹H NMR (500 MHz, CDCl₃) δ 8.54 (d, *J* = 8.8 Hz, 1H), 7.92 (s, 1H), 7.63 (s, 1H), 7.01 (d, *J* = 6.9 Hz, 2H), 5.32 - 5.24 (m, 1H), 3.93 (s, 3H), 3.86 - 3.54 (m, 6H), 3.30 (d, *J* = 6.1 Hz, 2H), 3.11 (d, *J* = 4.9 Hz, 3H), 2.40 (s, 4H), 2.18 (d, *J* = 7.0 Hz, 2H), 2.02 - 1.86 (m, 2H), 1.84 - 1.77 (m, 2H), 1.71 - 1.61 (m, 1H), 1.31 - 1.27 (m, 1H), 1.00 - 0.75 (m, 2H); ¹³C NMR (126 MHz, CDCl₃) δ 170.39, 158.58, 157.84, 152.68, 147.41, 131.28, 128.02, 120.16, 116.77, 109.59, 105.50, 65.35, 55.86, 53.78, 40.58, 39.89, 36.81, 34.88, 32.54, 31.84, 31.37, 28.07, 25.49. LC-MS, ESI⁺, m/z 565 [M+H]⁺, 567 [M+H+2]⁺. 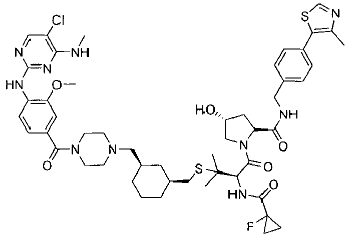 *(2R,4R)-1-((R)-3-((((1S,3R)-3-((4-(4-((5-chloro-4-(methylamino)pyrimidin-2-yl)omino)-3-methoxybenzoyl)piperazin-1-yl)methyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide* **(40, XL02063)**

**XL02063** was prepared following general procedure 8. 5.56 mg **XL02063** was obtained from 5.5 mg thiol compound, 53% yield. ¹H NMR (500 MHz, CDCl₃) δ 8.67 (s, 1H), 8.54 (d, *J* = 8.1 Hz, 1H), 7.92 (s, 1H), 7.63 (s, 1H), 7.40 - 7.31 (m, 5H), 7.24 - 7.19 (m, 1H), 7.02 - 6.98 (m, 2H), 5.39 - 5.23 (m, 1H), 4.79 (t, *J* = 7.7 Hz, 1H), 4.74 (dd, *J* = 7.5, 4.9 Hz, 1H), 4.53 (s, 1H), 4.46 (d, *J* = 5.7 Hz, 2H), 4.06 - 3.99 (m, 1H), 3.92 (s, 3H), 3.76 (dt, *J* = 11.0, 3.7 Hz, 1H), 3.65 (d, *J* = 20.0 Hz, 4H), 3.11 (d, *J* = 4.9 Hz, 3H), 2.56 - 2.48 (m, 4H), 2.46 - 2.31 (m, 6H), 2.21 (t, *J* = 10.9 Hz, 1H), 2.13 (d, *J* = 7.0 Hz, 2H), 1.89 (d, *J* = 11.6 Hz, 1H), 1.84 - 1.71 (m, 3H), 1.51 - 1.41 (m, 2H), 1.41 - 1.28 (m, 11H), 1.22 - 1.13 (m, 1H), 0.90 - 0.82 (m, 1H), 0.80 - 0.69 (m, 1H), 0.57 (q, *J* = 11.7 Hz, 1H); ¹³C NMR (126 MHz, CDCl₃) δ 170.67, 170.57, 170.54, 170.22, 158.74, 157.99, 152.84, 150.39, 148.72, 147.58, 138.18, 131.70, 131.48, 131.23, 129.70, 128.23, 128.11, 120.33, 116.93, 109.76, 105.69, 79.23, 70.20 (d, *J* = 5.5 Hz), 65.60 (d, *J* = 5.8 Hz), 58.95, 56.50 (d, *J* = 4.9 Hz), 56.30, 56.03, 53.94, 47.65, 47.58, 43.30, 38.38 (d, *J* = 13.2 Hz), 38.03, 36.62, 35.63 (d, *J* = 11.9 Hz), 35.15 (d, *J* = 4.8 Hz), 33.23 (d, *J* = 12.4 Hz), 31.59, 28.23, 25.85, 25.77 (d, *J* = 4.0 Hz), 25.48, 16.26, 14.11 (d, *J* = 10.4 Hz), 13.95 (d, *J* = 10.2 Hz). ¹⁹F NMR (471 MHz, CDCl₃) δ -197.77, -197.79. HRMS (ESI⁺) *m*/*z,* calcd for C₅₀H₆₄ClFN₁₀O₆S₂: 1019.4197 [M + H]⁺, found 1019.3952.

### Exemplary synthesis of Exemplary compound 41 (XL02067B)

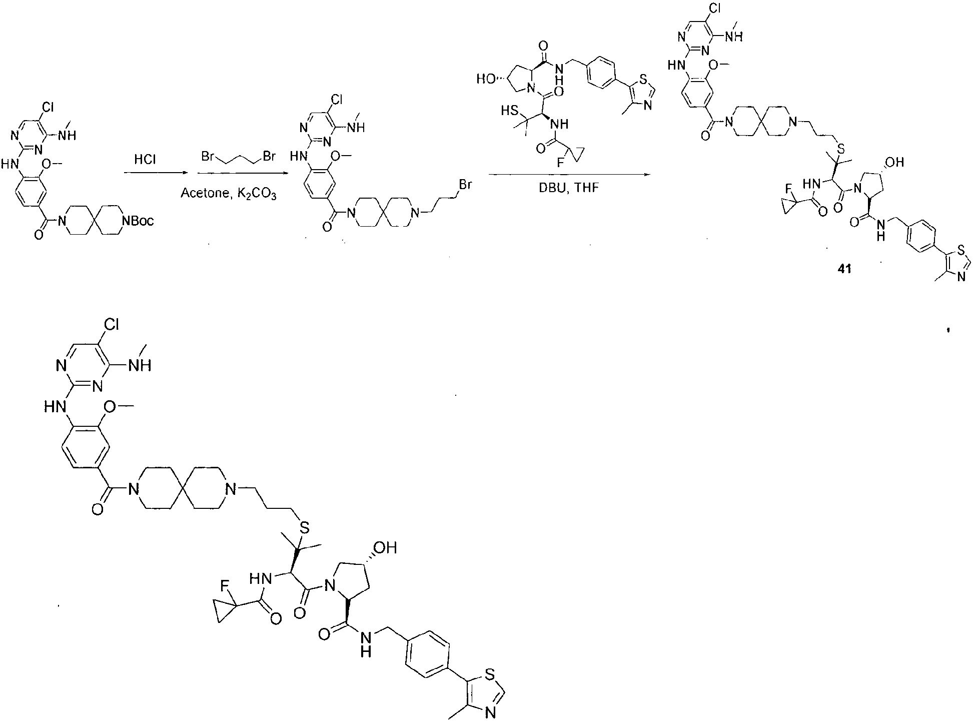

*(2R,4R)-1-((R)-3-((3-(9-(4-((5-chloro-4-(methylamino)pyrimidin-2-yl)omino)-3-methoxybenzoyl)-3,9-diozospiro[5.5]undecon-3-yl)propyl)thio)-2-(1-fluorocyclopropone-1-corboxamido)-3-methylbutonoyl)-4-hydroxy-N-(4-(4-methylthiozol-5-yl)benzyl)pyrrolidine-2-corboxamide (41, **XL02067B)***

To a solution of *tert*-butyl 9-(4-((5-chloro-4-(methylamino)pyrimidin-2-yl)amino)-3-methoxybenzoyl)-3,9-diazaspiro[5.5]undecane-3-carboxylate (200 mg, 0.367mmol) in DCM (1 mL) was added HCl (4N in 1,4-dioxane, 1 mL). The mixture was stirred at room temperature overnight, then condensed to afford a white solid (180 mg, 91% yield). The solid (60 mg, 0.125 mmol) was suspended in 2 mL acetone, followed by addition of 1,3-dibromoprane (75.5 mg, 0.374 mmol) and K₂CO₃ (86 mg, 0.623 mmol). After stirring at room temperature overnight, the mixture was added with water, extracted with DCM (3x), the combined organic phase was washed with water and brine, dried over sodium sulfate, filtered, and condensed to afford a residue which was purified through flash chromatography on silica gel (0%-10% methanol in DCM) to give the bromide compound as impure product which was used to next step without further purification. (9-(3-bromopropyl)-3,9-diazaspiro[5.5]undecan-3-yl)(4-((5-chloro-4-(methylamino)pyrimidin-2-yl)amino)-3-methoxyphenyl)methanone (10 mg, 0.018 mmol) and (2*S*,4*R*)-1-((R)-2-(1-fluorocyclopropane-1-carboxamido)-3-mercapto-3-methylbutanoyl)-4-hydroxy-N-(4-(4-methylthiazol-5-yl)benzyl)pyrrolidine-2-carboxamide (10 mg, 0.019 mmol) were dissolved in THF (1.5), followed by addition of DBU (16 µL, 0.112 mmol). After stirring at room temperature overnight, the mixture was condensed and purified through flash column chromatography on silica gel (0-10% methanol in DCM) to give **XL02067B** as white solid (13.6 mg, 71% yield). ¹H NMR (400 MHz, CDCl₃) δ 8.67 (s, 1H), 8.53 (d, *J* = 8.7 Hz, 1H), 7.92 (s, 1H), 7.62 (s, 1H), 7.42 - 7.28 (m, 5H), 7,22 (d, *J* = 4.5 Hz, 1H), 7.02 - 6.96 (m, 2H), 5.29 (q, *J* = 4.6 Hz, 1H), 4.82 - 4.70 (m, 2H), 4.61 - 4.39 (m, 3H), 4.06 (d, *J* = 11.3 Hz, 1H), 3.92 (s, 3H), 3.74 (d, *J* = 7.9 Hz, 1H), 3.57 (s, 4H), 3.11 (d, *J* = 4.8 Hz, 3H), 2.85- 2.55 (m, 3H), 2.55 - 2.30 (m, 3H), 2.51 - 2.30 (m, 6H), 2.27 - 2.16 (m, 1H), 1.65 (s, 4H), 1.49 (s, 4H), 1.38 - 1.23 (m, 12H). ¹³C NMR (101 MHz, CDCl₃) δ 170.72, 170.55 (d, *J* = 21.8 Hz), 170.09, 158.73, 157.99, 152.83, 150.39, 148.71, 147.57, 138.19, 131.70, 131.40, 131.21, 129.67, 128.40, 128.26, 120.09, 116.95, 109.62, 105.66, 79.45, 70.24, 59.07, 57.63, 56.69, 56.26, 56.03, 49.20, 48.13, 43.30, 36.65, 35.40, 30.16, 28.23, 26.35, 25.74, 25.49, 16.28, 14.09 (d, J = 11.0 Hz), 13.97 (d, J = 11.6 Hz). HRMS (ESI⁺) *m*/*z,* calcd for C₅₀H₆₄ClFN₁₀O₆S₂: 1019.4197 [M + H]⁺, found 1019.4191.

### Exemplary synthesis of Exemplary compound 42 (XL02068)

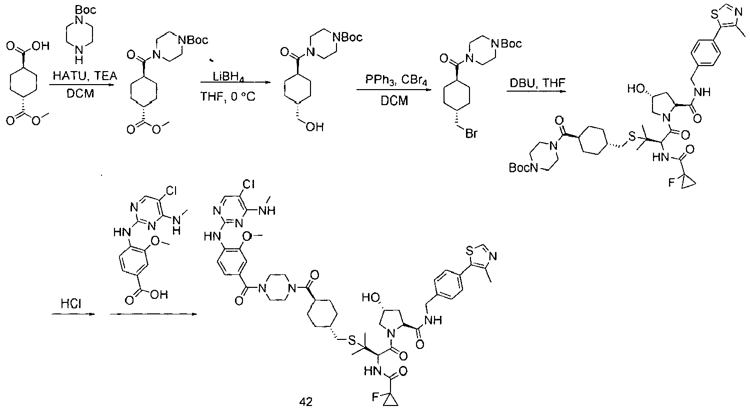

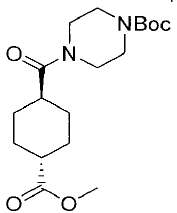

*tert-butyl 4-(trans-4-(methoxycorbonyl)cyclohexone-1-carbonyl)piperazine-1-carboxylote*

To a solution of 1-Boc-piperazine (1 g, 5.38 mmol), trans-4-(methoxycarbonyl)cyclohexane-1-carboxylic acid (1g, 5.38 mmol), and TEA (1.5 mL, 10.8 mmol) in DCM (25 mL) was added HATU (2.145 g, 5.65 mmol). After stirring at room temperature overnight, the mixture was diluted with DCM (25 mL), washed with water and brine, dried over sodium sulfate, filtered, and condensed to afford a residue which was purified through flash column chromatography on silica gel (0%-100% ethyl acetate in heptane) to give *tert-butyl 4-(trans-4-(methoxycarbonyl)cyclohexane-1-carbonyl)piperazine-1-carboxylate* (1.2 g, 63% yield). ¹H NMR (400 MHz, CDCl₃) δ 3.66 (s, 3H), 3.57 (s, 2H), 3.50 - 3.35 (m, 6H), 2.45 (tt, *J* = 11.6, 3.4 Hz, 1H), 2.33 (tt, *J* = 12.0, 3.6 Hz, 1H), 2.14 - 2.00 (m, 2H), 1.80 (dd, *J* = 14.0, 3.1 Hz, 2H), 1.70 - 1.54 (m, 2H), 1.51 - 1.37 (m, 11H); ¹³C NMR (101 MHz, CDCl₃) δ 176.08, 174.15, 154.70, 80.44, 51.72, 45.40, 43.90, 42.60, 41.63, 39.78, 28.52, 28.36; LC-MS, ESI⁺, m/z 299 [M-56+H]⁺. 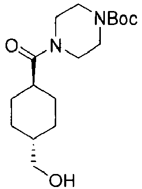 *tert-butyl 4-(trans-4-(hydroxymethyl)cyclohexone-1-carbonyl)piperazine-1-carboxylate*

To a solution of *tert*-butyl 4-(trans-4-(methoxycarbonyl)cyclohexane-1-carbonyl)piperazine-1-carboxylate (200 mg, 0.564 mmol) in THF (5 mL) was added LiBH₄ (0.846 mL, 1.69 mmol) dropwise at 0 °C. The reaction mixture was stirred at 0 °C for 2-3h then quenched with methanol and added with water. The mixture was extracted with DCM (3x) and the combined organic phase was washed with water and brine, dried over sodium sulfate, filtered, and condensed to give a residue which was purified through flash column chromatography on silica gel (0%-10% methanol in DCM) to yield *tert-butyl 4-(trans-4-(hydroxymethyl)cyclohexone-1-carbonyl)piperazine-1-carboxylate* (180 mg, 98% yield). ¹H NMR (400 MHz, CDCl₃) δ 3.69 - 3.26 (m, 10H), 2.41 (tt, *J* = 11.8, 3.4 Hz, 1H), 1.95 - 1.83 (m, 2H), 1.81 - 1.72 (m, 2H), 1.66 - 1.48 (m, 4H), 1.45 (s, 9H), 1.00 (qd, *J* = 13.1, 3.4 Hz, 2H) ;¹³C NMR (101 MHz, CDCl₃) δ 174.68, 154.68, 80.37, 68.28, 45.31, 43.93, 41.54, 40.59, 39.88, 28.85, 28.82, 28.46; LC-MS, 349, [M+Na]⁺. 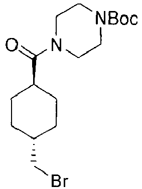 *tert-butyl 4-((1R,4R)-4-(bromomethyl)cyclohexane-1-carbonyl)piperazine-1-carboxylate*

CBr₄ (152 mg, 0.46 mmol) was dissolved in DCM (5 mL) and added dropwise to a solution of *tert*-butyl 4-(trans-4-(hydroxymethyl)cyclohexane-1-carbonyl)piperazine-1-carboxylate (100 mg, 0.306 mmol) and triphenyl phosphine (161 mg, 0.61 mmol) in DCM (6 mL) at 0 °C. After stirring at 0 °C for 2-3h, the reaction was quenched with water, extracted with DCM (3x), and the combined organic phase was washed with water and brine, dried over sodium sulfate, filtered, and condensed to give the crude product which was purified through flash column chromatography on silica gel (0%-100% ethyl acetate in heptane) to give *tert-butyl 4-((1R,4R)-4-(bromomethyl)cyclohexane-2-carbonyl)piperazine-1-carboxylate* (93 mg, 78% yield). ¹H NMR (400 MHz, CDCl₃) δ 3.65 - 3.35 (m, 8H), 3.31 (d, *J* = 6.0 Hz, 2H), 2.42 (tt, *J* = 11.8, 3.4 Hz, 1H), 1.96 (dd, *J* = 9.6, 4.1 Hz, 2H), 1.84 - 1.75 (m, 2H), 1.74 - 1.53 (m, 3H), 1.47 (s, 9H), 1.11 (qd, *J* = 13.2, 3.5 Hz, 2H); ¹³C NMR (101 MHz, CDCl₃) δ 174.30, 154.73, 80.45, 45.41, 43.96, 41.66, 40.30, 40.25, 39.23, 30.95, 28.89, 28.54. LC-MS, ESI⁺, m/z 333 [M+H-56], 335 [M+H+2-56]. 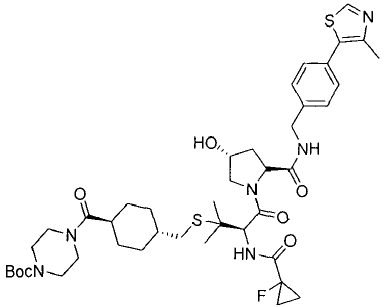 *tert-butyl 4-((1R,4R)-4-((((R)-3-(1-fluorocyclopropane-1-carboxamido}-4-((2R,4R)-4-hydroxy-2-((4-(4-methylthiazol-5-yl)benzyl)carbamoyl)pyrrolidin-1-yl)-2-methyl-4-oxobutan-2-yl)thio)methyl)cyclohexane-1-carbonyl)piperazine-1-carboxylate* was prepared following general procedure 8. 22 mg *tert-butyl 4-((1R,4R)-4-((((R)-3-(1-fluorocyclopropane-1-carboxamido)-4-((2R,4R)-4-hydroxy-2-((4-(4-methylthiazol-5-yl)benzyl)carbamoyl)pyrrolidin-1-y*/*)-2-methyl-4-oxobuton-2-yl)thio)methyl)cyclohexane-1-carbonyl)piperazine-1-carboxylate* was obtained from 15 mg thiol compound, 100% yield. ¹H NMR (500 MHz, CDCl₃) δ 8.66 (s, 1H), 7.39 - 7.31 (m, 5H), 7.25 - 7.23 (m, 1H), 4.78 - 4.70 (m, 2H), 4.52 (s, 1H), 4.49 - 4.37 (m, 2H), 4.01 (d, *J* = 11.2 Hz, 1H), 3.73 (dd, *J* = 11.1, 3.9 Hz, 1H), 3.55 (s, 2H), 3.46 - 3.32 (m, 6H), 3.19 (d, *J* = 2.9 Hz, 1H), 2.51 (s, 3H), 2.46 - 2.30 (m, 4H), 2.28 - 2.17 (m, 1H), 1.92 - 1.84 (m, 3H), 1.75 - 1.67 (m, 2H), 1.59 - 1.48 (m, 2H), 1.46 (s, 9H), 1.37 - 1.19 (m, 10H), 1.03 - 0.86 (m, 2H); ¹³C NMR (126 MHz, CDCl₃) δ 174.37, 170.89, 170.51 (d, *J* = 20.9 Hz), 170.00, 154.72, 150.40, 148.65, 138.16, 131.69, 131.14, 129.63, 128.14, 80.45, 78.27 (d, J = 232.2 Hz), 70.23, 59.20, 56.74, 56.30, 47.98, 45.36, 43.85, 43.24, 41.60, 40.28, 37.58, 36.92, 35.25, 32.20, 32.14, 29.09, 28.52, 25.61, 16.22, 13.99 (d, *J* = 10.04 Hz), 13.95 (d, *J* = 10.03 Hz); ¹⁹F NMR (471 MHz, CDCl₃) δ -197.69. LC-MS, ESI⁺, 843.8 [M+H] ⁺. 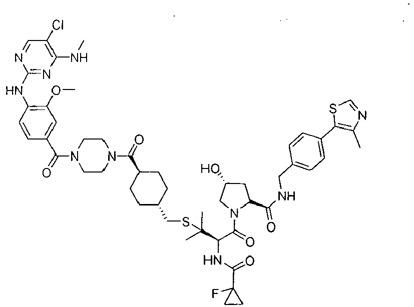 *(2R,4R)-1-((R)-3-((((1R,4R)-4-(4-(4-((5-chloro-4-(methylamino)pyrimidin-2-yl)cimino)-3-methoxybenzoyl)piperazine-1-corbonyl)cyclohexyl)methyl)thio)-2-(1-fluorocyclopropane-1-carboxamido)-3-methylbutanoyl)-4-hydroxy-N-(4-(4-methylthiazol-5-yl)benzyl)pyrrolidine-2-carboxamide **(42, XL02068)***

To a solution of *tert-*butyl 4-((1*R*,4*R*)-4-((((*R*)-3-(1-fluorocyclopropane-1-carboxamido)-4-((2*R*,4*R*)-4-hydroxy-2-((4-(4-methylthiazol-5-yl)benzyl)carbamoyl)pyrrolidin-1-yl)-2-methyl-4-oxobutan-2-yl)thio)methyl)cyclohexane-1-carbonyl)piperazine-1-carboxylate (22 mg, 0.026 mmol) in DCM (0.5 ml) was added HCl (4N HCl in 1,4-dioxane, 0.5 mL). After stirring at room temperature overnight, the mixture was condensed and washed with ethyl ether to afford a residue which was used to next step. The residue was dissolved in DMF (2 mL), followed by addition of 4-((5-chloro-4-(methylamino)pyrimidin-2-yl)amino)-3-methoxybenzoic acid (9 mg, 0.029 mmol), N,N-diisopropanylethylamine (0.021 mL, 0.117 mmol) and Pyoxim (17.2 mg, 0.033 mmol). The resulting mixture was stirred at room temperature for 2h. Water was added, and the mixture was extracted with DCM (3x). The combined organic phase was washed with water and brine, dried over sodium sulfate, filtered and condensed to afford the crude product which was purified though flash column chromatography on silica gel (0%-10% methanol in DCM) to yield **XL02068** (18.36 mg, 60% yield). ¹H NMR (500 MHz, CDCl₃) δ 8.66 (s, 1H), 8.57 (d, *J* = 8.1 Hz, 1H), 7.92 (s, 1H), 7.65 (s, 1H), 7.39 - 7.29 (m, 5H), 7.26 - 7.22 (m, 1H), 7.03 - 6.99 (m, 2H), 5.33 (q, *J* = 4.8 Hz, 1H), 4.80 - 4.71 (m, 2H), 4.53 (s, 1H), 4.45 (d, *J* = 5.9 Hz, 2H), 4.03 (d, *J* = 11.1 Hz, 1H), 3.93 (s, 3H), 3.76 - 3.44 (m, 9H), 3.12 - 3.04 (m, 4H), 2.51 (s, 3H), 2.48 - 2.33 (m, 4H), 2.25 - 2.16 (m, 1H), 1.89 (d, *J* = 12.9 Hz, 2H), 1.73 (d, *J* = 12.9 Hz, 2H), 1.60 - 1.50 (m, 2H), 1.48 - 1.39 (m, 1H), 1.37 - 1.24 (m, 10H), 1.03 - 0.90 (m, 2H); ¹³C NMR (126 MHz, CDCl₃) δ 174.46, 171.00, 170.84, 170.54 (d, *J* = 20.9 Hz), 170.03, 158.73, 157.87, 152.75, 150.41, 148.66, 147.63, 138.15, 131.97, 131.69, 131.16, 129.63, 128.15, 127.11, 120.40, 116.92, 109.75, 105.83, 79.20, 70.24, 59.15, 56.72, 56.32, 56.04, 47.94, 45.53, 43.26, 41.98, 40.29, 37.57, 36.85, 35.23, 32.18, 32.12, 29.10, 28.22, 25.63, 16.23, 14.02 (d, *J* = 9.6 Hz), 13.95 (d, *J* = 9.5 Hz); ¹⁹F NMR (471 MHz, CDCl₃) δ -197.71; HRMS (ESI⁺) *m*/*z,* calcd for C₅₀H₆₂ClFN₁₀O₇S₂: 1033.3989 [M + H]⁺, found 1033.3987.

### Exemplary synthesis of Exemplary compound 43 (XL02093)

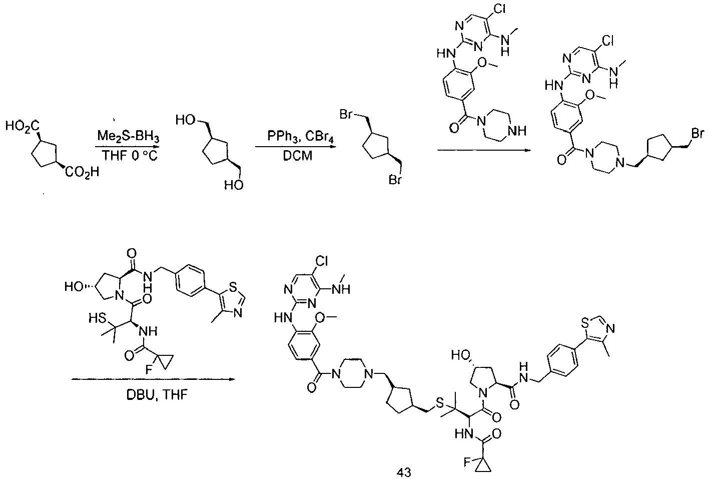

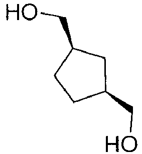

*(cis-cyclopentane-1,3-diyl)dimethanol*

To a solution of (1*R*,3*S*)-cyclopentane-1,3-dicarboxylic acid (500 mg, 3.125 mmol) in THF (10 mL) was added Mes₂S-BH₃ (2 M in THF, 3.48 mL, 6.96 mmol) at 0 °C. After stirring at 0 °C for 2-3h, the reaction is quenched with methanol and condensed under reduced pressure to afford a crude product which was purified with flash column chromatography on silica gel to give *(cis-cyclopentane-1,3-diyl)dimethanol* (400 mg, 97% yield). ¹H NMR (400 MHz, CDCl₃) δ 3.56 - 3.52 (m, 4H), 2.23 - 2.09 (m, 2H), 2.00 - 1.90 (m, 1H), 1.85 - 1.64 (m, 4H), 1.44 - 1.29 (m, 2H), 0.94 (dt, *J* = 12.6, 9.2 Hz, 1H). ¹³C NMR (101 MHz, CDCl₃) δ 67.40, 42.43, 32.89, 28.43. 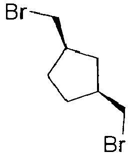 *cis-1,3-bis(bromomethyl)cyclopentane*

CBr₄ (955 mg, 2.88 mmol) was dissolved in DCM (5mL) and added to a solution of (cis-cyclopentane-1,3-diyl)dimethanol (150 mg, 1.15 mmol) and triphenyl phosphine (906 mg, 3.46 mmol) in DCM (10 mL) at -15 °C. The mixture was slowly warmed to room temperature and stirred at room temperature overnight. Water was added and the mixture was extracted with DCM. The organic phase was then washed with water and brine, dried over sodium sulfate, filtered and condensed to afford a residue which was purified through flash chromatography on silica gel (0-20% Ethyl acetate in heptane) to *cis-1,3-bis(bromomethyl)cyclopentane* (51 mg, 17% yield). ¹H NMR (400 MHz, CDCl₃) δ 3.40 (d, *J* = 6.7 Hz, 4H), 2.47 - 2.26 (m, 2H), 2.14 (dt, *J* = 13.6, 7.0 Hz, 1H), 1.97 - 1.80 (m, 2H), 1.52 - 1.42 (m, 2H), 1.09 (dt, *J* = 12.7, 10.0 Hz, 1H). ¹³C NMR (101 MHz, CDCl₃) δ 42.55, 38.68, 38.18, 30.85. 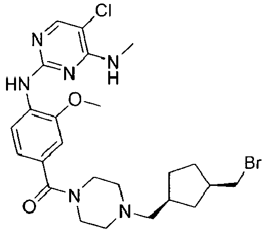 *(4-(((1S,3R)-3-(bromomethyl)cyclopentyl)methyl)piperazin-1-yl)(4-((5-chloro-4-(methylomino)pyrimidin-2-yl)omino)-3-methoxyphenyl)methonone*

To a solution of (4-((5-chloro-4-(methylamino)pyrimidin-2-yl)amino)-3-methoxyphenyl)(piperazin-1-yl)methanone HCl salt (30 mg, 0.067 mmol) in acetone (1 mL) was added cis-1,3-bis(bromomethyl)cyclopentane (56 mg, 0.217 mmol) and K₂CO₃ (60 mg, 0.434 mmol). After stirring at 50 °C for two days, the mixture was cooled down, diluted with water and extracted with DCM (3x). The combined organic phase was washed with water and brine, dried over sodium sulfate, filtered and condensed to afford a residue which was purified through flash column chromatography on silica gel (0%-10% methanol in DCM) to give *(4-(((1S,3R)-3-(bromomethyl)cyclopentyl)methyl)piperazin-1-yl)(4-((5-chloro-4-(methylamino)pyrimidin-2-yl)cimino)-3-methoxyphenyl)methanone* (8 mg, 20% yield). ¹H NMR (400 MHz, CDCl₃) δ 8.54 (d, *J* = 8.8 Hz, 1H), 7.92 (s, 1H), 7.63 (s, 1H), 7.03 - 6.97 (m, 2H), 5.33 - 5.26 (m, 1H), 3.92 (s, 3H), 3.64 (s, 4H), 3.39 (d, *J* = 6.7 Hz, 2H), 3.10 (d, *J* = 4.8 Hz, 3H), 2.43 (s, 4H), 2.36 - 2.27 (m, 3H), 2.23 - 2.13 (m, 1H), 2.11 - 2.01 (m, 1H), 1.90 - 1.75 (m, 2H), 1.46 - 1.30 (m, 2H), 0.97 (dd, *J* = 22.1, 9.7 Hz, 1H). ¹³C NMR (126 MHz, CDCl₃) δ 170.53, 158.67, 157.92, 152.77, 147.51, 131.38, 128.03, 120.26, 116.81, 109.66, 105.61, 64.50, 55.98, 53.77, 42.39, 39.33, 38.05, 37.56, 30.63, 30.59, 28.23. LC-MS, ESI⁺, m/z 551 [M+H]⁺. 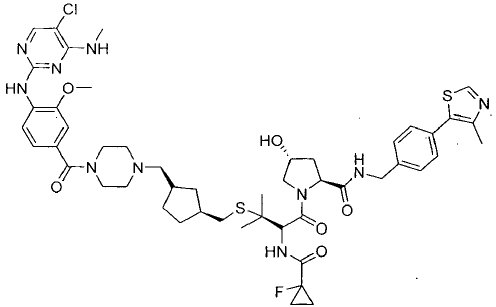 *(2S,4R)-1-((S)-3-((((1S,3S)-3-((4-(4-((5-chloro-4-(methylamino)pyrimidin-2-yl)omino)-3-methoxybenzoyl)piperazin-1-yl)methyl)cyclopentyl)methyl)thio)-2-(1-fluorocyclopropone-1-carboxamido)-3-methylbutonoyl)-4-hydroxy-N-(4-(4-methylthiazol-5-yl)benzyl)pyrrolidine-2-carboxamide* **(43, XL02093)**

**XL02093** was prepared following general procedure 8. 10.6 mg **XL02093** was obtained from 10 mg thiol compound, 58% yield. ¹H NMR (400 MHz, CDCl₃) δ 8.66 (s, 1H), 8.54 (d, *J* = 8.4 Hz, 1H), 7.92 (s, 1H), 7.63 (s, 1H), 7.36 (dd, *J* = 17.1, 8.2 Hz, 5H), 7.23 (d, *J* = 8.2 Hz, 1H), 7.00 (d, *J* = 7.6 Hz, 2H), 5.29 (d, *J* = 4.4 Hz, 1H), 4.87 - 4.68 (m, 2H), 4.49 (d, *J* = 28.7 Hz, 3H), 4.02 (dd, *J* = 20.0, 11.4 Hz, 1H), 3.92 (s, 3H), 3.74 (ddd, *J* = 44.8, 23.8, 18.6 Hz, 5H), 3.11 (d, *J* = 4.8 Hz, 3H), 2.58 - 2.34 (m, 10H), 2.24 (d, *J* = 13.6 Hz, 3H), 2.00 (dd, *J* = 35.3, 28.9 Hz, 3H), 1.75 (s, 2H), 1.30 (dd, *J* = 17.4, 11.0 Hz, 13H), 0.84 (dd, *J* = 21.2, 11.2 Hz, 1H); ¹³C NMR (101 MHz, CDCl₃) δ 170.72, 170.56, 170.20, 170.16, 158.73, 157.98, 152.82, 150.39, 148.70, 147.58, 138.19, 131.69, 131.48, 131.21, 129.67, 128.23, 128.20, 120.32, 116.92, 109.75, 105.69, 79.45, 70.20 (d, *J* = 15.6 Hz), 64.72 (d, *J* = 7.82 Hz), 59.01, 56.57 (d, *J* = 15.3 Hz), 56.28 (d, *J* = 12.2 Hz), 56.03, 53.71, 47.75, 43.27, 39.75, 39.12 (d, *J* = 11.6 Hz), 37.23 (d, *J* = 15.9 Hz), 36.73, 36.69, 34.52 (d, *J* = 9.0 Hz), 31.62, 30.36, 28.23, 25.77 (d, *J* = 2.87 Hz), 25.44 (d, *J* = 4.36 Hz), 16.26, 14.06 (d, *J* = 12.5 Hz), 13.95 (d, *J* = 10.51 Hz). ¹⁹F NMR (471 MHz, CDCl₃) δ -197.71, -197.72. HRMS (ESI⁺) *m*/*z,* calcd for C₄₉H₆₂ClFN₁₀O₆S₂: 1005.4040 [M + H]⁺, found 1005.3815. 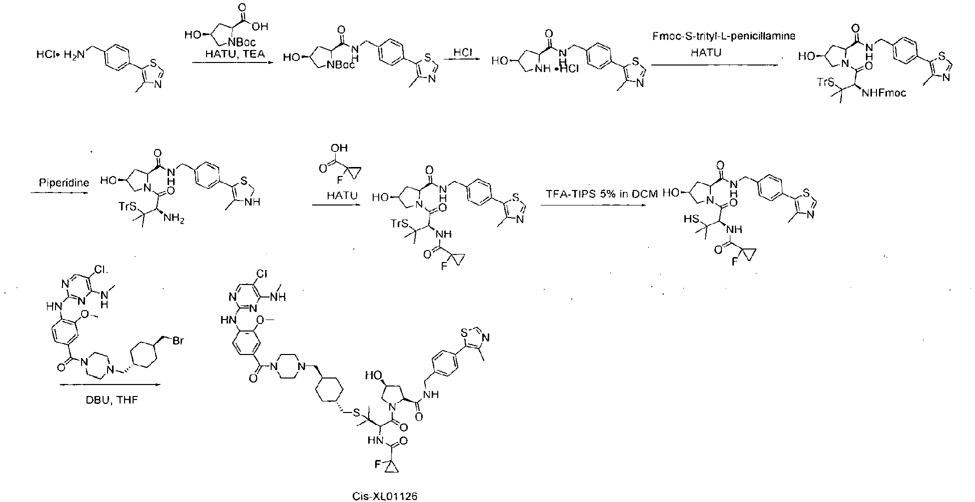 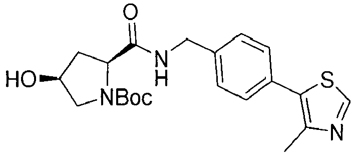 *tert-butyl (2S,4S)-4-hydroxy-2-((4-(4-methylthiozol-5-yl)benzyl)carbamoyl)pyrrolidine-1-carboxylote* was prepared following general proceure 3. 560 mg *tert-butyl (2S,4S)-4-hydroxy-2-((4-(4-methylthiazol-5-yl)benzyl)carbomoyl)pyrrolidine-1-carboxylate* was obtained from 500 mg amine compound, 65% yield. ¹H NMR (400 MHz, CDCl₃) δ 8.67 (s, 1H), 7.52 (s, 1H), 7.36 (dd, *J* = 22.1, 7.8 Hz, 4H), 5.15 (d, *J* = 9.2 Hz, 1H), 4.58 (dd, *J* = 15.2, 6.3 Hz, 1H), 4.50 - 4.33 (m, 3H), 3.58 - 3.42 (m, 2H), 2.52 (s, 3H), 2.38 (d, *J* = 14.1 Hz, 1H), 2.22 - 2.12 (m, 1H), 1.45 (s, 9H). LC-MS, ESI⁺, m/z 418.0 [M+H]⁺. 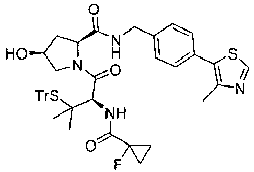 *(2S,4S)-1-((R)-2-(1-fluorocyclopropone-1-carboxamido)-3-methyl-3-(tritylthio)butanoyl)-4-hydroxy-N-(4-(4-methylthiazol-5-yl)benzyl)pyrrolidine-2-carboxamide*

To a solution of *tert*-butyl (2*S*,4*S*)-4-hydroxy-2-((4-(4-methylthiazol-5-yl)benzyl)carbamoyl)pyrrolidine-1-carboxylate (568 mg, 1.36 mmol) in DCM (6.8 mL) was added 4N HCl in 1,4-dioxane (6.8 ml). The resulting mixture was stirred at room temperature overnight and condensed to afford a solid (530 mg, 100% yield). To a solution of the obtained solid (200 mg, 0.57 mmol) and TEA (236 µL) in DMF (5 ml) was added dropwise with a mixture of Fmoc-S-trityl-L-penicillamine (329 mg, 0.54 mmol), HATU (215 mg, 0.57 mmol) and TEA (79 µL) in DMF (5 mL). After stirring at room temperature overnight, the mixture was diluted with DCM, added with water and the organic layer was collected. The organic layer was then washed with water and brine, dried over sodium sulfate, filtered and condensed to afford a residue which was purified with flash column to afford a residue as an amine compound (2*S*,4*S*)-1-((*R*)-2-amino-3-methyl-3-(tritylthio)butanoyl)-4-hydroxy-N-(4-(4-methylthiazol-5-yl)benzyl)pyrrolidine-2-carboxamide ( 120 mg, 32% yield, LC-MS, ESI⁻, 689.4 [M-H]⁻ ) which was used to the next step. To a solution of the amine compound (60 mg, 0.087 mmol) in DMF (1.5 ml) was added TEA (24 µL, 0.174 mmol), HATU (35 mg, 0.092 mmol), and 1-fluorocyclopropane-1-carboxylic acid (9 mg, 0.087 mmol) separately. After stirring at room temperature for 4h, the resulting mixture was diluted with ethyl acetate, and washed with water and brine, dried over sodium sulfate, filtered and condensed to afford a residue which was purified via flash column chromatography on silica gel to give *(2S,4S)-1-((R)-2-(1-fluorocyclopropone-1-carboxamido)-3-methyl-3-(tritylthio)butanoyl)-4-hydroxy-N-(4-(4-methylthiazol-5-yl)benzyl)pyrrolidine-2-carboxamide* (57 mg, 85% yield). ¹H NMR (400 MHz, CDCl₃) δ 8.62 (s, 1H), 7.60 (t, *J* = 6.0 Hz, 1H), 7.52 - 7.45 (m, 6H), 7.25 - 7.21 (m, 2H), 7.19 - 7.07 (m, 12H), 5.26 (d, *J* = 9.6 Hz, 1H), 4.54 (d, *J* = 8.7 Hz, 1H), 4.35 - 4.23 (m, 2H), 4.16 (dd, *J* = 15.1, 5.4 Hz, 1H), 3.40 (d, *J* = 5.1 Hz, 1H), 3.34 (dd, *J* = 11.0, 4.0 Hz, 1H), 3.22 (d, *J* = 10.9 Hz, 1H), 2.43 (s, 3H), 2.12 (d, *J* = 14.0 Hz, 1H), 2.08 - 1.97 (m, 1H), 1.30 - 1.12 (m, 4H), 1.07 (s, 3H), 1.01 (s, 3H). ¹³C NMR (101 MHz, CDCl₃) δ 172.74, 169.95, 169.73 (d, *J* = 20.35 Hz), 150.40, 148.65, 144.50, 137.65, 131.66, 131.14, 129.84, 129.62, 128.06, 127.92, 126.89, 78.30 (d, *J* = 226.86 Hz), 71.12, 68.37, 60.15, 58.94, 56.77, 53.73, 43.38, 35.48, 26.19, 25.71, 16.21, 13.66 (d, *J* = 9.46 Hz), 13.57 (d, *J* = 9.33 Hz). LC-MS, ESI⁺, m/z, 777.5 [M+H]⁺. 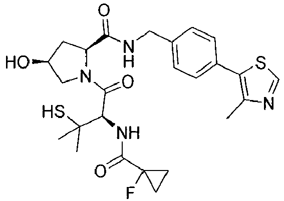 *(2S,4S)-1-((R)-2-(1-fluorocyclopropone-1-carb6xamido)-3-mercapto-3-methylbutonoyl)-4-hydroxy-N-(4-(4-methylthiazol-5-yl)benzyl)pyrrolidine-2-carboxomide* was prepared following general procedure 7. 36 mg *(2S,4S)-1-((R)-2-(1-fluorocyclopropone-1-carboxamido)-3-mercopto-3-methylbutonoyl)-4-hydroxy-N-(4-(4-methylthiazol-5-yl)benzyl)pyrrolidine-2-carboxamide* was obtained from 57 mg trityl protected compound, 92% yield. ¹H NMR (400 MHz, CDCl₃) δ 8.82 (s, 1H), 7.52 (t, *J* = 5.9 Hz, 1H), 7.41 - 7.34 (m, 4H), 6.63 (br, s, 2H), 4.74 - 4.60 (m, 3H), 4.48 (t, *J* = 4.2 Hz, 1H), 4.31 (dd, *J* = 15.0, 5.0 Hz, 1H), 3.96 (dd, *J* = 11.0, 4.0 Hz, 1H), 3.91 (d, *J* = 11.0 Hz, 1H), 2.52 (s, 3H), 2.39 - 2.29 (m, 2H), 2.27 - 2.17 (m, 1H), 1.40 - 1.23 (m, 10H). ¹³C NMR (101 MHz, CDCl₃) δ 172.53, 170.72, 170.14 (d, *J* = 20.51 Hz), 151.22, 147.71, 137.83, 132.26, 130.84, 129.84, 128.39, 78.21 (d, *J* = 231Hz), 71.15, 60.36, 58.70, 57.19, 46.43, 43.69, 35.65, 30.44, 28.93, 15.64, 13.96, 13.86. LC-MS, ESI⁺, m/z 535.4 [M+H]⁺. 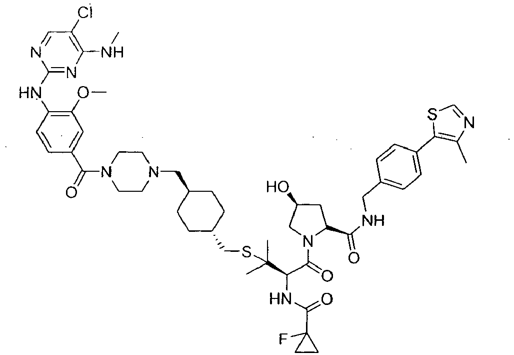 *(2R,4S)-1-((R)-3-((((1r,4R)-4-((4-(4-((5-chloro-4-(methylomino)pyrimidin-2-yl)amino)-3-methoxybenzoyl)piperazin-1-yl)methyl)cyclohexyl)methyl)thio)-2-(1-fluorocyclopropone-1-carboxamido)-3-methylbutonoyl)-4-hydroxy-N-(4-(4-methylthicizol-5-yl)benzyl)pyrrolidine-2-carboxamide* (Cis-XL01126)

**Cis-XL01126** was prepared following general procedure 8. 11.92 mg **Cis-XL01126** was obtained from 10 mg thiol compound, 65% yield. ¹H NMR (400 MHz, CDCl₃) δ 8.67 (s, 1H), 8.53 (d, *J* = 8.8 Hz, 1H), 7.92 (s, 1H), 7.62 (s, 1H), 7.45 (t, *J* = 5.9 Hz, 1H), 7.41 - 7.33 (m, 4H), 7.17 (dd, *J* = 8.1, 3.3 Hz, 1H), 7.03 - 6.98 (m, 2H), 5.31 - 5.28 (m, 1H), 4.73 (dd, *J* = 18.0, 8.3 Hz, 2H), 4.58 (dd, *J* = 15.0, 6.6 Hz, 1H), 4.53 - 4.46 (m, 1H), 4.37 (dd, *J* = 15.0, 5.3 Hz, 1H), 3.97 - 3.84 (m, 5H), 3.63 (s, 4H), 3.10 (d, *J* = 4.9 Hz, 3H), 2.52 (s, 3H), 2.43 - 2.32 (m, 7H), 2.21 (ddd, *J* = 14.0, 9.2, 4.8 Hz, 1H), 2.12 (d, *J* = 7.0 Hz, 2H), 1.82 (t, *J* = 13.3 Hz, 4H), 1.44 - 1.28 (m, 12H), 1.03 - 0.76 (m, 4H); ¹³C NMR (101 MHz, CDCl₃) δ 172.38, 170.76, 170.64, 170.13, 169.93, 158.89, 150.54, 148.75, 148.24, 137.42, 131.64, 131.47, 129.81, 128.29, 120.61, 109.94, 105.84, 79.48, 77.48, 77.16, 76.84, 71.23, 63.99, 60.36, 58.70, 56.13, 55.95, 52.79, 47.72, 43.71, 37.55, 35.52, 35.12, 33.31, 32.07, 31.98, 30.91, 28.36, 25.70, 25.47, 16.25, 13.95, 13.84. HRMS (ESI⁺) *m*/*z,* calcd for C₅₀H₆₄ClFN₁₀O₆S₂: 1019.4197 [M + H]⁺, found 1019.4206.

## Claims

1. A compound of formula (I):
X-L1-L2-Y Formula (I)
Wherein X is: 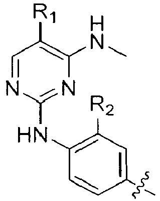
Wherein R₁ is selected from: F, Cl, Br, -CF₃, CN, NO₂, -CHF₂, -CH₂F;
Wherein R₂ is selected from: - Me, -CD₃, -OMe, -OCD₃, OCF₃, -OCHF₂, Ethyl, -OCH₂CH₃, isopropyl, cyclopropane, and the following groups: 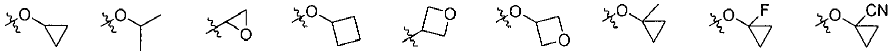
Wherein L1 is selected from: 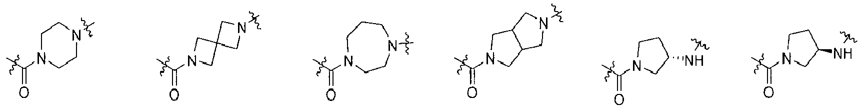 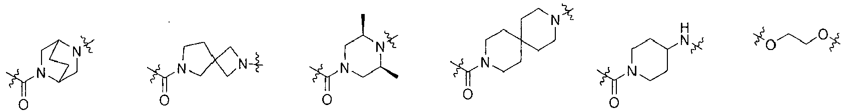 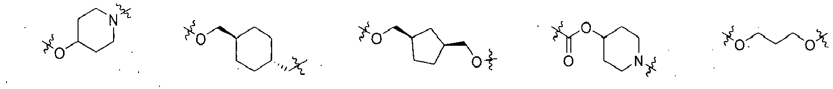 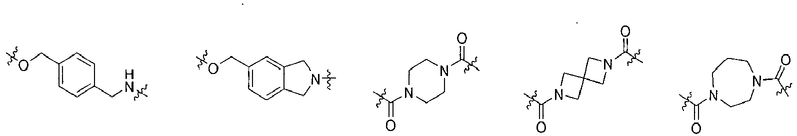 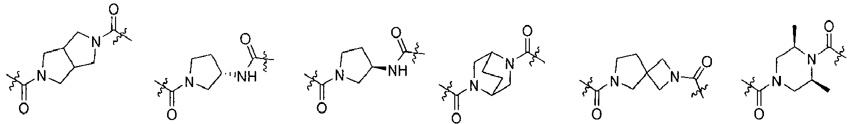 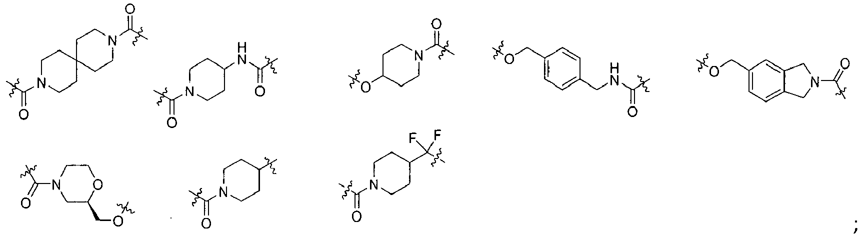
Wherein L2 is selected from: 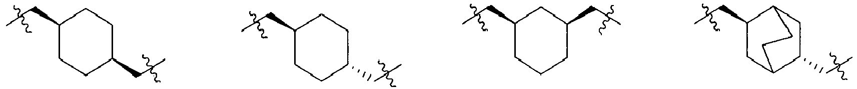 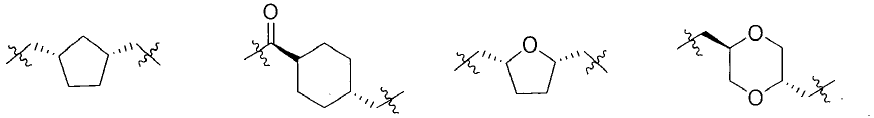 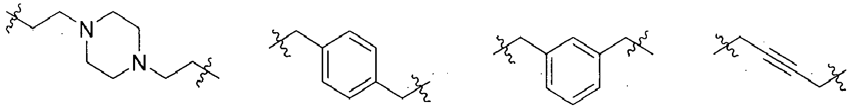 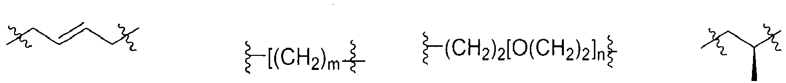 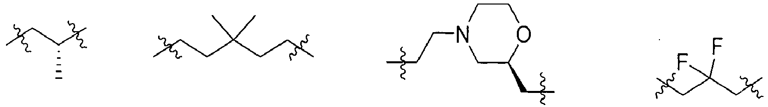 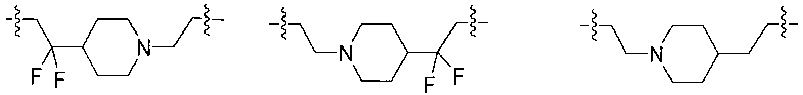 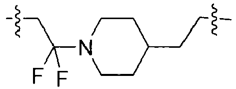
wherein m is independently selected from 2, 3, 4, 5, 6, 7, and 8; and
wherein n is independently selected from 1, 2, 3, 4, 5, and 6;
Wherein Y is selected from: 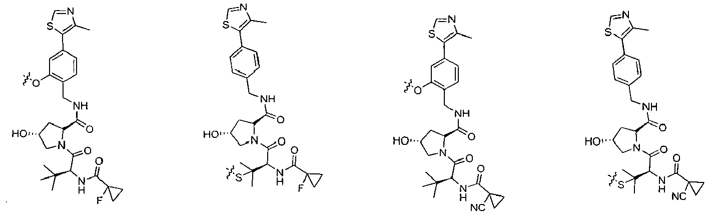 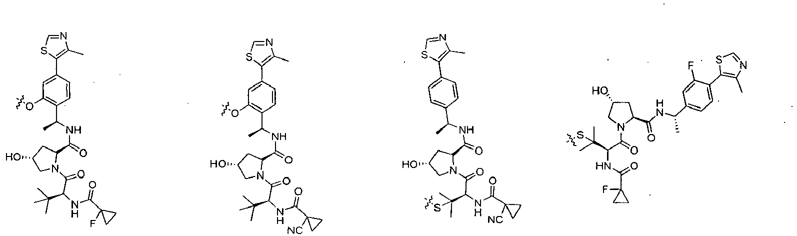 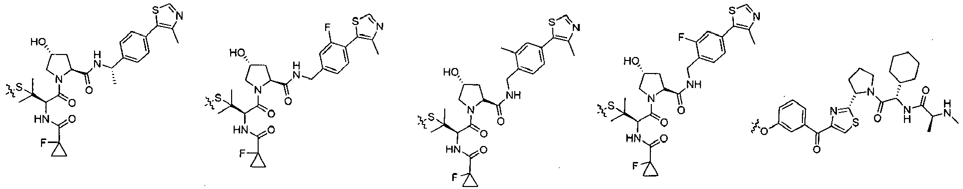 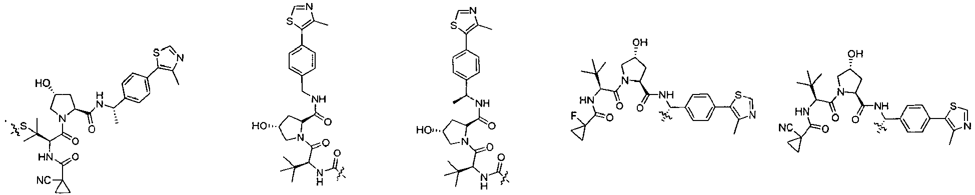 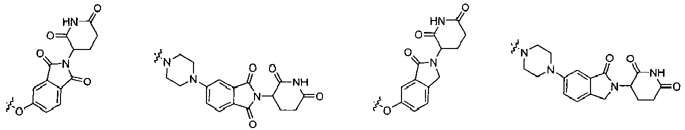 or a pharmaceutically acceptable, salt, enantiomer, stereoisomer, hydrate, solvate, or polymorph thereof.

2. The compound of formula (I) according to claim 1, wherein X is selected from 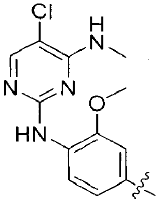 , and

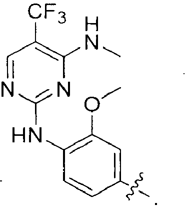

3. The compound of formula (I) according to claim 1 or 2, wherein L1 is selected from:

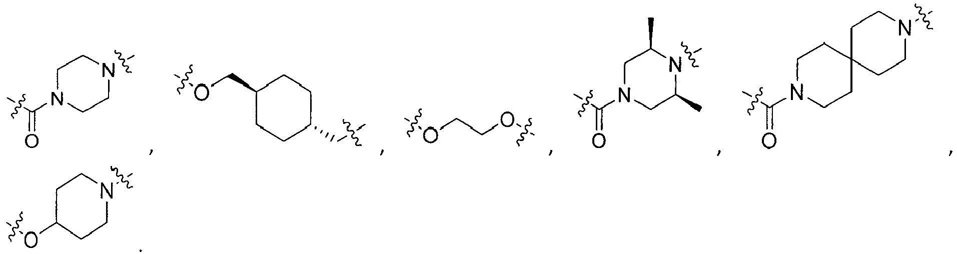

4. The compound of formula (I) according to any one of claims 1 to 3, wherein L2 is selected from: 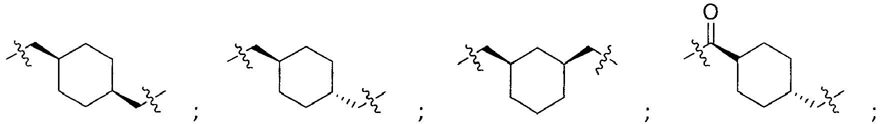 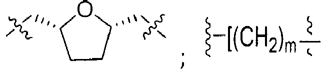 wherein m is independently selected from 3, 4, and 5; 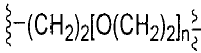 wherein n is independently selected from 1, 2, 3, and 4;

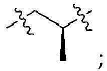

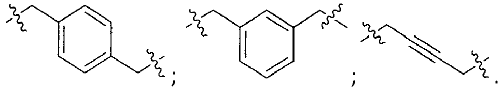

5. The compound of formula (I) according to anyone of claims 1 to 4, wherein Y is selected from:

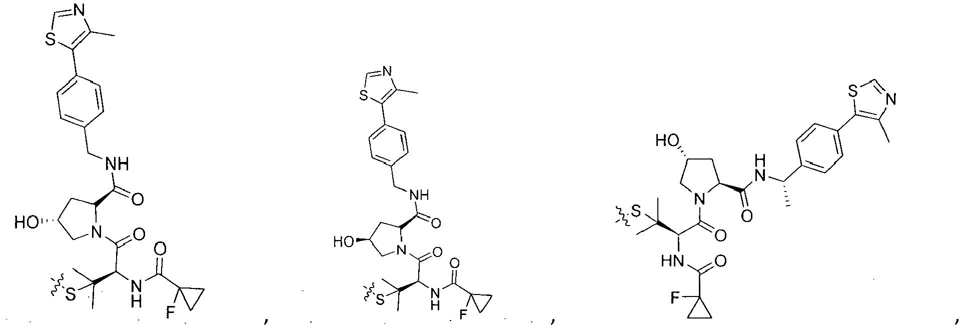

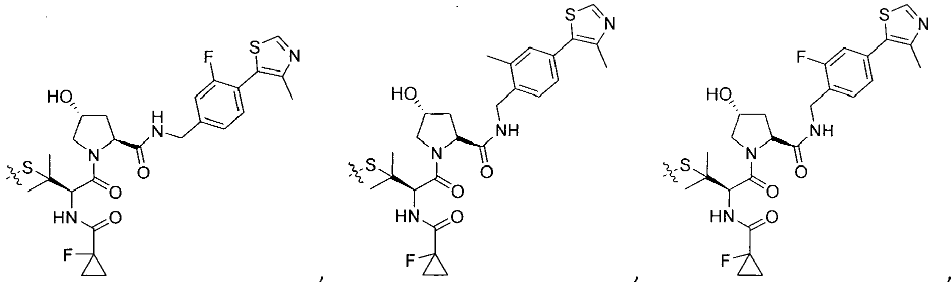

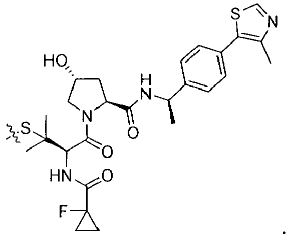

6. The compound of formula (I) according to any one of claims 1 to 5, wherein the compound is selected from: 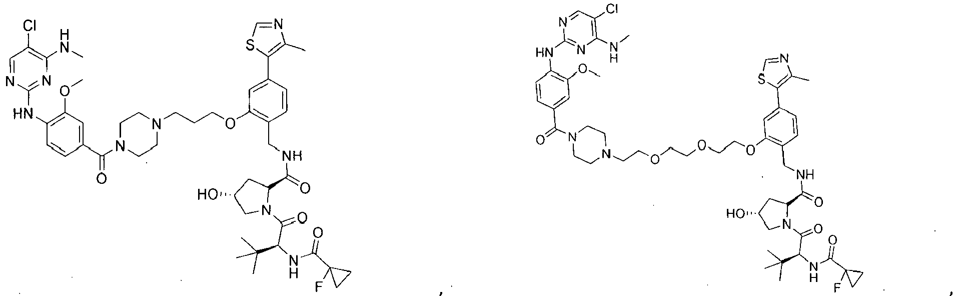 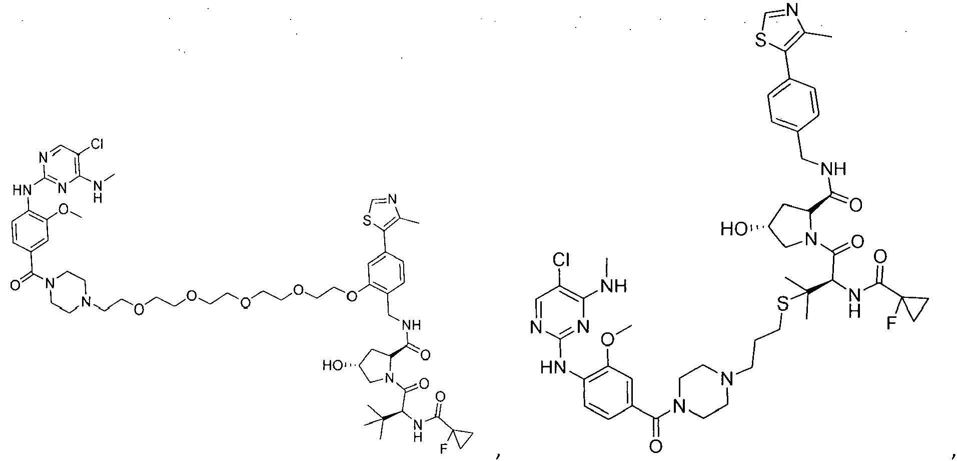 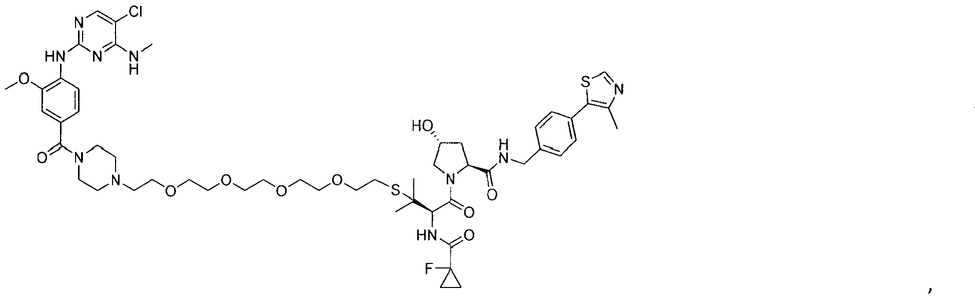 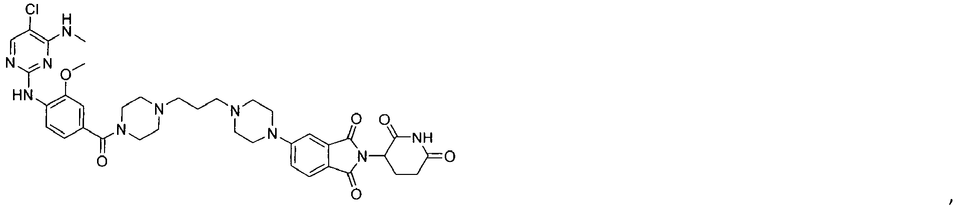 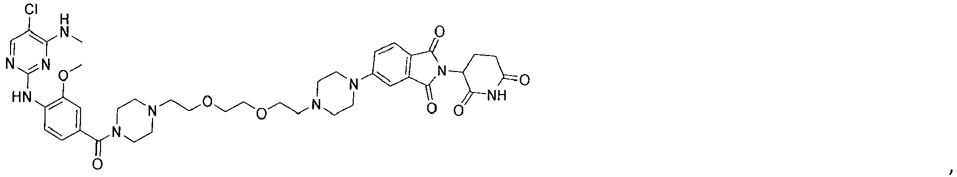 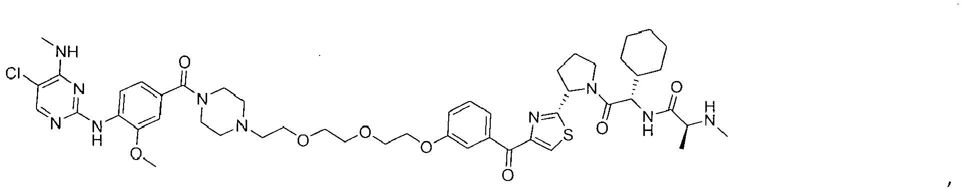 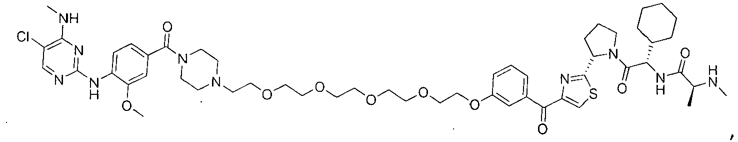 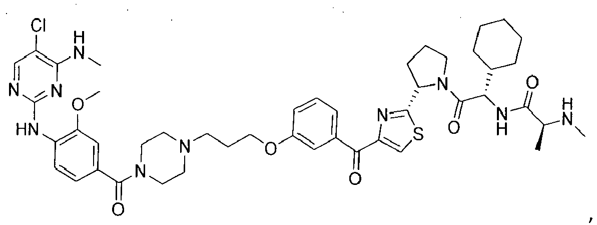 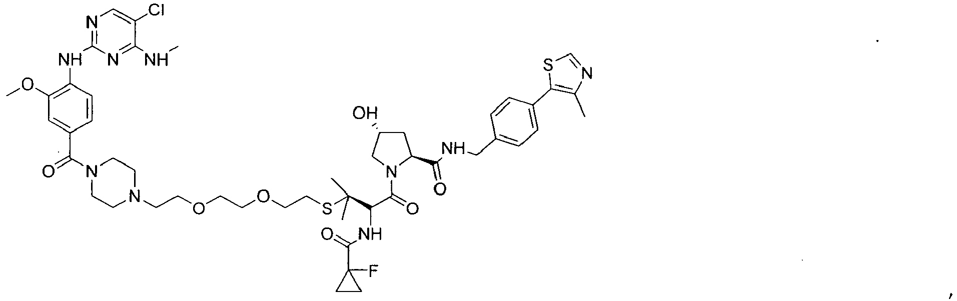 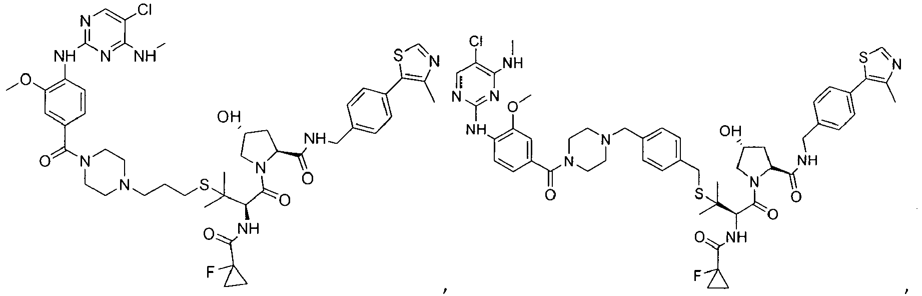 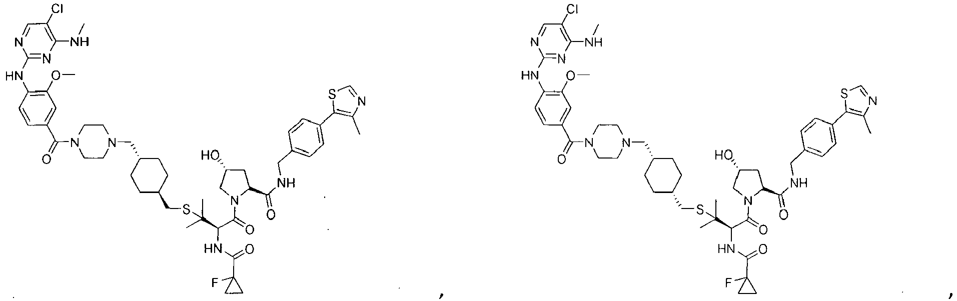 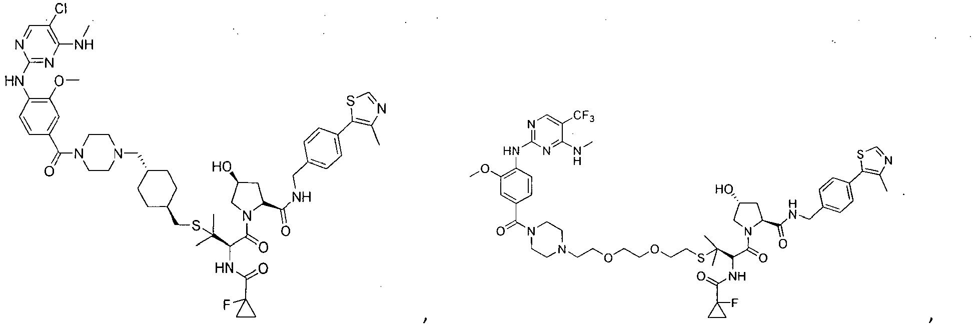 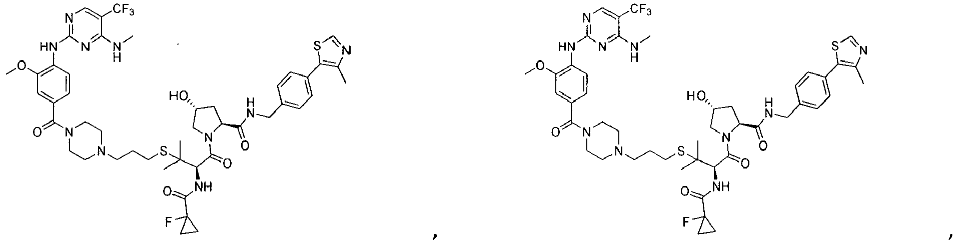 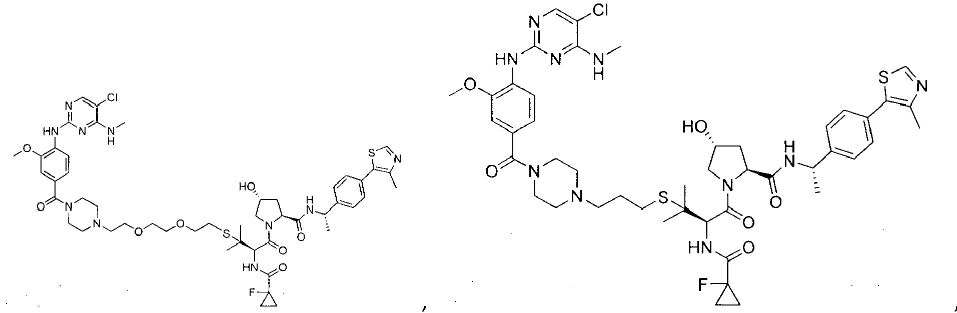 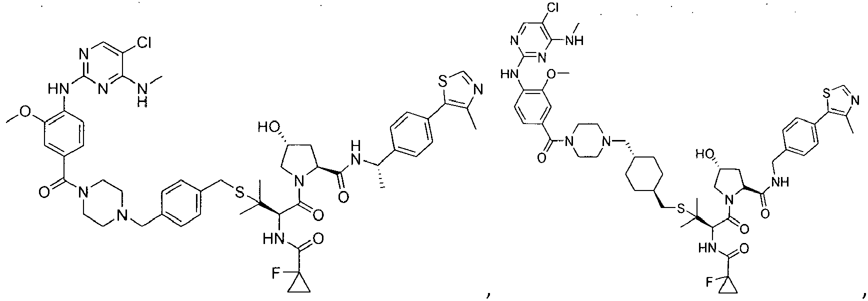 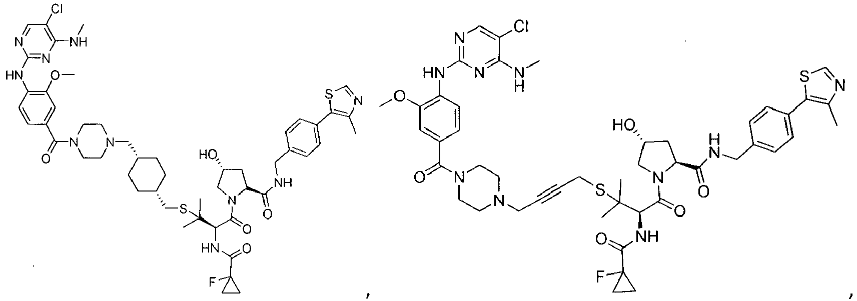 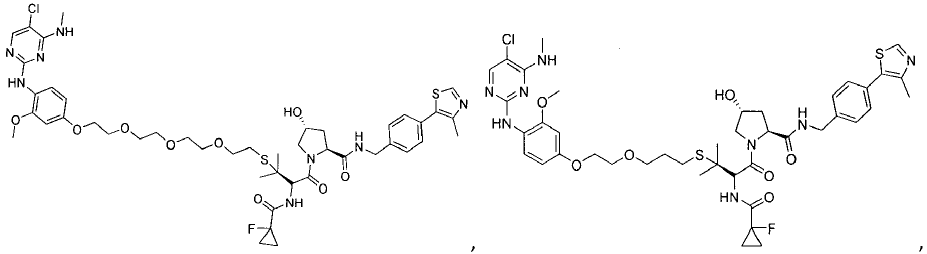 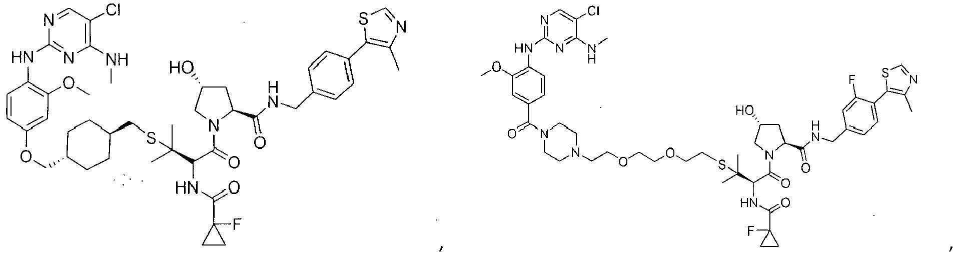 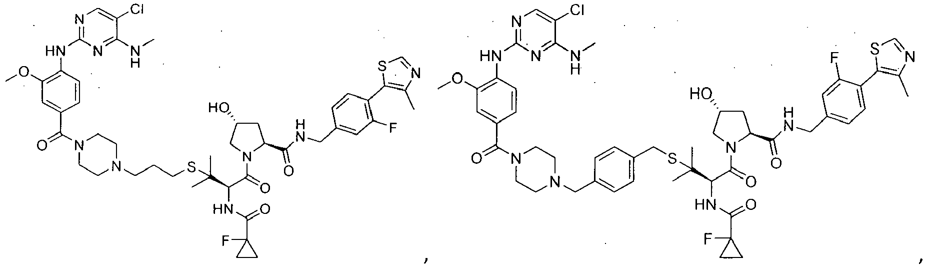 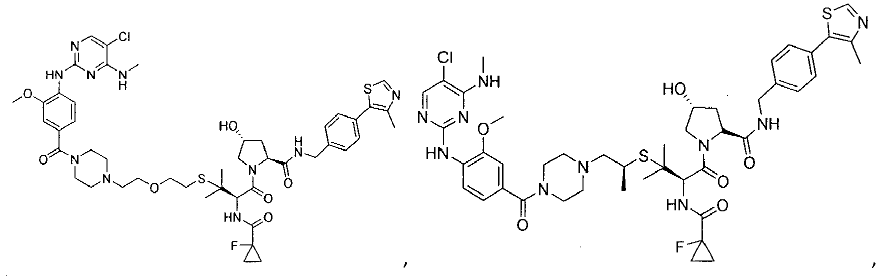 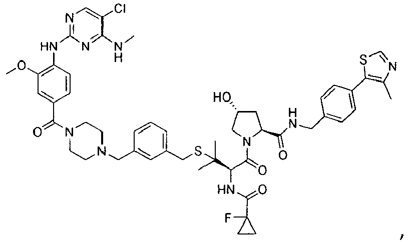 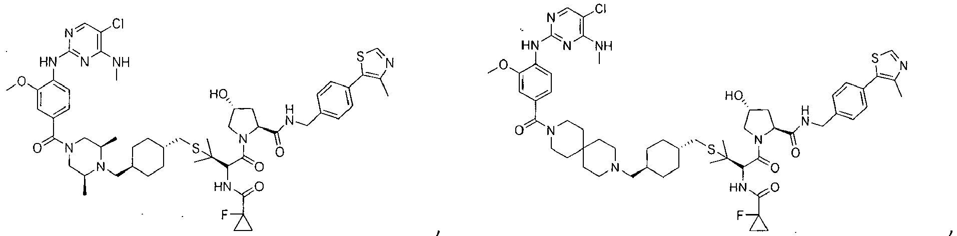 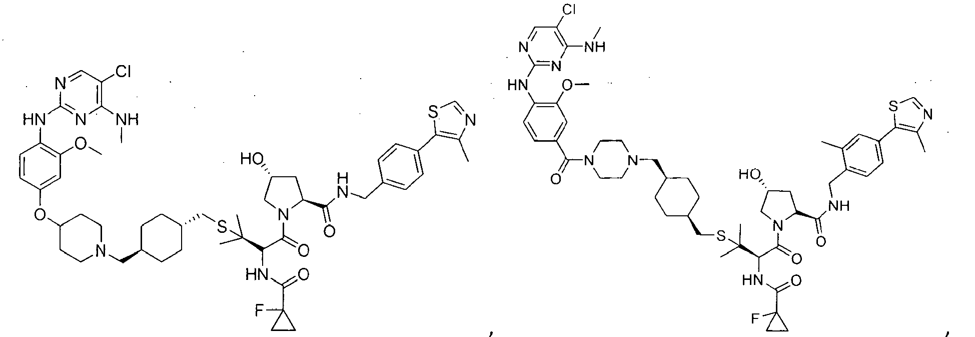 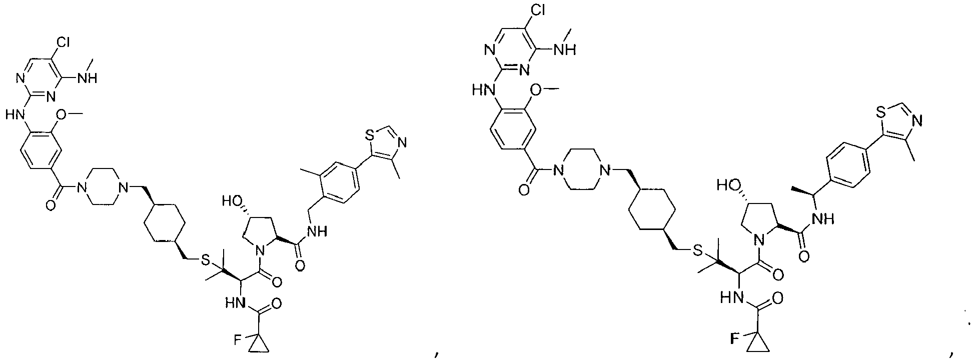 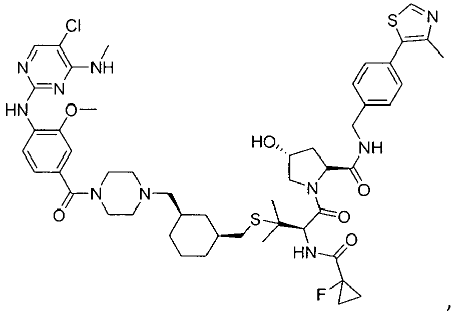 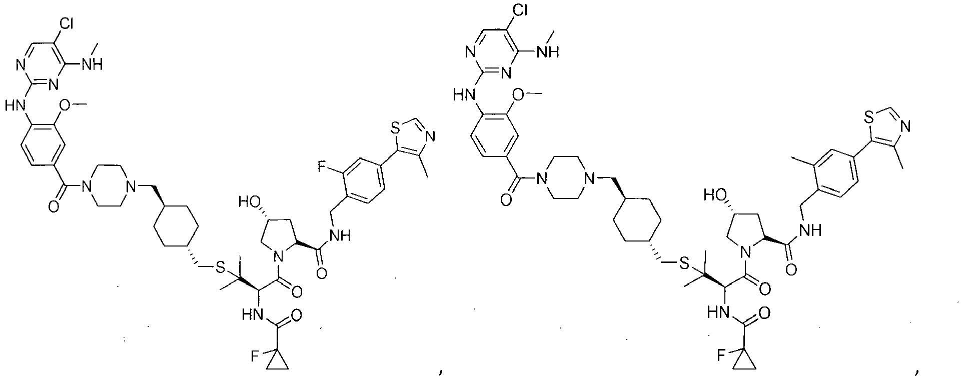 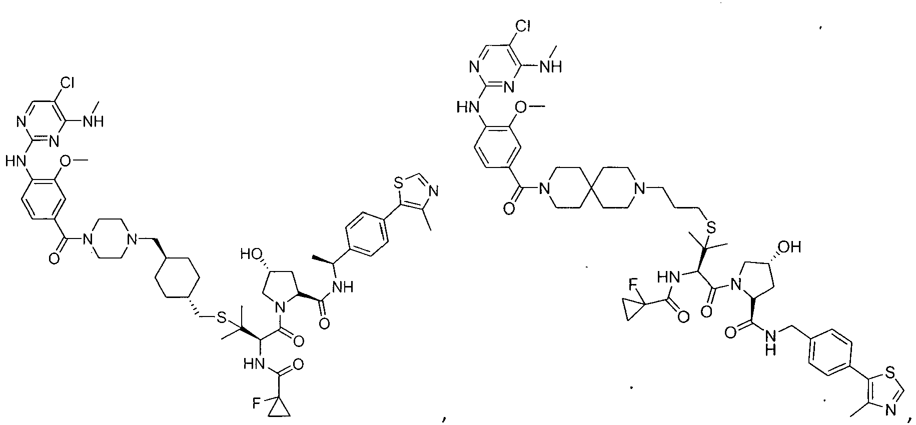 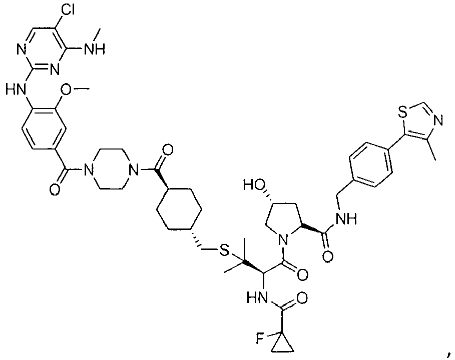 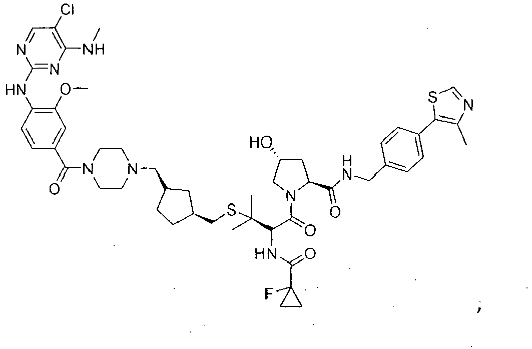 or a pharmaceutically acceptable, salt, enantiomer, stereoisomer, hydrate, solvate, or polymorph thereof.

7. The compound of formula (I) according to any one of claims 1 to 6, wherein one of:
the compound is selected from: 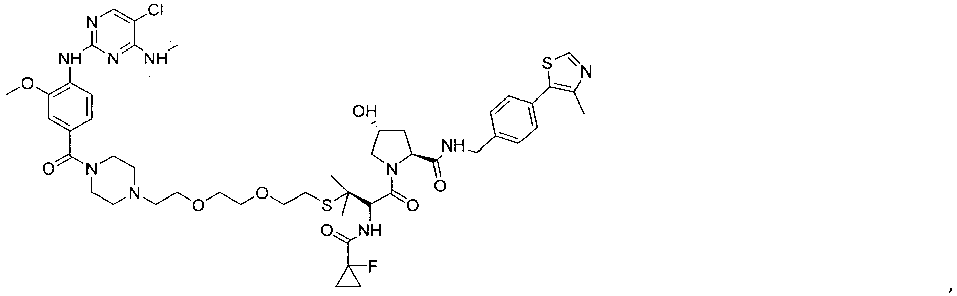 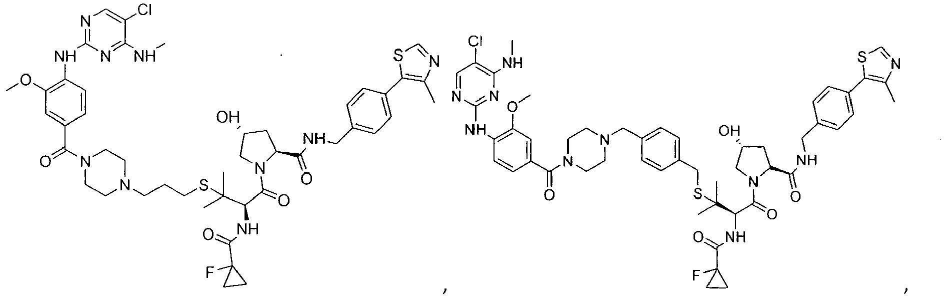 or a pharmaceutically acceptable, salt, enantiomer, stereoisomer, hydrate, solvate, or polymorph thereof; or
the compound is selected from: 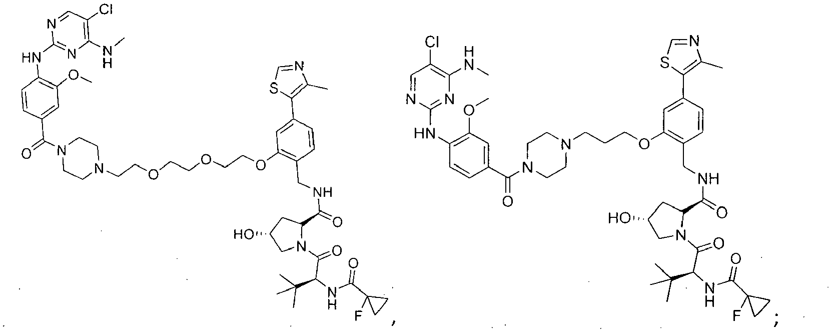 or a pharmaceutically acceptable, salt, enantiomer, stereoisomer, hydrate, solvate, or polymorph thereof.

8. The compound of formula (I) according to any one of claims 1 to 6, wherein the compound is selected from: 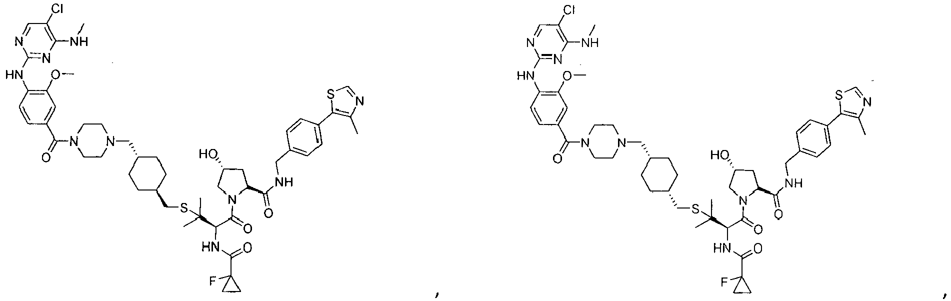 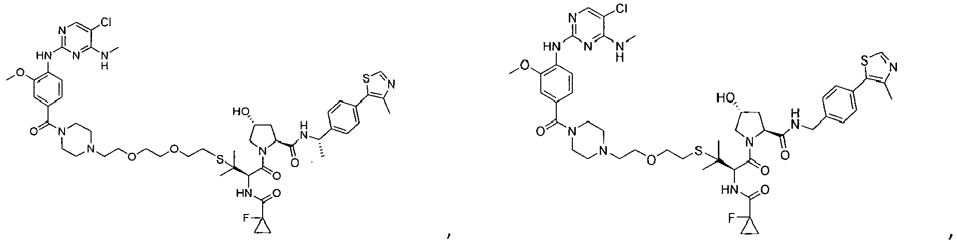 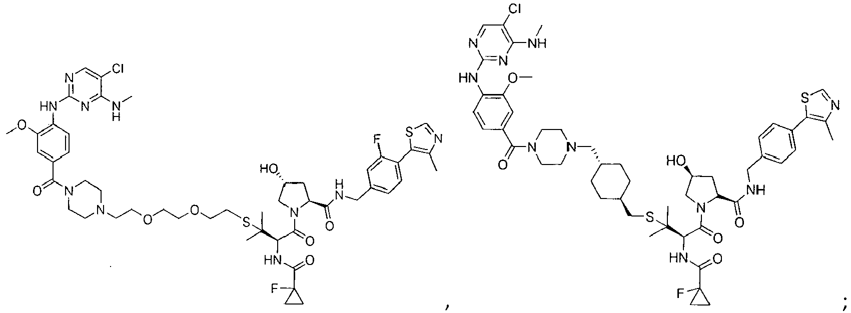 or a pharmaceutically acceptable, salt, enantiomer, stereoisomer, hydrate, solvate, or polymorph thereof.

9. The compound of formula (I) according to any one of claims 1 to 6, wherein the compound is selected from: 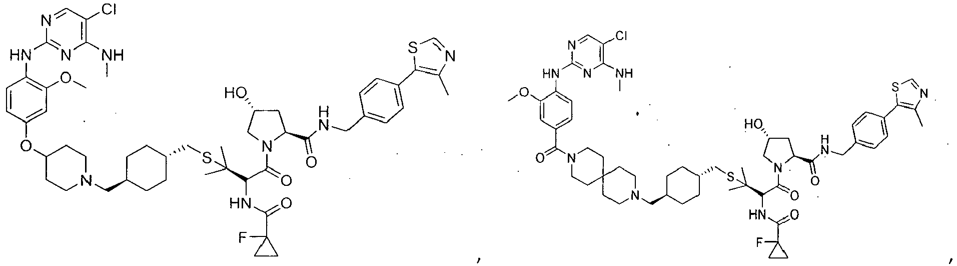 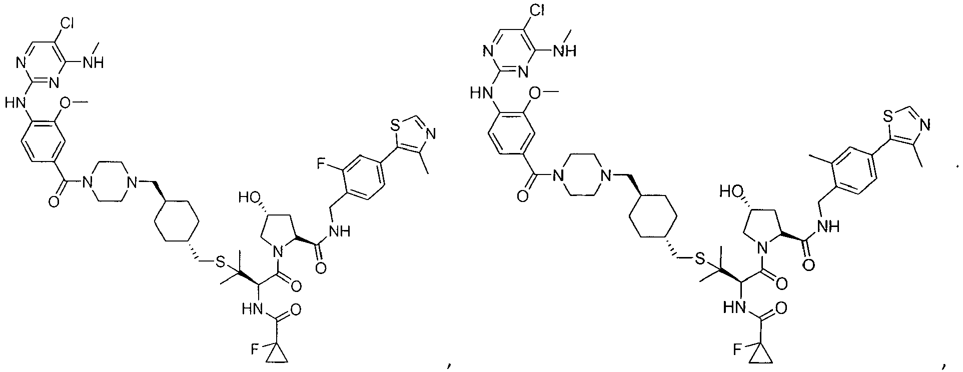 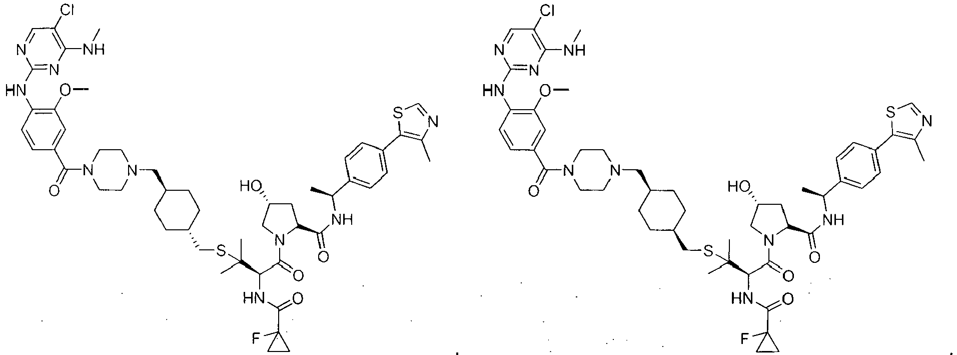 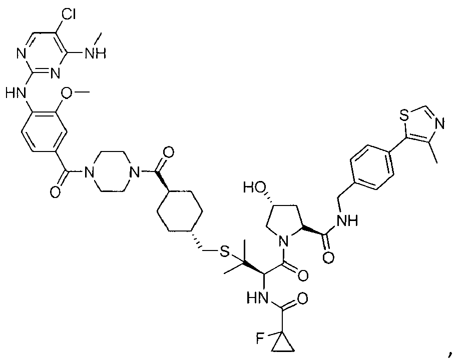 or a pharmaceutically acceptable, salt, enantiomer, stereoisomer, hydrate, solvate, or polymorph , thereof.

10. The compound of formula (I) according to any one of claims 1 to 6 or 8, wherein the compound is selected from: 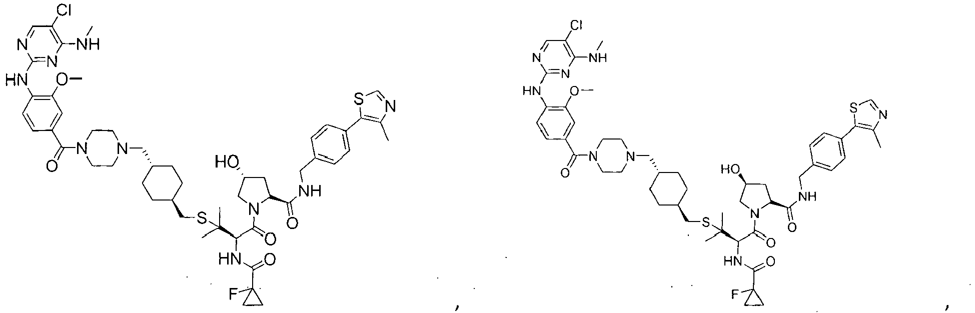 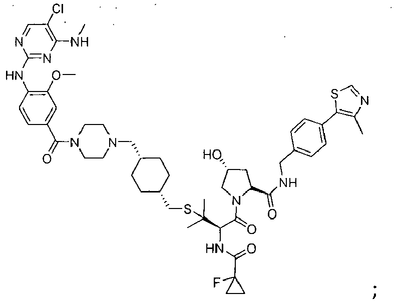 or a pharmaceutically acceptable, salt, enantiomer, stereoisomer, hydrate, solvate, or polymorph thereof.

11. The compound of formula (I) according to any one of claims 1 to 6 or 9, wherein at least one of:
the compound is selected from: 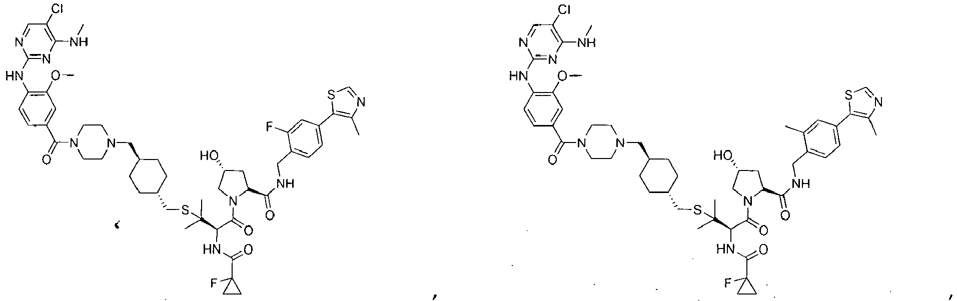 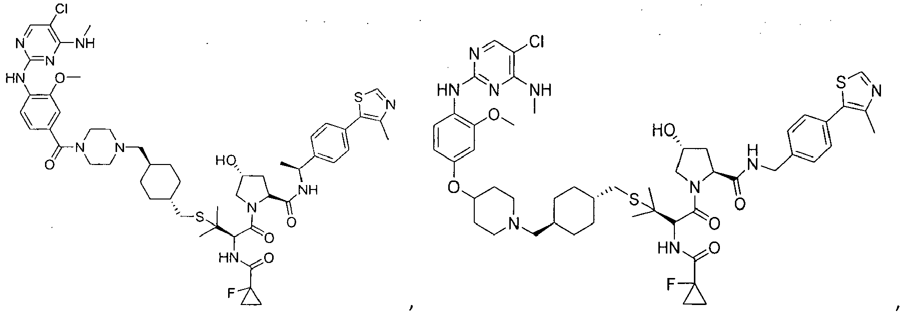 and 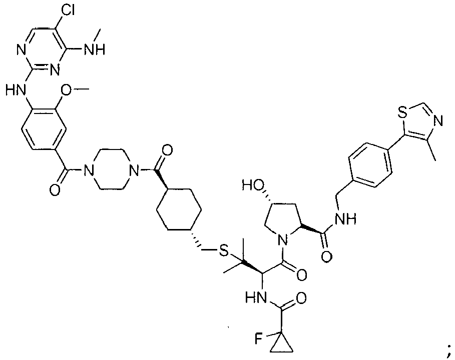 or a pharmaceutically acceptable, salt, enantiomer, stereoisomer, hydrate, solvate, or polymorph thereof; or
the compound is: 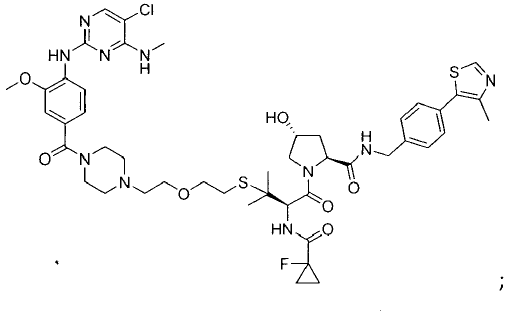 or a pharmaceutically acceptable, salt, enantiomer, stereoisomer, hydrate, solvate, or polymorph thereof.

12. A pharmaceutical composition comprising a compound as defined in any preceding claim and a pharmaceutically acceptable vehicle or diluent therefor, optionally, wherein the pharmaceutical composition comprises an effective amount of a compound as defined in any one of claims 1 to 11 in combination with a pharmaceutically acceptable carrier, additive or excipient.

13. A compound as defined in any of claims 1-11 for use in medicine.

14. A compound as defined in any of claims 1-11 for use in the prophylaxis or treatment of a disease or condition associated with altered GTPase and/or kinase activity of the protein Leucine-rich repeat kinase 2 (LRRK2) comprising the administration of a PROTAC compound of Formula (I) to a subject suffering from or likely to be exposed to said disease or condition.

15. A compound as defined in any of claims 1-11 for use in the prophylaxis or treatment of a disease or condition associated with altered PDE6D activity, comprising the administration of a PROTAC compound of Formula (I) as defined herein to a subject suffering from or likely to be exposed to a PDE6D-related disease or condition.

16. A compound as defined in any of claims 1-11 for use in the prophylaxis or treatment of a disease or condition independently selected from: Parkinson's Disease, idiopathic Parkinson's Disease, idiopathic late-onset Parkinson's Disease, familial Parkinson's Disease, LRRK2 mutation associated Parkinson's disease, Lewy body dementia, primary tauopathy, or inflammation-associated disease such as Leprosy, neuroinflammation, and Crohn's disease.
